(19) Europäisches Patentamt
European Patent Office
Office européen des brevets

(11) EP 4 778 933 A1

(12) **EUROPEAN PATENT APPLICATION**
published in accordance with Art. 153(4) EPC

(43) Date of publication:
**22.07.2026 Bulletin 2026/30**

(21) Application number: **24876582.8**

(22) Date of filing: **10.10.2024**

(51) International Patent Classification (IPC):
***C07D 519/00*** (2006.01) ***C07D 401/14*** (2006.01)
***C07D 487/04*** (2006.01) ***C07D 487/08*** (2006.01)
***C07D 495/00*** (2006.01) ***A61K 31/505*** (2006.01)
***A61K 31/435*** (2006.01) ***A61K 31/553*** (2006.01)
***A61P 35/00*** (2006.01)

(52) Cooperative Patent Classification (CPC):
**A61K 31/435; A61K 31/505; A61K 31/553; A61P 35/00; C07D 401/14; C07D 487/04; C07D 487/08; C07D 495/00; C07D 519/00**

(86) International application number:
**PCT/CN2024/123898**

(87) International publication number:
**WO 2025/077770 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.10.2023 CN 202311307546**
**06.02.2024 CN 202410168656**
**15.07.2024 CN 202410944473**

(71) Applicants:
- **Chengdu Hyperway Pharmaceuticals Co., Ltd.**
  **Chengdu, Sichuan 610041 (CN)**
- **Shenzhen Hyperway Pharmaceuticals Co., Ltd.**
  **Shenzhen, Guangdong 518116 (CN)**

(72) Inventors:
- **REN, Junfeng**
  **Chengdu, Sichuan 610041 (CN)**
- **LIU, Guanfeng**
  **Chengdu, Sichuan 610041 (CN)**
- **WU, Xiancai**
  **Chengdu, Sichuan 610041 (CN)**
- **YANG, Mao**
  **Chengdu, Sichuan 610041 (CN)**
- **LI, Hongbo**
  **Chengdu, Sichuan 610041 (CN)**
- **LI, Yingfu**
  **Chengdu, Sichuan 610041 (CN)**

(74) Representative: **De Arpe Tejero, Manuel**
**Paseo de la Castellana 93b**
**28046 Madrid (ES)**

# (54) FUSED RING COMPOUND AND USE THEREOF IN KRAS INHIBITOR

(57) The present disclosure relates to the field of medical technologies, and provides a fused ring compound, a pharmaceutical composition containing the same, and the use thereof. The compound has a structure as represented by formula (I), or is a tautomer, a mesomer, a racemate, an enantiomer, a diastereoisomer or a mixture thereof, a metabolite, a metabolic precursor, an isotope substitution form, a pharmaceutically acceptable salt, a hydrate, a solvate, a polymorph or a cocrystal thereof. The compound can be used for treating one or more cancers caused by KRAS mutation.

EP 4 778 933 A1

## Description

### TECHNICAL FIELD

The present disclosure relates to the field of medical technologies, in particular to a fused ring compound, a pharmaceutical composition containing the same, and use thereof.

### BACKGROUND

The KRAS (Kirsten Rat Sarcoma Viral Oncogene Homolog) gene belongs to the RAS family. The other two genes in the family are respectively HRAS and NRAS, and the homology between the genes can reach 85%. KRAS is essentially a monomeric G protein, which is located on both sides of the cell membrane, and exhibits GTPase activity. Its encoded protein consists of 188 to 189 amino acids and has a molecular weight of 21KD. KRAS plays an important regulatory role in signal transduction pathways of tumor cell growth and angiogenesis and other processes. Under normal circumstances, it can control the path of regulating cell growth; when abnormalities occur, it causes cells to continuously grow and prevents cells from self-destruction. When the KRAS gene mutates, it is permanently activated to continuously stimulate cell growth, so that the signal transduction in cells is disordered, and cell proliferation is uncontrolled, leading to carcinogenesis.

KRAS is the most common cancer mutation gene in humans. About 30% of all the cancers are associated with the activation of KRAS mutation, including 95% of pancreatic ductal adenocarcinoma (PDAC), 45% of colon and rectal cancer (CRC), 35% of non-small cell lung cancer (NSCLC), etc. Due to protein structure problems, KRAS was once considered an "undruggable" target in the field of oncology drug development. However, as the research deepened, researchers found that some specific mutant KRAS proteins would form a site that could act on drugs. After binding to small molecule drugs, KRAS can be locked in an inactive KRAS protein-GDP state, which inhibits the activation of the KRAS pathway and thus initiates the antitumor effect. In recent years, several KRAS G12C drug candidates have entered clinical trials, among which AMG 510 from Amgen and MRTX849 from Mirati Therapeutics have made the fastest progress. The former has been approved by FDA for marketing. However, the inhibitory effect of these drugs requires an active cysteine residue, making them unusable for the treatment of non-G12C mutants (such as G12D, G12V, G12R, G12S, G12A and G13D), while the latter constitutes the majority of KRAS changes in cancer. Therefore, it remains an ongoing effort to seek therapeutic options that can inhibit various KRAS mutants.

At present, there are many patents reporting on pan-KRAS inhibitor compounds, such as KUMQUAT BIOSCIENCES INC. and China Jiakos Co., Ltd. have publicly reported a class of pan-KRAS inhibitor compounds (WO2022173870) and (WO2023020519). However, many pan-KRAS inhibitors reported have the problems such as low exposure and low bioavailability. Therefore, it is of important significance for seeking and developing new and efficient pan-KRAS inhibitors.

### SUMMARY

In view of this, the present disclosure provides a fused ring compound, a pharmaceutical composition comprising the same, and use thereof. The provided compound can be used to treat one or more cancers caused by KRAS mutations, has relatively good pharmacokinetic properties, can be used to prepare drugs for preventing and/or treating diseases mediated by various KRAS mutants, and has good application prospects.

The present disclosure provides a compound represented by Formula I, a tautomer, a mesomer, a racemate, an enantiomer, a diastereomer or a mixture thereof, a metabolite, a metabolic precursor, an isotope substitution form, a pharmaceutically acceptable salt, a hydrate, a solvate, a polymorph or a cocrystal thereof:

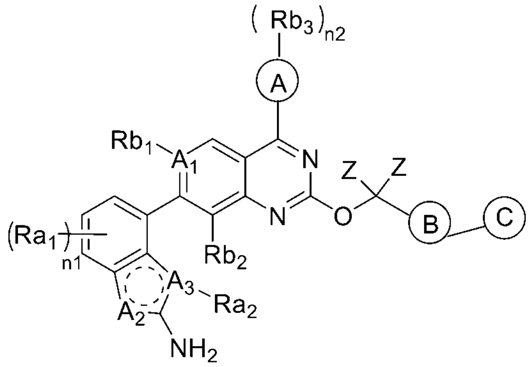

I

$A_1$ is selected from C or N;

$A_2$ is selected from S, N, or C; A3 is selected from N, O, or C; and $A_2$ and $A_3$ are not simultaneously C;

Z at each occurrence is independently selected from H or deuterium;

$Ra_1$ is selected from H, halogen, $C(CH_3)=CH-$ or $-CH=CH_2$, $-CF=CH_2$, -CN, OH, $NH_2$, $-CF_3$, $-CHF_2$, $-CH_2F$, $-CF_2CH_3$, $-CF_2CF_3$, $-OCHF_2$, $-OCF_3$, aroyl, ethynyl, propynyl, butynyl, pentynyl, hexynyl, cyclopropyl, cyclopentenyl, cyclohexyl, cycloheptyl, methyl, ethyl, propyl, butyl, pentyl, vinyl, propenyl, allyl, butenyl, butenyl, pentadienyl, dipentenyl, hexenyl, hexadienyl, methoxy, ethoxy, propoxy, isopropoxy, butoxy, tert-butoxy, pentooxy, or hexoxy;

$n_1$ is an integer from 0 to 3, for example, 0, 1, 2 or 3;

$Ra_2$ is selected from -CN, H, or absent;

$Rb_1$, $Rb_2$, and $Rb_3$ are selected from absent, hydrogen, deuterium, halogen, substituted or unsubstituted alkyl or cycloalkyl, substituted or unsubstituted heteroalkyl or heterocycloalkyl, substituted or unsubstituted alkoxy, hydroxyl, cyano, amino, ester group, amide, aryl, heteroaryl, acylguanidyl, sulfonyl, phosphoryl, sulfonic acid group, and phosphate group, wherein the substituents are each independently selected from deuterium, halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group;

$n_2$ is an integer from 0 to 6, for example 0, 1, 2, 3, 4, 5 or 6;

Ring A is a substituted or unsubstituted 4- to 12-membered heterocycle, with heteroatoms being N and/or O; and

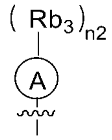

does not have the following structure:

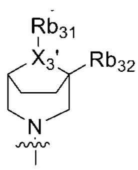

;

wherein $Rb_{31}$ and $Rb_{32}$ are each independently selected from -H, substituted or unsubstituted $C_{1-10}$ alkyl, substituted or unsubstituted $C_{1-10}$ heteroalkyl, -CO(substituted or unsubstituted $C_{0-10}$ alkyl), $-(CH2)_{n21}CO$(substituted or unsubstituted $C_{0-10}$ alkyl), -COO(substituted or unsubstituted $C_{0-10}$ alkyl), $-COO-(CH_2)_{n22}-OCO$(substituted or unsubstituted $C_{0-10}$ alkyl), and $-COO-CH(CH_3)-OCO$(substituted or unsubstituted $C_{0-10}$ alkyl), and the substituents are selected from deuterium, halogen, hydroxyl, cyano, amino, ester group, and amide; $n_{21}$ and $n_{22}$ are each independently an integer from 0 to 5, for example, 0, 1, 2, 3, 4 or 5; $X_{3'}$ is C, N or O;

Ring B is a substituted or unsubstituted 4- to 12-membered heterocycle with nitrogen as the heteroatom;

Ring C is a substituted or unsubstituted 4- to 12-membered carbon ring or heterocycle, with N and/or O as the heteroatom;

The Ring A, the Ring B, and the Ring C are each independently selected from monocyclic, fused, bridged and spirocyclic rings, and the substituents are selected from -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl,

alkoxy, alkynyl, ester group, amide, aryl, heteroaryl, acylguanidyl, sulfonyl, phosphoryl, sulfonic acid group, and phosphate group.

In the present disclosure,

“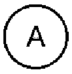”

refers to Ring A;

“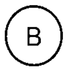”

refers to Ring B; and

‘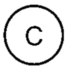”

refers to Ring C.

Further, the Ring A is selected from one of the following substituted or unsubstituted structures, in which $n_2$ is 0:

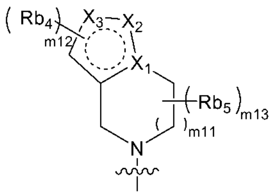 , 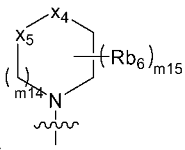 , 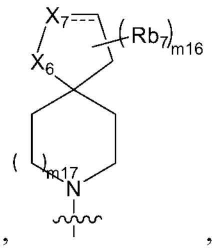 , 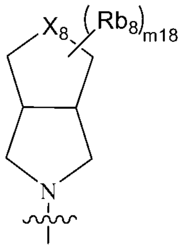 ,

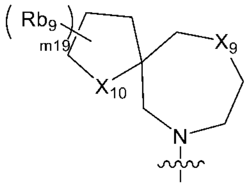 , 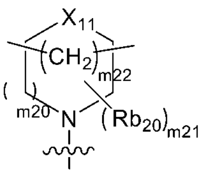 , 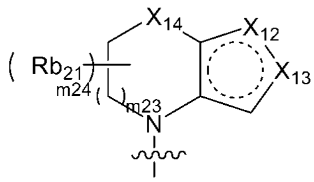 , 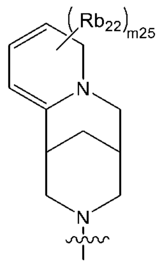 ,

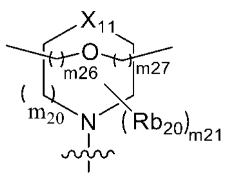 , 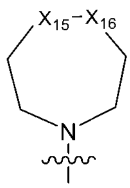 , and 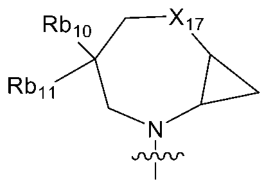 ;

$X_1$ and $X_2$ are each independently selected from N or C; $X_3$ is selected from N, O, or C; and only one of $X_1$, $X_2$, and $X_3$ is C; $Rb_4$ and $Rb_5$ at each occurrence are independently selected from absent, H, halogen, deuterium, substituted or unsubstituted alkyl, hydroxyl, cyano, $-C(=O)NRb_{41}Rb_{42}$, $-(CH_2)m_{121}C(=O)NRb_{41}Rb_{42}$, $-(CH_2)m_{121}NRb_{41}Rb_{42}$, $-NRb_{41}Rb_{42}$, $-C(=O)Rb_{41}$, and $-C(=O)ORb_{41}$; $Rb_{41}$ and $Rb_{42}$ are each independently selected from H, and substituted or unsubstituted alkyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl,

phosphoryl, sulfonic acid group, and phosphate group; $m_{11}$ is an integer from 0 to 3, for example 0, 1, 2 or 3; $m_{12}$, $m_{13}$, and $m_{121}$ are each independently an integer from 0 to 6, for example 0, 1, 2, 3, 4, 5, or 6;

$X_4$ and $X_5$ are each independently selected from N, O, or C; $Rb_6$ at each occurrence is independently selected from absent, H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{14}$ is an integer from 0 to 3, for example, 0, 1, 2, or 3; and $m_{15}$ is an integer from 0 to 6, for example, 0, 1, 2, 3, 4, 5, or 6;

$X_6$ is selected from N or O; $X_5$ is selected from N, O or C; $Rb_7$ at each occurrence is independently selected from absent, H, halogen, deuterium, substituted or unsubstituted alkyl, hydroxyl, cyano, carbonyl, $-C(=O)NRb_{41}Rb_{42}$, $-(CH2)m_{121}C(=O)NRb_{41}Rb_{42}$, $-(CH_2)m_{121}NRb_{41}Rb_{42}$, $-NRb_{41}Rb_{42}$, $-C(=O)Rb_{41}$, $-C(=O)ORb_{41}$; $Rb_{41}$ and $Rb_{42}$ are each independently selected from H, and substituted or unsubstituted alkyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{16}$ is an integer from 0 to 6; and $m_{17}$ is an integer from 0 to 3, for example, 0, 1, 2, or 3;

$X_8$ is selected from N, O, or C; $Rb_8$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{18}$ is an integer from 0 to 6, for example 0, 1, 2, 3, 4, 5, or 6;

$X_9$ and $X_{10}$ are each independently selected from N or O; $Rb_9$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{19}$ is an integer from 0 to 6, for example 0, 1, 2, 3, 4, 5 or 6;

$X_{11}$ is selected from N, O, or C; $Rb_{20}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{21}$ is an integer from 0 to 6, for example 0, 1, 2, 3, 4, 5, or 6; and $m_{20}$ and $m_{22}$ are integers from 0 to 3, for example 0, 1, 2, or 3;

$X_{12}$, $X_{13}$, and $X_{14}$ are each independently selected from N or O; $Rb_{21}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{23}$ is an integer from 1 to 3, for example 1, 2, or 3; and $m_{24}$ is an integer from 0 to 6, for example 0, 1, 2, 3, 4, 5, or 6;

$Rb_{22}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{25}$ is an integer from 0 to 6, for example 0, 1, 2, 3, 4, 5 or 6;

$m_{26}$ and $m_{27}$ are integers from 0 to 3, such as 0, 1, 2 or 3;

$X_{15}$ and $X_{16}$ are each independently selected from O and -NH;

$X_{17}$ is selected from O or N; $Rb_{10}$ and $Rb_{11}$ are independently selected from -OH, $-CH_3$, and H.

Further, the Ring B is selected from the following substituted or unsubstituted structure:

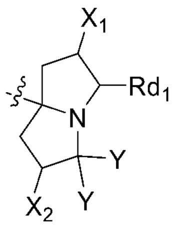

wherein,

$X_1$ and $X_2$ are independently selected from hydrogen, deuterium, and halogen; Y is selected from hydrogen or deuterium;
$Rd_1$ is Ring C.

Further, the Ring C is selected from one of the following:

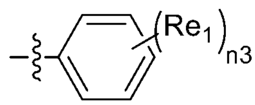 , 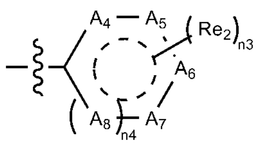 , and 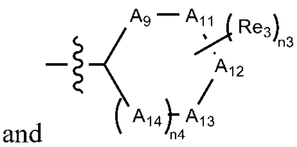

$Re_1$, $Re_2$, and $Re_3$ at each occurrence are each independently selected from -H, -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl, substituted or unsubstituted alkoxy, alkynyl, ester group, amide, aryl, heteroaryl, acylguanidyl, sulfonyl, phosphoryl, sulfonic acid group, and phosphate group, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group;
$n_3$ at each occurrence is independently an integer from 0 to 5, for example, 0, 1, 2, 3, 4 or 5; and $n_4$ at each occurrence is independently selected from 0 or 1;
or, when $n_3$ is greater than or equal to 2, two adjacent $Re_1$, $Re_2$ or $Re_3$ together with atoms connected therebetween also form a 4 to 7-membered ring;
$A_4$ to $A_8$ are each independently selected from a heteroatom or C, and are not all C. The number of heteroatoms in $A_4$ to $A_8$ is 1, 2 or 3, and the heteroatoms are selected from N and/or O; and
$A_9$ to $A_{14}$ are each independently selected from a heteroatom or C, and are not all C. The number of heteroatoms in $A_9$ to $A_{14}$ is 1 or 2, and the heteroatoms are selected from N and/or O.

Further, $Re_1$, $Re_2$, and $Re_3$ at each occurrence are each independently selected from -H, -OH, halogen, $-NO_2$, -CN, $-CF_3$, $-C_2F_5$, $-OCF_3$, $-OCHF_2$, $-OCH_2F$, phosphate group, monomethyl phosphate group, dimethyl phosphate group, linear or branched $C_{1-5}$ alkyl, $C_{1-5}$ alkoxy, $C_{3-10}$ cycloalkyl, $C_{3-10}$ heterocycloalkyl, $-N(C_{0-10}$ alkyl$)(C_{0-10}$ alkyl$)$, $-S(C_{0-10}$ alkyl$)$, $-OCON(C_{0-10}$ alkyl$)(C_{0-10}$ alkyl$)$, $C_{5-6}$ aryl, five-membered heteroaryl, and six-membered heteroaryl; and
or, when $n_3$ is greater than or equal to 2, two adjacent $Re_1$, $Re_2$ or $Re_3$ together with atoms connected therebetween also form saturated or unsaturated $C_{3-8}$ cycloalkyl, saturated or unsaturated $C_{3-8}$ heterocycloalkyl, saturated or unsaturated cyclolactone, $C_{5-6}$ aryl, bridged cycloalkyl, spirocycloalkyl, wherein the H in the above-mentioned groups may be substituted by -D, -OH, -F, $-NO_2$, -CN, $-CF_3$, $-C_2F_5$, $C_{1-3}$ alkyl, or amide.

Further, the Ring C is selected from:

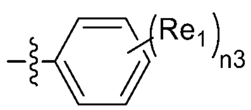

$Re_1$ at each occurrence is independently selected from -H, -OH, -F, -Cl, -CN, $-CF_3$, $-C_2F_5$, $-OCF_3$, monomethylphosphoryl, dimethylphosphoryl, linear or branched $C_{1-5}$ alkyl, $C_{1-5}$ alkoxy, $C_{3-10}$ cycloalkyl, $C_{3-10}$ heterocycloalkyl, $-S(C_{0-10}$ alkyl$)$, $-OCON(C_{0-10}$ alkyl$)(C_{0-10}$ alkyl$)$, $C_{5-6}$ aryl, five-membered heteroaryl, and six-membered heteroaryl; and
or, when $n_3$ is greater than or equal to 2, two adjacent $Re_1$ together with carbon atoms therebetween form saturated or unsaturated $C_{3-8}$ cycloalkyl, saturated or unsaturated $C_{3-8}$ heterocycloalkyl, five-membered cyclolactone, $C_{5-6}$ aryl, bridged cycloalkyl, and spirocycloalkyl, wherein the H in the above-mentioned groups may be substituted by -D, -OH,

-F, $-NO_2$, -CN, $-CF_3$, $-C_2F_5$, $C_{1-3}$ alkyl, or amide.

Further, $n_3$ is selected from 1, 2, 3, 4 or 5.

More further, $n_3$ is selected from 1, 2, or 3.

Further, $Re_1$ is not simultaneously H.

More further, $Re_1$ at each occurrence is independently selected from halogen, $-CF_3$, $-C_2F_5$, $-OCF_3$, $-OCHF_2$, and $-OCH_2F$.

More further, $Re_1$ at each occurrence is independently selected from H or halogen.

More further,

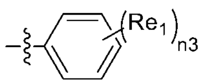

is selected from

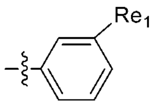 , 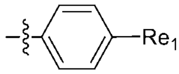 ,

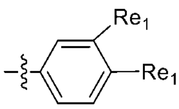 , 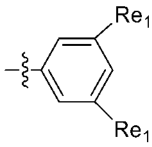 , and 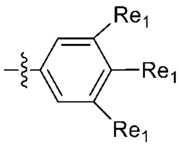 .

In the present disclosure, when $Re_1$ is selected from halogen, F, Cl and the like may be preferred.

Further, when $n_3$ is greater than or equal to 2, two adjacent $Re_1$ together a benzene ring connected therebetween also form one of the following structures:

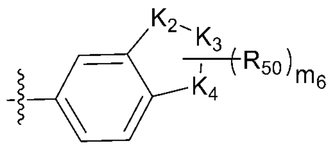 , 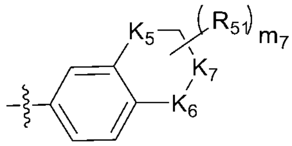 , 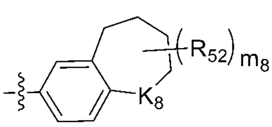 ,

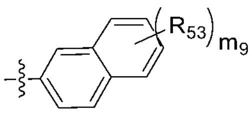 , 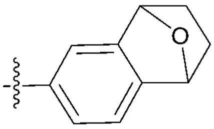 , 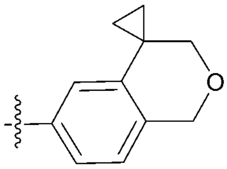 , 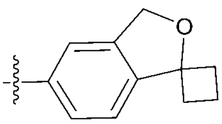 ,

and

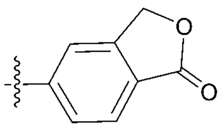 ;

wherein $K_2$, $K_3$, and $K_4$ are fused with the benzene ring to form a saturated or unsaturated five-membered ring; $K_2$, $K_3$, and $K_4$ are independently selected from C, N, O, and S; $K_5$, $K_6$, and $K_7$ are independently selected from C, N, O, and S; $K_8$ is independently selected from C, N, O, and S; $R_{50}$, $R_{51}$, $R_{52}$, and $R_{53}$ are independently selected from -H, -D, -OH, -F, $-NO_2$, -CN, $-CF_3$, $-C_2F_5$, $C_{1-3}$ alkyl, $C_{1-3}$ alkoxy, and $C_{3-6}$ cycloalkyl;$m_6$, $m_7$, $m_8$, and $m_9$ are integers from 0 to 6, for example, 0, 1, 2, 3, 4, 5, or 6.

Further,

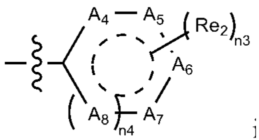

is selected from

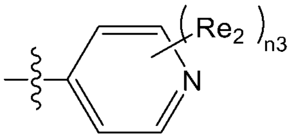, 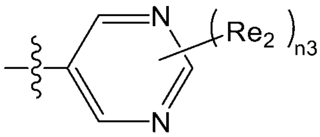,

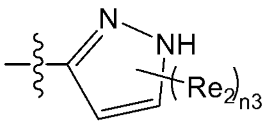, 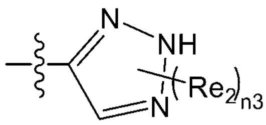;

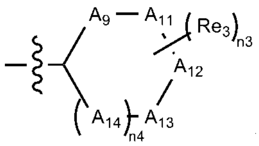

is selected from

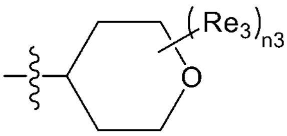, 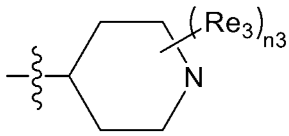,

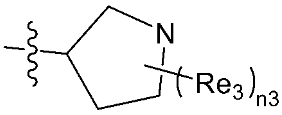, and 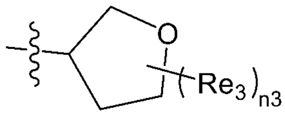.

When the Ring B is selected from the following structure:

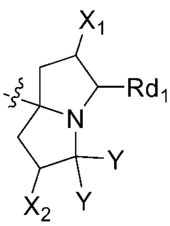,

and the Ring C is selected from:

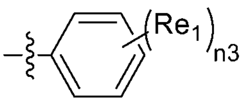,

Formula I presents the following structure:

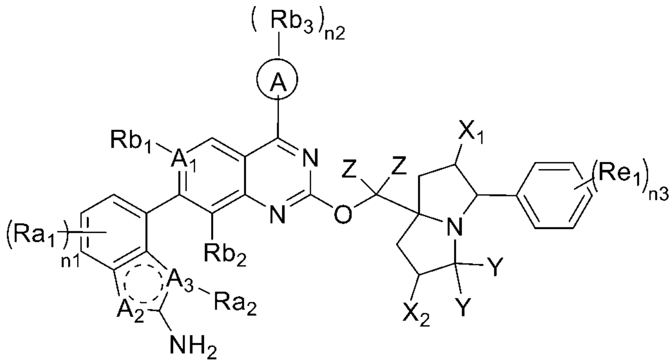

Further, in

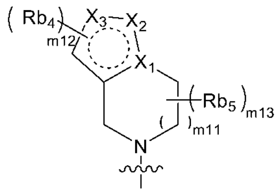

,

$X_1$, $X_2$, and $X_3$ are selected from one of the following combinations:

$X_1$ and $X_3$ are N, and $X_2$ is C;
$X_1$ is N, $X_2$ is N, and $X_3$ is O; and
$X_1$ and $X_2$ are N, and $X_3$ is C.

Further, $m_{11}$ is selected from 1 or 2.

Further, in

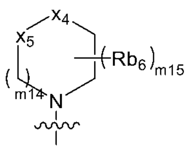

,

$X_4$ and $X_5$ are selected from one of the following combinations:

$X_4$ is O, and $X_5$ is C;
$X_4$ and $X_5$ are C;
$X_4$ is N, and $X_5$ is C; and
$X_4$ is O, and $X_5$ is N.

Further, $m_{14}$ is selected from 0, 1, or 2.

More further, $m_{14}$ is selected from 1 or 2, and $m_{15}$ is selected from 1 or 2.

Preferably, $m_{14}$ is selected from 2, and $X_4$ is O and $X_5$ is C. Based on this,

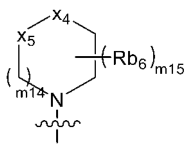

is a structure as follows:

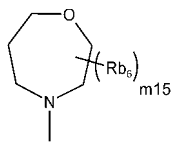

.

Further, $m_{15}$ is selected from 2.
More further,

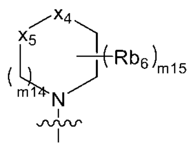

is a structure as follows:

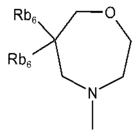

.

Further, $Rb_6$ at each occurrence is independently selected from hydroxyl, and substituted or unsubstituted $C_{1-3}$ alkyl; the substituents are each independently selected from halogen and hydroxyl.
Preferably, in

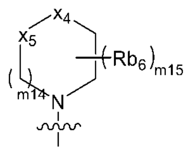

,

two $Rb_6$ are different.
Further, in

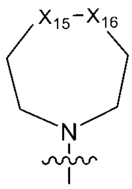

,

$X_{15}$ and $X_{16}$ are each independently selected from O and -NH, and are different from each other.
Further, in

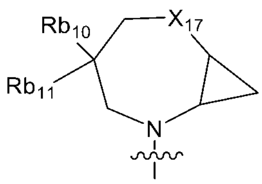

,

$X_{17}$ is selected from O; $Rb_{10}$ and $Rb_{11}$ are selected from one of the following combinations:

$Rb_{10}$ and $Rb_{11}$ are each independently selected from -OH and $-CH_3$, and are different from each other;
$Rb_{10}$ and $Rb_{11}$ are selected from H.

Further, $A_1$ is selected from C.

Further, $Rb_1$ is selected from halogen, substituted or unsubstituted alkyl or cycloalkyl, -CN, and phosphoryl, and the substituents are each independently selected from deuterium and halogen; and $Rb_2$ is selected from halogen.

Further, $Rb_1$ is selected from Cl, Br, and I; and $Rb_2$ is selected from F.

Further, $A_2$ is selected from S; and $A_3$ is selected from C.

Further, $Ra_1$ is selected from H and halogen; more further, $Ra_1$ is selected from H, F, Cl, and Br.

Further, $n_1$ is selected from 1 or 2.

Further, $Ra_2$ is selected from -CN.

Further, Formula I is selected from one of the following:

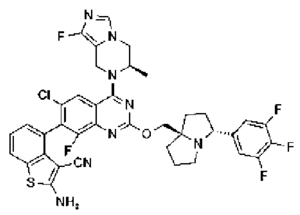
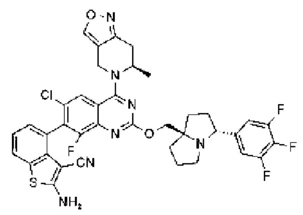
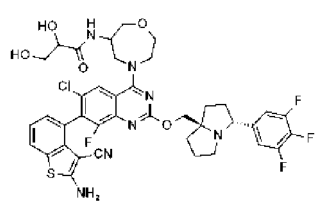

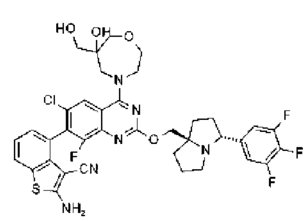
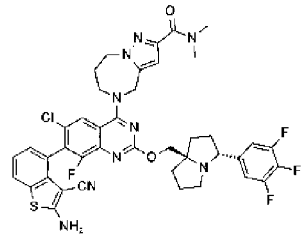

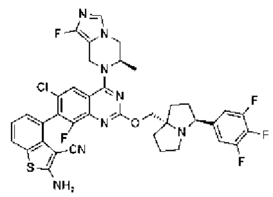
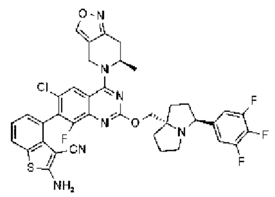
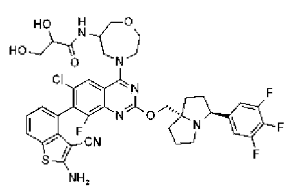

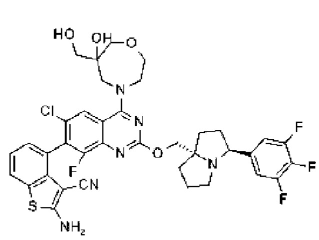
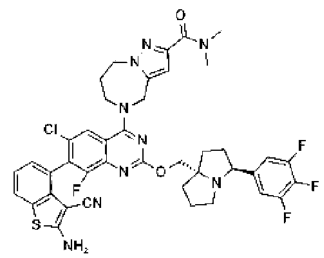

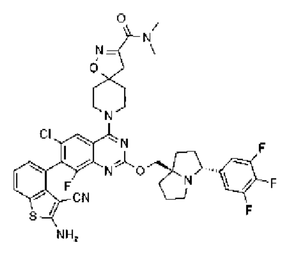
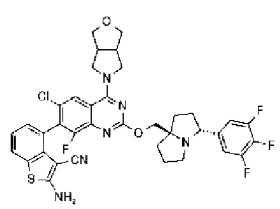
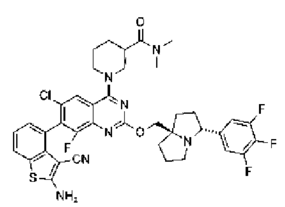

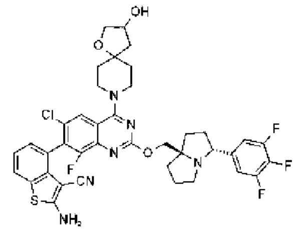
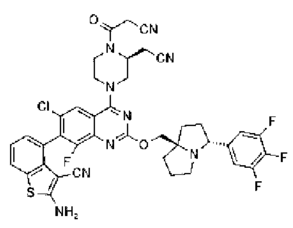

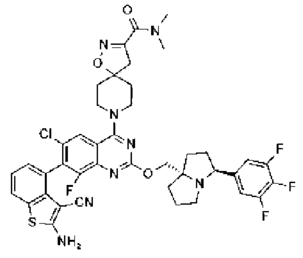
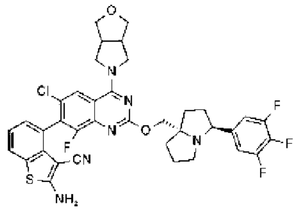
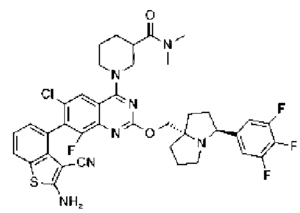

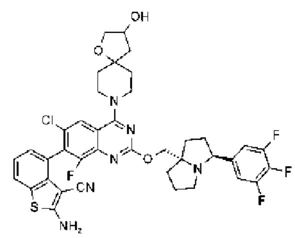
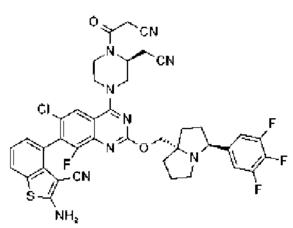

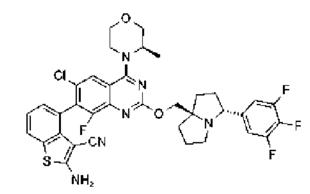
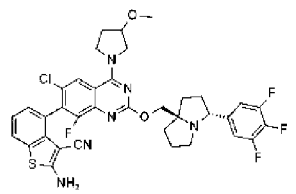
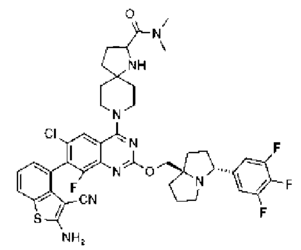

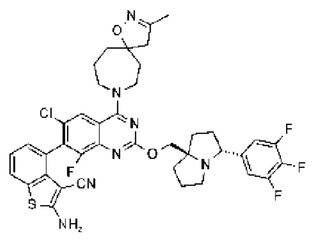
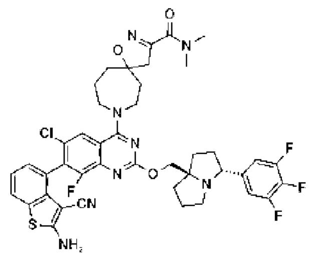

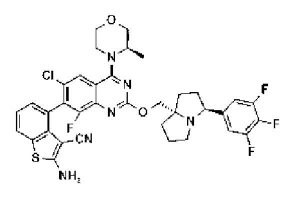
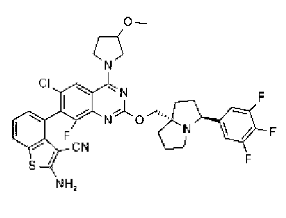
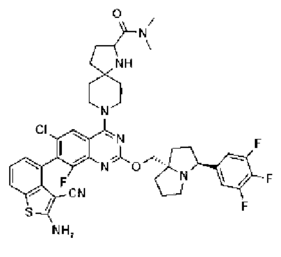

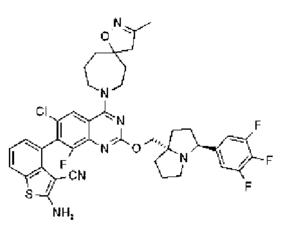
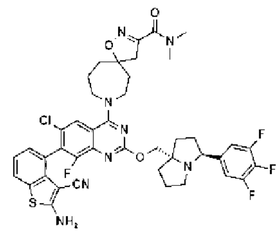

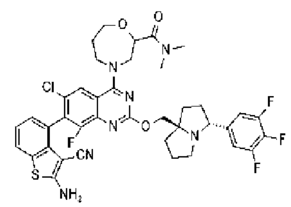
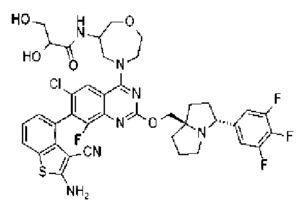
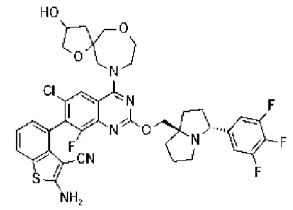

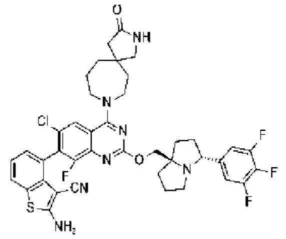
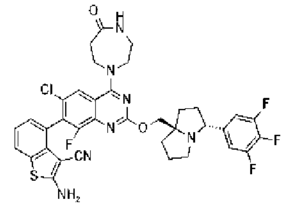

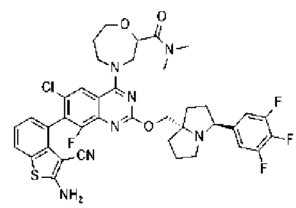
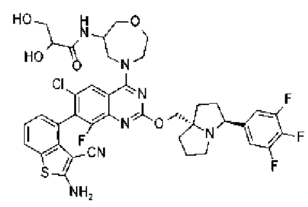
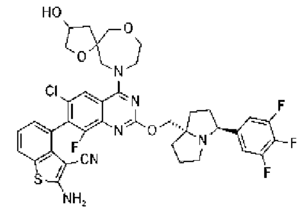

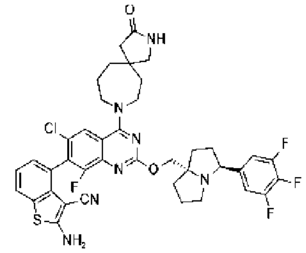
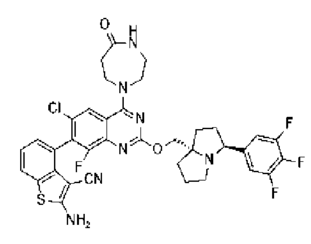

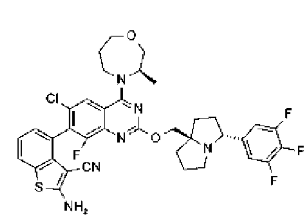
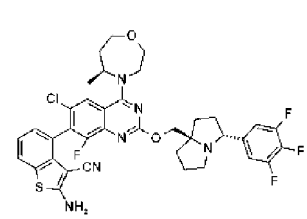
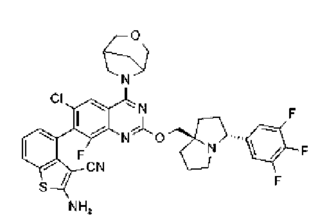

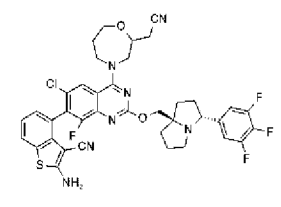
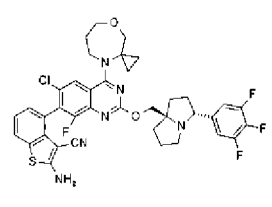

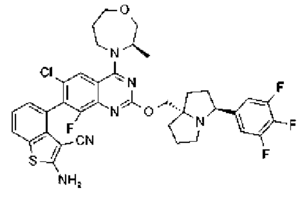

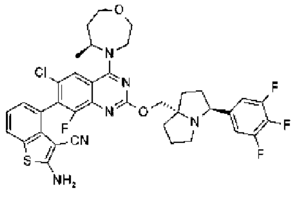

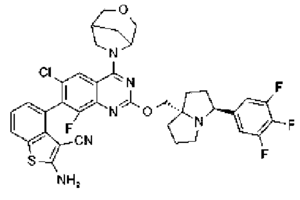

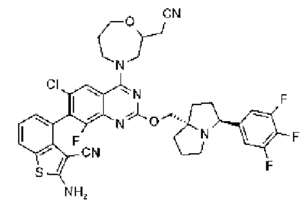

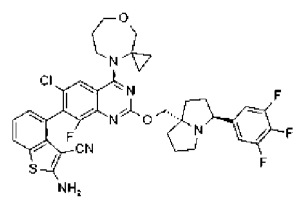

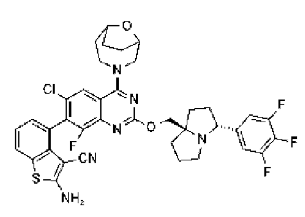

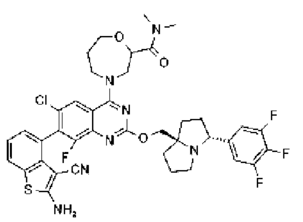

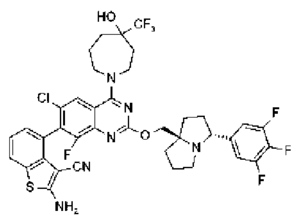

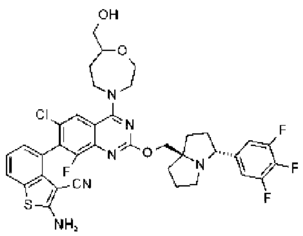

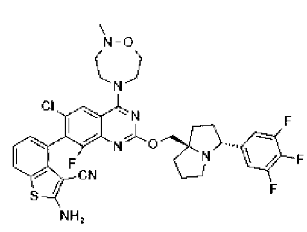

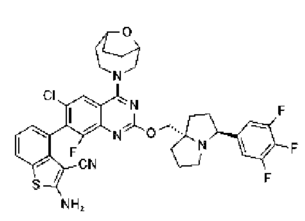

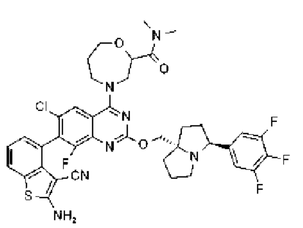

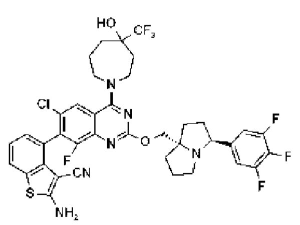

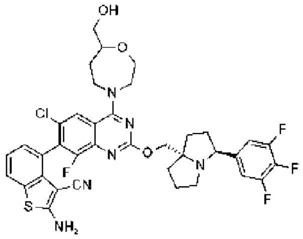

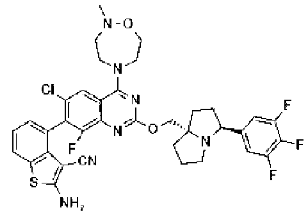

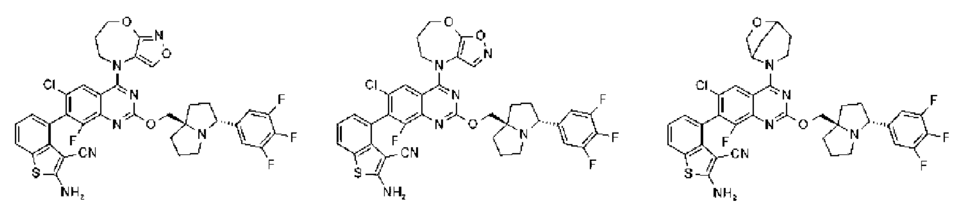

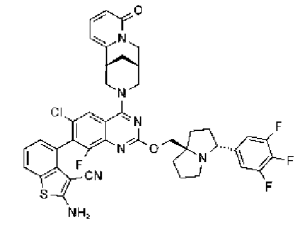
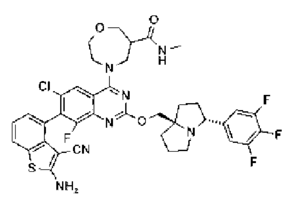
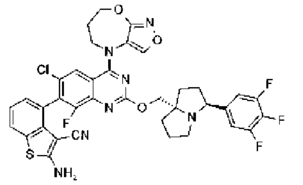
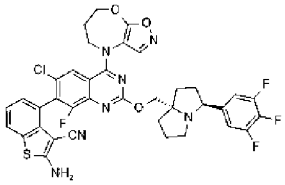
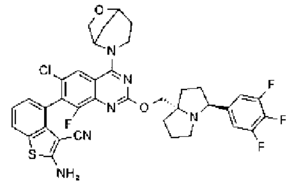
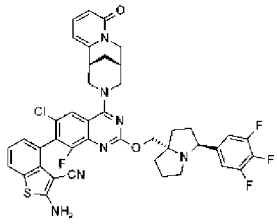
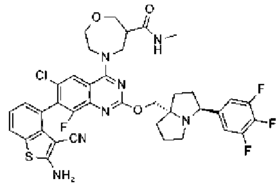
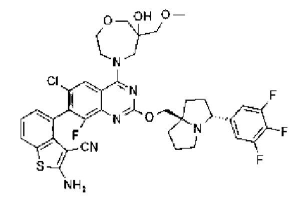
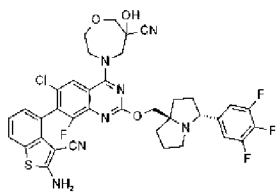
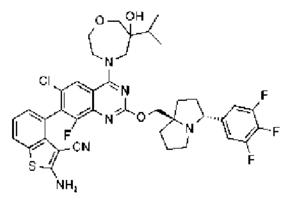
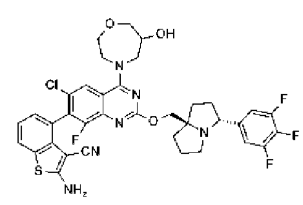
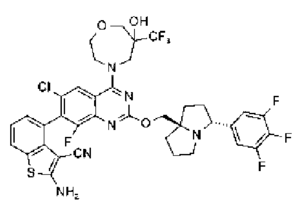
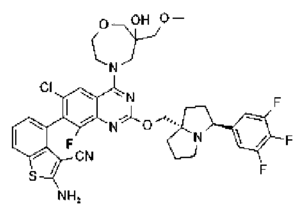
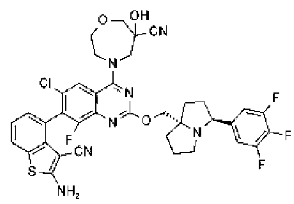
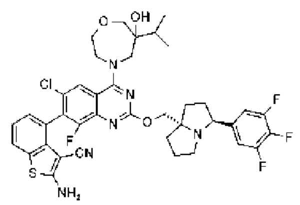
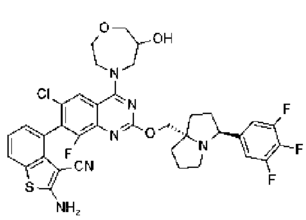
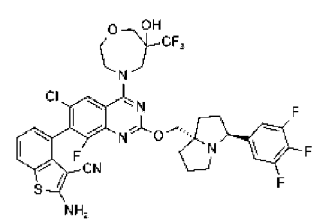

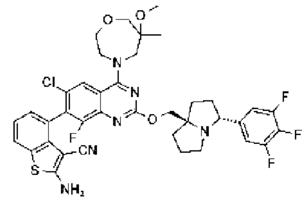
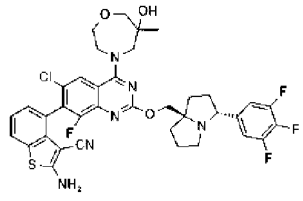
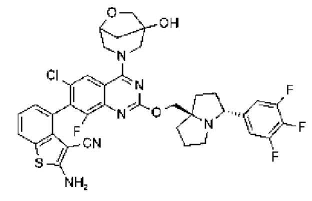

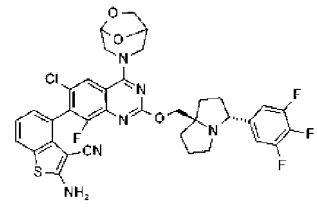
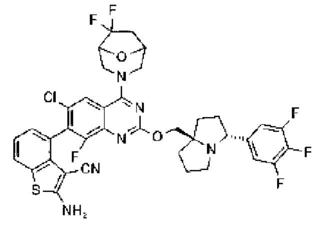

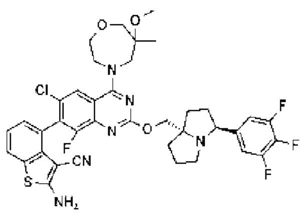
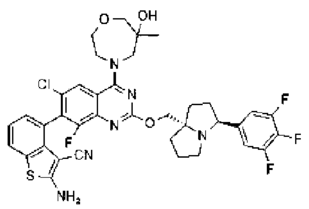
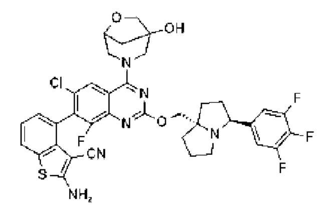

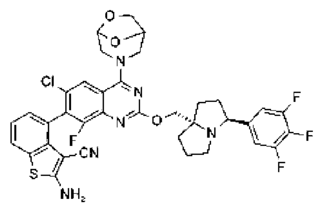
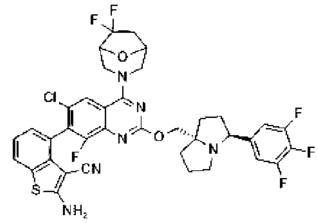

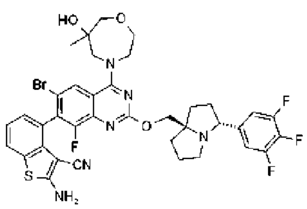
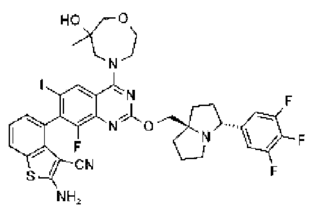
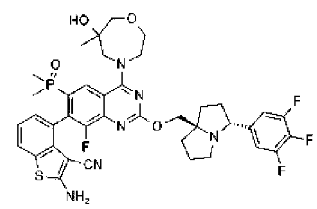

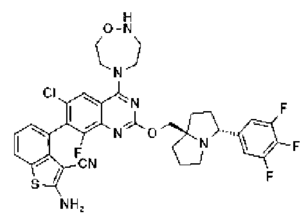
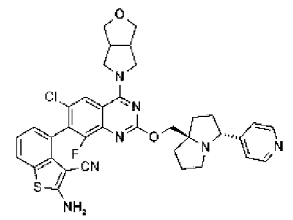

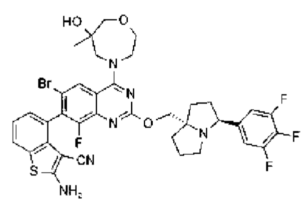
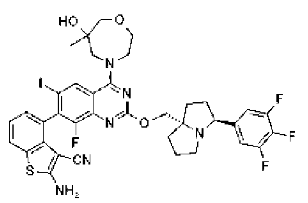
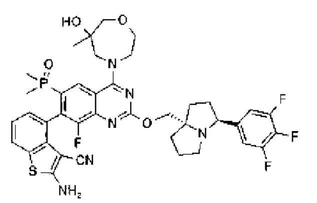

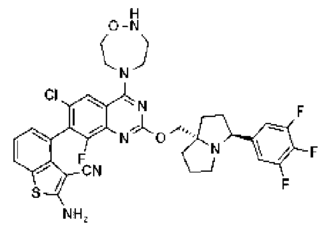

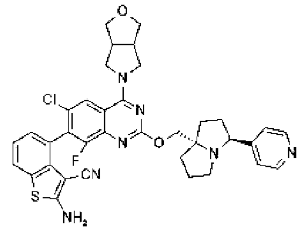

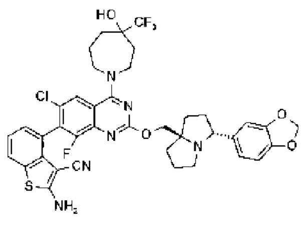

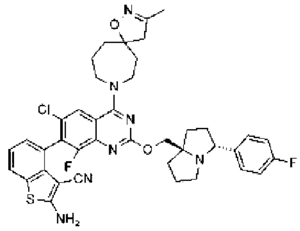

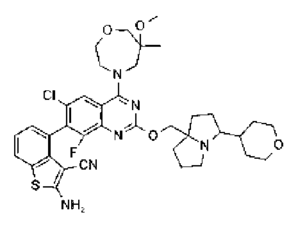

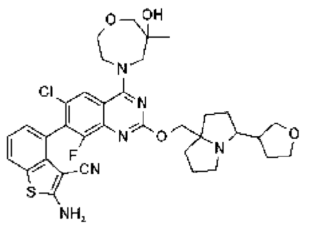

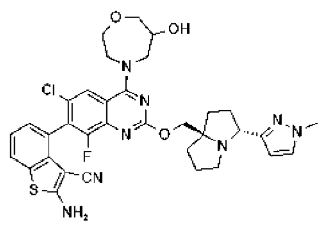

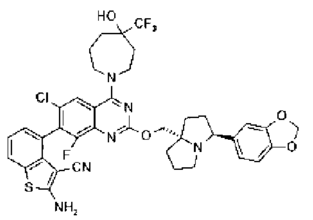

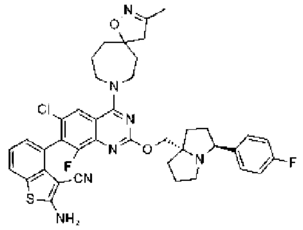

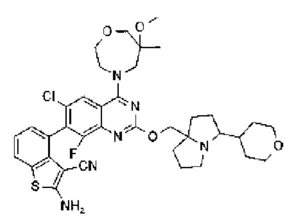

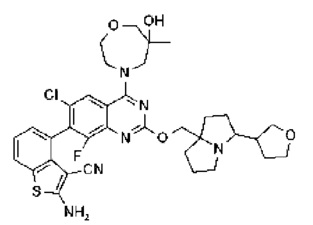

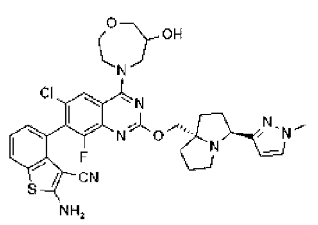

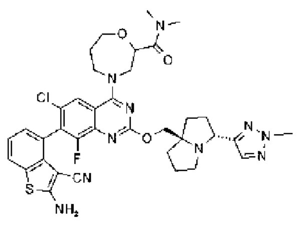

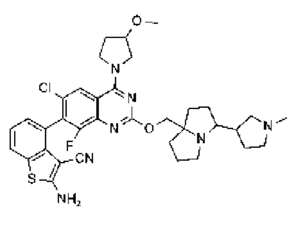

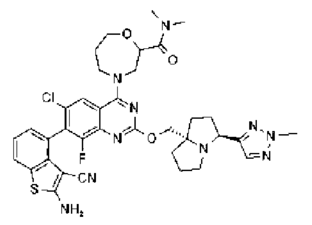

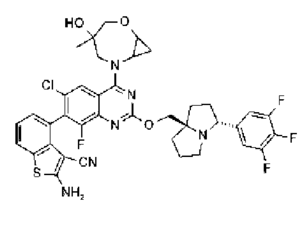

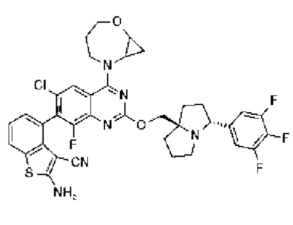

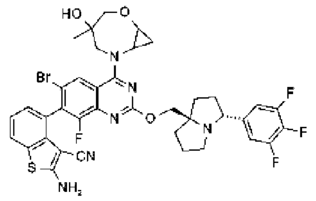
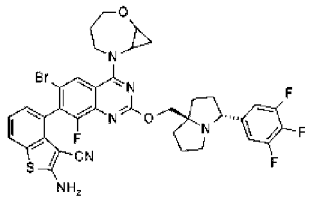
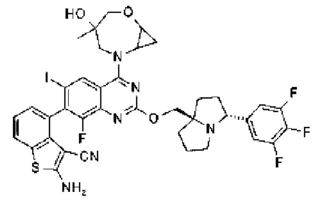

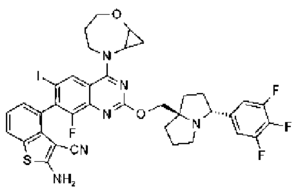
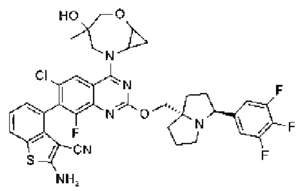

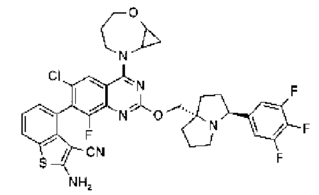
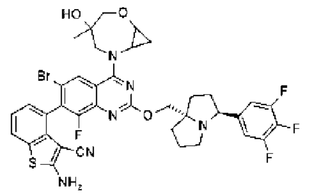
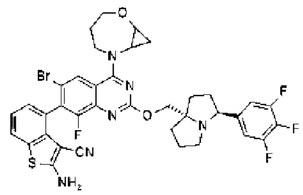

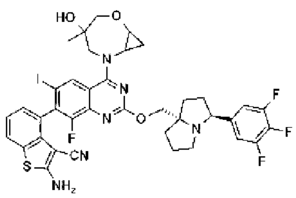
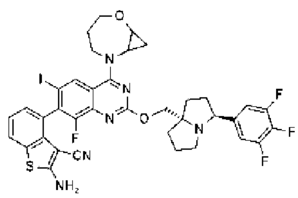

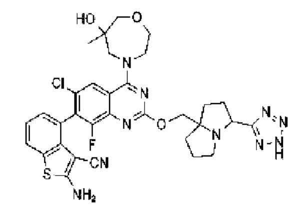
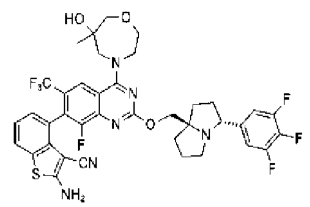
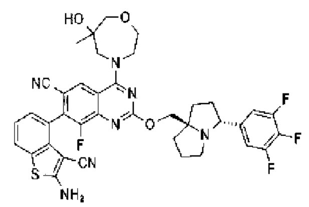

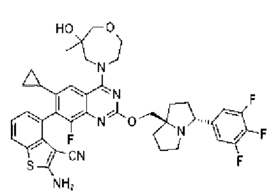
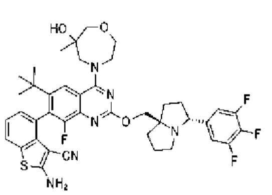

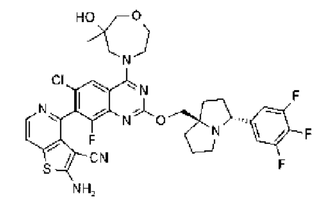
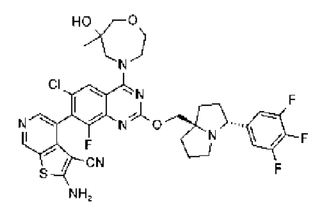
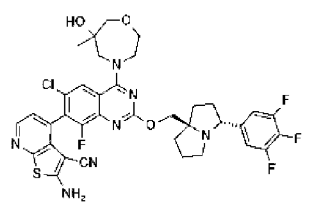

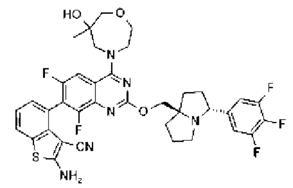

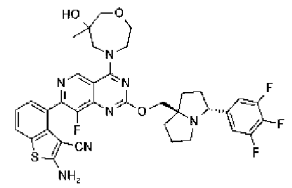

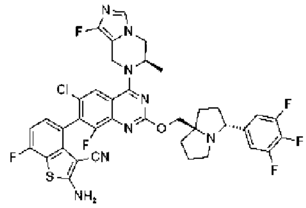

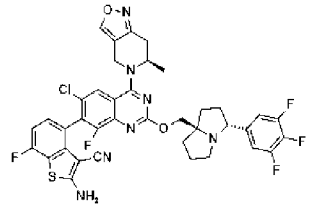

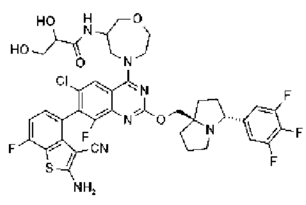

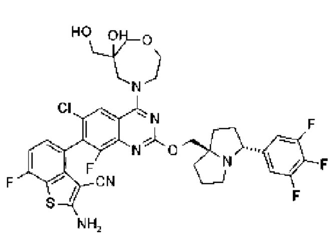

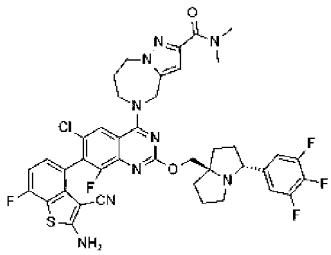

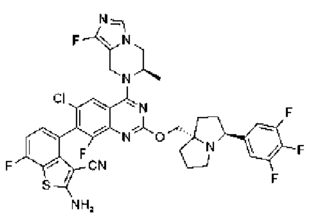

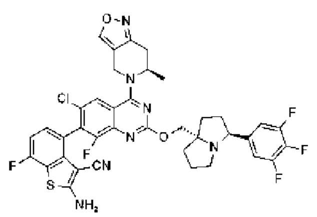

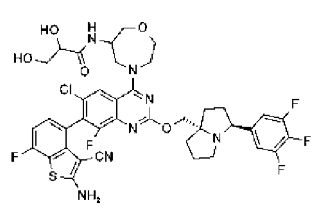

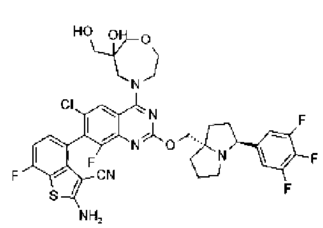

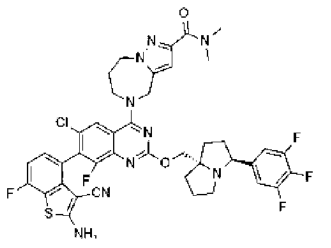

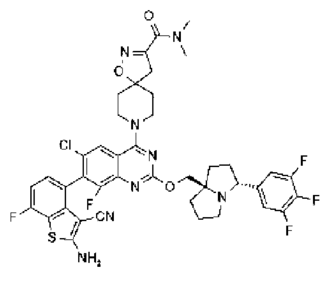

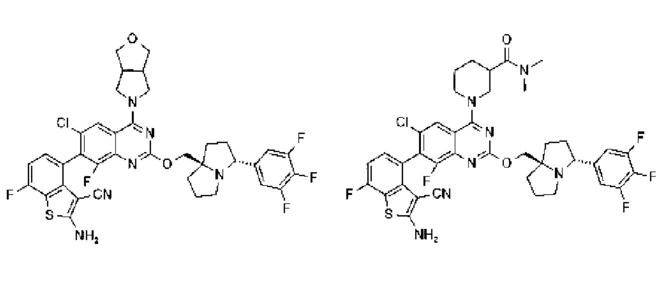

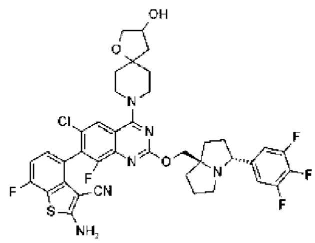
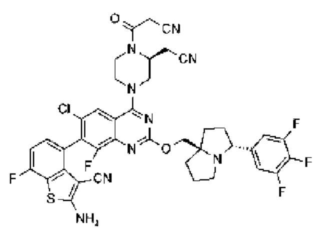

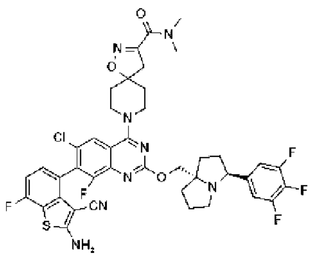
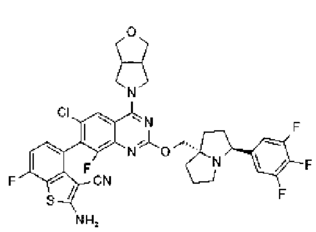
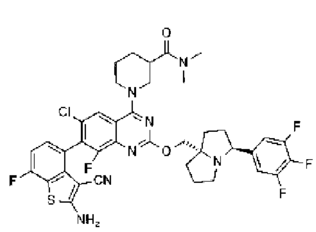

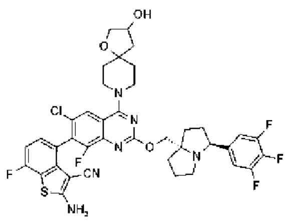
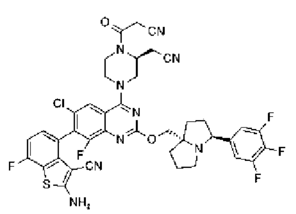

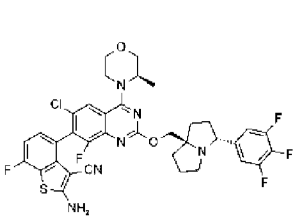
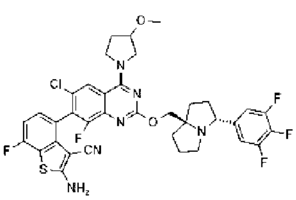
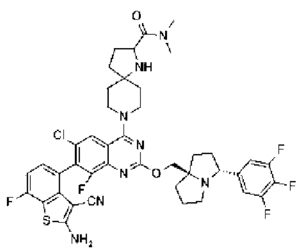

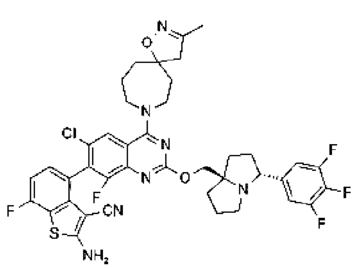
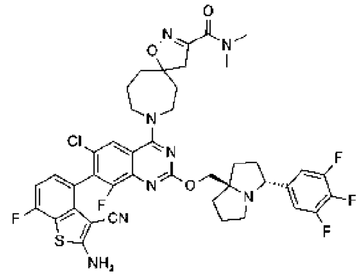

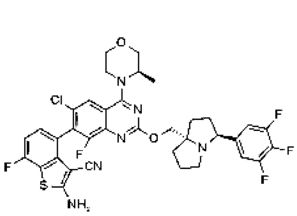
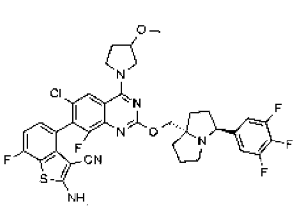
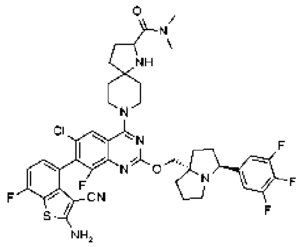

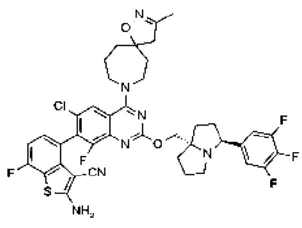
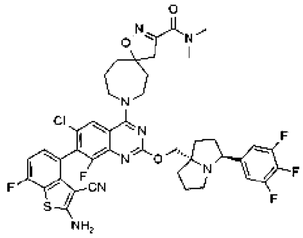

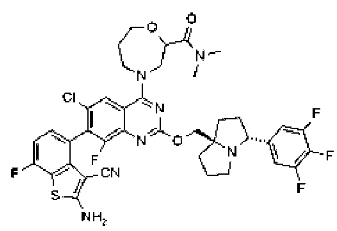
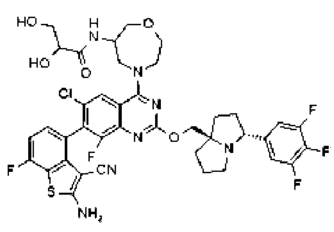
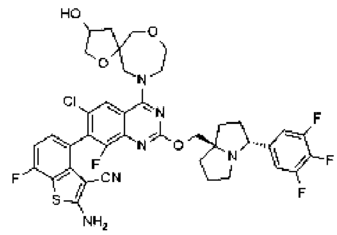

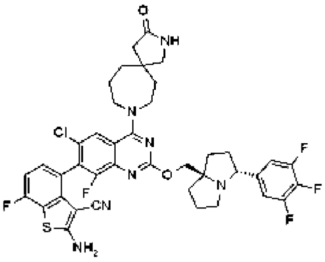
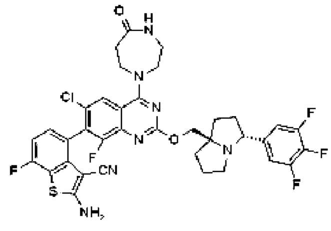

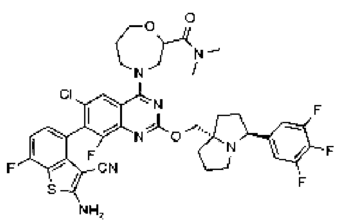
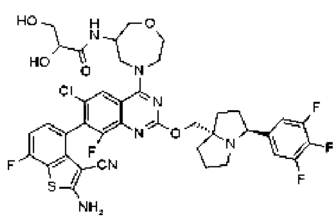
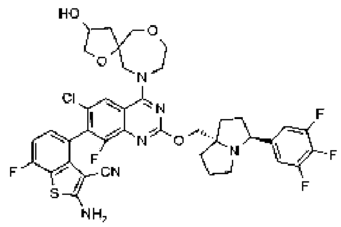

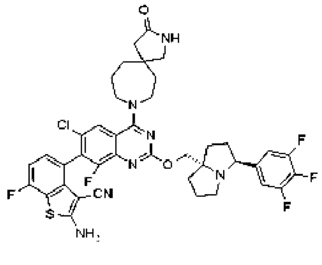
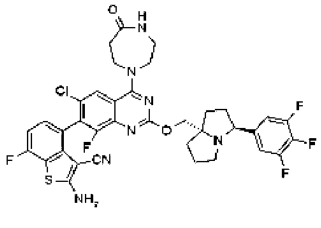

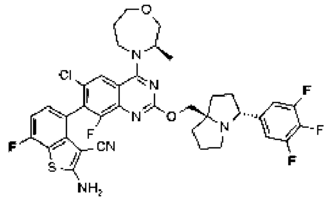
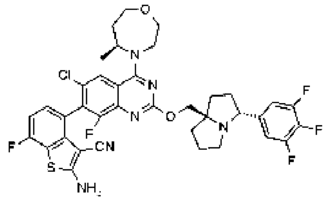
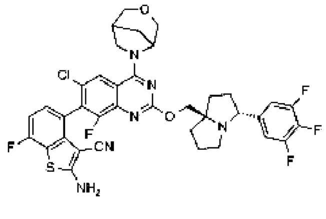

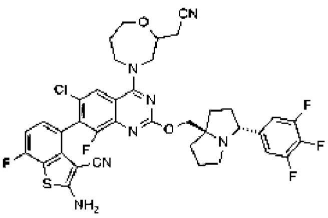
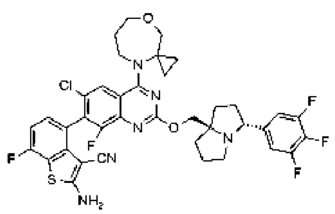

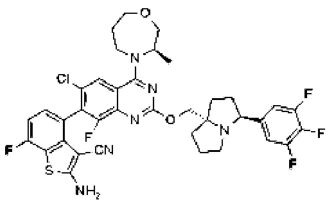
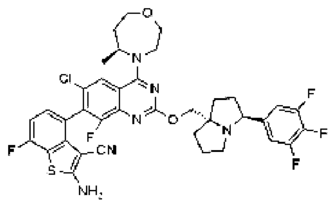
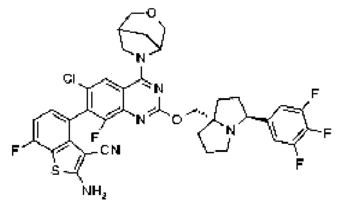

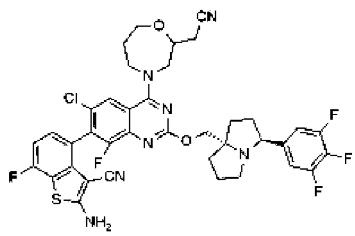
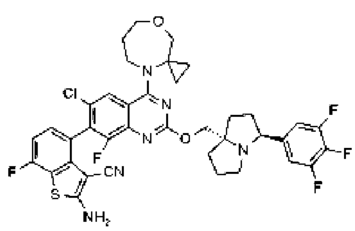

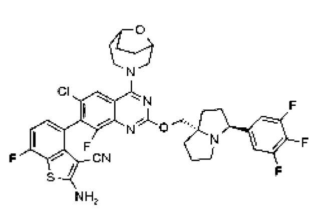
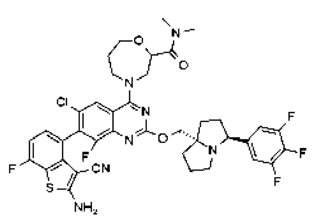
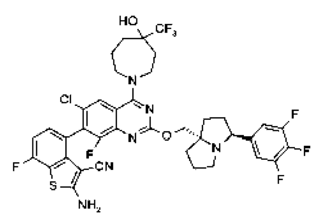

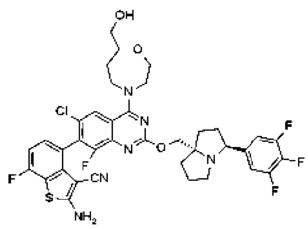
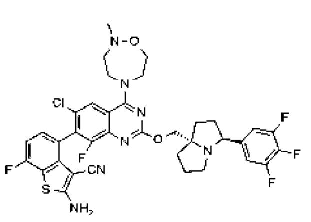

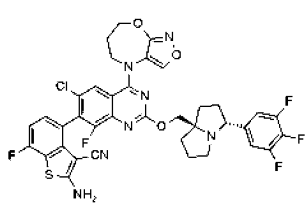
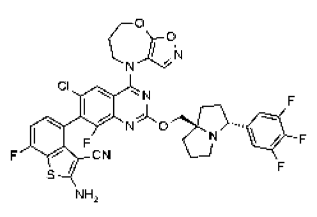
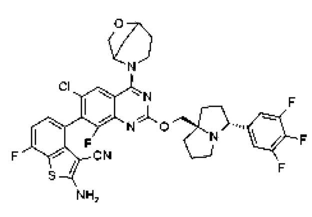

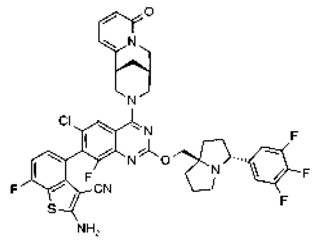
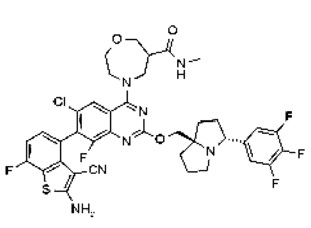

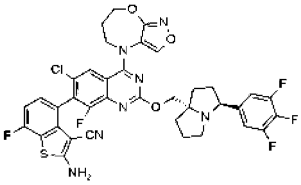
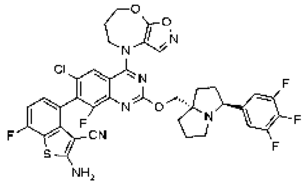
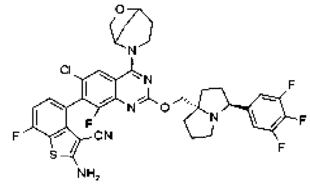

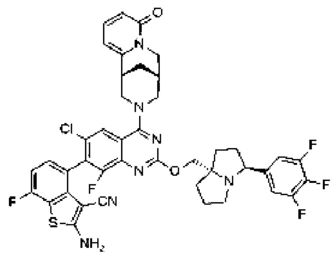
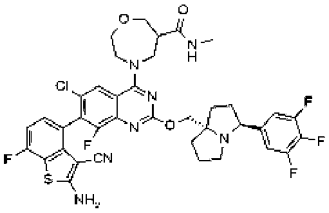

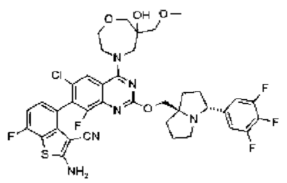
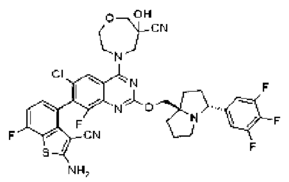
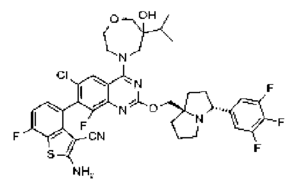

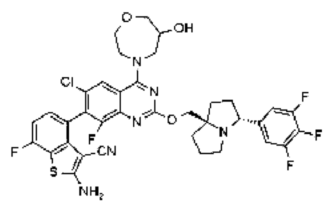
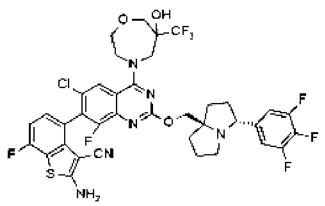

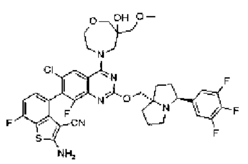
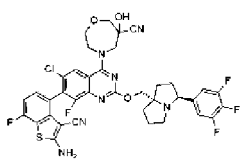
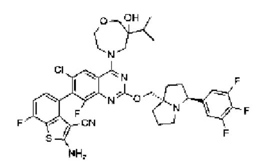

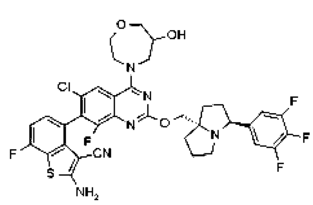
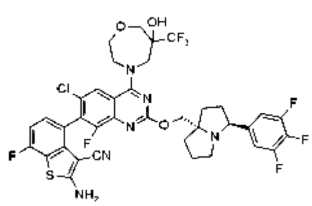

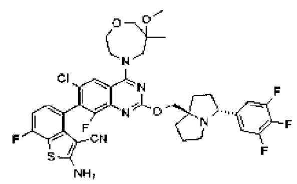
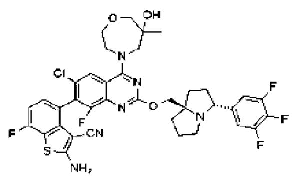
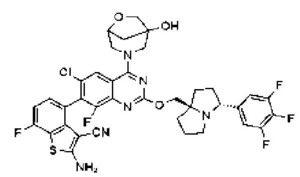

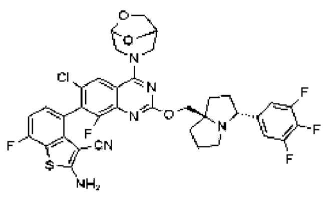
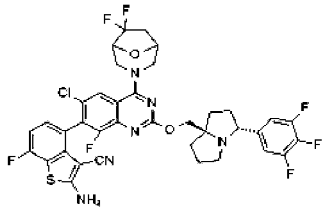

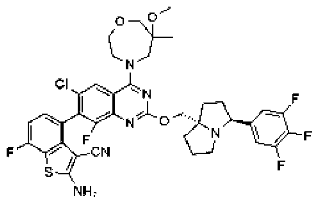
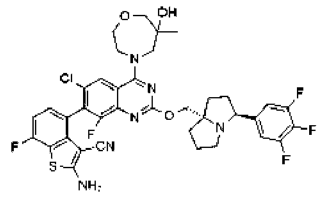
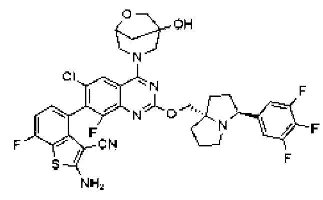

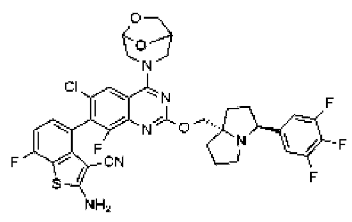
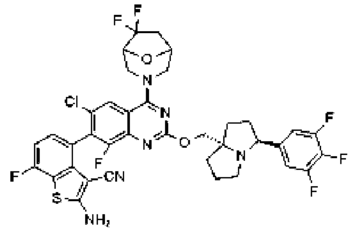

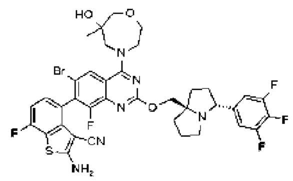
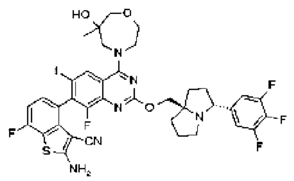
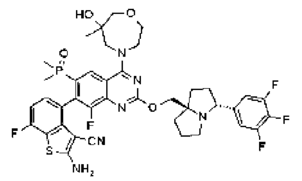

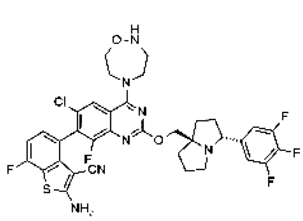
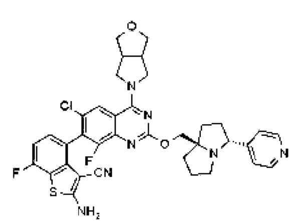

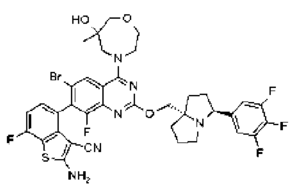
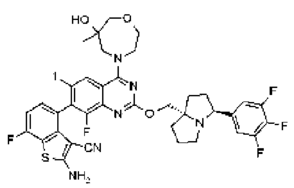
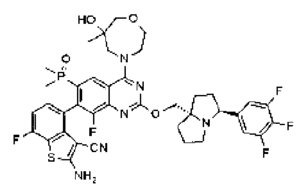

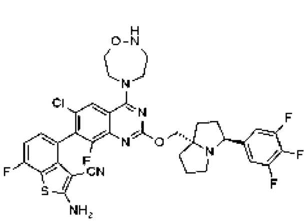
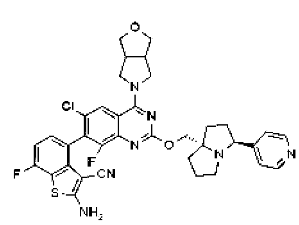

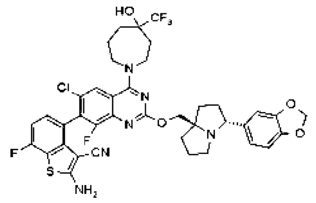
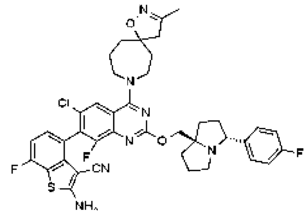
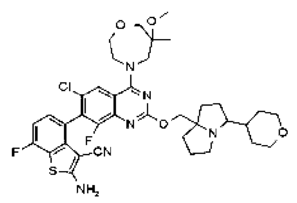

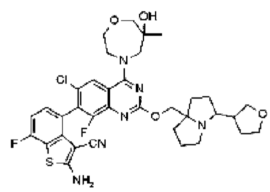
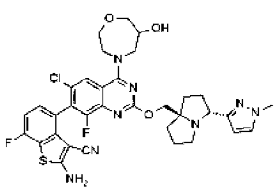

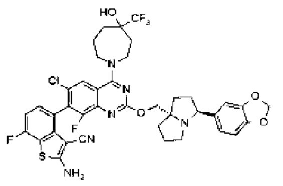
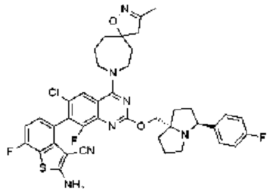
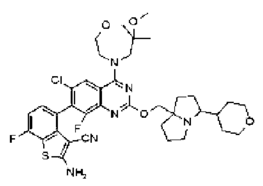

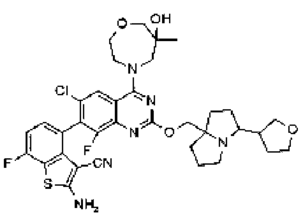
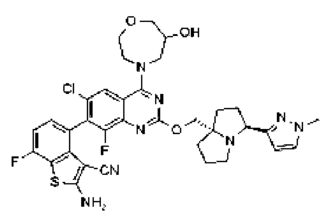

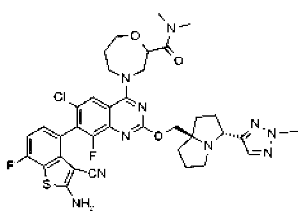
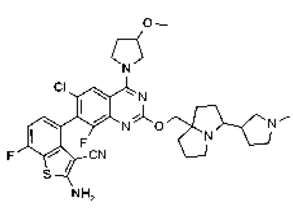
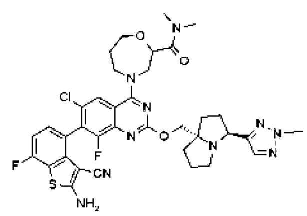

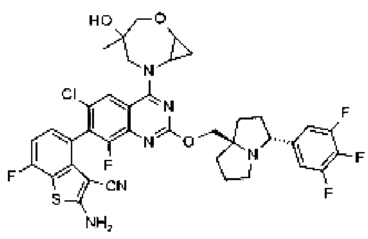
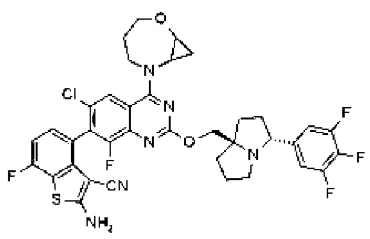

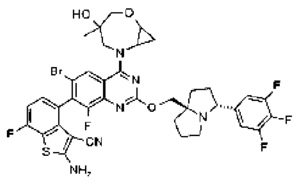
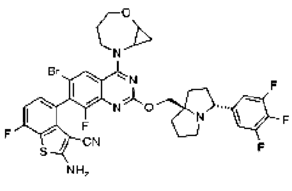
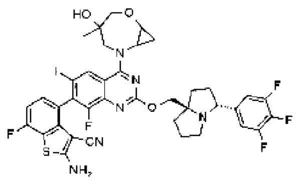

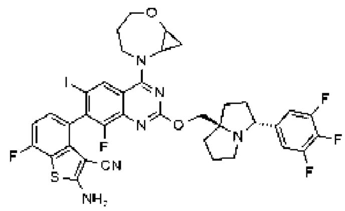
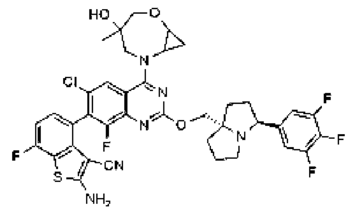

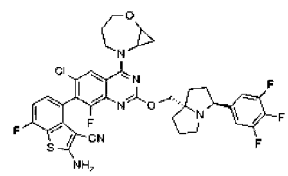
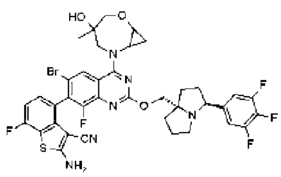
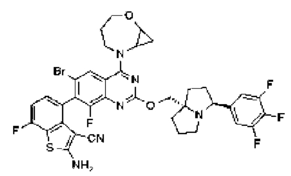

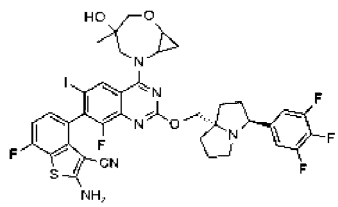
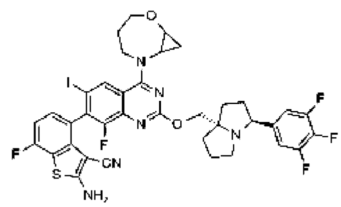

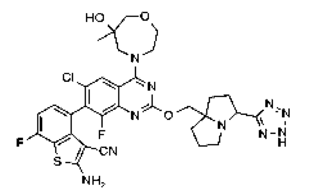
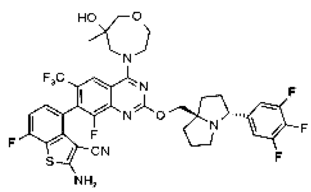
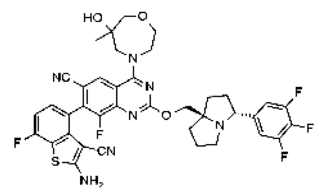

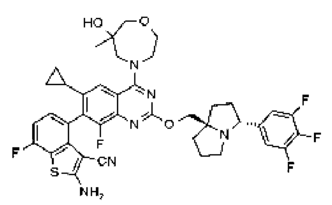
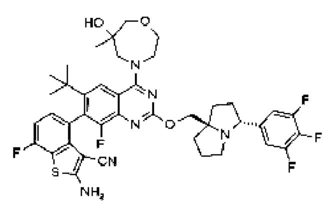

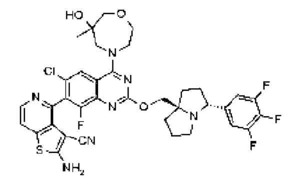
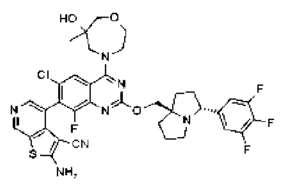
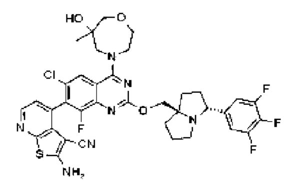

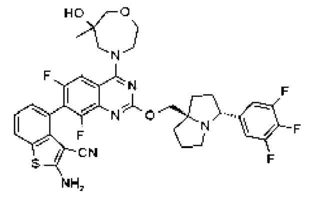
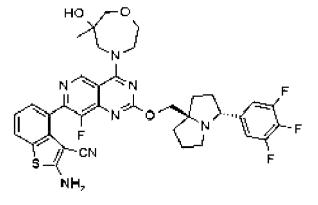

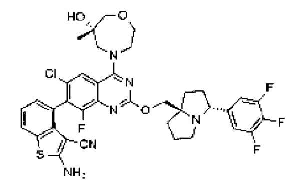
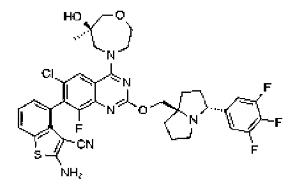
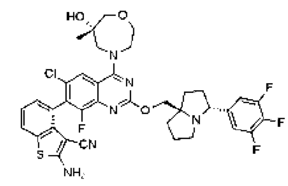

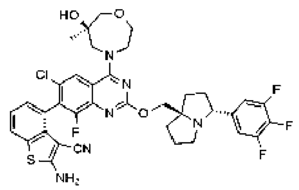
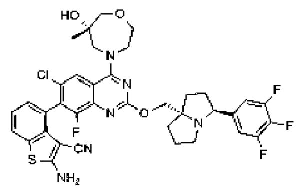

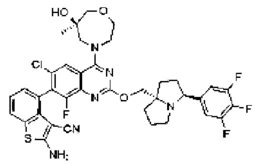
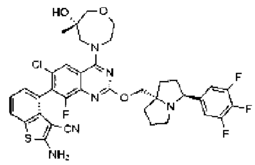
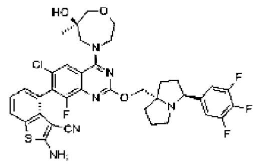

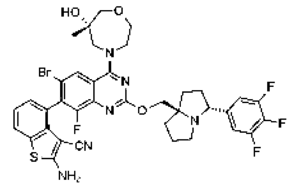
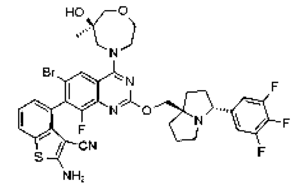

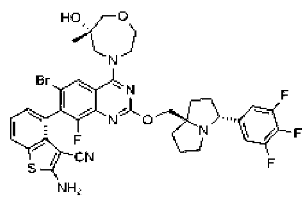
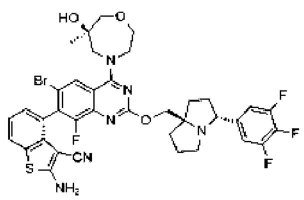
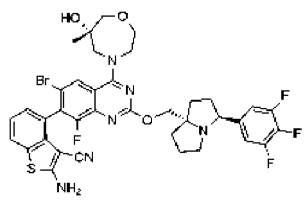

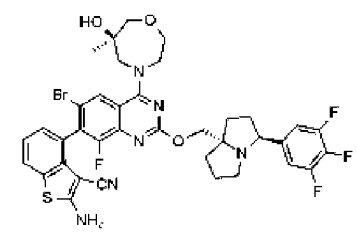
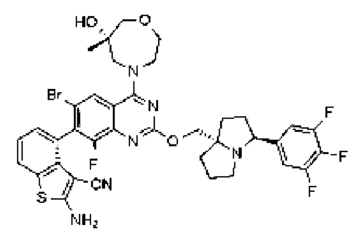

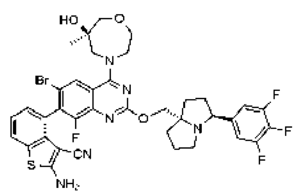
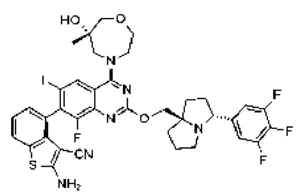
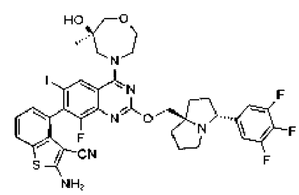

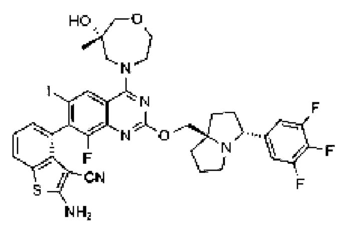
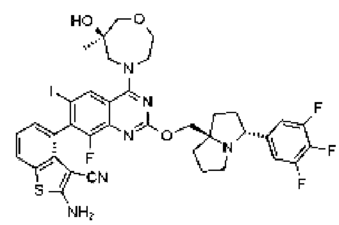

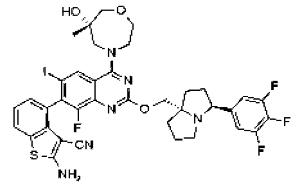
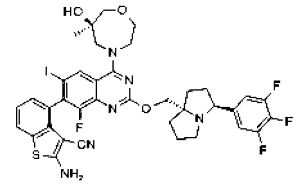
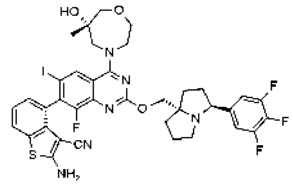

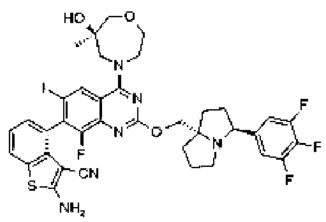
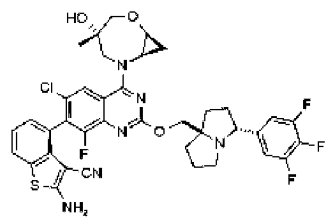

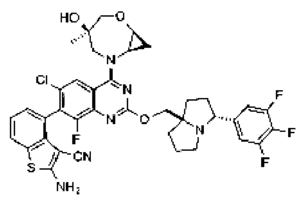
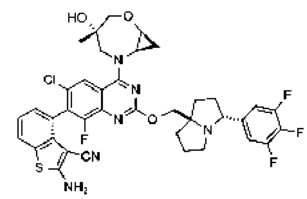
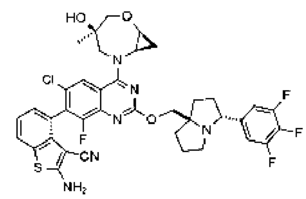

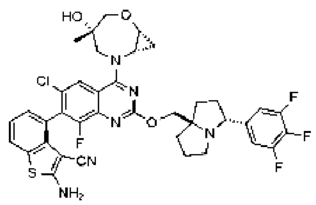
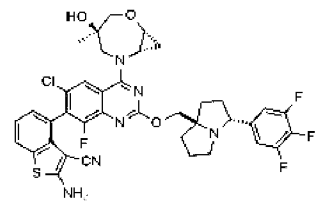

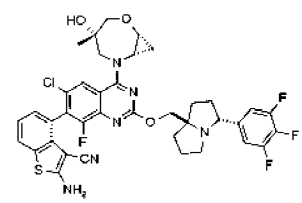
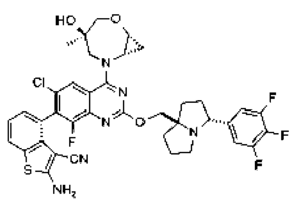
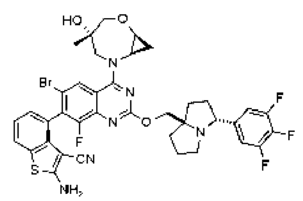

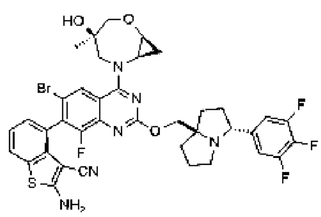
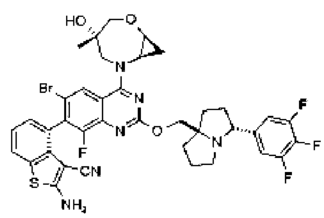

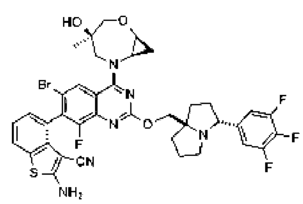
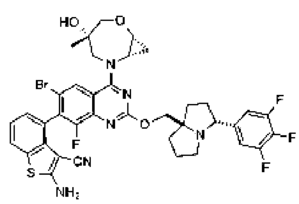
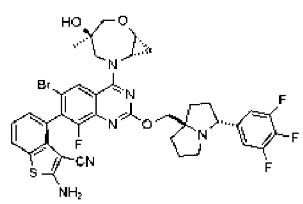

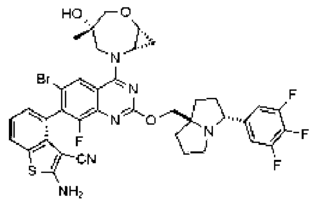
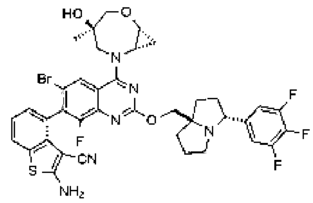

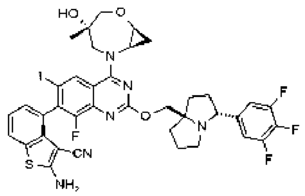
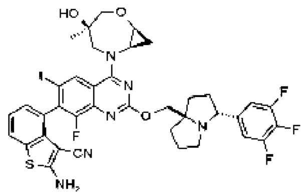
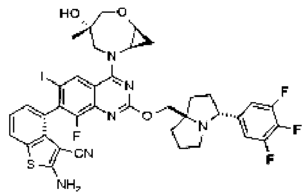

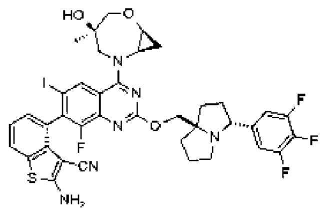
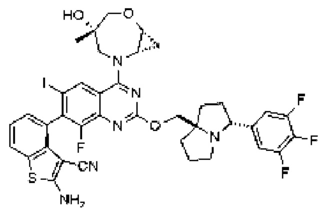

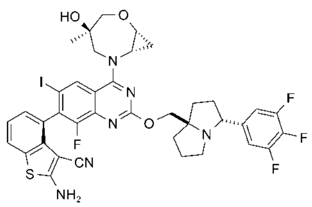
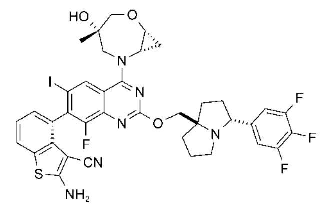
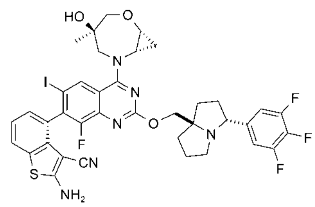
.

The present disclosure also provides a pharmaceutical composition, wherein an active compound in the pharmaceutical composition comprises one or more selected from the aforementioned compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof.

The present disclosure also provides use of the above-mentioned compound, tautomer, mesomer, racemate, enantiomer, diastereomer or a mixture thereof, metabolite, metabolic precursor, isotope-substituted form (such as hydrogen atom(s) being replaced by deuterium atom(s)), pharmaceutically acceptable salt, hydrate, solvate, polymorph or cocrystal thereof in the preparation of KRAS inhibitor drugs.

The present disclosure provides a pharmaceutical composition, wherein an active compound in the pharmaceutical composition comprises one or more selected from the above-mentioned compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof.

Preferably, the pharmaceutical composition further comprises a pharmaceutically acceptable adjuvant, wherein the pharmaceutically acceptable adjuvant is selected from one or more of a carrier, a diluent, or an excipient. Furthermore, the present disclosure does not impose any particular limitations on the formulation type and the like of the pharmaceutical composition.

The present disclosure also provides use of the above-mentioned compound, tautomer, mesomer, racemate, enantiomer, diastereomer or a mixture thereof, metabolite, metabolic precursor, isotope-substituted form (such as hydrogen atom(s) being replaced by deuterium atom(s)), pharmaceutically acceptable salt, hydrate, solvate, polymorph or cocrystal thereof in the preparation of KRAS inhibitor drugs.

Preferably, the present disclosure provides use of the above-mentioned compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof in the preparation of KRAS mutation inhibitor drugs.

The present disclosure also provides use of the above-mentioned compound, tautomer, mesomer, racemate, enantiomer, diastereomer or a mixture thereof, the metabolite, metabolic precursor, isotope-substituted form, pharmaceutically acceptable salt, hydrate, solvate, polymorph or cocrystal thereof in the preparation of a drug for the treatment,

relief, or prevention of diseases or disorders associated with Noonan syndrome, LEOPARD syndrome, leukemia, neuroblastoma, lymphoma, melanoma, esophageal cancer, head and neck tumors, breast cancer, lung cancer, bone cancer, biliary tract cancer, glioma, nasopharyngeal carcinoma, gastric cancer, kidney cancer, liver cancer, cervical cancer, thyroid cancer, bladder cancer, prostate cancer, testicular cancer, endometrial cancer, pancreatic cancer, pancreatic ductal adenocarcinoma, ovarian cancer, rectal cancer and colon cancer.

The present disclosure also provides a method for inhibiting KRAS, which involves administering to a patient an effective dose of the aforementioned compound, tautomer, mesomer, racemate, enantiomer, diastereomer or a mixture thereof, metabolite, metabolic precursor, isotope-substituted form (such as hydrogen atom(s) being replaced by deuterium atom(s)), pharmaceutically acceptable salt, hydrate, solvate, polymorph or cocrystal thereof.

The present disclosure also provides a method for inhibiting various KRAS mutations, which involves administering to a patient a certain effective dose of the aforementioned compound, tautomer, mesomer, racemate, enantiomer, diastereomer or a mixture thereof, metabolite, metabolic precursor, isotope-substituted form (such as hydrogen atom(s) being replaced by deuterium atom(s)), pharmaceutically acceptable salt, hydrate, solvate, polymorph or cocrystal thereof.

The present disclosure also provides a method for treating, alleviating, or preventing diseases, which involves administering to a patient a certain effective dose of the aforementioned compound, tautomer, mesomer, racemate, enantiomer, diastereomer or a mixture thereof, metabolite, metabolic precursor, isotope-substituted form (such as hydrogen atom(s) being replaced by deuterium atom(s)), pharmaceutically acceptable salt, hydrate, solvate, polymorph or cocrystal thereof; the diseases include, but are not limited to: the use of a medicament for diseases or disorders associated with Noonan syndrome, LEOPARD syndrome, leukemia, neuroblastoma, lymphoma, melanoma, esophageal cancer, head and neck tumors, breast cancer, lung cancer, bone cancer, biliary tract cancer, glioma, nasopharyngeal carcinoma, gastric cancer, kidney cancer, liver cancer, cervical cancer, thyroid cancer, bladder cancer, prostate cancer, testicular cancer, endometrial cancer, pancreatic cancer, pancreatic ductal adenocarcinoma, ovarian cancer, rectal cancer and colon cancer.

To more clearly describe the content of the present disclosure, the terms used are defined as follows:

The term "pharmaceutically acceptable" as described in the present disclosure refers to any substance that does not interfere with the bioactivity of the active ingredient and is non-toxic to the host to which it is given.

The pharmaceutically acceptable adjuvants as described in the present disclosure are a general term for all additional materials in a drug other than the active pharmaceutical ingredient (API). The adjuvants should possess the following properties: (1) non-toxic to the human body and with virtually no side effects; (2) chemically stable and not easily affected by temperature, pH, storage time, etc.; (3) without incompatibility with the API and not affecting the efficacy and quality control of the API; and (4) not interacting with packaging materials. The adjuvants in the present disclosure include, but are not limited to, fillers (diluents), lubricants (flow aids or anti-adhesion agents), dispersants, wetting agents, adhesives, regulators, solubilizers, antioxidants, antibacterial agents, emulsifiers, disintegrants, etc. The adhesives include syrups, gum arabic, gelatin, sorbitol, astragalus gum, cellulose and its derivatives (such as microcrystalline cellulose, sodium carboxymethyl cellulose, ethyl cellulose, or hydroxypropyl methyl cellulose), gelatin paste, syrup, starch paste, or polyvinylpyrrolidone, etc.; the fillers include lactose, powdered sugar, dextrin, starch and its derivatives, cellulose and its derivatives, inorganic calcium salts (such as calcium sulfate, calcium phosphate, dicalcium phosphate, precipitated calcium carbonate, etc.), sorbitol, or glycine, etc.; the lubricants include micronized silica gel, magnesium stearate, talc, aluminum hydroxide, boric acid, hydrogenated vegetable oil, polyethylene glycol, etc.; the disintegrants include starch and its derivatives (such as sodium carboxymethyl starch, sodium starch glycolate, etc.). The ingredients include: pregelatinized starch, modified starch, hydroxypropyl starch, corn starch, etc.; polyvinylpyrrolidone or microcrystalline cellulose, etc.; the humectants include sodium dodecyl sulfate, water or alcohol, etc.; the antioxidants include sodium sulfite, sodium bisulfite, sodium metabisulfite, dibutylbenzoic acid, etc.; the antibacterial agents include 0.5% phenol, 0.3% cresol, 0.5% chlorobutanol, etc.; the regulators include hydrochloric acid, citric acid, potassium hydroxide (sodium), sodium citrate and buffers (including sodium dihydrogen phosphate and disodium hydrogen phosphate), etc.; the emulsifiers include polysorbate-80, sorbitan oleate, poloxamer F-68, lecithin, soybean lecithin, etc.; and the solubilizers include Tween-80, bile, glycerin, etc.

There are no particular limitations on the administration mode of the compound or pharmaceutical composition described in the present disclosure. Representative administration modes include (but are not limited to): oral, parenteral (intravenous, intramuscular, or subcutaneous) and topical administration.

Solid dosage forms for oral administration include capsules, tablets, pills, powders, and granules. In these solid dosage forms, the active compound is mixed with at least one conventional inert excipient (or carrier), such as sodium citrate or dicalcium phosphate, or is mixed with the following components: (a) fillers or compatibilizers, such as starch, lactose, sucrose, glucose, mannitol, and silica; (b) binders, such as hydroxymethyl cellulose, alginate, gelatin, polyvinylpyrrolidone, sucrose, and gum arabic; (c) humectants, such as glycerin; (d) disintegrants, such as agar, calcium carbonate, potato starch or cassava starch, alginate, certain complex silicates, and sodium carbonate; (e) slowing agents, such as paraffin; (f) absorption accelerators, such as quaternary ammonium compounds; (g) wetting agents, such as cetyl alcohol and glyceryl monostearate; (h) adsorbents, such as kaolin; and (i) lubricants, such as talc, calcium stearate,

magnesium stearate, solid polyethylene glycol, sodium dodecyl sulfate, or mixtures thereof. Buffers may also be included in capsules, tablets, and pills.

Solid dosage forms such as tablets, sugar pills, capsules, pellets, and granules can be prepared using coatings and shells, such as casings and other materials known in the art. They may contain opacifying agents, and the release of the active compound or compound from such composition can be delayed in a portion of the digestive tract. Examples of encapsulating components that can be used are polymeric substances and waxes. If necessary, the active compound may also form microcapsules with one or more of the excipients described above.

Liquid dosage forms for oral administration include pharmaceutically acceptable emulsions, solutions, suspensions, syrups, or tinctures. In addition to the active compound, the liquid dosage forms may contain inert diluents conventionally used in the art, such as water or other solvents, solubilizers, and emulsifiers, such as ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures of these substances.

In addition to these inert diluents, the composition may also contain auxiliaries such as wetting agents, emulsifiers and suspending agents, sweeteners, flavoring agents and fragrances.

In addition to the active compound, the suspension may contain suspending agents such as ethoxylated isooctadecyl alcohol, polyoxyethylene sorbitol and dehydrated sorbitol esters, microcrystalline cellulose, aluminum methoxide and agar, or mixtures of these substances.

Compositions for parenteral injection may comprise physiologically acceptable sterile aqueous or anhydrous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

Dosage forms of the compounds of the present disclosure for topical administration include ointments, powders, patches, sprays, and inhalers. The active ingredient is mixed under sterile conditions with a physiologically acceptable carrier and any preservatives, buffers, or propellants that may be necessary.

The compounds in the present disclosure can also be used for injectable formulations. The injectable formulation is selected from liquid injections (water injection), sterile powders for injection (powder injection), or tablets for injection (referring to molded or machine-compressed tablets made by an aseptic method, dissolved in water for injection before use, for subcutaneous or intramuscular injection).

In addition to the aforementioned compounds, the injectable powder also contains at least an excipient. The excipient described in the present disclosure is a component intentionally added to the drug. It should not possess pharmacological properties in the amount used; however, the excipient may contribute to drug processing, dissolution or release, drug delivery via targeted administration routes, or contribute to stability.

"- - - - - -" refers to a single or double bond that satisfies all valence states.

The ring structure contains "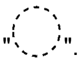". It indicates that the ring structure is a conjugate system.

"Substituted" refers to the replacement of hydrogen atoms in a molecule by other different atoms or molecules.

"Membered" refers to the number of skeleton atoms that make up the ring.

The term "one-bond" as described in the present disclosure refers to the fact that there is only one bond at that point, or it can be understood as "absent".

"Alkyl" refers to an aliphatic hydrocarbon group, and refers to a saturated hydrocarbon group. The alkyl moiety can be linear alkyl or branched alkyl. The typical alkyl includes, but is not limited to, methyl, ethyl, propyl, isopropyl, butyl, isobutyl, tert-butyl, pentyl, hexyl, etc.

As used herein, $C_1$-$C_n$ includes $C_1$-$C_2$, $C_1$-$C_3$...$C_1$-$C_n$, where n is an integer greater than one; prefixes used as substituents indicate the minimum and maximum number of carbon atoms in the substituent; for example, " $C_1$-$C_6$ alkyl" refers to linear or branched alkyl containing one to six carbon atoms. In the present disclosure, "substituted or unsubstituted $C_0$ alkyl" and "$C_0$ alkyl" refer to H.

Further, the alkyl as described above is selected from $C_{0-10}$ alkyl. Further, the $C_{0-10}$ alkyl is selected from $C_{1-10}$ alkyl; more further, the $C_{1-10}$ alkyl may be selected from $C_{1-9}$ alkyl, $C_{1-8}$ alkyl, $C_{1-7}$ alkyl, $C_{1-6}$ alkyl, $C_{1-5}$ alkyl, $C_{1-4}$ alkyl, $C_{1-3}$ alkyl, and $C_{1-2}$ alkyl.

More further, the alkyl is preferably $C_{1-3}$ alkyl.

More further, the alkyl is selected from methyl, ethyl, n-propyl, and isopropyl.

Further, the substituted alkyl is selected from $-CF_3$ and $-C_2F_5$.

"Heteroalkyl" refers to an alkyl containing heteroatoms, including but not limited to O, S, N, P, Si, etc. The heteroalkyl includes alkoxy, such as methoxy ($CH_3O$-), ethoxy ($C_2H_5O$-), propoxy ($C_3H_7O$-), etc.

"Alkenyl" refers to an aliphatic hydrocarbon group having at least one carbon-carbon double bond. The alkenyl can be linear or branched.

"Alkynyl" refers to an aliphatic hydrocarbon group having at least one carbon-carbon triple bond. The alkynyl group can be linear or branched.

"Amide" refers to a chemical structure with the formula -C(O)NHR or -NHC(O)R, where R can be selected from alkyl, heteroalkyl, cycloalkyl, heterocycloalkyl, aryl, heteroaryl, etc.

"Sulfonyl" refers to a chemical structure with the formula $-S(=O)_2R$, including sulfonamide, wherein R can be selected from alkyl, heteroalkyl, cycloalkyl, heterocycloalkyl, aryl, heteroaryl, amino, etc.

"Phosphoryl" refers to a chemical structure with the formula -P(=O)RR', where R and R' can be each independently selected from alkyl, heteroalkyl, cycloalkyl, heterocycloalkyl, aryl, heteroaryl, hydroxyl, amino, etc.; among them, the alkyl includes methyl, ethyl, propyl, isopropyl, butyl, isobutyl, tert-butyl, pentyl, hexyl, etc.

"Ester group" refers to a chemical structure with the formula -COOR, where R is selected from alkyl, heteroalkyl, cycloalkyl, heterocycloalkyl, aryl, heteroaryl, etc.

"Acyl" refers to a chemical structure with the formula -C(O)R, where R is selected from alkyl, heteroalkyl, cycloalkyl, heterocycloalkyl, aryl, heteroaryl, etc.

"Ring" refers to any covalently closed structure, including, for example, a carbocyclic ring (e.g., aryl or cycloalkyl), a heterocyclic ring (e.g., heteroaryl or heterocycloalkyl), an aromatic ring (e.g., aryl or heteroaryl), and a non-aromatic ring (e.g., cycloalkyl or heterocycloalkyl). The "ring" described in the present disclosure can be monocyclic or polycyclic, and can be fused, spirocyclic, or bridged.

"Cycloalkyl" refers to a saturated or unsaturated cyclic hydrocarbon substituent. The cycloalkyl is generally selected from $C_{1-10}$ cycloalkyl.

The typical cycloalkyl includes, but is not limited to:

cyclopropyl, cyclobutyl, cyclopentyl, cyclohexyl

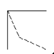, 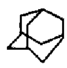,

etc.

"Heterocycloalkyl" refers to a cycloalkyl containing at least one heteroatom in its ring skeleton. The heterocycloalkyl is generally selected from $C_{1-10}$ cycloalkyl.

The typical heterocycloalkyl includes, but is not limited to:

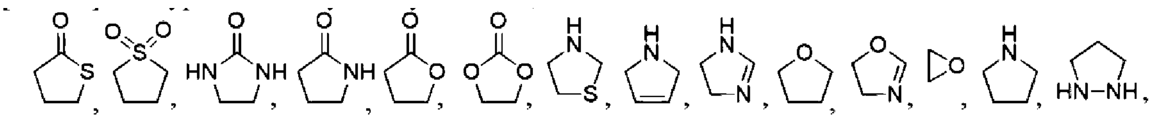

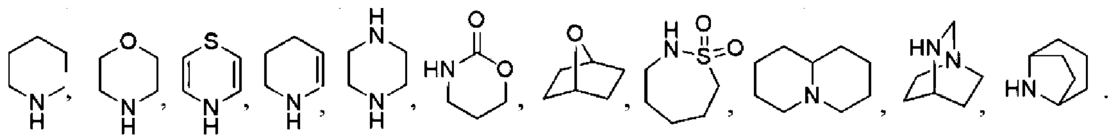

"Aryl" refers to a planar ring with a delocalized π-electron system containing 4n+2 π electrons, where n is an integer. An aryl ring can consist of five, six, seven, eight, nine, or more atoms. The aryl includes, but is not limited to, phenyl, naphthyl, phenanthryl, anthracenyl, fluorenyl, and indenyl.

The typical heteroaryl includes, but is not limited to:

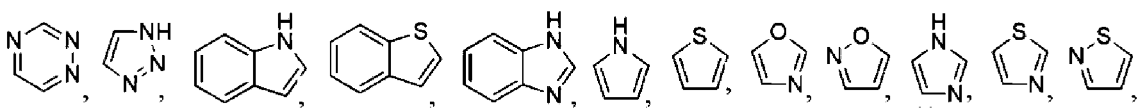

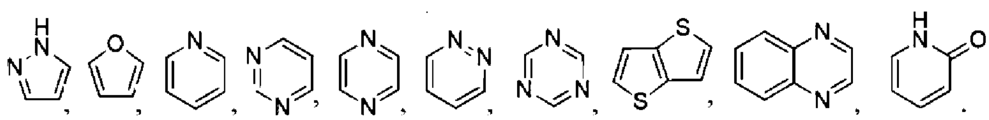

"Halogen" or "halo" refers to fluorine, chlorine, bromine, or iodine.

"Deuterium" refers to an isotope of hydrogen (H), also known as heavy hydrogen, and its element symbol is

generally D or $^2H$.

The alkyl, heteroalkyl, cyclyl group, heterocyclyl, amino, ester group, carbonyl, amide group, sulfonyl, phosphoryl, boronic acid group, boronic ester group, guanidino, acylguanidyl, aryl, heteroaryl, and imino as described in this context can be non-substituted alkyl, heteroalkyl, cyclyl group, heterocyclyl, amino, ester group, carbonyl, amide, sulfonyl, phosphoryl, boronic acid group, boronic ester group, guanidino, acylguanidyl, aryl, heteroaryl, or imino, or substituted alkyl, heteroalkyl, cyclyl group, heterocyclyl, amino, ester group, carbonyl, amide, sulfonyl, phosphoryl, boronic acid group, boronic ester group, guanidino, acylguanidyl, aryl, heteroaryl, or imino.

In the above context, unless otherwise specified, the "substituted" means that the mentioned group can be substituted by one or more additional groups, wherein the additional groups are each independently selected from alkyl, cycloalkyl, aryl, carboxyl, heteroaryl, heterocycloalkyl, hydroxyl, alkoxy, alkylthio, aryloxy, O=, guanidino, cyano, nitro, acyl, halogen, haloalkyl, amino, etc.

The term "pharmaceutically acceptable salt" refers to a salt formed by the compound of the present disclosure with an acid or base that is suitable for use as a medicine. The aforementioned acids and bases are Lewis acids and bases in a broad sense. Acids suitable for forming salts include, but are not limited to: inorganic acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, phenylmethanesulfonic acid, and benzenesulfonic acid; and acidic amino acids such as aspartic acid and glutamic acid.

"Optional" or "optionally" means that the event or environment described below may but does not have to occur, and the description includes the possibility or absence of such event or environment. For example, "optionally alkyl-substituted heterocyclic group" means that the alkyl may but does not have to be present, and the description includes cases where the heterocyclic group is substituted with alkyl and cases where the heterocyclic group is not substituted with alkyl.

"KRAS inhibitor" is a drug that inhibits KRAS activity.

The present disclosure is described in detail below compared with the prior art:

The present disclosure provides a class of novel compounds targeting pan-KRAS, exhibiting unexpectedly excellent activity and pharmacokinetic property.

The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof of the present disclosure can be used in the preparation of the drug for the treatment, relief or prevention of diseases or disorders associated with Noonan syndrome, LEOPARD syndrome, leukemia, neuroblastoma, melanoma, esophageal cancer, head and neck tumors, breast cancer, lung cancer, pancreatic cancer, pancreatic ductal adenocarcinoma, colorectal and colon cancer, and have significant development value.

The compound provided by the present disclosure can be used to treat one or more cancers caused by KRAS mutations.

The compound provided by the present disclosure can effectively block KRAS activity, and has good oral exposure levels.

The synthesis method in the present disclosure is simple to operate and has a high yield.

## DETAILED DESCRIPTION OFTHE EMBODIMENTS

In order to enable those skilled in the art to better understand the technical solution of the present disclosure, the present disclosure is further described in detail below in conjunction with specific embodiments.

In the present disclosure, the structure of the compound is determined by mass spectrometry (MS) and/or nuclear magnetic resonance ($^1H$ NMR) equipment. Chemical abbreviations have the following meanings:

DMF: N,N-dimethylformamide
THF: tetrahydrofuran
DIPEA: N,N-diisopropylethylamine
PE: petroleum ether
EA: ethyl acetate
DCM: dichloromethane
HATU: O-(7-azabenzotriazol-1-yl)-N,N,N',N'-tetramethyluronium hexafluorophosphate
HBTU: benzotriazole-N,N,N',N'-tetramethyluronium hexafluorophosphate
DMSO: dimethyl sulfoxide
BOP-Cl: bis(2-oxo-3-oxazolidinyl)phosphinoyl chloride
$(BOC)_2O$: di-tert-butyl dicarbonate
SEM-Cl: 2-(trimethylsilyl)ethoxymethyl chloride

NCS: N-chlorosuccinimide
NBS: N-bromosuccinimide
NIS: N-iodosuccinimide
LHMDS: lithium bis(trimethylsilyl)amide
TBAF: tetrabutylammonium fluoride
$TMSCF_3$: (trifluoromethyl)trimethylsilane
$CS_2CO_3$: cesium carbonate
NMP: N-methylpyrrolidone
NMO: N-methylmorpholine oxide
$K_2OsO_4$: potassium osmate
Pd/C: palladium on carbon
SFC: supercritical fluid chromatography
Synthesis of intermediates

**Preparation of Intermediate 1:** 7-bromo-2,6-dichloro-8-fluoroquinazolin-4-ol

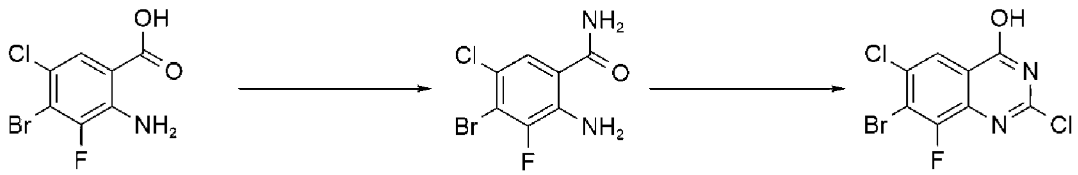

Intermediate 1

Step 1: Synthesis of 2-amino-4-bromo-5-chloro-3-fluorobenzene-1-carboxamide

2-amino-4-bromo-5-chloro-3-fluorobenzene-1-carboxylic acid (76.0 g, 284.6 mmol), ammonium chloride (91.2 g, 1704.7 mmol) and DMF (800 mL) were added to a reaction flask, followed by the sequential addition of DIPEA (365.6 g, 2834.1 mmol) and HATU (212.8 g, 560.0 mmol). The mixture was stirred at room temperature for 3 h. The reaction solution was poured into water (3 L), and a large amount of brown solid precipitated. After stirring for 0.5 h, the mixture was subjected to suction filtration, and the filter cake was rinsed with a small amount of water and then dried to obtain 42.5 g of target product (total yield: 56.1%).
LC/MS: m/z=267.0 $[M+H]^+$.

Step 2: Synthesis of 7-bromo-2,6-dichloro-8-fluoroquinazolin-4-ol

2-amino-4-bromo-5-chloro-3-fluorobenzene-1-carboxamide (23.2 g, 87.2 mmol) was dissolved in 1,4-dioxane (200 mL). After the reaction system was purged with nitrogen, a solution of thiophosgene (30.0 g, 260.9 mmol) in 1,4-dioxane (50 mL) was added dropwise at room temperature. After completion of the addition, the mixture was stirred at room temperature for 1 h, and then heated to 100°C and stirred for 1 h. The reaction solution was concentrated to dryness, and then slurried with a mixed solution of PE/EA = 3/1 (150 mL) for 1 h. The filter cake was filtered and dried to obtain 23.0 g of off-white solid (yield: 85.2%).
LC/MS: m/z = 311.1 $[M+H]^+$.

**Preparation of Intermediate 2:** 2-methylprop-2-yl {[3-cyano-4-(5,5-dimethyl-1,3,2-dioxaborinan-2-yl)-7-fluorobenzo [b]thiophen-2-yl]amino}formate

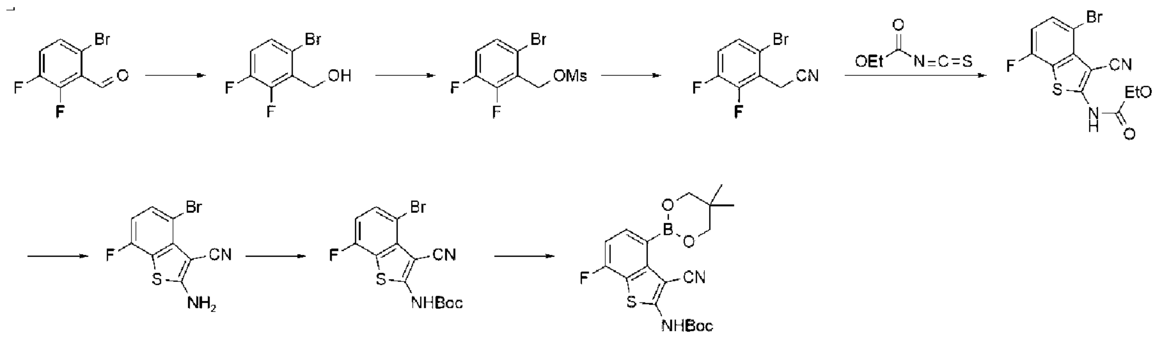

Intermediate 2

Step 1: Synthesis of (6-bromo-2,3-difluorophenyl)methanol

6-bromo-2,3-difluorobenzene-1-carboxaldehyde (100.0 g, 450 mmol) was weighed and dissolved in ethanol (1 L). Sodium borohydride (18.0 g, 480 mmol) was added in portions at 0°C, and the mixture was stirred at room temperature for 0.5 h. The reaction system was poured into water (3 L), and then extracted with dichloromethane. The organic phase was backwashed once with saturated brine, dried, and to obtain 100.9 g of white solid. The white solid was used directly for the next step.
LC/MS: m/z = 223.1 $[M+H]^+$.

Step 2: Synthesis of (4-bromo-1,2-difluorobenzen-3-yl)methyl methanesulfonate

(6-bromo-2,3-difluorophenyl)methanol (the product from the previous step) and triethylamine (91.6 g, 900 mmol) were dissolved in dichloromethane (1 L). Methanesulfonyl chloride (57.0 g, 500 mmol) was added dropwise at 0°C. After completion of the addition, the mixture was stirred at 0°C for 1 h. The reaction system was poured into water (3 L), and then extracted with dichloromethane. The organic phase was backwashed once with saturated brine, dried, and concentrated under reduced pressure to obtain 119.7 g of off-white solid (two-step yield: 87.5%).

Step 3: Synthesis of (6-bromo-2,3-difluorophenyl)acetonitrile

(4-bromo-1,2-difluorobenzen-3-yl)methyl methanesulfonate (119.7 g, 397.7 mmol) and trimethylcyanosilane (78.9 g, 795.3 mmol) were dissolved in acetonitrile (1 L). A solution of tetrabutylammonium fluoride trihydrate (250.9 g, 795.3 mmol) in tetrahydrofuran (500 mL) was added dropwise under ice bath conditions. After completion of the addition, the mixture was stirred at room temperature for 1 h. The reaction system was poured into saturated brine (3 L), and then extracted with ethyl acetate. The organic phase was backwashed once with saturated brine, dried, and concentrated under reduced pressure to obtain 89.0 g of yellow solid (yield: 96.0%).
LC/MS: m/z = 231.2 $[M+H]^+$.

Step 4: Synthesis of ethyl [(4-bromo-3-cyano-7-fluorobenzo[b]thiophen-2-yl)amino]carbamate

(6-bromo-2,3-difluorophenyl)acetonitrile (89.0 g, 380 mmol) was dissolved in 900 mL DMF. Potassium tert-butoxide (64.4 g, 580 mmol) was added in portions at 0°C. After stirring at 0°C for 20 min, ethyl isothiocyanatoformate (75.4 g, 580 mmol) was added dropwise. After completion of the addition, the mixture was stirred at room temperature for 1 h, followed by stirring at 100°C for 0.5 h. After the reaction solution was cooled to room temperature, the reaction system was poured into water (3 L), and a large amount of solid precipitated. The resulting suspension was filtered, and the filter cake was washed with a small amount of water and dried to obtain a crude product. The crude product was used directly for the next step.
LC/MS: m/z = 343.1 $[M+H]^+$.

Step 5: Synthesis of 2-amino-4-bromo-7-fluorobenzo[b]thiophene-3-carbonitrile

Ethyl [(4-bromo-3-cyano-7-fluorobenzo[b]thiophen-2-yl)amino]carbamate (the product from the previous step) was dissolved in DMSO (680 mL), and then a solution of sodium hydroxide (112.0 g, 2800 mmol) in water (400 mL) was added. The mixture was reacted overnight at 100°C. After the reaction solution was cooled to room temperature, the reaction system was poured into water (1 L) and slurried for 1 h. The resulting slurry was filtered, and the filter cake was

washed once with a small amount of water, and dried to give 88.0 g of yellow solid (two-step yield: 84.6%). LC/MS: m/z = 271.1 $[M+H]^+$.

Step 6: Synthesis of 2-methylpropan-2-yl[(4-bromo-3-cyano-7-fluorobenzo[b]thiophen-2-yl)amino]formate

2-amino-4-bromo-7-fluorobenzo[b]thiophene-3-carbonitrile (88.0 g, 320 mmol) and 4-dimethylaminopyridine (4.0 g, 30 mmol) were dissolved in THF/DMF (450 mL/450 mL), and then $(Boc)_2O$ (99 g, 450 mmol) was added. The reaction system was stirred overnight at room temperature. The reaction solution was poured into water (2 L) and extracted with ethyl acetate. Activated carbon (360 g) and silica gel (50 g) were added to the organic phase, and then stirred overnight. The mixture was subjected to suction filtration, and then the filtrate was dried and concentrated under reduced pressure to obtain 86.0 g of yellow solid (yield: 72.0%).
LC/MS: m/z = 371.1 $[M+H]^+$.

Step 7: Synthesis of 2-methylpropan-2-yl { [3-cyano-4-(5,5-dimethyl-1,3,2-dioxaborinan-2-yl)-7-fluorobenzo [b]thiophen-2-yl]amino}formate

2-methylpropan-2-yl[(4-bromo-3-cyano-7-fluorobenzo[b]thiophen-2-yl)amino]carbamate (24.0 g, 64.7 mmol), 2-(5,5-dimethyl-1,3,2-dioxaborinan-2-yl)-5,5-dimethyl-1,3,2-dioxaborinane (43.9 g, 194.1 mmol), potassium acetate (19.0 g, 194.1 mmol), and dioxane (450 mL) were added to a reaction flask. After the reaction system was purged with nitrogen, $Pd(dppf)Cl_2$ (2.3 g, 3.2 mmol) was added. The reaction mixture was heated to 100°C and stirred overnight under nitrogen. After being cooled to room temperature, the reaction system was poured into water (1.5 L), and then extracted with ethyl acetate. The organic phase was dried, concentrated under reduced pressure, and then purified by silica gel column chromatography (PE/EA=50/1) to obtain 11.8 g of white solid (yield: 45.5%).
LC/MS: m/z = 405.2 $[M+H]^+$.

**Preparation of Intermediate 3:** 2-methylpropan-2-yl{[3-cyano-4-(4,4,5,5-tetramethyl-1,3,2-dioxaborolan-2-yl)benzo [b]thiophen-2-yl] amino}formate

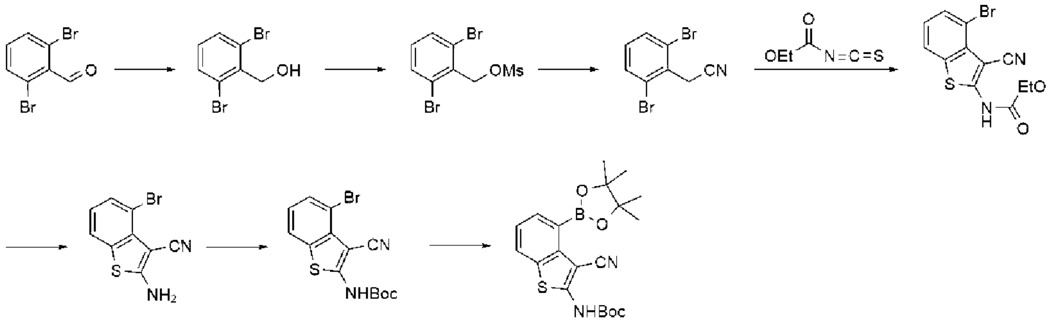

Intermediate 3

Step 1: Synthesis of (2,6-dibromophenyl)methanol

2,6-dibromobenzene-1-carboxaldehyde (25.0 g, 94.7 mmol) was dissolved in ethanol (250 mL), and sodium borohydride (3.8 g, 100.0 mmol) was added at 0°C. The mixture was stirred at room temperature for 0.5 h. The reaction mixture was poured into water (1 L), and then extracted with dichloromethane. The organic phase was backwashed once with saturated brine, dried, and concentrated under reduced pressure to obtain 25.0 g of white solid (yield: 99.3%). LC/MS: m/z=265.0 $[M+H]^+$.

Step 2: Synthesis of (1,3-dibromobenzen-2-yl)methyl methanesulfonate

(2,6-dibromophenyl)methanol (15.0 g, 56.4 mmol) and triethylamine (11.4 g, 112.8 mmol) were dissolved in dichloromethane (150 mL). Methanesulfonyl chloride (7.1 g, 62.1 mmol) was added dropwise at 0°C. After completion of the dropwise addition, the mixture was stirred at 0°C for 1 h. The reaction system was poured into water (500 mL), and then extracted with dichloromethane. The organic phase was backwashed once with saturated brine, dried, and concentrated under reduced pressure to obtain 16.6 g of off-white solid (yield: 85.6%).

Step 3: Synthesis of (2,6-dibromophenyl)acetonitrile

(1,3-dibromobenzen-2-yl)methyl methanesulfonate (16.6 g, 48.3 mmol) and trimethylcyanosilane (9.6 g, 96.5 mmol) were dissolved in acetonitrile (170 mL). A solution of tetrabutylammonium fluoride trihydrate (30.4 g, 96.5 mmol) in tetrahydrofuran (90 mL) was added dropwise under ice bath conditions. After completion of the addition, the mixture was stirred at room temperature for 1 h. The reaction mixture was then poured into saturated brine (500 mL), and then extracted with ethyl acetate. The organic phase was backwashed once with saturated brine. The organic phase was dried, concentrated under reduced pressure, and purified by silica gel column chromatography (PE/EA = 10/1) to obtain 11.9 g of off-white solid (yield: 89.4%).
LC/MS: m/z = 273.1 $[M+H]^+$.

Step 4: Synthesis of ethyl [(4-bromo-3-cyanobenzo[b]thiophen-2-yl)amino]carbamate

(2,6-dibromophenyl)acetonitrile (11.9 g, 43.2 mmol) was dissolved in DMF (120 mL). After the reaction system was purged with nitrogen, NaH (60%, 1.7 g, 43.1 mmol) was added at 0°C. The mixture was stirred at 0°C for 20 min, followed by dropwise addition of ethyl isothiocyanatoformate (6.2 g, 47.4 mmol) at 0°C. After completion of the dropwise addition, the mixture was stirred at room temperature for 20 min. Cuprous iodide (822 mg, 4.3 mmol) and L-proline (994 mg, 8.6 mmol) were added, and then the mixture was stirred overnight at 60°C. After being cooled to room temperature, the reaction system was poured into water containing 2.0% ethylenediaminetetraacetic acid (300 mL) and stirred overnight. The reaction mixture was filtered, and the filter cake was washed three times with a small amount of water, and dried to obtain 11.8 g of pale yellow solid (yield: 84.0%).
LC/MS: m/z = 325.1 $[M+H]^+$.

Step 5: Synthesis of 2-amino-4-bromobenzo[b]thiophene-3-carbonitrile

Ethyl [(4-bromo-3-cyanobenzo[b]thiophen-2-yl)amino]carbamate (11.8 g, 36.2 mmol) was dissolved in DMSO (55 mL), and a solution of sodium hydroxide (10.1 g, 253.6 mmol) in water (36 mL) was added. The mixture was reacted overnight at 110°C. After the reaction solution was cooled to room temperature, the reaction system was poured into ice water (300 mL) and slurried for 1 h. The resulting slurry was filtered, and the filter cake was washed once with a small amount of water and dried to obtain 9.0 g of pale yellow solid. The pale yellow solid was used directly for the next-step reaction.
LC/MS: m/z = 253.1 $[M+H]^+$.

Step 6: Synthesis of 2-methylprop-2-yl[(4-bromo-3-cyanobenzo[b]thiophen-2-yl)amino]formate

2-amino-4-bromobenzo[b]thiophene-3-carbonitrile (the crude product from the previous step) and 4-dimethylaminopyridine (442 mg, 3.6 mmol) were dissolved in THF/DMF (50 mL/50 mL), and $(Boc)_2O$ (10.3 g, 47.1 mmol) was added. The reaction system was stirred overnight at room temperature. The reaction solution was poured into water (300 mL), and then extracted with ethyl acetate. The organic phase was dried, concentrated under reduced pressure, and purified by silica gel column chromatography (PE/EA = 10/1) to obtain 10.5 g of pale yellow solid (two-step yield: 82.4%).
LC/MS: m/z=353.0 $[M+H]^+$.

Step 7: Synthesis of 2-methylpropan-2-yl{[3-cyano-4-(4,4,5,5-tetramethyl-1,3,2-dioxaborolan-2-yl)benzo[b]thiophen-2-yl] amino}formate

2-methylpropan-2-yl[(4-bromo-3-cyanobenzo[b]thiophen-2-yl)amino]formate (10.5 g, 29.8 mmol), pinacol diboronate (15.1 g, 59.7 mmol), potassium acetate (8.8 g, 89.6 mmol), and dioxane (100 mL) were added to a reaction flask. After the reaction system was purged with nitrogen, $Pd(dppf)Cl_2$ (1.1 g, 1.5 mmol) was added. The reaction mixture was heated to 90°C and stirred for 6 h under nitrogen. After being cooled to room temperature, the reaction system was poured into water (300 mL), and then extracted with ethyl acetate. The organic phase was dried, concentrated under reduced pressure, and purified by silica gel column chromatography (PE/EA = 30/1) to obtain 5.2 g of off-white solid (yield: 43.9%).
LC/MS: m/z = 401.2 [M+H]+.

**Preparation of Intermediate 4:** [3-(3,4,5-trifluorophenyl)-2,3,5,6,7,7a-hexahydro-1H-pyrrolizin-7a-yl]methanol (Trans racemate)

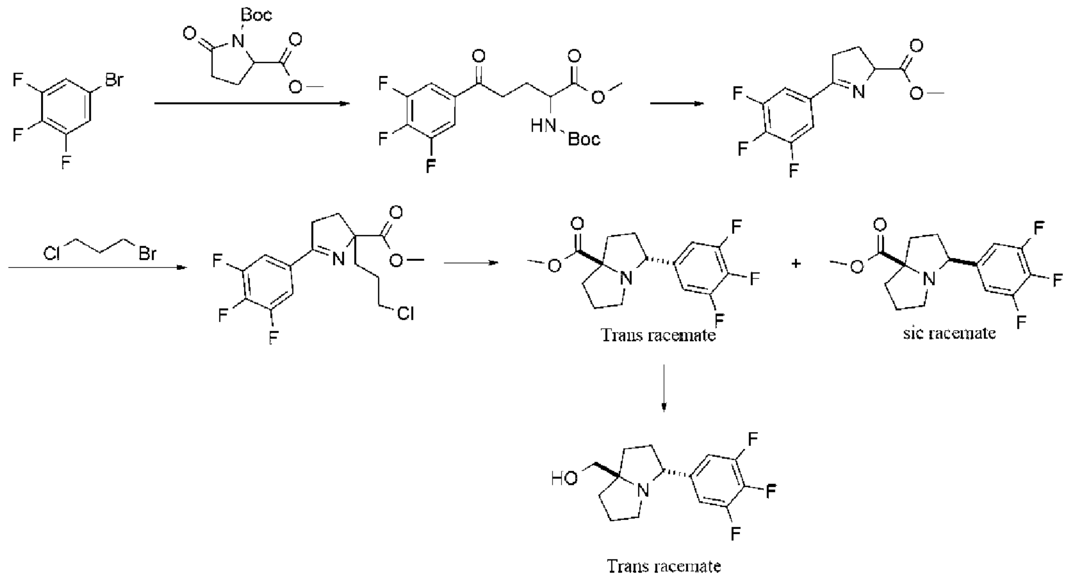

Trans racemate

sic racemate

Trans racemate

Intermediate 4

Step 1: Synthesis of methyl 2-({[(2-methylpropan-2-yl)oxy]carbonyl}amino)-5-oxo-5-(3,4,5-trifluorophenyl)valerate

Magnesium shavings (4.8 g, 200 mmol) were weighed into a reaction flask A, and 3,4,5-trifluorobromobenzene (1 g, 4.8 mmol), iodine (0.2 g), and tetrahydrofuran (5 mL) were added. After heating to initiate the reaction, a solution of 3,4,5-trifluorobromobenzene (20.0 g, 95.2 mmol) in tetrahydrofuran (150 mL) was added dropwise. The reaction system was maintained under reflux. After completion of the dropwise addition, reflux was continued for 0.5 h. 1-(tert-butyl)-2-methyl-5-oxopyrrolidine-1,2-dicarboxylate (24.3 g, 100 mmol) and tetrahydrofuran (250 mL) were added to another reaction flask B. The reaction mixture was cooled to -70°C under nitrogen using a dry ice-ethanol bath. The p-methoxyphenyl magnesium bromide solution from the reaction flask A was added in portions to the reaction flask B, and the reaction was carried out at this temperature for 3 h. A saturated ammonium chloride solution (500 mL) was added. The reaction system was extracted three times with ethyl acetate. The organic phase was dried, concentrated under reduced pressure, and then purified by column chromatography (PE/EA=1/1) to obtain 26.4 g of target product (yield: 70.0%).
LC/MS: m/z = 376.1 $[M+H]^+$.

Step 2: Synthesis of methyl 5-(3,4,5-trifluorophenyl)-3,4-dihydro-2H-pyrrole-2-carboxylate

Methyl 2-({[(2-methylpropan-2-yl)oxy]carbonyl}amino)-5-oxo-5-(3,4,5-trifluorophenyl)valerate (26.4 g, 70.4 mmol) was added to a reaction flask, followed by the addition of HCl/MeOH (4 M, 200 mL) at room temperature. The mixture was stirred overnight at room temperature. The reaction system was directly concentrated under reduced pressure, and 100 mL of saturated sodium bicarbonate aqueous solution was added. The mixture was extracted with ethyl acetate. The organic phase was dried and concentrated under reduced pressure to obtain 17.0 g of product (yield: 94.0%).
LC/MS: m/z = 258.1 $[M+H]^+$.

Step 3: Synthesis of methyl 2-(3-chloropropyl)-5-(3,4,5-trifluorophenyl)-3,4-dihydro-2H-pyrrole-2-carboxylate

Methyl 5-(3,4,5-trifluorophenyl)-3,4-dihydro-2H-pyrrole-2-carboxylate (17.0 g, 66.1 mmol) and 1-bromo-3-chloropropane (20.6 g, 132.3 mmol) were dissolved in a mixed solution of DMF (100 mL) and THF (200 mL). Sodium hydride (60%, 4.0 g, 99.2 mmol) was added in portions under ice bath conditions, and the mixture was stirred at room temperature for 2 h. The reaction mixture was then poured into a saturated ammonium chloride aqueous solution (1 L), and then extracted with ethyl acetate. The organic phase was dried, and concentrated under reduced pressure. The crude product was purified by column chromatography (PE/EA = 8/1) to obtain 16.6 g of off-white solid (yield: 75.5%).
LC/MS: m/z = 334.1 $[M+H]^+$.

Step 4: Synthesis of methyl 3-(3,4,5-trifluorophenyl)-2,3,5,6,7,7a-hexahydro-1H-pyrrolizin-7a-carboxylate (Trans racemate)

Methyl 2-(3-chloropropyl)-5-(3,4,5-trifluorophenyl)-3,4-dihydro-2H-pyrrole-2-carboxylate (16.6 g, 49.8 mmol) was dissolved in methanol (200 mL), acetic acid (2 mL) was added, and then $NaBH_3CN$ (6.3 g, 100.3 mmol) was added in portions under ice bath conditions. The mixture was stirred at room temperature for 3 h. The reaction mixture was concentrated to dryness, and water (200 mL) was added. The solution was adjusted to weakly basic with sodium carbonate, and then extracted with ethyl acetate. The organic phase was dried, and concentrated under reduced pressure. The crude product was purified by column chromatography (PE/EA=5/1) and then eluted to obtain 6.3 g of cis product as pale yellow oil (cis racemate); subsequently, elution was continued with PE/EA = 2:1, affording 5.0 g of the trans racemate as an off-white solid (trans racemate), with a total yield of 75.8%.
LC/MS: m/z = 300.2 $[M+H]^+$.

Step 5: Synthesis of [3-(3,4,5-trifluorophenyl)-2,3,5,6,7,7a-hexahydro-1H-pyrrolizin-7a-yl]methanol (Trans racemate)

Methyl 3-(3,4,5-trifluorophenyl)-2,3,5,6,7,7a-hexahydro-1H-pyrrolizin-7a-carboxylate (Trans racemate) (5.0 g, 16.7 mmol) was dissolved in tetrahydrofuran (50 mL), and lithium aluminum hydride (1.14 g, 30 mmol) was added at 0°C, and the mixture was stirred for 1 h. Water (200 mL) was added to the reaction system. The mixture was extracted with ethyl acetate, and then washed with saturated brine. The organic phase was dried over anhydrous sodium sulfate and then filtered. The filtrate was concentrated under reduced pressure, and then purified by column chromatography (DCM/MeOH = 20/1) to obtain 4.1 g of target product (yield: 91%).
LC/MS: m/z = 272.2 $[M+H]^+$.

**Preparation of Intermediate 5:** [3-(3,4,5-trifluorophenyl)-2,3,5,6,7,7a-hexahydro-1H-pyrrolizin-7a-yl]methanol (Trans isomer 2)

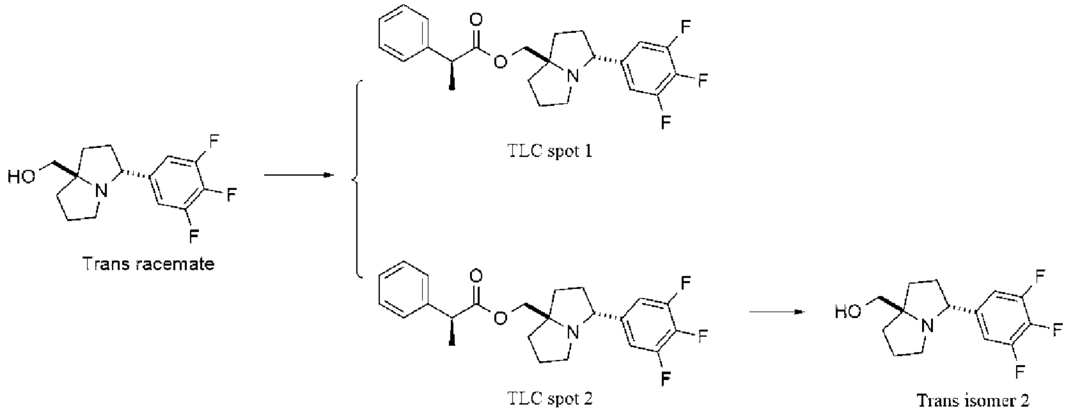


Intermediate 5

Step 1: Synthesis of (2S)-[3-(3,4,5-trifluorophenyl)-2,3,5,6,7,7a-hexahydro-1H-pyrrolizin-7a-yl]methyl2-phenylpropanoat e (TLC spot 2)

[3-(3,4,5-trifluorophenyl)-2,3,5,6,7,7a-hexahydro-1H-pyrrolizin-7a-yl]methanol (Trans racemate) (1.0 g, 3.7 mmol), (S)-2-phenylpropionic acid (554 mg, 3.7 mmol), 4-dimethylaminopyridine (24 mg, 0.2 mmol), and dichloromethane (10 mL) were added to a reaction flask. 1-ethyl-(3-dimethylaminopropyl)carbodiimide hydrochloride (1.0 g, 5.2 mmol) was added in portions at room temperature. The mixture was stirred overnight at room temperature. The reaction solution was poured into water (30 mL), and then extracted with dichloromethane. The organic phase was dried, concentrated under reduced pressure, and purified by column chromatography (DCM/MeOH = 50/1) to obtain 560 mg of top spot (TLC spot 1) as a pale yellow solid and 570 mg of bottom spot (TLC spot 2) as a pale yellow solid (total yield: 75.8%).
LC/MS: m/z=404.2 $[M+H]^+$.

Step 2: Synthesis of [3-(3,4,5-trifluorophenyl)-2,3,5,6,7,7a-hexahydro-1H-pyrrolizin-7a-yl]methanol (Trans isomer 2)

(2S)-[3-(3,4,5-trifluorophenyl)-2,3,5,6,7,7a-hexahydro-1H-pyrrolizin-7a-yl]methyl 2-phenylpropanoate (TLC spot 2 of the previous product, 570 mg, 1.4 mmol) was dissolved in MeOH/$H_2O$ (5 mL/0.5 mL), and NaOH (80 mg, 2.0 mmol) was added. The mixture was stirred at room temperature for 1 h. The reaction mixture was poured into water (20 mL), and then extracted with ethyl acetate. The organic phase was backwashed once with 1M NaOH aqueous solution, dried, and concentrated under reduced pressure to obtain 380 mg of target product (Trans isomer 2) (yield: 99%).
LC/MS: m/z = 272.2 $[M+H]^+$.

**Preparation of Intermediate 6:** Synthesis of 2-methylpropan-2-yl[(4-bromo-3-cyanothieno[2,3-c]pyridin-2-yl)amino] formate

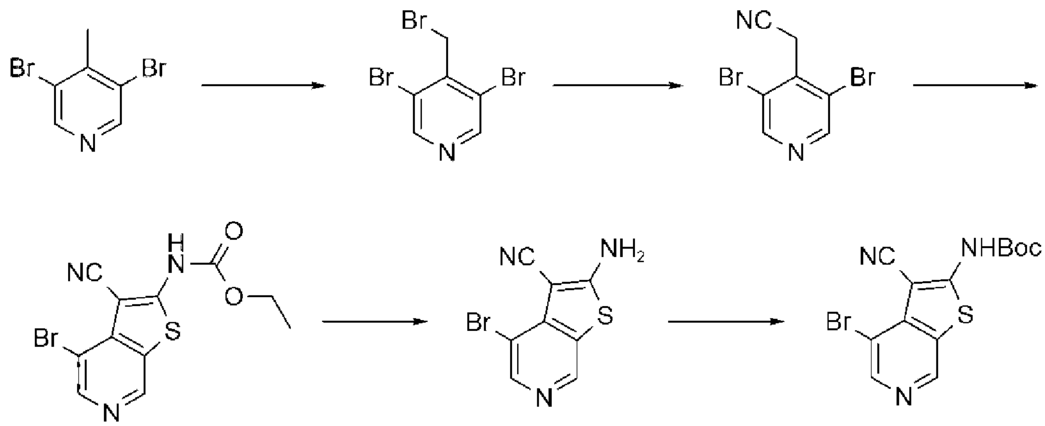

Intermediate 6

Step 1: Synthesis of 3,5-dibromo-4-(bromomethyl)pyridine

3,5-dibromo-4-methylpyridine (10.0 g, 40 mmol) was dissolved in acetonitrile (100 mL), and NBS (8.5 g, 48 mmol) was added in portions at room temperature. The mixture was then heated to 80°C and reacted for 3 h. The reaction system was poured into water (300 mL), and then extracted with ethyl acetate. The organic phase was backwashed once with saturated brine, dried, and concentrated under reduced pressure to obtain 18.9 g of black oil. The black oil was used directly for the next step.
LC/MS: m/z=325.9 $[M-H]^-$.

Step 2: Synthesis of 3,5-dibromo-4-(cyanomethyl)pyridine

3,5-dibromo-4-(bromomethyl)pyridine was dissolved in acetonitrile (100 mL), and trimethylcyanosilane (5.9 g, 60 mmol) was added at 0°C. Then, a solution of tetrabutylammonium fluoride in tetrahydrofuran (60 mL, 60 mmol) was added dropwise at 0°C. After completion of the dropwise addition, the reaction mixture was allowed to react at room temperature for 3 h. The reaction system was poured into water (300 mL), and then extracted with ethyl acetate. The organic phase was dried, concentrated under reduced pressure, and purified by silica gel column chromatography (PE/EA = 6/1) to obtain 6.9 g of yellow solid (two-step total yield: 62.7%).
LC/MS: m/z=272.8 $[M-H]^-$.

Step 3: Synthesis of ethyl [(4-bromo-3-cyanothieno[2,3-c]pyridin-2-yl)amino]formate

3,5-dibromo-4-(cyanomethyl)pyridine (5.0 g, 18.3 mmol) was dissolved in DMF (50 mL). Sodium hydride (730 mg, 20.1 mmol) was added at 0°C. After completion of the addition, the mixture was stirred at 0°C for 0.5 h. Then, ethyl isothiocyanatoformate (2.6 g, 20.1 mmol) was added dropwise at 0°C. After completion of the dropwise addition, the mixture was reacted at room temperature for 1 h. Then, cuprous iodide (347 mg, 1.8 mmol) and L-proline (210 mg, 1.8 mmol) were added, and the mixture was heated to 65°C and reacted for 16 h. Ammonia water (150 mL) was added dropwise to the reaction system, and a large amount of solids precipitated. The resulting suspension was stirred at room temperature for 6 h and then subjected to suction filtration. The filter cake was washed with a small amount of water and

dried to obtain 3.2 g of green solid (yield: 53.9%).
LC/MS: m/z=323.9 [M-H]$^-$.

Step 4: Synthesis of 2-amino-4-bromothieno[2,3-c]pyridine-3-carbonitrile

Ethyl [(4-bromo-3-cyanothieno[2,3-c]pyridin-2-yl)amino]formate (3.2 g, 10 mmol) was dissolved in DMSO (15 mL), followed by the addition of an aqueous sodium hydroxide solution (5.0 mol/L, 15 mL). The mixture was heated to 60°C and reacted for 3 h. After the reaction solution cooled to room temperature, water (90 mL) was added dropwise, resulting in the precipitation of a large amount of solid. The resulting suspension was filtered. The filter cake was washed with a small amount of water and dried to obtain 2.2 g of green solid (yield: 88.3%).
LC/MS: m/z=251.9 [M-H]$^-$.

Step 5: Synthesis of 2-methylpropan-2-yl[(4-bromo-3-cyanothieno[2,3-c]pyridin-2-yl)amino]formate

2-amino-4-bromothieno[2,3-c]pyridine-3-carbonitrile (2.2 g, 9 mmol) was dissolved in DMF (11 mL) and tetrahydrofuran (11 mL), and di-tert-butyl dicarbonate (3.0 g, 14 mmol) was added. The reaction mixture was reacted at room temperature for 3 h. The reaction system was poured into water (50 mL), and then extracted with ethyl acetate. The organic phase was dried, concentrated under reduced pressure, and purified by silica gel column chromatography (PE/EA = 3/1) to obtain 664 mg of yellow solid (yield: 21.6%).
LC/MS: m/z=351.9 [M-H]$^-$.

Example 1

2-methylpropan-2-yl({4-[6-chloro-4-(6-{[(2,2-dimethyl-1,3-dioxacyclopentan-4-yl)carbonyl ]amino}-1,4-oxazepan-4-yl)-8-fluoro-2-({[3-(3,4,5-trifluorophenyl)-2,3,5,6,7,7a-hexahydro-1H-pyrrol izin-7a-yl]methyl}oxy)quinazolin-7-yl]-3-cyano-7-fluorobenzo[b]thiophen-2-yl}amino)formate (Trans racemate)

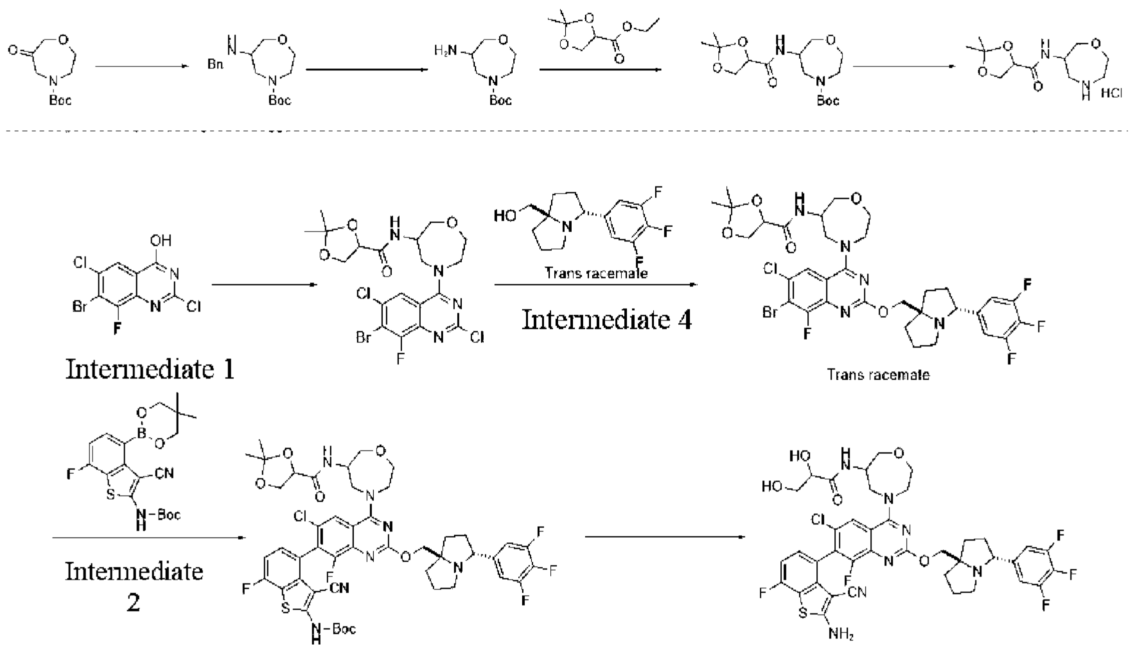


Step 1: Synthesis of 2-methylpropan-2-yl 6-(benzylamino)-1,4-oxazepane-4-carboxylate

2-methylpropan-2-yl 6-oxo-1,4-oxazepane-4-carboxylate (1.0 g, 4.69 mmol), benzylamine (502 mg, 4.69 mmol) and 4A molecular sieve (100 mg) were weighed and dissolved in DCM (15 mL). The materials were stirred at room temperature and reacted for 2 h, then sodium borohydride acetate (2.0 g, 9.38 mmol) was added, and the mixture was stirred at room temperature for 2 h. The reaction solution was added to water (50 mL) and then filtered, the filtrate was collected and then extracted three times with DCM. The organic phase was dried and concentrated under reduced pressure, and then purified by silica gel column chromatography (PE/EA = 1/1) to obtain 1.0 g of colorless oil (yield: 58.8%).
LC/MS: m/z = 367.2 [M+H]$^+$.

Step 2: Synthesis of 2-methylpropan-2-yl 6-amino-1,4-oxazepane-4-carboxylate

2-methylpropan-2-yl 6-(benzylamino)-1,4-oxazepane-4-carboxylate (1.0 g, 2.73 mmol) was added to a reaction flask and dissolved in methanol (10 mL). After purging with hydrogen, Pd/C (500 mg) was added. The reaction mixture was stirred at room temperature for 16 h under a hydrogen atmosphere. The reaction mixture was filtered through a diatomaceous earth, and the filtrate was collected and concentrated under reduced pressure to obtain 782 mg of colorless oil. The obtained colorless oil was used directly for the next step.
LC/MS: m/z = 217.2 $[M+H]^+$.

Step 3: Synthesis of 2-methylpropan-2-yl 6-{[(2,2-dimethyl-1,3-dioxolan-4-yl)carbonyl]amino}-1,4-oxazepane-4-carboxylate

Ethyl 2,2-dimethyl-1,3-dioxolane-4-carboxylate (200 mg, 1.25 mmol) and 2-methylpropan-2-yl 6-amino-1,4-oxazepan-4-carboxylate (430 mg, crude product) were weighed and dissolved in tetrahydrofuran (5 mL). After the system was cooled to 0°C, lithium hexamethyldisilazide (LHMDS) (1.9 mL, 2 M) was slowly added. After completion of the addition, the mixture was slowly heated to room temperature and reacted under stirring for 16 h. The reaction solution was added to a saturated ammonium chloride aqueous solution (50 mL), and then extracted with ethyl acetate. The organic phase was dried, concentrated under reduced pressure, and purified by column chromatography (DCM/MeOH = 20/1) to obtain 110 mg of white oil (two-step yield: 21.3%).
LC/MS: m/z = 345.2 $[M+H]^+$.

Step 4: Synthesis of 2,3-dihydroxy-N-(1,4-oxazepan-6-yl)propionamide hydrochloride

2-methylpropan-2-yl 6-{[(2,2-dimethyl-1,3-dioxolan-4-yl)carbonyl]amino}-1,4-oxazepane-4-carboxylate (210 mg, 0.21 mmol) was weighed and dissolved in DCM (3 mL), and then dioxane hydrochloride (1 mL) was added. The system was stirred at room temperature for 2 h, concentrated under reduced pressure, and used directly in the next step.
LC/MS: m/z = 245.1 $[M+H]^+$.

Step 5: Synthesis of N-[4-(7-bromo-2,6-dichloro-8-fluoroquinazolin-4-yl)-1,4-oxazepan-6-yl]-2,2-dimethyl-1,3-dioxolan-4 -carboxamide

7-bromo-2,6-dichloro-8-fluoroquinazolin-4-ol (200 mg, 0.65 mmol), 2,3-dihydroxy-N-(1,4-oxazepan-6-yl)propionamide hydrochloride (182 mg, 0.65 mmol), DIPEA (168 mg, 1.30 mmol) and dichloromethane (6 mL) were added to a reaction flask. After the reaction system was purged with nitrogen, bis(2-oxo-3-oxazolyl)phosphine chloride (249 mg, 0.98 mmol) was added. The mixture was stirred at room temperature for 2 h. The reaction solution was poured into water (30 mL), and then extracted with dichloromethane. The organic phase was dried, concentrated under reduced pressure, and purified by silica gel column chromatography (PE/EA = 30/1) to obtain 150 mg of yellow solid (yield: 43.4%).
LC/MS: m/z=537.0 $[M+H]^+$.

Step 6: Synthesis of N-{4-[7-bromo-6-chloro-8-fluoro-2-({[3-(3,4,5-trifluorophenyl)-2,3,5,6,7,7a-hexahydro-1H-pyrrolizin -7a-yl]methyl}oxy)quinazolin-4-yl]-1,4-oxazepan-6-yl}-2,2-dimethyl-1,3- dioxolane-4-carboxamide (Trans racemate)

N-[4-(7-bromo-2,6-dichloro-8-fluoroquinazolin-4-yl)-1,4-oxazepan-6-yl]-2,2-dimethyl-1,3-d ioxolane-4-carboxamide (150 mg, 0.28 mmol), [3-(3,4,5-trifluorophenyl)-2,3,5,6,7,7a-hexahydro-1H-pyrrolizin-7a-yl]methanol (Trans racemate) (76 mg, 0.28 mmol), DMF/THF (2 mL/2 mL), $CS_2CO_3$ (183 mg, 0.56 mmol) and 1,4-diazabicyclo[2.2.2]octane (DABCO) (31 mg, 0.28 mmol) were added to a reaction flask. The mixture was stirred at 60°C for 5 h. The reaction solution was poured into water (20 mL), and then extracted with ethyl acetate. The organic phase was dried, concentrated under reduced pressure, and purified by silica gel column chromatography (PE/EA=2/1) to obtain 74 mg of yellow solid (yield: 34.3%).
LC/MS: m/z = 772.1 $[M+H]^+$.

Step 7: Synthesis of 2-methylpropan-2-yl ({4-[6-chloro-4-(6-{[(2,2-dimethyl-1,3-dioxolan-4-yl)carbonyl]amino}-1,4-oxazepan-4-yl)-8-fluoro-2 -({[1-(3,4,5-trifluorophenyl)octahydrocyclopenta[1,2-a][5]annulen-3a-yl]methyl}oxy)quinazolin-7-yl] -3-cyano-7-fluorobenzo[b]thiophen-2-yl}amino)formate (Trans racemate)

N-{4-[7-bromo-6-chloro-8-fluoro-2-({[3-(3,4,5-trifluorophenyl)-2,3,5,6,7,7a-hexahydro-1H-pyrrolizin-7a-yl] methyl}oxy)quinazolin-4-yl]-1,4-oxazepan-6-yl}-2,2-dimethyl-1,3-dioxolane-4-carbo xamide (Trans racemate) (74 mg,

0.1 mmol), 2-methylpropan-2-yl {[3-cyano-4-(5,5-dimethyl-1,3,2-dioxaborinan-2-yl)-7-fluorobenzo[b]thiophen-2-yl]amino}formate (40 mg, 0.1 mmol), $CS_2CO_3$(49 mg, 0.15 mmol) and toluene (2 mL) were added to a reaction flask. After the reaction system was purged with nitrogen, Pd(DPEPhos)$Cl_2$ (7 mg, 0.01 mmol) was added. The reaction mixture was heated to 100°C and stirred for 6 h under nitrogen. After the reaction system was cooled to room temperature, the reaction solution was poured into water (10 mL), and then extracted with ethyl acetate. The organic phase was dried, concentrated under reduced pressure, and purified by silica gel column chromatography (DCM/MeOH = 20/1) to obtain 18 mg of yellow solid (yield: 19.1%).
LC/MS: m/z=984.3 $[M+H]^+$.

Step 8: Synthesis of N-{4-[7-(2-amino-3-cyano-7-fluorobenzo[b]thiophen-4-yl)-6-chloro-8-fluoro-2-({[1-(3,4,5-trifluoroph enyl)octahydrocyclopenta[1,2-a][5]annulen-3a-yl]methyl}oxy)quinazolin-4-yl]-1,4-oxazepan-6-yl}-2, 3-dihydroxypropionamide (Trans racemate)

2-methylpropan-2-yl ({4-[6-chloro-4-(6-{[(2,2-dimethyl-1,3-dioxolan-4-yl)carbonyl]amino}-1,4-oxazacycloheptane-4-yl)-8 -fluoro-2-({[3-(3,4,5-trifluorophenyl)-2,3,5,6,7,7a-hexahydro-1H-pyrrolizin-7a-yl]methyl}oxy)quinaz olin-7-yl]-3-cyano-7-fluorobenzo[b]thiophen-2-yl}amino)formate (Trans racemate) (18 mg) was added to a reaction flask and dissolved in dichloromethane (2 mL), trifluoroacetic acid (1 mL) was then added, and the materials were stirred at room temperature for 30 min. The resulting reaction mixture was concentrated under reduced pressure, added to a saturated $NaHCO_3$ aqueous solution (10 mL), and then extracted with ethyl acetate. The organic phase was dried over anhydrous sodium sulfate, filtered, concentrated under reduced pressure, and purified by column chromatography (DCM/MeOH=10/1) to obtain 7 mg of target product (yield: 45.5%).
LC/MS: m/z = 844.2 $[M+H]^+$.

Example 2

2-amino-4-{6-chloro-8-fluoro-4-[6-hydroxy-6-(hydroxymethyl)-1,4-oxazepan-4-yl]-2-({[3-( 3,4,5-trifluorophenyl)-2,3,5,6,7,7a-hexahydro-1H-pyrrolizin-7a-yl]methyl}oxy)quinazolin-7-yl}-7-flu orobenzo[b]thiophene-3-carbonitrile (Trans racemate)

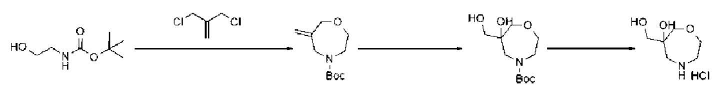

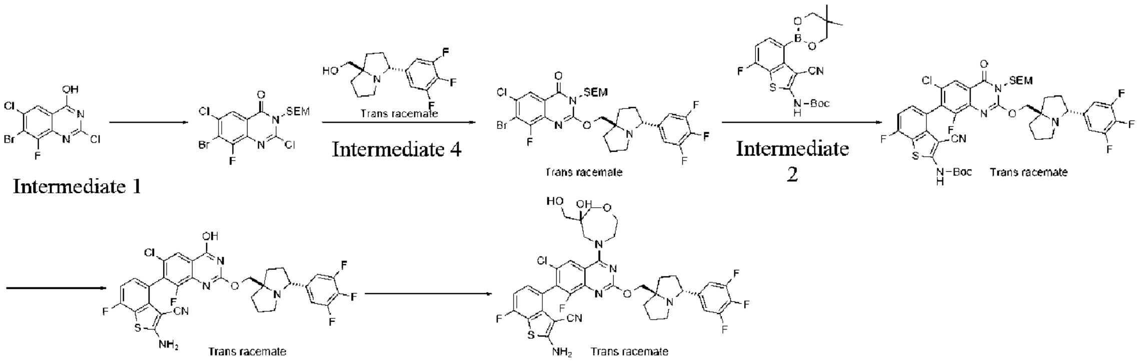


Step 1: Synthesis of 2-methylpropan-2-yl 6-methylene-1,4-oxazepane-4-carboxylate

NaH (141 mg, 3.5 mmol, 60%) was weighed and dissolved in DMF (3 mL). After the system was cooled to 0°C, 3-chloro-2-(chloromethyl)prop-1-ene (200 mg, 1.6 mmol) was slowly added. After 10 min, 2-methylpropan-2-yl [(2-hydroxyethyl)amino]formate (258 mg, 1.6 mmol) was added. After completion of the addition, the mixture was heated to room temperature and reacted under stirring for 2.5 h. The reaction solution was added to water (20 mL), and then extracted three times with ethyl acetate. The organic phase was dried, concentrated under reduced pressure, and purified by silica gel column chromatography (PE/EA = 10/1) to obtain 170 mg of yellow oil (yield: 64.2%).
LC/MS: m/z = 214.1 $[M+H]^+$.

Step 2: Synthesis of 2-methylpropan-2-yl 6-hydroxy-6-(hydroxymethyl)-1,4-oxazepane-4-carboxylate

2-methylpropan-2-yl 6-methylene-1,4-oxazepane-4-carboxylate (2.6 g, 12.2 mmol), N-methylmorpholine N-oxide (NMO) (1.4 g, 12.2 mmol) and $K_2OsO_4$ (747 mg, 2.44 mmol) were added to a reaction flask, followed by addition of THF/$H_2O$ (26 mL/3.3 mL). The system was reacted at room temperature for 2 h, then $H_2O$ was added, and the mixture was extracted three times with ethyl acetate. The organic phase was dried and concentrated under reduced pressure, and purified by silica gel column chromatography (PE/EA = 1/1) to obtain 780 mg of brown oil (yield: 26.0%).
LC/MS: m/z=248.1 $[M+H]^+$.

Step 3: Synthesis of (6-methyl-1,4-oxazepan-6-yl)methanol hydrochloride

2-methylpropan-2-yl 6-hydroxy-6-(hydroxymethyl)-1,4-oxazepane-4-carboxylate (200 mg, 0.81 mmol) was weighed and dissolved in a hydrochloric acid/methanol solution (4 M, 4 mL). The mixture was stirred at room temperature for 2 h and then concentrated under reduced pressure. The crude product was used directly for the next step.
LC/MS: m/z = 148.1 [M+H]+.

Step 4: Synthesis of 7-bromo-2,6-dichloro-3-(5,5-dimethyl-2-oxa-5-silahexan-1-yl)-8-fluoro-3,4-dihydroquinazolin-4-one

7-bromo-2,6-dichloro-8-fluoroquinazolin-4-ol (2.0 g, 6.4 mmol) was weighed and dissolved in DCM (30 mL), followed by the addition of DIEA (2.0 g, 16 mmol). After completion of the addition, SEM-Cl (1.38 g, 8.3 mmol) and TBAI (230 mg, 0.64 mmol) were added under stirring. The system was stirred at room temperature for 2 h, and then water (150 mL) was added. The mixture was extracted with DCM. The organic phase was dried, concentrated under reduced pressure, and purified by silica gel column chromatography (PE/EA = 3/1) to obtain 1.89 g of pale yellow solid (yield: 95.9%).
LC/MS: m/z=310.0 $[M+H]^+$.

Step 5: Synthesis of 7-bromo-6-chloro-3-(5,5-dimethyl-2-oxa-5-silahexan-1-yl)-8-fluoro-2-({[3-(3,4,5-trifluorophenyl)-2,3 ,5,6,7,7a-hexahydro-1H-pyrrolizin-7a-yl]methyl}oxy)-3,4-dihydroquinazolin-4-one (Trans racemate)

[3-(3,4,5-trifluorophenyl)-2,3,5,6,7,7a-hexahydro-1H-pyrrolizin-7a-yl]methanol (Trans racemate) (1.65 g, 6.0 mmol) was weighed and dissolved in THF. NaH (480 mg, 12.0 mmol) was slowly added at 0°C. The system was reacted at this temperature for 0.5 h. Then, a solution of 7-bromo-2,6-dichloro-3-(5,5-dimethyl-2-oxa-5-silahexan-1-yl)-8-fluoro-3,4-dihydroquinazolin-4-one (1.89 g, 6.0 mmol) in THF (15 mL) was added. The system was stirred at room temperature for 4 h. Water (150 mL) was added, and the mixture was extracted with ethyl acetate. The organic phase was dried, concentrated under reduced pressure, and purified by silica gel column chromatography (PE/EA = 4/1) to obtain 2.2 g of white solid compound (yield: 67.4%).
LC/MS: m/z = 545.2 $[M+H]^+$.

Step 6: Synthesis of 2-methylpropan-2-yl ({4-[6-chloro-3-(5,5-dimethyl-2-oxa-5-silahexan-1-yl)-8-fluoro-4-oxo-2-({[3-(3,4,5-trifluorophenyl)-2 ,3,5,6,7,7a-hexahydro-1H-pyrrolizin-7a-yl]methyl}oxy)quinazolin-7-yl]-3-cyano-7-fluorobenzo[b]thio phen-2-yl}amino)formate (Trans racemate)

7-bromo-6-chloro-3-(5,5-dimethyl-2-oxa-5-silahexan-1-yl)-8-fluoro-2-({[3-(3,4,5-trifluorop henyl)-2,3,5,6,7,7a-hexahydro-1H-pyrrolizin-7a-yl]methyl}oxy)-3,4-dihydroquinazolin-4-one (Trans racemate) (2.2 g, 4.0 mmol), 2-methylpropan-2-yl {[3-cyano-4-(5,5-dimethyl-1,3,2-dioxaborinan-2-yl)-7-fluorobenzo[b]thiophen-2-yl]amino}formate (1.61 g, 4.0 mmol), cesium carbonate (2.6 g, 8.0 mmol) and toluene (25 mL) were added to a reaction flask. After the reaction system was purged with nitrogen, Pd(DPEPhos)$Cl_2$ (286 mg, 0.1 mmol) was added. The reaction mixture was heated to 100°C and stirred for 6 h under nitrogen. After the reaction system was cooled to room temperature, the reaction mixture was poured into water (200 mL), and then extracted with ethyl acetate. The organic phase was dried, and purified by silica gel column chromatography (DCM/MeOH = 20/1) to obtain 1.5 g of yellow solid (yield: 49.6%).
LC/MS: m/z = 757.2 $[M+H]^+$.

Step 7: Synthesis of 2-amino-4-[6-chloro-8-fluoro-4-hydroxy-2-({[3-(3,4,5-trifluorophenyl)-2,3,5,6,7,7a-hexahydro-1H-py rrolizin-7a-yl]methyl}oxy)quinazolin-7-yl]-7-fluorobenzo[b]thiophene-3-carbonitrile (Trans racemate)

2-methylpropan-2-yl ({4-[6-chloro-3-(5,5-dimethyl-2-oxa-5-silahexan-1-yl)-8-fluoro-4-oxo-2-({[3-(3,4,5-trifluorophenyl)-2 ,3,5,6,7,7a-hexahydro-1H-pyrrolizin-7a-yl]methyl}oxy)quinazolin-7-yl]-3-cyano-7-fluorobenzo[b]thio phen-2-yl}amino)formate (Trans racemate) (1.0 g, 1.3 mmol) was weighed and dissolved in DCM (10 mL). Then, trifluoroacetic acid (3 mL) was added. The system was stirred at room temperature for 2 h, and concentrated under

reduced pressure. The obtained crude product was used directly for next-step reaction.
LC/MS: m/z = 658.2 $[M+H]^+$.

Step 8: Synthesis of 2-amino-4-{6-chloro-8-fluoro-4-[6-hydroxy-6-(hydroxymethyl)-1,4-oxazepan-4-yl]-2-({[3-(3,4,5-trifluorophenyl)-2,3,5,6,7,7a-hexahydro-1H-pyrrolizin-7a-yl]methyl}oxy)quinazolin-7-yl}-7-fluorobenzo[ b]thiophene-3-carbonitrile (Trans racemate)

2-amino-4-[6-chloro-8-fluoro-4-hydroxy-2-({[3-(3,4,5-trifluorophenyl)-2,3,5,6,7,7a-hexahy dro-1H-pyrrolizin-7a-yl]methyl}oxy)quinazolin-7-yl]-7-fluorobenzo[b]thiophene-3-carbonitrile (Trans racemate) (50 mg, crude), (6-methyl-1,4-oxazepan-6-yl)methanol hydrochloride (14 mg, crude), DIPEA (0.1 mL), and dichloromethane (2 mL) were added to a reaction flask. After the reaction system was purged with nitrogen, bis(2-oxo-3-oxazolyl)phosphine chloride (20 mg, 0.08 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography (PE/EA=10/1) to obtain 4 mg of target compound (yield: 7.7%).
LC/MS: m/z = 787.2 $[M+H]^+$.

Example 3

2-amino-4-[6-chloro-8-fluoro-4-(3-methyl-2,8-diaza-1-oxaspiro[4.6]undec-2-en-8-yl)-2-({[3 -(3,4,5-trifluorophenyl)-2,3,5,6,7,7a-hexahydro-1H-pyrrolizin-7a-yl]methyl}oxy)quinazolin-7-yl]-7-fl uorobenzo[b]thiophene-3-carbonitrile (Trans racemate)

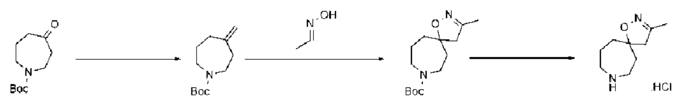

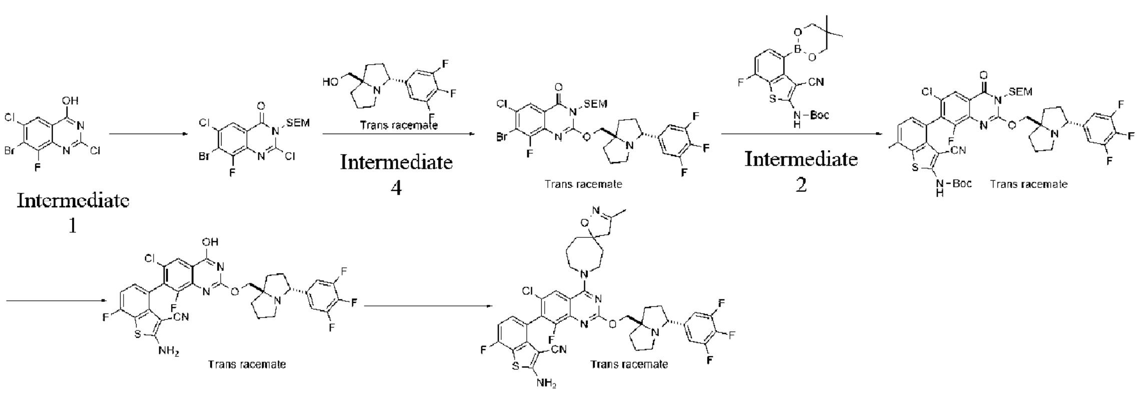


Step 1: Synthesis of 2-methylpropan-2-yl 4-methyleneazepane-1-carboxylate

Methyltriphenylphosphine bromide (1.67 g, 4.6 mmol) was weighed and dissolved in 15 mL of THF. After being purged three times with nitrogen, the reaction system was cooled to -30 to -35°C. n-butyllithium (2.7 mL, 6.9 mmol) was slowly added dropwise while maintaining the temperature at -30 to -35°C. After completion of the addition, the reaction was allowed to proceed for 1 h. 2-methylpropan-2-yl 4-oxoazepane-1-carboxylate (1.0 g, 4.6 mmol) was dissolved in 7 mL of THF and added dropwise to the above reaction system while maintaining the temperature at -10 to -20°C. After completion of the addition, the reaction was allowed to proceed from 0°C to -10°C for 1 h. The mixture was then allowed to warm to room temperature naturally and stirred for 16 h. The reaction system was then poured into a saturated $NH_4Cl$ aqueous solution, and a small amount of NaCl solids were added. Phase separation was conducted, and the upper-layer organic phase was collected and concentrated under reduced pressure. The residue was dissolved in EA and water was added. Phase separation was conducted. The EA phase was collected and purified by column chromatography (PE/EA=6/1) to obtain 560 mg of target product (yield: 56.0%).
LC/MS: m/z = 212.2 $[M+H]^+$.

Step 2: Synthesis of 2-methylpropan-2-yl 3-methyl-2,8-diaza-1-oxaspiro [4.6]undec-2-ene-8-carboxylate

Acetaldehyde oxime (185 mg, 3.1 mmol) was weighed and dissolved in DCM/DMF (5 mL/5 mL), and NCS (412

mg, 3.1 mmol) was added. The materials were reacted at room temperature for 1 h. 2-methylpropan-2-yl 4-methyleneazepane-1-carboxylate (450 mg, 2.1 mmol) and TEA (636 mg, 6.3 mmol) were added to the above reaction system, and the mixture was stirred at room temperature for 16 h. DCM and water were added to the reaction, phase separation was conducted, and the DCM phase was collected. The organic phase was washed with saturated brine, dried, concentrated under reduced pressure, and purified by column chromatography (PE/EA = 3/1) to obtain 300 mg of target product (yield: 53.3%).
LC/MS: m/z = 269.2 $[M+H]^+$.

Step 3: Synthesis of 3-methyl-2,8-diaza-1-oxaspiro[4.6]undec-2-ene hydrochloride.

2-methylpropan-2-yl 3-methyl-2,8-diaza-1-oxaspiro[4.6]undec-2-ene-8-carboxylate (300 mg,0.99 mmol) was weighed and dissolved in dioxane (2 mL), and then HCl/dioxane (4 M, 5 mL) was added. The reaction mixture was stirred at room temperature for 16 h. The reaction mixture was directly concentrated under reduced pressure to obtain 140 mg of target product (yield: 84.3%).
LC/MS: m/z = 169.2 $[M+H]^+$.

Step 4: Synthesis of 7-bromo-2,6-dichloro-3-(5,5-dimethyl-2-oxa-5-silahexan-1-yl)-8-fluoro-3,4-dihydroquinazolin-4-one

7-bromo-2,6-dichloro-8-fluoroquinazolin-4-ol (2.0 g, 6.4 mmol) was weighed and dissolved in DCM (30 mL), and then DIEA (2.0 g, 16 mmol) was added. After that, SEM-Cl (1.38 g, 8.3 mmol) and TBAI (230 mg, 0.64 mmol) were added under stirring. The system was stirred at room temperature for 2 h, water was added, and the mixture was extracted with DCM (150 mL). The organic phase was dried and concentrated under reduced pressure, and then purified by silica gel column chromatography (PE/EA = 3/1) to obtain 1.89 g of pale yellow solid (yield: 95.9%).
LC/MS: m/z = 310.2 $[M+H]^+$.

Step 5: Synthesis of 7-bromo-6-chloro-3-(5,5-dimethyl-2-oxa-5-silahexan-1-yl)-8-fluoro-2-({[3-(3,4,5-trifluorophenyl)-2,3 ,5,6,7,7a-hexahydro-1H-pyrrolizin-7a-yl]methyl}oxy)-3,4-dihydroquinazolin-4-one (Trans racemate)

[3-(3,4,5-trifluorophenyl)-2,3,5,6,7,7a-hexahydro-1H-pyrrolizin-7a-yl]methanol (1.65 g, 6.0 mmol) was weighed and dissolved in THF. NaH (480 mg, 12.0 mmol) was slowly added at 0°C. The system was reacted at this temperature for 0.5 h. Then, a solution of 7-bromo-2,6-dichloro-3-(5,5-dimethyl-2-oxa-5-silahexan-1-yl)-8-fluoro-3,4-dihydroquinazolin-4-one (1.89 g, 6.0 mmol) in THF (15 mL) was added. The system was stirred at room temperature for 4 h. Water (150 mL) was added, and the mixture was extracted with ethyl acetate. The organic phase was dried, concentrated under reduced pressure, and then purified by silica gel column chromatography (PE/EA = 4/1) to obtain 2.2 g of white solid compound (yield: 67.4%).
LC/MS: m/z = 545.2 $[M+H]^+$.

Step 6: Synthesis of 2-methylpropan-2-yl ({4-[6-chloro-3-(5,5-dimethyl-2-oxa-5-silahexan-1-yl)-8-fluoro-4-oxo-2-({[3-(3,4,5-trifluorophenyl)-2 ,3,5,6,7,7a-hexahydro-1H-pyrrolizin-7a-yl]methyl}oxy)quinazolin-7-yl]-3-cyano-7-fluorobenzo[b]thio phen-2-yl}amino)formate (Trans racemate)

7-bromo-6-chloro-3-(5,5-dimethyl-2-oxa-5-silahexan-1-yl)-8-fluoro-2-({[3-(3,4,5-trifluorop henyl)-2,3,5,6,7,7a-hexahydro-1H-pyrrolizin-7a-yl]methyl}oxy)-3,4-dihydroquinazolin-4-one (Trans racemate) (2.2 g, 4.0 mmol), 2-methylpropan-2-yl {[3-cyano-4-(5,5-dimethyl-1,3,2-dioxaborinan-2-yl)-7-fluorobenzo[b]thiophen-2-yl]amino}formate (1.61 g, 4.0 mmol), $Cs_2CO_3$ (2.6 g, 8.0 mmol), and toluene (25 mL) were added to a reaction flask. After the reaction system was purged with nitrogen, Pd(DPEPhos)$Cl_2$ (286 mg, 0.1 mmol) was added. The reaction mixture was heated to 100°C and stirred for 6 h under nitrogen. After the reaction system was cooled to room temperature, the reaction mixture was poured into water (200 mL), and then extracted with ethyl acetate. The organic phase was dried, concentrated under reduced pressure, and purified by silica gel column chromatography (DCM/MeOH = 20/1) to obtain 1.5 g of yellow solid (yield: 49.6%).
LC/MS: m/z = 757.2 $[M+H]^+$.

Step 7: Synthesis of 2-amino-4-[6-chloro-8-fluoro-4-hydroxy-2-({[3-(3,4,5-trifluorophenyl)-2,3,5,6,7,7a-hexahydro-1H-py rrolizin-7a-yl]methyl}oxy)quinazolin-7-yl]-7-fluorobenzo[b]thiophene-3-carbonitrile (Trans racemate)

2-methylpropan-2-yl ({4-[6-chloro-3-(5,5-dimethyl-2-oxa-5-silahexan-1-yl)-8-fluoro-4-oxo-2-({[3-(3,4,5-trifluorophenyl)-2 ,3,5,6,7,7a-hexahydro-1H-pyrrolizin-7a-yl]methyl}oxy)quinazolin-7-yl]-3-cyano-7-fluorobenzo[b]thio phen-2-yl}amino)formate (Trans racemate) (1.0 g, 1.3 mmol) was weighed and dissolved in DCM (10 mL). Then, trifluoroacetic acid (3 mL) was added. The system was stirred at room temperature for 2 h and concentrated under

reduced pressure. The crude product was used directly for next-step reaction.
LC/MS: m/z = 658.2 [M+H]+.

Step 8: Synthesis of 2-amino-4-[6-chloro-8-fluoro-4-(3-methyl-2,8-diaza-1-oxaspiro[4.6]undec-2-en-8-yl)-2-({[3-(3,4,5-tri fluorophenyl)-2,3,5,6,7,7a-hexahydro-1H-pyrrolizin-7a-yl]methyl}oxy)quinazolin-7-yl]-7-fluorobenz o[b]thiophene-3-carbonitrile (Trans racemate)

2-amino-4-[6-chloro-8-fluoro-4-hydroxy-2-({[3-(3,4,5-trifluorophenyl)-2,3,5,6,7,7a-hexahy dro-1H-pyrrolizin-7a-yl]methyl}oxy)quinazolin-7-yl]-7-fluorobenzo[b]thiophene-3-carbonitrile (Trans racemate) (10 mg, 0.015 mmol), 3-methyl-2,8-diaza-1-oxaspiro[4.6]undec-2-ene hydrochloride (6 mg, 0.03 mmol), DIPEA (6 mg, 0.045 mmol) and dichloromethane (1.5 mL) were added to a reaction flask. After the reaction system was purged with nitrogen, bis(2-oxo-3-oxazolyl)phosphine chloride (6 mg, 0.02 mmol) was added. The mixture was stirred at room temperature for 2 h. The reaction solution was poured into water (10 mL), and extracted with dichloromethane. The organic phase was dried, concentrated under reduced pressure, and purified by pre-TLC (DCM/MeOH=15/1) to obtain 1 mg of pale yellow solid (yield: 8.3%).
LC/MS: m/z = 808.2 [M+H]+.

Example 4

8-[7-(2-amino-3-cyano-7-fluorobenzo[b]thiophen-4-yl)-6-chloro-8-fluoro-2-({[3-(3,4,5-trifl uorophenyl)-2,3,5,6,7,7a-hexahydro-1H-pyrrolizin-7a-yl]methyl}oxy)quinazolin-4-yl]-N,N-dimethyl-2,8-diaza-1-oxaspiro[4.6]undec-2-ene-3-carboxamide (Trans racemate)

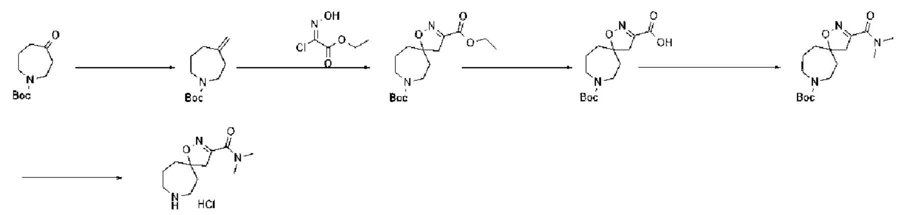

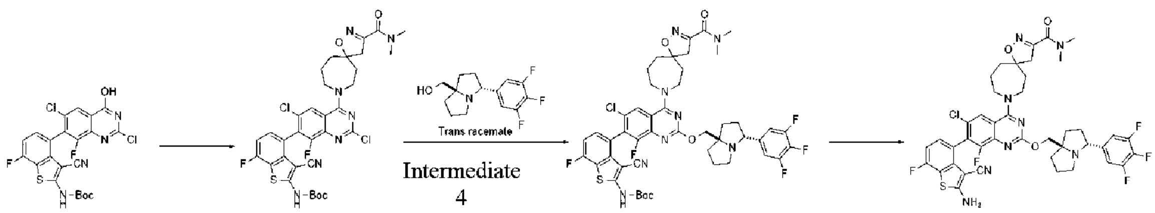


Step 1: Synthesis of 2-methylpropan-2-yl 4-methyleneazepane-1-carboxylate

Methyltriphenylphosphine bromide (1.670 g, 4.6 mmol) was weighed and dissolved in 15 mL of THF. After being purged three times with nitrogen, the reaction system was cooled to -30 to -35°C. n-butyllithium (2.7 mL, 6.9 mmol) was slowly added dropwise while maintaining the temperature at -30 to -35°C. After completion of the addition, the reaction was allowed to proceed for 1 h. 2-methylpropan-2-yl 4-oxoazepane-1-carboxylate (1.002 g, 4.6 mmol) was dissolved in 7 mL of THF and added dropwise to the above reaction system while maintaining the temperature at -10 to -20°C. After completion of the addition, the reaction was allowed to proceed from 0°C to -10°C for 1 h. The mixture was then allowed to warm to room temperature naturally and stirred for 16 h. The reaction mixture was poured into a saturated $NH_4Cl$ aqueous solution and a small amount of NaCl solid was added. Phase separation was conducted, the upper organic phase was collected and concentrated under reduced pressure. The residue was dissolved in ethyl acetate and water was added. Phase separation was conducted, and the organic phase was collected, and purified by column chromatography (PE/EA=6/1) to obtain 560 mg of target product (yield: 56%).
LC/MS: m/z = 212.2 [M+H]+.

Step 2: Synthesis of ethyl 8-{[(2-methylpropan-2-yl)oxy]carbonyl}-2,8-diaza-1-oxaspiro[4.6]undec-2-ene-3-carboxylate

2-methylpropan-2-yl 4-methyleneazepane-1-carboxylate (290 mg, 1.3 mmol) was weighed and dissolved in a

DCM/$H_2O$ (5 mL/5 mL) mixed solvent. Then, chlorooxime ethyl acetate (249 mg, 1.3 mmol) was added, and the reaction was stirred at room temperature for 16 h. DCM and water were added to the reaction mixture, phase separation was conducted and the DCM phase was collected. The organic phase was washed with saturated brine, dried, and concentrated under reduced pressure. The residue was purified by pre-TLC (PE/EA=3/1) to obtain 140 mg of target product (yield: 31%).
LC/MS: m/z = 327.2 $[M+H]^+$.

Step 3: Synthesis of 8-{[(2-methylpropan-2-yl)oxy]carbonyl}-2,8-diaza-1-oxaspiro[4.6]undec-2-ene-3-carboxylic acid

Ethyl 8-{[(2-methylpropan-2-yl)oxy]carbonyl}-2,8-diaza-1-oxaspiro[4.6]undec-2-ene-3-carboxylate (140 mg, 0.4 mmol) was weighed and dissolved in ethanol/$H_2O$ (3 mL/0.3 mL), and then a NaOH (34 mg, 0.8 mmol) solid was added. The mixture was stirred at room temperature for 16 h. The reaction solution was concentrated under reduced pressure, water was added, and the pH was adjusted to 3-4 with a hydrochloric acid aqueous solution (2 M). EA was added for extraction and phase separation. The EA phase was collected, dried over sodium sulfate, and concentrated under reduced pressure to obtain 90 mg of target product, yield: 70%.
LC/MS: m/z = 299.2 $[M+H]^+$.

Step 4: Synthesis of 2-methylpropan-2-yl 3-[(dimethylamino)carbonyl]-2,8-diaza-1-oxaspiro[4.6]undec-2-ene-8-carboxylate

8-{[(2-methylpropan-2-yl)oxy]carbonyl}-2,8-diaza-1-oxaspiro[4.6]undec-2-ene-3-carboxyli c acid (90 mg, 0.3 mmol) was weighed and dissolved in THF (2 mL). Then, DIEA (116 mg, 0.9 mmol) and HBTU (148 mg, 0.45 mmol) were added, and the mixture was stirred at room temperature for 10 min. Then, dimethylamine (2 M in THF) solution (0.22 mL, 0.45 mmol) was added, and the mixture was reacted at room temperature for 2 h. Water and EA were added to the reaction system to separate the phases. The EA phase was collected, dried over sodium sulfate, and concentrated under reduced pressure. The residue was purified by Pre-TLC (PE/EA = 2/1) to obtain 42 mg of target product (yield: 42%).
LC/MS: m/z = 326.2 $[M+H]^+$.

Step 5: Synthesis of N,N-dimethyl-2,8-diaza-1-oxaspiro[4.6]undec-2-ene-3-carboxamide hydrochloride.

2-methylpropan-2-yl 3-[(dimethylamino)carbonyl]-2,8-diaza-1-oxaspiro[4.6]undec-2-ene-8-carboxylate (40 mg, 0.12 mmol) was weighed and dissolved in dioxane (0.5 mL). Then, HCl/dioxane (4 M, 2 mL) was added. The reaction was stirred at room temperature for 16 h. The reaction system was directly concentrated under reduced pressure to obtain 25 mg of target product (yield: 78.1%).
LC/MS: m/z = 226.2 $[M+H]^+$.

Step 6: Synthesis of 2-methylpropan-2-yl {[3-cyano-4-(2,6-dichloro-4-{3-[(dimethylamino)carbonyl]-2,8-diaza-1-oxaspiro[4.6]undec-2-en-8-yl }-8-fluoroquinazolin-7-yl)-7-fluorobenzo[b]thiophen-2-yl]amino}formate

2-methylpropan-2-yl {[3-cyano-4-(2,6-dichloro-8-fluoro-4-hydroxyquinazolin-7-yl)-7-fluorobenzo[b]thiophen-2-yl]amino} formate (48 mg, 0.09 mmol) was weighed and dissolved in DCM (2 mL). DIEA (34 mg, 0.27 mmol) and N,N-dimethyl-2,8-diaza-1-oxaspiro[4.6]undec-2-ene-3-carboxamide hydrochloride (25 mg, 0.09 mmol) were added. Finally, bis(2-oxo-3-oxazolyl)phosphine chloride (45 mg, 0.18 mmol) was added. The reaction mixture was stirred at room temperature for 4 h. DCM and water were added to the reaction system. Phase separation was conducted and the organic phase was collected. The collected organic phase was dried over anhydrous sodium sulfate and concentrated under reduced pressure. The residue was purified by Pre-TLC (PE/EA=2/1) to obtain 35 mg of target product (yield: 54.6%).
LC/MS: m/z = 730.2 $[M+H]^+$.

Step 7: Synthesis of 2-methylpropan-2-yl {[4-(6-chloro-4-{3-[(dimethylamino)carbonyl]-2,8-diaza-1-oxaspiro[4.6]undec-2-en-8-yl}-8-fluoro-2-({[3-(3,4,5-trifluorophenyl)-2,3,5,6,7,7a-hexahydro-1H-pyrrolizin-7a-yl]methyl}oxy)quinazolin-7-yl)-3-cyano-7-fluorobenzo[b]thiophen-2-yl]amino}formate (Trans racemate)

2-methylpropan-2-yl {[3-cyano-4-(2,6-dichloro-4-{3-[(dimethylamino)carbonyl]-2,8-diaza-1-oxaspiro[4.6]undec-2-en-8-yl }-8-fluoroquinazolin-7-yl)-7-fluorobenzo[b]thiophen-2-yl]amino}formate (35 mg, 0.05 mmol) was weighed and dissolved in DMF/THF (2 mL/2 mL). $Cs_2CO_3$ (49 mg, 0.15 mmol), DABCO (28 mg, 0.025 mmol) and [3-(3,4,5-trifluorophenyl)-2,3,5,6,7,7a-hexahydro-1H-pyrrolizin-7a-yl]methanol (Trans racemate) (20 mg, 0.075 mmol) were added. The mixture was stirred at 60°C for 16 h. The reaction solution was added to water (20 mL), and then extracted three times with ethyl acetate. The organic phase was dried and concentrated under reduced pressure, and purified by pre-TLC (DCM/MeOH=20/1) to obtain 11 mg of pale yellow solid (yield: 23.4%).
LC/MS: m/z = 965.2 [M+H]+.

Step 8: Synthesis of 8-[7-(2-amino-3-cyano-7-fluorobenzo[b]thiophen-4-yl)-6-chloro-8-fluoro-2-({[3-(3,4,5-trifluoropheny 1)-2,3,5,6,7,7a-hexahydro-1H-pyrrolizin-7a-yl]methyl}oxy)quinazolin-4-yl]-N,N-dimethyl-2,8-diaza-1 -oxaspiro [4.6]undec-2-ene-3-carboxamide (Trans racemate)

2-methylpropan-2-yl {[4-(6-chloro-4-{3-[(dimethylamino)carbonyl]-2,8-diaza-1-oxaspiro[4.6]undec-2-en-8-yl}-8-fluoro-2-({[3-(3,4,5-trifluorophenyl)-2,3,5,6,7,7a-hexahydro-1H-pyrrolizin-7a-yl]methyl}oxy)quinazolin-7-yl)-3-cyano-7-fluorobenzo[b]thiophen-2-yl]amino}formate (Trans racemate) (11 mg,0.01 mmol) was weighed and dissolved in DCM/TFA (1.2 mL/0.4 mL). The reaction mixture was stirred at room temperature for 30 min, and concentrated under reduced pressure. The dried material was added to a saturated $NaHCO_3$ aqueous solution (10 mL) and then extracted with ethyl acetate. The organic phase was dried over anhydrous sodium sulfate, filtered, concentrated under reduced pressure, and purified by pre-TLC (DCM/MeOH=10/1) to obtain 1 mg of target product (yield: 11.8%).
LC/MS: m/z = 865.2 [M+H]+.

Example 5

2-methylpropan-2-yl [(4-{6-chloro-8-fluoro-4-[4-hydroxy-4-(trifluoromethyl)azacycloheptyl-1-yl]-2-({[3-(3,4,5-trifluoroph enyl)-2,3,5,6,7,7a-hexahydro-1H-pyrrolizin-7a-yl]methyl}oxy)quinazolin-7-yl}-3-cyano-7-fluorobenz o[b]thiophen-2-yl)amino]formate (Trans racemate)

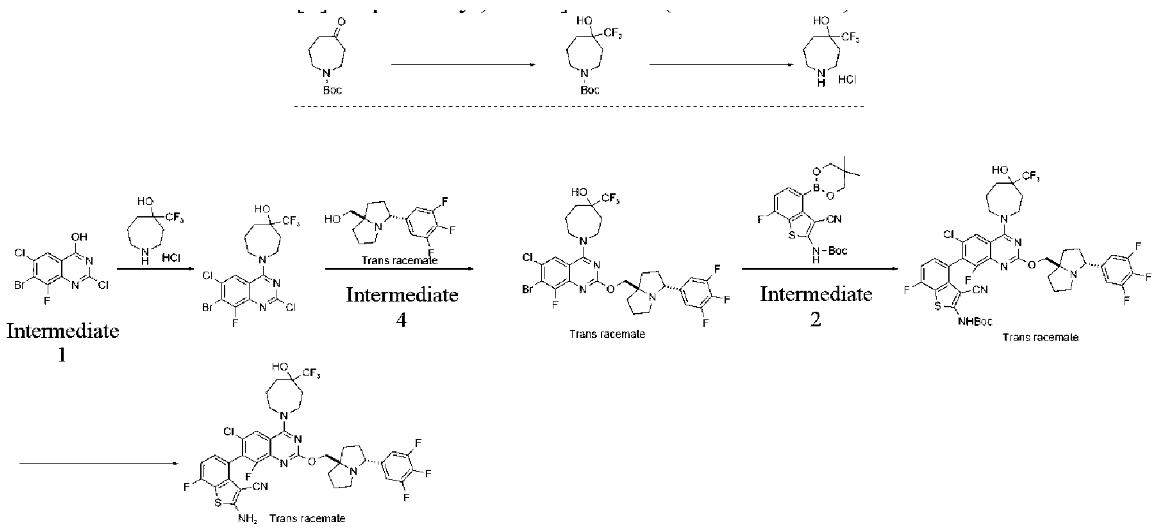


Step 1: Synthesis of 2-methylpropan-2-yl 4-hydroxy-4-(trifluoromethyl)azacycloheptane-1-carboxylate

2-methylpropan-2-yl 4-oxoazepane-1-carboxylate (1.0 g, 4.69 mmol) was weighed and dissolved in THF (10 mL). The reaction solution was cooled to 0°C, and then $TMSCF_3$ (1.0 g, 7.0 mmol) and TBAF (2 mL, 1 M in THF) were added. The mixture was stirred at room temperature for 3 h. The reaction solution was added to water (100 mL), and then extracted three times with ethyl acetate. The organic phase was dried and concentrated under reduced pressure to obtain 1.5 g (crude product). The crude product was used directly for the next step.
LC/MS: m/z = 284.1 [M+H]+.

Step 2: Synthesis of 6-(trifluoromethyl)-1,4-oxazepan-6-ol hydrochloride.

2-methylpropan-2-yl 4-hydroxy-4-(trifluoromethyl)azepane-1-carboxylate (1.5 g, crude product) was weighed and dissolved in hydrochloric acid/dioxane (4M, 20 mL). The reaction solution was stirred at room temperature for 2 h, and concentrated under reduced pressure. The crude product was used directly for next-step reaction.
LC/MS: m/z = 184.1 [M+H]+.

Step 3: Synthesis of 1-(7-bromo-2,6-dichloro-8-fluoroquinazolin-4-yl)-4-(trifluoromethyl)azepan-4-ol

7-bromo-2,6-dichloro-8-fluoroquinazolin-4-ol (200 mg, 0.65 mmol), 6-(trifluoromethyl)-1,4-oxazepan-6-ol hydrochloride (142 mg, 0.65 mmol), DIPEA (170 mg, 1.30 mmol) and dichloromethane (10 mL) were added to a reaction flask. After the reaction system was purged with nitrogen, bis(2-oxo-3-oxazolyl)phosphine chloride (249 mg, 0.98 mmol) was added, and the mixture was stirred at room temperature for 2 h. The reaction solution was poured into water (100 mL), and then extracted with dichloromethane. The organic phase was dried, concentrated under reduced pressure, and purified by silica gel column chromatography (PE/EA = 2/1) to obtain 142 mg of pale yellow solid (yield: 46.3%). LC/MS: m/z=476.0 $[M+H]^+$.

Step 4: Synthesis of 1-[7-bromo-8-fluoro-2-({[3-(3,4,5-trifluorophenyl)-2,3,5,6,7,7a-hexahydro-1H-pyrrolizin-7a-yl] methy 1}oxy)quinazolin-4-yl]-4-(trifluoromethyl)azepan-4-ol (Trans racemate)

1-(7-bromo-2,6-dichloro-8-fluoroquinazolin-4-yl)-4-(trifluoromethyl)azepan-4-ol (142 mg, 0.30 mmol), [3-(3,4,5-trifluorophenyl)-2,3,5,6,7,7a-hexahydro-1H-pyrrolizin-7a-yl]methanol (Trans racemate) (81 mg, 0.30 mmol) and DMF/THF (2 mL/2 mL) were added to a reaction flask, followed by the addition of $CS_2CO_3$(196 mg, 0.6 mmol) and DABCO (34 mg, 0.30 mmol). The mixture was stirred at 60°C for 5 h. The reaction solution was poured into water (20 mL), and then extracted with dichloromethane. The organic phase was dried, concentrated under reduced pressure, and purified by silica gel column chromatography (PE/EA = 20/1) to obtain 82 mg of yellow solid (yield: 38.7%). LC/MS: m/z=711.1 $[M+H]^+$.

Step 5: Synthesis of 2-methylpropan-2-yl [(4-{6-chloro-8-fluoro-4-[4-hydroxy-4-(trifluoromethyl)azacycloheptan-1-yl]-2-({[3-(3,4,5-trifluoroph enyl)-2,3,5,6,7,7a-hexahydro-1H-pyrrolizin-7a-yl]methyl}oxy)quinazolin-7-yl}-3-cyano-7-fluorobenz o[b]thiophen-2-yl)amino]formate (Trans racemate)

1-[7-bromo-8-fluoro-2-({[3-(3,4,5-trifluorophenyl)-2,3,5,6,7,7a-hexahydro-1H-pyrrolizin-7a -yl]methyl}oxy)quinazolin-4-yl]-4-(trifluoromethyl)azacycloheptan-4-ol (Trans racemate) (82 mg, 0.12 mmol), 2-methylpropan-2-yl {[3-cyano-4-(5,5-dimethyl-1,3,2-dioxaborinan-2-yl)-7-fluorobenzo[b]thiophen-2-yl]amino}formate (48 mg, 0.12 mmol), $CS_2CO_3$(59 mg, 0.18 mmol) and toluene (2 mL) were added to a reaction flask. After the reaction system was purged with nitrogen, Pd(DPEPhos)$Cl_2$(8.4 mg, 0.012 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, concentrated under reduced pressure, and purified by silica gel column chromatography (DCM/MeOH = 20/1) to obtain 23 mg of yellow solid (yield: 21.7%). LC/MS: m/z = 923.2 $[M+H]^+$.

Step 6: Synthesis of 2-amino-4-{6-chloro-8-fluoro-4-[4-hydroxy-4-(trifluoromethyl)azepan-1-yl]-2-({[1-(3,4,5-trifluoroph enyl)octahydrocyclopenta[1,2-a][5]annulen-3a-yl]methyl}oxy)quinazolin-7-yl}-7-fluorobenzo[b]thiop hene-3-carbonitrile (Trans racemate)

2-methylpropan-2-yl [(4-{6-chloro-8-fluoro-4-[4-hydroxy-4-(trifluoromethyl)azepan-1-yl]-2-({[3-(3,4,5-trifluorophenyl)-2, 3,5,6,7,7a-hexahydro-1H-pyrrolizin-7a-yl]methyl}oxy)quinazolin-7-yl}-3-cyano-7-fluorobenzo[b]thio phen-2-yl)amino]formate (Trans racemate) (23 mg, 0.025 mmol) was added to a reaction flask and dissolved in dichloromethane (3 mL), and trifluoroacetic acid (1 mL) was added. The reaction solution was stirred at room temperature for 30 min. The mixture was concentrated under reduced pressure, added to a saturated $NaHCO_3$ aqueous solution (10 mL), and then extracted with ethyl acetate. The organic phase was dried over anhydrous sodium sulfate, filtered, concentrated under reduced pressure, and purified by column chromatography (DCM/MeOH=10/1) to obtain 9 mg of target product (yield: 43.9%).

LC/MS: m/z = 823.2 $[M+H]^+$.
$^1H$ NMR (400 MHz, d6-DMSO) $\delta$ 1.27-1.49 (2H, m), 1.53-1.64 (3H, m), 1.72-2.34 (11H, m), 3.14-3.29 (1H, m), 3.58-3.88 (2H, m), 4.01-4.28 (4H, m), 6.04 (1H, brs), 7.12-7.31 (3H, m), 7.79 (1H, d, J =8.0Hz), 7.83 (1H, s), 7.96-8.01 (1H, m).

Example 6

2-amino-4-[6-chloro-8-fluoro-4-(4-hydroxy-4-methylazepan-1-yl)-2-({[3-(3,4,5-trifluorophe nyl)-2,3,5,6,7,7a-hexahydro-1H-pyrrolizin-7a-yl]methyl}oxy)quinazolin-7-yl]-7-fluorobenzo[b]thioph ene-3-carbonitrile (Trans racemate)

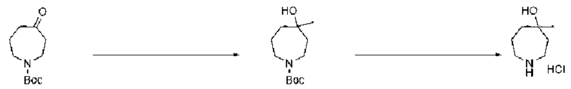

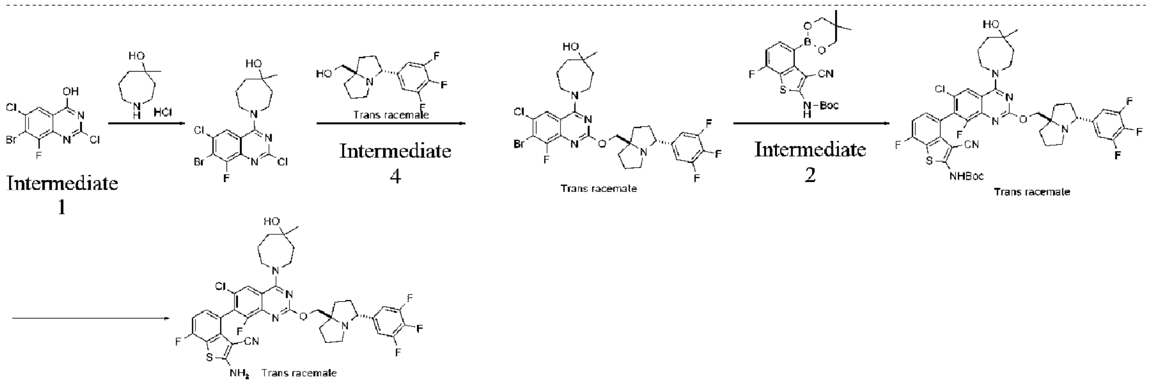


Step 1: Synthesis of 2-methylpropan-2-yl 4-hydroxy-4-methylazepane-1-carboxylate

2-methylpropan-2-yl 4-oxoazepane-1-carboxylate (1.0 g, 4.69 mmol) was weighed and dissolved in 10 mL of THF. The solution was cooled to 0°C, and then methylmagnesium bromide (3.1 mL, 3 M in THF) was added. The mixture was stirred at room temperature for 3 h. The reaction solution was added to 50 mL of saturated ammonium chloride aqueous solution, and the mixture was extracted three times with ethyl acetate. The organic phase was dried and concentrated under reduced pressure. The residue was purified by Prep-TLC (PE/EA = 4/1) to obtain 1.3 g of colorless oil (yield: 31.0%).
LC/MS: m/z = 230.2 $[M+H]^+$.

Step 2: Synthesis of 4-methylazepan-4-ol hydrochloride

2-methylpropan-2-yl 4-hydroxy-4-(methyl)methylazepane-1-carboxylate (1.3 g, 5.67 mmol) was weighed and dissolved in hydrochloric acid/dioxane (4 M, 10 mL). The reaction solution was stirred at room temperature for 2 h, and concentrated under reduced pressure. The crude product was used directly for next-step reaction.
LC/MS: m/z = 130.1 $[M+H]^+$.

Step 3: Synthesis of 1-(7-bromo-2,6-dichloro-8-fluoroquinazolin-4-yl)-4-methylazepan-4-ol

7-bromo-2,6-dichloro-8-fluoroquinazolin-4-ol (50 mg, 0.32 mmol), 4-methylazepan-4-ol hydrochloride (53 mg, 0.32 mmol), DIPEA (124 mg, 0.96 mmol), and dichloromethane (3 mL) were added to a reaction flask. After the reaction system was purged with nitrogen, bis(2-oxo-3-oxazolyl)phosphine chloride (122 mg, 0.48 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography (PE/EA = 2/1) to obtain 23 mg of pale yellow solid (yield: 33.8%).
LC/MS: m/z=422.0 $[M+H]^+$.

Step 4: Synthesis of 1-[7-bromo-6-chloro-8-fluoro-2-({[1-(3,4,5-trifluorophenyl)octahydrocyclopenta[1,2-a][5]annulen-3a-yl]methyl}oxy)quinazolin-4-yl]-4-methylazepan-4-ol (Trans racemate)

1-(7-bromo-2,6-dichloro-8-fluoroquinazolin-4-yl)-4-methylazepan-4-ol (23 mg, 0.055 mmol) and [3-(3,4,5-trifluorophenyl)-2,3,5,6,7,7a-hexahydro-1H-pyrrolizin-7a-yl]methanol (Trans racemate) (14 mg, 0.055 mmol) were added to a reaction flask, and DMF/THF (2 mL/2 mL) was added, followed by the addition of $CS_2CO_3$ (27 mg, 0.083 mmol) and DABCO (6 mg, 0.055 mmol). The mixture was stirred at 60°C for 5 h. The reaction solution was poured into water (10 mL), and then extracted with dichloromethane. The organic phase was dried, concentrated under reduced pressure, and purified by silica gel column chromatography (DCM/MeOH = 30/1) to obtain 15 mg of yellow solid (yield: 41.9%).
LC/MS: m/z = 657.1 $[M+H]^+$.

Step 5: Synthesis of 2-methylpropan-2-yl ({4-[6-chloro-8-fluoro-4-(4-hydroxy-4-methylazepan-1-yl)-2-({[3-(3,4,5-trifluorophenyl)-2,3,5,6,7,7a -hexahydro-1H-pyrrolizin-7a-yl]methyl}oxy)quinazolin-7-yl]-3-cyano-7-fluorobenzo[b]thiophen-2-yl }amino) formate (Trans racemate)

1-[7-bromo-6-chloro-8-fluoro-2-({[1-(3,4,5-trifluorophenyl)octahydrocyclopenta[1,2-a][5]a nnulen-3a-yl] methyl}oxy)quinazolin-4-yl]-4-methylazepan-4-ol (Trans racemate) (15 mg, 0.023 mmol), 2-methylpropan-2-yl {[3-cyano-4-(5,5-dimethyl-1,3,2-dioxaborinan-2-yl)-7-fluorobenzo[b]thiophen-2-yl]amino} formate (9 mg, 0.023 mmol), $CS_2CO_3$ (11 mg, 0.035 mmol), and toluene (2 mL) were added to a reaction flask. After the reaction system was purged with nitrogen, Pd(DPEPhos)$Cl_2$ (1.6 mg, 0.0023 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, and purified by silica gel column chromatography (DCM/MeOH = 20/1) to obtain 6 mg of yellow solid (yield: 30.3%).
LC/MS: m/z=869.3 $[M+H]^+$.

Step 6: Synthesis of 2-amino-4-[6-chloro-8-fluoro-4-(4-hydroxy-4-methylazepan-1-yl)-2-({[3-(3,4,5-trifluorophenyl)-2,3,5 ,6,7,7a-hexahydro-1H-pyrrolizin-7a-yl]methyl}oxy)quinazolin-7-yl]-7-fluorobenzo[b]thiophene-3-car bonitrile (Trans racemate)

2-methylpropan-2-yl ({4-[6-chloro-8-fluoro-4-(4-hydroxy-4-methylazepan-1-yl)-2-({[3-(3,4,5-trifluorophenyl)-2,3,5,6,7,7a -hexahydro-1H-pyrrolizin-7a-yl]methyl}oxy)quinazolin-7-yl]-3-cyano-7-fluorobenzo[b]thiophen-2-yl }amino)formate (Trans racemate) (6 mg, 0.007 mmol) was added to a reaction flask and dissolved in dichloromethane (2 mL), and then trifluoroacetic acid (1 mL) was added. The mixture was stirred at room temperature for 30 min. The solution was concentrated under reduced pressure and added to a saturated $NaHCO_3$ aqueous solution (10 mL). The mixture was extracted with ethyl acetate. The organic phase was dried over anhydrous sodium sulfate, filtered, concentrated under reduced pressure, and purified by column chromatography (DCM/MeOH = 10/1) to obtain 2 mg of target product (yield: 37.7%).

LC/MS: m/z=769.2 $[M+H]^+$.
$^1$H NMR (400 MHz, d6-DMSO) δ 1.56-1.71 (4H, m), 1.78-1.91 (4H, m), 1.92-2.02 (4H, m), 2.06-2.27 (5H, m), 3.54-3.65 (1H, m), 3.73-3.92 (2H, m), 4.10-4.26 (2H, m), 4.41-4.43 (1H, m), 5.31-5.33 (1H, m), 7.12-7.33 (3H, m), 7.48-7.65(2H, m), 7.97 (0.6H, s), 8.12 (1.4H, s).

Example 7

2-amino-4-{6-chloro-8-fluoro-4-[(1S)-6-oxo-7,11-diazatricyclo[7.3.1.02,7]trideca-2(3),4-die n-11-yl]-2-({[3-(3,4,5-trifluorophenyl)-2,3,5,6,7,7a-hexahydro-1H-pyrrolizin-7a-yl]methyl}oxy)quina zolin-7-yl}-7-fluorobenzo [b]thiophene-3-carbonitrile (Trans racemate)

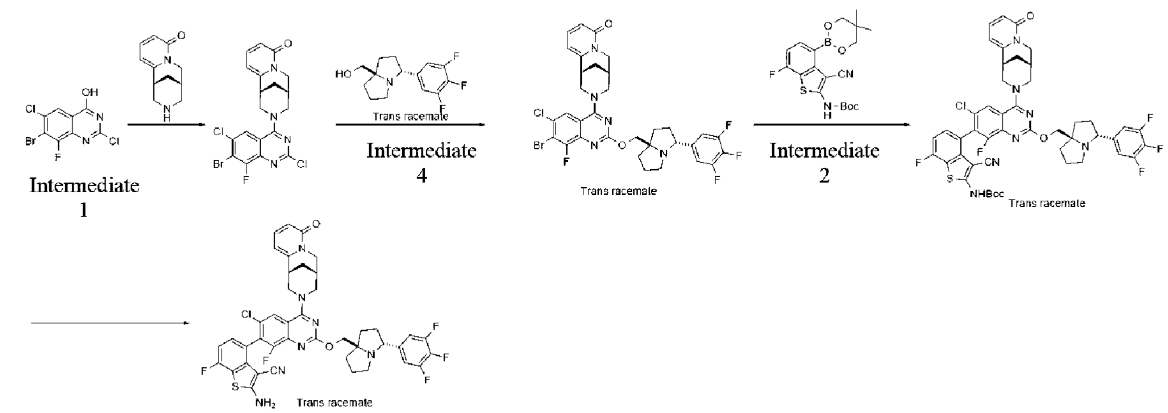


Step 1: Synthesis of (1S)-11-(7-bromo-2,6-dichloro-8-fluoroquinazolin-4-yl)-7,11-diazatricyclo[7.3.1.02,7]trideca-2(3),4-d ien-6-one

7-bromo-2,6-dichloro-8-fluoroquinazolin-4-ol (60 mg, 0.19 mmol), cytisine (36 mg, 0.19 mmol), DIPEA (49 mg, 0.38 mmol), and dichloromethane (3 mL) were added to a reaction flask. After the reaction system was purged with nitrogen, bis(2-oxo-3-oxazolyl)phosphine chloride (74 mg, 0.29 mmol) was added. The mixture was stirred at room

temperature for 2 h. The reaction solution was poured into water (10 mL), extracted with dichloromethane. The organic phase was dried, concentrated under reduced pressure, and purified by silica gel column chromatography (PE/EA = 1/1) to obtain 36 mg of pale yellow solid (yield: 38.7%).
LC/MS: m/z=483.0 $[M+H]^+$.

Step 2: Synthesis of (1S)-11-[7-bromo-6-chloro-8-fluoro-2-({[3-(3,4,5-trifluorophenyl)-2,3,5,6,7,7a-hexahydro-1H-pyrroli zin-7a-yl]methyl}oxy)quinazolin-4-yl]-7,11-diazatricyclo[7.3.1.02,7]trideca-2(3),4-dien-6-one (Trans racemate)

(1S)-11-(7-bromo-2,6-dichloro-8-fluoroquinazolin-4-yl)-7,11-diazatricyclo[7.3.1.02,7]tridec a-2(3),4-dien-6-one (36 mg, 0.075 mmol) and [3-(3,4,5-trifluorophenyl)-2,3,5,6,7,7a-hexahydro-1H-pyrrolizin-7a-yl]methanol (Trans racemate) (20 mg, 0.075 mmol) were added to a reaction flask, DMF/THF (2 mL/2 mL) was added, and then $CS_2CO_3$ (36 mg, 0.11 mmol) and DABCO (8.4 mg, 0.075 mmol) were added. The mixture was stirred at 60°C for 5 h. The reaction solution was poured into water (10 mL), extracted with dichloromethane. The organic phase was dried,concentrated under reduced pressure, and purified by silica gel column chromatography (DCM/MeOH=20/1) to obtain 22 mg of pale yellow solid (yield: 41.0%).
LC/MS: m/z=718.1 $[M+H]^+$.

Step 3: Synthesis of 2-methylpropan-2-yl [(4-{6-chloro-8-fluoro-4-[(1S,9R)-6-oxo-7,11-diazatricyclo[7.3.1.02,7]trideca-2(3),4-dien-11-yl]-2-({[ 3-(3,4,5-trifluorophenyl)-2,3,5,6,7,7a-hexahydro-1H-pyrrolizin-7a-yl]methyl}oxy)quinazolin-7-yl}-3-cyano-7-fluorobenzo[b]thiophen-2-yl)amino]formate (Trans racemate)

(1S)-11-[7-bromo-6-chloro-8-fluoro-2-({[3-(3,4,5-trifluorophenyl)-2,3,5,6,7,7a-hexahydro-1 H-pyrrolizin-7a-yl] methyl}oxy)quinazolin-4-yl]-7,11-diazatricyclo[7.3.1.02,7]trideca-2(3),4-dien-6-o ne (Trans racemate) (22 mg, 0.03 mmol), {[3-cyano-4-(5,5-dimethyl-1,3,2-dioxaborinan-2-yl)-7-fluorobenzo[b]thiophen-2-yl]amino}formate (12 mg, 0.03 mmol), $CS_2CO_3$(15 mg, 0.045 mmol) and toluene (2 mL)were added to a reaction flask. After the reaction system was purged with nitrogen, Pd(DPEPhos)$Cl_2$ (2.1 mg, 0.003 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, concentrated under reduced pressure, and purified by silica gel column chromatography (DCM/MeOH = 20/1) to obtain 14 mg of yellow solid (yield: 49.1%).
LC/MS: m/z = 930.2 $[M+H]^+$.

Step 4: Synthesis of 2-amino-4-{6-chloro-8-fluoro-4-[(1S)-6-oxo-7,11-diazatricyclo[7.3.1.02,7]trideca-2(3),4-dien-11-yl]-2-({[3-(3,4,5-trifluorophenyl)-2,3,5,6,7,7a-hexahydro-1H-pyrrolizin-7a-yl]methyl}oxy)quinazolin-7-y 1}-7-fluorobenzo [b]thiophene-3-carbonitrile (Trans racemate)

2-methylpropan-2-yl [(4-{6-chloro-8-fluoro-4-[(1S,9R)-6-oxo-7,11-diazatricyclo[7.3.1.02,7]trideca-2(3),4-dien-11-yl]-2-({[ 3-(3,4,5-trifluorophenyl)-2,3,5,6,7,7a-hexahydro-1H-pyrrolizin-7a-yl]methyl}oxy)quinazolin-7-yl}-3-cyano-7-fluorobenzo[b]thiophen-2-yl)amino]formate (Trans racemate) (14 mg, 0.015 mmol) was added to a reaction flask and dissolved in dichloromethane (2 mL), and trifluoroacetic acid (1 mL) was added. The mixture was stirred at room temperature for 30 min. The product was concentrated under reduced pressure, added to a saturated $NaHCO_3$ aqueous solution (10 mL), and then extracted with ethyl acetate. The organic phase was dried over anhydrous sodium sulfate, filtered, concentrated under reduced pressure, and purified by column chromatography (DCM/MeOH=10/1) to obtain 4 mg of target product (yield: 32.0%).

LC/MS: m/z = 830.2 $[M+H]^+$.
$^1$H NMR (400 MHz, d6-DMSO) δ 1.64-2.19 (6H, m), 2.31-2.40 (1H, m), 2.63-2.70 (2H, m), 3.43-3.55 (2H, m), 3.59-3.82 (2H, m), 3.93-4.60 (6H, m), 4.65-4.98 (1H, m), 6.23 (1H, d, J = 8.0Hz), 6.29-6.44 (1H, m), 7.14-7.42 (5H, m), 7.55-7.68(1H, m), 8.07-8.19 (2H, m).

Example 8

4-[4-(3,3a,4,5,6,6a-hexahydro-1H-furano[4,3-c]pyrrolo-5-yl)-6-chloro-8-fluoro-2-({[3-(3,4, 5-trifluorophenyl)-2,3,5,6,7,7a-hexahydro-1H-pyrrolizin-7a-yl]methyl}oxy)quinazolin-7-yl]-2-amino-7-fluorobenzo[b]thiophene-3-carbonitrile (Trans racemate)

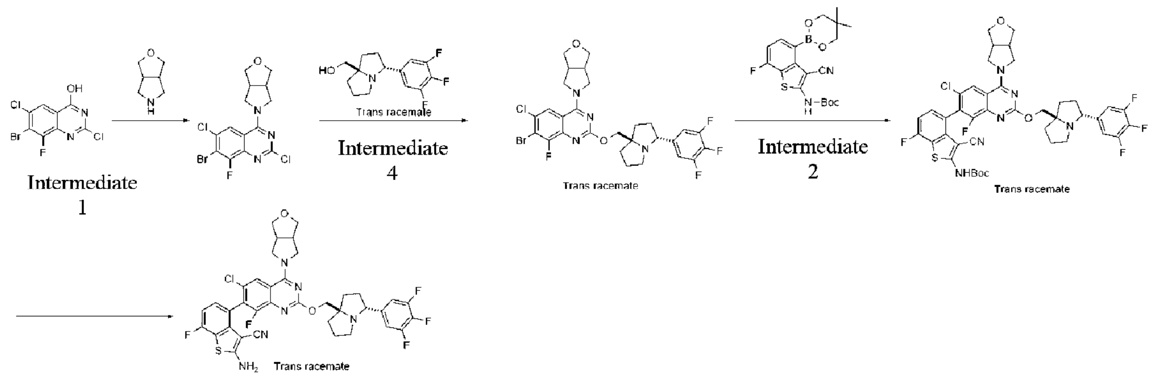


Step 1: Synthesis of 4-(3,3a,4,5,6,6a-hexahydro-1H-furano[4,3-c]pyrrolo-5-yl)-7-bromo-2,6-dichloro-8-fluoroquinazoline

7-bromo-2,6-dichloro-8-fluoroquinazolin-4-ol (100 mg, 0.32 mmol), hexahydro-1H-furano[3,4-C]pyrrole (36 mg, 0.32 mmol), DIPEA (83 mg, 0.64 mmol), and dichloromethane (4 mL) were added to a reaction flask. After the reaction system was purged with nitrogen, bis(2-oxo-3-oxazolyl)phosphine chloride (122 mg, 0.48 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography (PE/EA = 2/1) to obtain 97 mg of pale yellow solid (yield: 74.3%).
LC/MS: m/z=406.0 $[M+H]^+$.

Step 2: Synthesis of 4-(3,3a,4,5,6,6a-hexahydro-1H-furano[4,3-c]pyrrolo-5-yl)-7-bromo-6-chloro-8-fluoro-2-({[3-(3,4,5-tri fluorophenyl)-2,3,5,6,7,7a-hexahydro-1H-pyrrolizin-7a-yl]methyl}oxy)quinazoline (Trans racemate)

4-(3,3a,4,5,6,6a-hexahydro-1H-furano[4,3-c]pyrrolo-5-yl)-7-bromo-2,6-dichloro-8-fluoroqui nazoline (97 mg, 0.24 mmol) and [3-(3,4,5-trifluorophenyl)-2,3,5,6,7,7a-hexahydro-1H-pyrrolizin-7a-yl]methanol (Trans racemate) (65 mg, 0.24 mmol) were added to a reaction flask, and then DMF/THF (2 mL/2 mL) was added, followed by the addition of $CS_2CO_3$ (117 mg, 0.36 mmol) and DABCO (27 mg, 0.24 mmol). The mixture was stirred at 60°C for 5 h. The reaction solution was poured into water (10 mL), extracted with dichloromethane. The organic phase was dried, concentrated under reduced pressure, and purified by silica gel column chromatography (DCM/MeOH=30/1) to obtain 56 mg of yellow solid (yield: 36.6%).
LC/MS: m/z = 641.1 $[M+H]^+$.

Step 3: Synthesis of 2-methylpropan-2-yl ({4-[4-(3,3a,4,5,6,6a-hexahydro-1H-furano[4,3-c]pyrrolo-5-yl)-6-chloro-8-fluoro-2-({[3-(3,4,5-trifluo rophenyl)-2,3,5,6,7,7a-hexahydro-1H-pyrrolizin-7a-yl]methyl}oxy)quinazolin-7-yl]-3-cyano-7-fluoro benzo[b]thiophen-2-yl}amino)formate (Trans racemate)

4-(3,3a,4,5,6,6a-hexahydro-1H-furano[4,3-c]pyrrolo-5-yl)-7-bromo-6-chloro-8-fluoro-2-({[3 -(3,4,5-trifluorophenyl)-2,3,5,6,7,7a-hexahydro-1H-pyrrolizin-7a-yl]methyl}oxy)quinazoline (Trans racemate) (56 mg, 0.09 mmol), 2-methylpropan-2-yl {[3-cyano-4-(5,5-dimethyl-1,3,2-dioxaborinan-2-yl)-7-fluorobenzo[b]thiophen-2-yl]amino}formate (36 mg, 0.09 mmol), $CS_2CO_3$ (46 mg, 0.14 mmol), and toluene (2 mL) were added to a reaction flask. After the reaction system was purged with nitrogen, Pd(DPEPhos)$Cl_2$ (6.3 mg, 0.009 mmol) was added. The reaction mixture was heated to 100°C and stirred for 6 h under nitrogen. After the reaction system was cooled to room temperature, the reaction mixture was poured into water (20 mL), extracted with ethyl acetate. The organic phase was dried, concentrated under reduced pressure, and purified by silica gel column chromatography (DCM/MeOH = 20/1) to obtain 36 mg of yellow solid (yield: 48.3%).
LC/MS: m/z = 853.2 $[M+H]^+$.

Step 4: Synthesis of 4-[4-(3,3a,4,5,6,6a-hexahydro-1H-furano[4,3-c]pyrrolo-5-yl)-6-chloro-8-fluoro-2-({[3-(3,4,5-trifluoro phenyl)-2,3,5,6,7,7a-hexahydro-1H-pyrrolizin-7a-yl]methyl}oxy)quinazolin-7-yl]-2-amino-7-fluorobe nzo[b]thiophene-3-carbonitrile (Trans racemate)

2-methylpropan-2-yl ({4-[4-(3,3a,4,5,6,6a-hexahydro-1H-furano[4,3-c]pyrrolo-5-yl)-6-chloro-8-fluoro-2-({[3-(3,4,5-trifluo rophenyl)-2,3,5,6,7,7a-hexahydro-1H-pyrrolizin-7a-yl]methyl}oxy)quinazolin-7-yl]-3-cyano-7-fluoro benzo[b]thiophen-2-yl}amino)formate (Trans racemate) (36 mg, 0.04 mmol) was added to a reaction flask and

dissolved in dichloromethane (3 mL), and trifluoroacetic acid (1 mL) was added. The mixture was stirred at room temperature for 30 min. The product was concentrated under reduced pressure, added to a saturated $NaHCO_3$ aqueous solution (10 mL), and then extracted with ethyl acetate. The organic phase was dried over anhydrous sodium sulfate, filtered, concentrated under reduced pressure, and purified by column chromatography (DCM/MeOH=10/1) to obtain 13 mg of target product (yield: 40.9%).

LC/MS: m/z = 753.2 $[M+H]^+$.

$^1$H NMR (400 MHz, d6-DMSO) δ 1.64-1.69 (2H, m), 1.77-1.96 (3H, m), 2.04-2.24 (3H, m), 2.30-2.40 (2H, m), 2.98-3.17 (3H, m), 3.51-3.97 (6H, m), 4.06-4.29 (4H, m), 7.13-7.37 (3H, m), 8.11 (1H, s), 8.16 (1H, s), 7.70-7.77 (1H, m), 7.95 (1H, s), 8.17 (2H, s).

Example 9

2-amino-4-[6-chloro-8-fluoro-4-(6-hydroxy-6-methyl-1,4-oxazepan-4-yl)-2-({[3-(3,4,5-triflu orophenyl)-2,3,5,6,7,7a-hexahydro-1H-pyrrolizin-7a-yl]methyl}oxy)quinazolin-7-yl]-7-fluorobenzo[b ]thiophene-3-carbonitrile (Trans isomer 2)

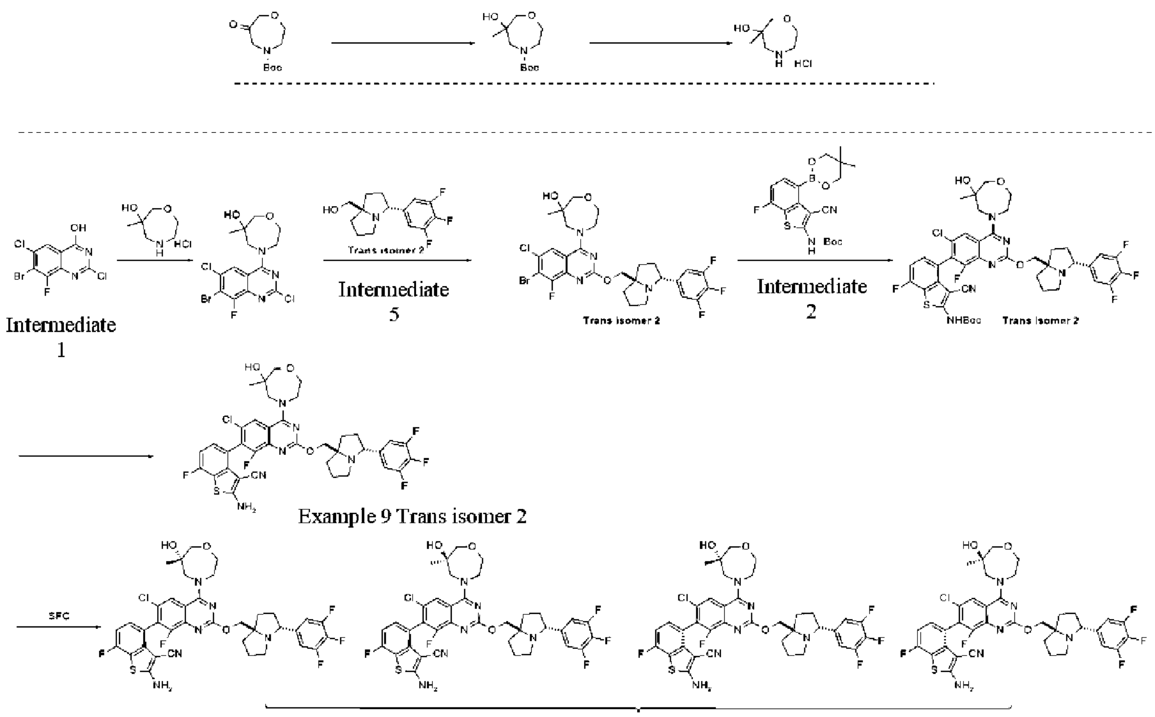


| | | | |
|---|---|---|---|
| Example 10: | Trans isomer 2, Axial chiral isomer 1 | Example 11: | Trans isomer 2, Axial chiral isomer 2 |
| Example 12: | Trans isomer 2, Axial chiral isomer 3 | Example 14: | Trans isomer 2, Axial chiral isomer 4 |

Step 1: Synthesis of 2-methylpropan-2-yl 6-hydroxy-6-methyl-1,4-oxazepane -4-carboxylate

2-methylpropan-2-yl 6-oxo-1,4-oxazepan-4-carboxylate (1.0 g, 4.65 mmol) was weighed and dissolved in THF (10 mL). The solution was cooled to 0°C, and then methyl magnesium bromide (3.1 mL, 3 M) was added. The mixture was stirred at room temperature for 3 h. The reaction solution was added to saturated ammonium chloride solution (100 mL), and then extracted three times with ethyl acetate. The organic phase was dried, concentrated under reduced pressure, and purified by Prep-TLC (PE/EA = 3/1) to obtain 0.7 g of yellow oil (yield: 65.4%).
LC/MS: m/z = 232.2 $[M+H]^+$.

Step 2: Synthesis of 6-methyl-1,4-oxazepan-6-ol hydrochloride

2-methylpropan-2-yl 6-hydroxy-6-methyl-1,4-oxazepane-4-carboxylate (0.7 g, 3.03 mmol) was weighed and dissolved in hydrochloric acid/dioxane (4 M, 8 mL). The system was stirred at room temperature for 2 h. The solution was

concentrated under reduced pressure. The obtained crude product was used directly for next-step reaction.
LC/MS: m/z = 132.1 [M+H]+.

Step 3: Synthesis of 4-(7-bromo-2,6-dichloro-8-fluoroquinazolin-4-yl)-6-methyl-1,4-oxazepan-6-ol

7-bromo-2,6-dichloro-8-fluoroquinazolin-4-ol (100 mg, 0.32 mmol), 6-methyl-1,4-oxazepan-6-ol hydrochloride (53 mg, 0.32 mmol), DIPEA (124 mg, 0.96 mmol), and dichloromethane (5 mL) were added to a reaction flask. After the reaction system was purged with nitrogen, bis(2-oxo-3-oxazolyl)phosphine chloride (122 mg, 0.48 mmol) was added. The mixture was stirred at room temperature for 2 h. The reaction solution was poured into water (20 mL), and then extracted with dichloromethane. The organic phase was dried, concentrated under reduced pressure, and purified by silica gel column chromatography (PE/EA = 30/1) to obtain 46 mg of pale yellow solid (yield: 33.8%).
LC/MS: m/z=424.0 [M+H]+.

Step 4: Synthesis of 4-[7-bromo-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-6-methyl-1,4-oxazepan-6-ol (Trans isomer 2)

4-(7-bromo-2,6-dichloro-8-fluoroquinazolin-4-yl)-6-methyl-1,4-oxazepan-6-ol (46 mg, 0.11 mmol) and [3-(3,4,5-trifluorophenyl)-2,3,5,6,7,7a-hexahydro-1H-pyrrolizin-7a-yl]methanol (Trans isomer 2) (30 mg, 0.11 mmol) were added to a reaction flask, and then DMF/THF (2 mL/2 mL) was added, followed by the addition of $CS_2CO_3$(55 mg, 0.17 mmol) and DABCO (12 mg, 0.11 mmol). The mixture was stirred at 60°C for 5 h. The reaction solution was poured into water (10 mL), and then extracted with dichloromethane. The organic phase was dried, concentrated under reduced pressure, and purified by silica gel column chromatography (DCM/MeOH = 30/1) to obtain 22 mg of pale yellow solid (yield: 30.7%).
LC/MS: m/z=659.1 [M+H]+.

Step 5: Synthesis of 2-methylpropan-2-yl ({4-[6-chloro-8-fluoro-4-(6-hydroxy-6-methyl-1,4-oxazepan-4-yl)-2-({[3-(3,4,5-trifluorophenyl)-2,3,5 ,6,7,7a-hexahydro-1H-pyrrolizin-7a-yl]methyl}oxy)quinazolin-7-yl]-3-cyano-7-fluorobenzo[b]thiophe n-2-yl}amino)formate (Trans isomer 2)

4-[7-bromo-6-chloro-8-fluoro-2-({[3-(3,4,5-trifluorophenyl)-2,3,5,6,7,7a-hexahydro-1H-pyr rolizin-7a-yl]methyl}oxy)quinazolin-4-yl]-6-methyl-1,4-oxazepan-6-ol (Trans isomer 2) (22 mg, 0.033 mmol), 2-methylpropan-2-yl {[3-cyano-4-(5,5-dimethyl-1,3,2-dioxaborinan-2-yl)-7-fluorobenzo[b]thiophen-2-yl]amino}formate (13 mg, 0.033 mmol), $CS_2CO_3$ (15 mg, 0.045 mmol), and toluene (2 mL) were added to a reaction flask. After the reaction system was purged with nitrogen, Pd(DPEPhos)$Cl_2$ (2.3 mg, 0.0033 mmol) was added. The reaction mixture was heated to 100°C and stirred for 6 h under nitrogen. After the reaction system was cooled to room temperature, the reaction mixture was poured into water (20 mL), and then extracted with ethyl acetate. The organic phase was dried, concentrated under reduced pressure, and then purified by silica gel column chromatography (DCM/MeOH=20/1) to obtain 12 mg of yellow solid (yield: 41.4%).
LC/MS: m/z = 871.2 [M+H]+.

Step 6: Synthesis of 2-amino-4-[6-chloro-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]-7-fluorobenzo[b]thiophen e-3-carbonitrile (Trans isomer 2)

2-methylpropan-2-yl ({4-[6-chloro-8-fluoro-4-(6-hydroxy-6-methyl-1,4-oxazepan-4-yl)-2-({[3-(3,4,5-trifluorophenyl)-2,3,5 ,6,7,7a-hexahydro-1H-pyrrolizin-7a-yl]methyl}oxy)quinazolin-7-yl]-3-cyano-7-fluorobenzo[b]thiophe n-2-yl}amino)formate (Trans isomer 2) (12 mg, 0.014 mmol) was added to a reaction flask and dissolved in dichloromethane (2 mL), and trifluoroacetic acid (1 mL) was added. The mixture was stirred at room temperature for 30 min. The product was concentrated under reduced pressure, added to a saturated $NaHCO_3$ aqueous solution (10 mL), and then extracted with ethyl acetate. The organic phase was dried over anhydrous sodium sulfate, filtered, concentrated under reduced pressure, and purified by column chromatography (DCM/MeOH=10/1) to obtain 7 mg of target product (yield: 66.0%).

LC/MS: m/z = 771.2 [M+H]+.
$^1$H NMR (400 MHz, d6-DMSO) δ 1.52-1.70 (3H, m), 1.87-1.94 (2H, m), 1.97-2.02 (2H, m), 2.07-2.19 (3H, m), 2.30-2.38 (1H, m), 3.69-3.72 (2H, m), 3.77-3.83 (1H, m), 3.90-4.03 (3H, m), 4.07-4.31 (5H, m), 5.28-5.41 (2H, m), 7.13-7.38 (4H, m), 8.11 (2H, brs), 8.55 (1H, d, J = 24.0 Hz).

Step 7: Resolution of 2-amino-4-[6-chloro-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]-7-fluorobenzo[b]thiophen e-3-carbonitrile (Trans isomer 2)

Resolution method: Instrument: Waters SFC-80; Column: Chiralcel AD-3 (4.6*100 mm); Mobile phase: A (supercritical $CO_2$), B: (isopropanol, isopropanol solution containing 0.2% 7M ammonia); Gradient: B%=50%, Flow rate: 1.5 mL/min; Wavelength: 215 nm; Pressure: 2000 psi. Retention time of the first eluate Rt=1.446 min, retention time of the second eluate Rt=1.446 min, retention time of the third eluate Rt=2.247 min, retention time of the fourth eluate Rt=2.659 min.

The first eluate (retention time Rt = 1.446 min) was defined as the compound in Example 10:
LC/MS: m/z = 771.2$[M+H]^+$.

The second eluate (retention time Rt = 1.446 min) was defined as the compound in Example 11:
LC/MS: m/z = 771.2 $[M+H]^+$.

The third eluate (retention time Rt = 2.247 min) was defined as the compound in Example 12:

LC/MS: m/z = 771.2 $[M+H]^+$.
$^1$H NMR (400 MHz, d6- DMSO) δ 1.13 (s, 3H), 8.50 (s, 1H), 1.53 - 1.64 (m, 3H), 1.84 - 1.86 (m, 2H), 1.95-1.99 (m, 1H), 2.06-2.11 (m, 2H), 2.18-2.24 (m, 1H), 2.35-2.50 (m, 1H), 3.55 (s, 2H), 3.63-3.68 (m, 1H), 3.88-3.98 (m, 3H), 4.08-4.24 (m, 5H), 5.27 (s, 1H), 7.16 (dd, J = 4.0Hz, 8.0Hz, 1H), 7.25-7.32 (m, 3H), 8.10 (s, 2H), 8.51 (s, 1H).

The fourth eluate (retention time Rt = 2.659 min) was defined as the compound in Example 13:
LC/MS: m/z = 771.2 $[M+H]^+$.

Example 14

8-[7-(2-amino-7-fluorobenzo[b]thiophen-4-yl)-6-chloro-8-fluoro-2-({[3-(3,4,5-trifluorophen yl)-2,3,5,6,7,7a-hexahydro-1H-pyrrolizin-7a-yl]methyl}oxy)quinazolin-4-yl]-8-aza-1-oxaspiro[4.5]de c-3-ol (Trans racemate)

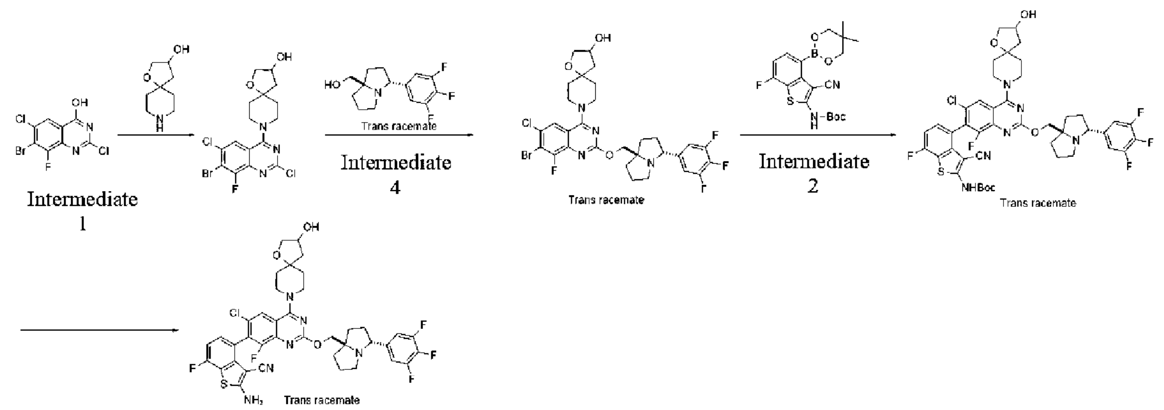


Step 1: Synthesis of 8-(7-bromo-2,6-dichloro-8-fluoroquinazolin-4-yl)-8-aza-1-oxaspiro[4.5]dec-3-ol

7-bromo-2,6-dichloro-8-fluoroquinazolin-4-ol (100 mg, 0.32 mmol), 8-aza-1-oxaspiro[4.5]dec-3-ol (50 mg, 0.32 mmol), DIPEA (83 mg, 0.64 mmol), and dichloromethane (5 mL) were added to a reaction flask. After the reaction system was purged with nitrogen, bis(2-oxo-3-oxazolyl)phosphine chloride (122 mg, 0.48 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography (PE/EA = 2/1) to obtain 70 mg of white solid (yield: 53.6%).
LC/MS: m/z = 406.1 $[M+H]^+$.

Step 2: Synthesis of 8-[7-bromo-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-8-aza-1-oxaspiro[4.5]dec-3-ol (Trans racemate)

4-(3,3a,4,5,6,6a-hexahydro-1H-furano[4,3-c]pyrrolo-5-yl)-7-bromo-2,6-dichloro-8-fluoroqui nazoline (70 mg, 0.17 mmol) and [3-(3,4,5-trifluorophenyl)-2,3,5,6,7,7a-hexahydro-1H-pyrrolizin-7a-yl]methanol (Trans racemate) (46 mg, 0.17 mmol) were added to a reaction flask, and then DMF/THF (2 mL/2 mL) was added, followed by the addition

of $CS_2CO_3$ (85 mg, 0.26 mmol) and DABCO (19 mg, 0.17 mmol). The mixture was stirred at 60°C for 5 h. The reaction solution was poured into water (10 mL), and then extracted with dichloromethane. The organic phase was dried, concentrated under reduced pressure, and purified by silica gel column chromatography (DCM/MeOH=30/1) to obtain 33 mg of yellow solid (yield: 28.0%).
LC/MS: m/z = 685.1 $[M+H]^+$.

Step 3: Synthesis of 2-methylpropan-2-yl ({4-[6-chloro-8-fluoro-4-(3-hydroxy-8-aza-1-oxaspiro[4.5]dec-8-yl)-2-({[3-(3,4,5-trifluorophenyl)-2,3 ,5,6,7,7a-hexahydro-1H-pyrrolizin-7a-yl]methyl}oxy)quinazolin-7-yl]-7-fluorobenzo[b]thiophen-2-yl }amino)formate (Trans racemate)

8-[7-bromo-6-chloro-8-fluoro-2-({[3-(3,4,5-trifluorophenyl)-2,3,5,6,7,7a-hexahydro-1H-pyr rin-7a-yl]methyl}oxy)quinazolin-4-yl]-8-aza-1-oxaspiro[4,5]dec-3-ol (Trans racemate) (33 mg, 0.048 mmol), 2-methylpropan-2-yl {[3-cyano-4-(5,5-dimethyl-1,3,2-dioxaborinan-2-yl)-7-fluorobenzo[b]thiophen-2-yl]amino}formate (19 mg, 0.048 mmol), $CS_2CO_3$(23 mg, 0.072 mmol), and toluene (2 mL) were added to a reaction flask. After the reaction system was purged with nitrogen, Pd(DPEPhos)$Cl_2$ (3.4 mg, 0.0048 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, concentrated under reduced pressure, and purified by silica gel column chromatography (DCM/MeOH=20/1) to obtain 16 mg of yellow solid (yield: 37.2%).
LC/MS: m/z = 897.2 $[M+H]^+$.

Step 4: Synthesis of 8-[7-(2-amino-7-fluorobenzo[b]thiophen-4-yl)-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-8-aza-1-oxaspiro[4,5]dec-3-ol (Trans racemate)

2-methylpropan-2-yl ({4-[6-chloro-8-fluoro-4-(3-hydroxy-8-aza-1-oxaspiro[4.5]dec-8-yl)-2-({[3-(3,4,5-trifluorophenyl)-2,3 ,5,6,7,7a-hexahydro-1H-pyrrolizin-7a-yl]methyl}oxy)quinazolin-7-yl]-7-fluorobenzo[b]thiophen-2-yl }amino) formate (Trans racemate) (16 mg, 0.018 mmol) was added to a reaction flask and dissolved in dichloromethane (3 mL), and then trifluoroacetic acid (1 mL) was added. The mixture was stirred at room temperature for 30 min. The solution was concentrated under reduced pressure and added to a saturated $NaHCO_3$ aqueous solution (10 mL). The mixture was extracted with ethyl acetate. The organic phase was dried over anhydrous sodium sulfate, filtered, concentrated under reduced pressure, and purified by column chromatography (DCM/MeOH = 10/1) to obtain 3 mg of target product (yield: 26.1%).
LC/MS: m/z = 797.2 $[M+H]^+$.

Example 15

2-amino-4-[6-amino-8-fluoro-4-(3-methoxytetrahydro-1H-pyrrol-1-yl)-2-({[3-(3,4,5-trifluor ophenyl)-2,3,5,6,7,7a-hexahydro-1H-pyrrolizin-7a-yl]methyl}oxy)quinazolin-7-yl]-7-fluorobenzo[b]t hiophene-3-carbonitrile (Trans racemate)

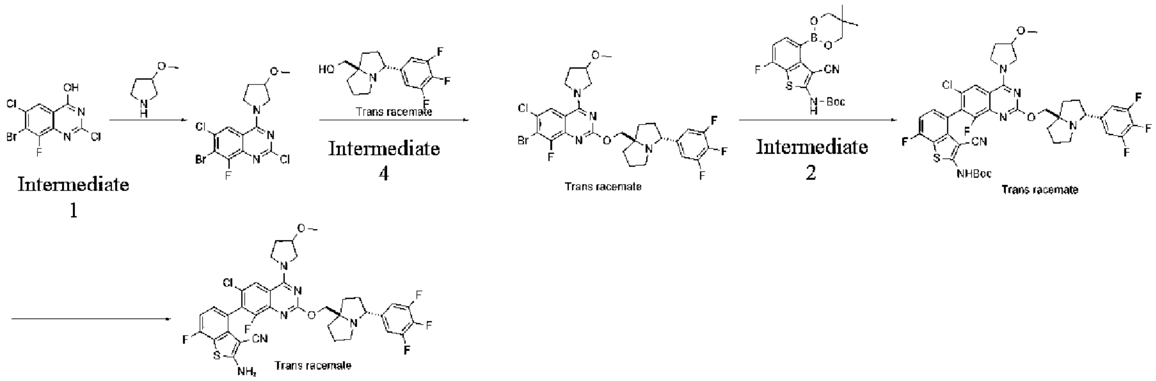


Step 1: Synthesis of 7-bromo-2,6-dichloro-8-fluoro-4-(3-methoxytetrahydro-1H-pyrrol-1-yl)quinazoline

7-bromo-2,6-dichloro-8-fluoroquinazolin-4-ol (100 mg, 0.32 mmol), 3-methoxytetrahydropyrrole (32 mg, 0.32 mmol), DIPEA (83 mg, 0.64 mmol), and dichloromethane (3 mL) were added to a reaction flask. After the reaction system was purged with nitrogen, bis(2-oxo-3-oxazolyl)phosphine chloride (122 mg, 0.48 mmol) was added. The mixture was

stirred at room temperature for 2 h. The reaction solution was poured into water (10 mL), and then extracted with dichloromethane. The organic phase was dried, concentrated under reduced pressure, and purified by silica gel column chromatography (PE/EA = 2/1) to obtain 88 mg of pale yellow solid (yield: 69.3%).
LC/MS: m/z=394.0 $[M+H]^+$.

Step 2: Synthesis of 7-bromo-6-chloro-8-fluoro-4-(3-methoxytetrahydro-1H-pyrrol-1-yl)-2-({[3-(3,4,5-trifluorophenyl)-2,3 ,5,6,7,7a-hexahydro-1H-pyrrolizin-7a-yl]methyl}oxy)quinazoline (Trans racemate)

7-bromo-2,6-dichloro-8-fluoro-4-(3-methoxytetrahydro-1H-pyrrol-1-yl)quinazoline (88 mg, 0.22 mmol), and [3-(3,4,5-trifluorophenyl)-2,3,5,6,7,7a-hexahydro-1H-pyrrol-7a-yl]methanol (Trans racemate) (60 mg, 0.22 mmol) were added to a reaction flask, and then DMF/THF (2 mL/2 mL) was added, followed by the addition of $CS_2CO_3$(108 mg, 0.33 mmol) and DABCO (25 mg, 0.22 mmol). The mixture was stirred at 60°C for 5 h. The reaction solution was poured into water (10 mL), and then extracted with dichloromethane. The organic phase was dried, concentrated under reduced pressure, and purified by silica gel column chromatography (DCM/MeOH = 30/1) to obtain 51 mg of pale yellow solid (yield: 36.2%).
LC/MS: m/z=629.1 $[M+H]^+$.

Step 3: Synthesis of 2-methylpropan-2-yl ({4-[6-amino-8-fluoro-4-(3-methoxytetrahydro-1H-pyrrol-1-yl)-2-({[3-(3,4,5-trifluorophenyl)-2,3,5,6, 7,7a-hexahydro-1H-pyrrolizin-7a-yl]methyl}oxy)quinazolin-7-yl]-3-cyano-7-fluorobenzo[b]thiophen-2-yl}amino)formate (Trans racemate)

7-bromo-6-chloro-8-fluoro-4-(3-methoxytetrahydro-1H-pyrrol-1-yl)-2-({[3-(3,4,5-trifluorop henyl)-2,3,5,6,7,7a-hexahydro-1H-pyrrolizin-7a-yl]methyl}oxy)quinazoline(Trans racemate) (51 mg, 0.08 mmol), 2-methylpropan-2-yl {[3-cyano-4-(5,5-dimethyl-1,3,2-dioxaborinan-2-yl)-7-fluorobenzo[b]thiophen-2-yl]amino }formate (32 mg, 0.08 mmol), $CS_2CO_3$ (39 mg, 0.12 mmol) and toluene (2 mL) were added to a reaction flask. After the reaction system was purged with nitrogen, $Pd(DPEPhos)Cl_2$ (5.6 mg, 0.008 mmol) was added. The reaction mixture was heated to 100°C and stirred for 6 h under nitrogen. After the reaction system was cooled to room temperature, the reaction solution was poured into water (20 mL), extracted with ethyl acetate. The organic phase was dried, and purified by silica gel column chromatography (DCM/MeOH=20/1) to obtain 15 mg of yellow solid (yield: 22.0%).
LC/MS: m/z = 841.2 $[M+H]^+$.

Step 4: Synthesis of 2-amino-4-[6-amino-8-fluoro-4-(3-methoxytetrahydro-1H-pyrrol-1-yl)-2-({[3-(3,4,5-trifluorophenyl)-2,3,5,6,7,7a-hexahydro-1H-pyrrolizin-7a-yl]methyl}oxy)quinazolin-7-yl]-7-fluorobenzo[b]thiophene-3-carbonitrile (Trans racemate)

2-methylpropan-2-yl ({4-[6-amino-8-fluoro-4-(3-methoxytetrahydro-1H-pyrrol-1-yl)-2-({[3-(3,4,5-trifluorophenyl)-2,3,5,6, 7,7a-hexahydro-1H-pyrrolizin-7a-yl]methyl}oxy)quinazolin-7-yl]-3-cyano-7-fluorobenzo[b]thiophen-2-yl}amino)formate (Trans racemate) (15 mg, 0.018 mmol) was added to a reaction flask and dissolved in dichloromethane (3 mL). Then, trifluoroacetic acid (1 mL) was added, and the mixture was stirred at room temperature for 30 min. The solution was concentrated under reduced pressure and added to a saturated $NaHCO_3$ aqueous solution (10 mL). The mixture was extracted with ethyl acetate. The organic phase was dried over anhydrous sodium sulfate, filtered, concentrated under reduced pressure, and purified by column chromatography (DCM/MeOH = 10/1) to obtain 6 mg of target product (yield: 45.5%).

LC/MS: m/z = 741.2 $[M+H]^+$.
$^1H$ NMR (400 MHz, d6-DMSO) δ 1.41-1.53 (2H, m), 1.58-1.64 (1H, m), 1.81-1.89 (1H, m), 1.97-2.04 (4H, m), 2.06-2.10 (1H, m), 2.15-2.24 (1H, m), 3.26-3.30 (3H, m), 3.48-3.52 (1H, m), 3.79-4.34 (8H, m), 5.27-5.38 (1H, m), 6.61-6.70 (1H, m), 7.08-7.33 (4H, m), 8.09-8.15 (2H, m).

## Example 16

4-[7-(2-amino-3-cyano-7-fluorobenzo[b]thiophen-4-yl)-6-chloro-8-fluoro-2-({[3-(3,4,5-trifl uorophenyl)-2,3,5,6,7,7a-hexahydro-1H-pyrrolizin-7a-yl]methyl}oxy)quinazolin-4-yl]-N,N-dimethyl-1,4-oxazepan-2-carboxamide (Trans racemate)

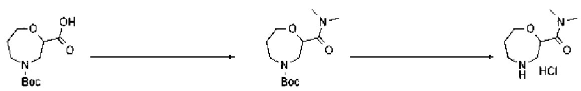

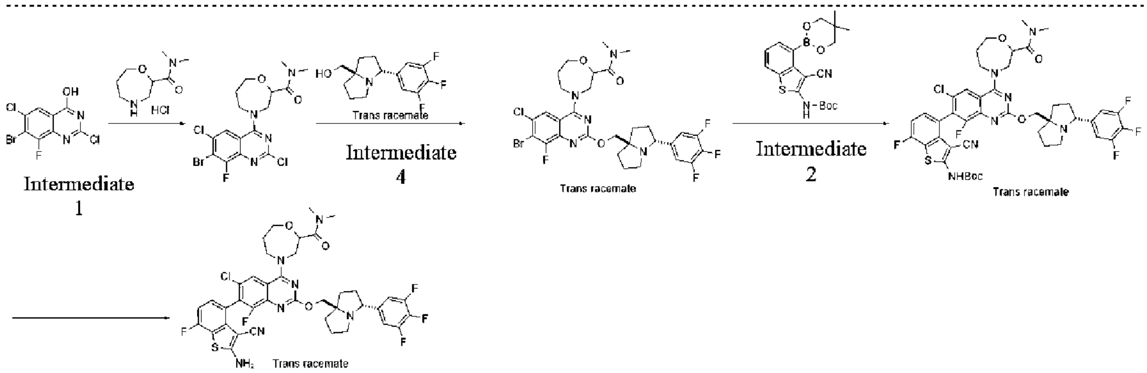


Step 1: Synthesis of 2-methylpropan-2-yl 2-[(dimethylamino)carbonyl]-1,4-oxazepane-4-carboxylate

4-{[(2-methylpropan-2-yl)oxy]carbonyl}-1,4-oxazepane-2-carboxylic acid (50 mg, 0.20 mmol), dimethylamine(0.1 mL, 0.30 mmol), DIEA (53 mg, 0.41 mmol), and HBTU (101 mg, 0.27 mmol) were weighed and dissolved in THF (2 mL). The reaction system was stirred at room temperature for 3 h. The reaction solution was added to water (10 mL), and then extracted three times with ethyl acetate. The organic phase was dried, concentrated under reduced pressure, and purified by Prep-TLC (PE/EA = 1/1) to obtain 50 mg of colorless oil (yield: 90.0%).
LC/MS: m/z = 273.2 $[M+H]^+$.

Step 2: Synthesis of N,N-dimethyl-1,4-oxazepan-2-carboxamide hydrochloride

2-methylpropan-2-yl 2-[(dimethylamino)carbonyl]-1,4-oxazepane-4-carboxylate (50 mg, 0.18 mmol) was weighed and dissolved in hydrochloric acid/dioxane (4 M, 2 mL). The system was stirred at room temperature for 2 h. The solution was concentrated under reduced pressure to obtain 43 mg of crude product. The crude product was used directly for next-step reaction.
LC/MS: m/z = 173.1 $[M+H]^+$.

Step 3: Synthesis of 4-(7-bromo-2,6-dichloro-8-fluoro-1,2-dihydroquinazolin-4-yl)-N,N-dimethyl-1,4-oxazepane-2-carbox amide

7-bromo-2,6-dichloro-8-fluoroquinazolin-4-ol (50 mg, 0.16 mmol), N,N-dimethyl-1,4-oxazepane-2-carboxamide hydrochloride (43 mg, 0.21 mmol), DIPEA (62 mg, 0.48 mmol), and dichloromethane (1 mL) were added to a reaction flask. After the reaction system was purged with nitrogen, bis(2-oxo-3-oxazolyl)phosphine chloride (61 mg, 0.24 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography (PE/EA = 1/1) to obtain 40 mg of pale yellow solid (yield: 53.5%).
LC/MS: m/z = 465.1 $[M+H]^+$.

Step 4: Synthesis of 4-[7-bromo-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-N,N-dimethyl-1,4-oxazepane-2-carboxamide (Trans racemate)

4-(7-bromo-2,6-dichloro-8-fluoro-1,2-dihydroquinazolin-4-yl)-N,N-dimethyl-1,4-oxazepane -2-carboxamide (40 mg, 0.09 mmol) and [3-(3,4,5-trifluorophenyl)-2,3,5,6,7,7a-hexahydro-1H-pyrrolizin-7a-yl]methanol (Trans racemate) (25 mg, 0.09 mmol) were added to a reaction flask, and then DMF/THF (2 mL/2 mL) was added, and further $CS_2CO_3$ (46 mg, 0.14 mmol) and DABCO (10 mg, 0.09 mmol) were added to the reaction flask. The mixture was stirred at 60°C for 5 h. The reaction solution was poured into water (10 mL), and then extracted with dichloromethane. The organic phase was dried, concentrated under reduced pressure, and purified by silica gel column chromatography (DCM/MeOH=30/1) to obtain 39 mg of yellow solid (yield: 65.0%).
LC/MS: m/z=700.1 $[M+H]^+$.

Step 5: Synthesis of 2-methylpropan-2-yl {[4-(6-chloro-4-{2-[(dimethylamino)carbonyl]-1,4-oxazepan-4-yl}-8-fluoro-2-({[3-(3,4,5-trifluorophe nyl)-2,3,5,6,7,7a-hexahydro-1H-pyrrolizin-7a-yl]methyl}oxy)quinazolin-7-yl)-3-cyano-7-fluorobenzo [b]thiophen-2-yl]amino}formate (Trans racemate)

4-[7-bromo-6-chloro-8-fluoro-2-({[3-(3,4,5-trifluorophenyl)-2,3,5,6,7,7a-hexahydro-1H-pyr rolizin-7a-yl]methyl}oxy)quinazolin-4-yl]-N,N-dimethyl-1,4-oxazepane-2-carboxamide (Trans racemate) (39 mg, 0.06 mmol), 2-methylpropan-2-yl {[3-cyano-4-(5,5-dimethyl-1,3,2-dioxaborinan-2-yl)-7-fluorobenzo[b]thiophen-2-yl]amino }formate (24 mg, 0.06 mmol), $CS_2CO_3$ (29 mg, 0.09 mmol), and toluene (2 mL) were added to a reaction flask. After the reaction system was purged with nitrogen, Pd(DPEPhos)$Cl_2$ (4.2 mg, 0.006 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, and purified by silica gel column chromatography (DCM/MeOH=20/1) to obtain 12 mg of yellow solid (yield: 23.5%).
LC/MS: m/z = 912.2 $[M+H]^+$.

Step 6: Synthesis of 4-[7-(2-amino-3-cyano-7-fluorobenzo[b]thiophen-4-yl)-6-chloro-8-fluoro-2-({[3-(3,4,5-trifluoropheny 1)-2,3,5,6,7,7a-hexahydro-1H-pyrrolizin-7a-yl]methyl}oxy)quinazolin-4-yl]-N,N-dimethyl-1,4-oxazep ane-2-carboxamide (Trans racemate)

2-methylpropan-2-yl {[4-(6-chloro-4-{2-[(dimethylamino)carbonyl]-1,4-oxazepan-4-yl}-8-fluoro-2-({[3-(3,4,5-trifluorophe nyl)-2,3,5,6,7,7a-hexahydro-1H-pyrrolizin-7a-yl]methyl}oxy)quinazolin-7-yl)-3-cyano-7-fluorobenzo [b]thiophen-2-yl]amino}formate (Trans racemate) (12 mg, 0.013 mmol) was added to a reaction flask and dissolved in dichloromethane (3 mL), and trifluoroacetic acid (1 mL) was added. The mixture was stirred at room temperature for 30 min. The solution was concentrated under reduced pressure and added to a saturated $NaHCO_3$ aqueous solution (10 mL). The solution was extracted with ethyl acetate. The organic phase was dried over anhydrous sodium sulfate, filtered, concentrated under reduced pressure, and purified by column chromatography (DCM/MeOH=10/1) to obtain 3 mg of target product (yield: 28.0%).
LC/MS: m/z = 812.2 $[M+H]^+$.

Example 17

2-amino-4-[6-chloro-8-fluoro-4-(1,4-oxazepan-4-yl)-2-({[1-(3,4,5-trifluorophenyl)octahydro cyclopenta[1,2-a][5]annulen-3a-yl]methyl}oxy)quinazolin-7-yl]-7-fluorobenzo[b]thiophene-3-carboni trile (Trans racemate)

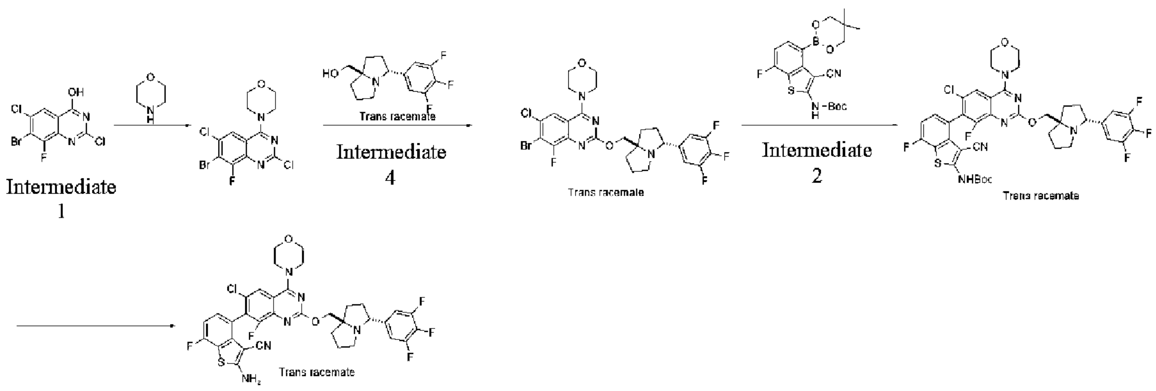


Step 1: Synthesis of 7-bromo-2,6-dichloro-8-fluoro-4-(1,4-oxazinane-4-yl)quinazoline

7-bromo-2,6-dichloro-8-fluoroquinazolin-4-ol (100 mg, 0.32 mmol), morpholine (28 mg, 0.32 mmol), DIPEA (83 mg, 0.64 mmol), and dichloromethane (3 mL) were added to a reaction flask. After the reaction system was purged with nitrogen, bis(2-oxo-3-oxazolyl)phosphine chloride (122 mg, 0.48 mmol) was added. The mixture was stirred at room temperature for 2 h. The reaction solution was poured into water (10 mL), extracted with dichloromethane. The organic phase was dried, concentrated under reduced pressure, and purified by silica gel column chromatography (PE/EA = 2/1) to obtain 91 mg of pale yellow solid (yield: 74.4%).
LC/MS: m/z = 380.1 $[M+H]^+$.

Step 2: Synthesis of 7-bromo-6-chloro-8-fluoro-4-(1,4-oxazepan-4-yl)-2-({[3-(3,4,5-trifluorophenyl)-2,3,5,6,7,7a-hexahyd ro-1H-pyrrolizin-7a-yl]methyl}oxy)quinazoline (Trans racemate)

7-bromo-2,6-dichloro-8-fluoro-4-(1,4-oxazepan-4-yl)quinazoline (91 mg, 0.24 mmol) and [3-(3,4,5-trifluorophenyl)-2,3,5,6,7,7a-hexahydro-1H-pyrrolizin-7a-yl]methanol (Trans racemate) (65 mg, 0.24 mmol) were added to a reaction flask, followed by the addition of DMF/THF (2 mL/2 mL), $CS_2CO_3$ (117 mg, 0.36 mmol) and DABCO (27 mg, 0.24 mmol).

The mixture was stirred at 60°C for 5 h. The reaction solution was poured into water (10 mL), extracted with dichloromethane. The organic phase was dried, concentrated under reduced pressure, and purified by silica gel column chromatography (DCM/MeOH = 30/1) to obtain 63 mg of pale yellow solid (yield: 42.7%).
LC/MS: m/z = 615.1 $[M+H]^+$.

Step 3: Synthesis of 2-methylpropan-2-yl ({4-[6-chloro-8-fluoro-4-(1,4-oxazepan-4-yl)-2-({[3-(3,4,5-trifluorophenyl)-2,3,5,6,7,7a-hexahydro-1 H-pyrrolizin-7a-yl]methyl}oxy)quinazolin-7-yl]-3-cyano-7-fluorobenzo[b]thiophen-2-yl}amino)forma te (Trans racemate)

7-bromo-6-chloro-8-fluoro-4-(1,4-oxazepan-4-yl)-2-({[3-(3,4,5-trifluorophenyl)-2,3,5,6,7,7a -hexahydro-1H-pyrrolizin-7a-yl]methyl}oxy)quinazoline (Trans racemate) (63 mg, 0.10 mmol), 2-methylpropan-2-yl {[3-cyano-4-(5,5-dimethyl-1,3,2-dioxaborinan-2-yl)-7-fluorobenzo[b]thiophen-2-yl]amino}formate (40 mg, 0.10 mmol), $CS_2CO_3$ (49 mg, 0.15 mmol), and toluene (2 mL) were added to a reaction flask. After the reaction system was purged with nitrogen, Pd(DPEPhos)$Cl_2$ (7.0 mg, 0.001 mmol) was added. The reaction mixture was heated to 100°C and stirred for 6 h under nitrogen. After the reaction system was cooled to room temperature, the reaction solution was poured into water (20 mL), and then extracted with ethyl acetate. The organic phase was dried, and purified by silica gel column chromatography (DCM/MeOH=20/1) to obtain 19 mg of yellow solid (yield: 22.4%).
LC/MS: m/z = 827.2 $[M+H]^+$.

Step 4: Synthesis of 2-amino-4-[6-chloro-8-fluoro-4-(1,4-oxazepan-4-yl)-2-({[3-(3,4,5-trifluorophenyl)-2,3,5,6,7,7a-hexah ydro-1H-pyrrolizin-7a-yl]methyl}oxy)quinazolin-7-yl]-7-fluorobenzo[b]thiophene-3-carbonitrile (Trans racemate)

2-methylpropan-2-yl ({4-[6-chloro-8-fluoro-4-(1,4-oxazacyclohexan-4-yl)-2-({[3-(3,4,5-trifluorophenyl)-2,3,5,6,7,7a-hexah ydro-1H-pyrrolizin-7a-yl]methyl}oxy)quinazolin-7-yl]-3-cyano-7-fluorobenzo[b]thiophen-2-yl}amino )formate (Trans racemate) (19 mg, 0.023 mmol) was added to a reaction flask and dissolved in dichloromethane (3 mL). Trifluoroacetic acid (1 mL) was added. The mixture was stirred at room temperature for 30 min. The solution was concentrated under reduced pressure and added to a saturated $NaHCO_3$ aqueous solution (10 mL). The mixture was extracted with ethyl acetate. The organic phase was dried over anhydrous sodium sulfate, filtered, concentrated under reduced pressure, and purified by column chromatography (DCM/MeOH = 10/1) to obtain 10 mg of target product (yield: 59.9%).

LC/MS: m/z = 727.2 $[M+H]^+$.
$^1H$ NMR (400 MHz, d6-DMSO) δ 1.41-1.53 (2H, m), 1.58-1.64 (1H, m), 1.81-1.89 (1H, m), 1.97-2.04 (4H, m), 2.06-2.10 (1H, m), 2.15-2.24 (1H, m), 3.26-3.30 (3H, m), 3.48-3.52 (1H, m), 3.79-4.34 (8H, m), 5.27-5.38 (1H, m), 6.61-6.70 (1H, m), 7.08-7.33 (4H, m), 8.09-8.15 (2H, m).

Example 18

2-amino-4-[6-chloro-8-fluoro-4-(6-hydroxy-6-methyl-1,4-oxazepan-4-yl)-2-({[3-(3,4,5-triflu orophenyl)-2,3,5,6,7,7a-hexahydro-1H-pyrrolizin-7a-yl]methyl}oxy)quinazolin-7-yl]benzo[b]thiophen e-3-carbonitrile (Trans isomer2)

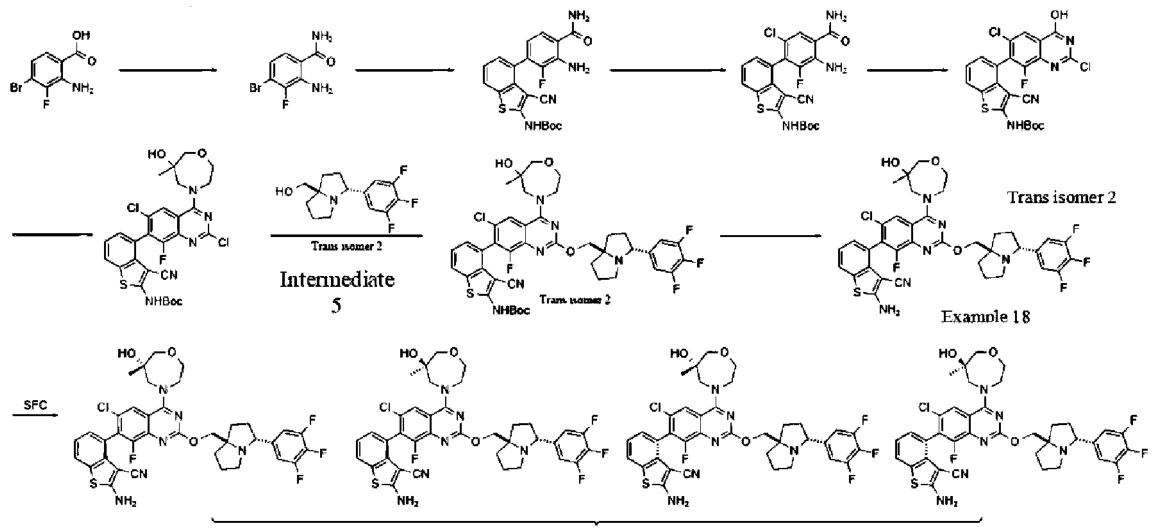


| | | | |
|---|---|---|---|
| Example 19: | Trans isomer 2. Axial chiral isomer 1 | Example 21: | Trans isomer 2, Axial chiral isomer 3 |
| Example 20: | Trans isomer 2, Axial chiral isomer 2 | Example 22: | Trans isomer 2, Axial chiral isomer 4 |

Step 1: Synthesis of 2-amino-4-bromo-3-fluorobenzamide

2-amino-4-bromo-3-fluorobenzene-1-carboxylic acid (350 g, 284.6 mol) and ammonium chloride (280 g, 5.233 mol), and DMF (3.5 L) were added to a reaction flask, followed by the addition of DIPEA (868 g, 6.729 mmol) and HBTU (850 g, 2.243 mol). The mixture was stirred at room temperature for 12 h. The reaction solution was poured into water (14 L), and a large amount of brown solid precipitated. After stirring for 0.5 h, the mixture was filtered, and the filter cake was washed with a small amount of water and dried to obtain 210 g of target product (total yield: 60.2%).
LC/MS: m/z = 233.1 $[M+H]^+$.

Step 2: Synthesis of 2-methylpropan-2-yl {[4-(3-amino-4-carbamoyl-2-fluorophenyl)-3 -cyanobenzo[b]thiophen-2-yl] amino}formate

2-amino-4-bromo-3-fluorobenzamide (68 g, 0.29 mol), potassium tert-butoxide (71.6 g, 0.73 mol), and pinacol diboronate (111.3 g, 0.44 mol) were added to a reaction flask, and then dioxane (680 mL) solution was added. After the reaction system was purged with nitrogen three times, Pd(dppf)$Cl_2$ (4.0 g, 0.005 mol) was added. The system was heated to 95°C and reacted for 3 h. Then, 2-methylpropan-2-yl {[3-cyano-4-(4,4,5,5-tetramethyl- 1,3,2-dioxaborolan-2-yl)benzo [b]thiophen-2-yl]amino }formate (8.5 g, 0.19 mol), $Cs_2CO_3$ (142.4 g, 0.44 mol) and water (136 mL) were added to the reaction system. Finally, Pd(dppf)$Cl_2$ (2.0 g, 0.002 mol) was added. The reaction mixture was heated to 95°C and stirred for 12 h under nitrogen. After the reaction system was cooled to room temperature, the reaction system was poured into water (1 L), and then extracted with ethyl acetate. The organic phase was dried and concentrated under reduced pressure to obtain 76.5 g of target product. The target product was used directly for the next step.
LC/MS: m/z=427.1 $[M+H]^+$.

Step 3: Synthesis of 2-methylpropan-2-yl {[4-(3-amino-6-chloro-4-carbamoyl-2-fluorophenyl)-3-cyanobenzo[b]thiophen-2-yl]amino}formate

2-methylpropan-2-yl{[4-(3-amino-4-carbamoyl-2-fluorophenyl)-3-cyanobenzo[b]thiophen-2 -yl]amino}formate (2 g, 4.69 mmol) was added to a reaction flask, and then (NMP) was added, followed by the slow addition of NCS (747 mg, 5.62 mmol). The system was reacted overnight at room temperature. The reaction mixture was poured into water, extracted with ethyl acetate, washed twice with saturated brine, dried, and concentrated to obtain a crude product. The crude product was purified by Prep-TLC to obtain 1.8 g of target product (yield: 85%).
LC/MS: m/z = 461.2 $[M+H]^+$.

Step 4: Synthesis of 2-methylpropan-2-yl {[4-(6-chloro-2-chloro-8-fluoro-4-hydroxyquinazolin-7-yl)-3-cyanobenzo[b]thiophen-2-yl]amino}for mate

2-methylpropan-2-yl [4-(3-amino-6-chloro-4-carbamoyl-2-fluorophenyl)-3-cyanobenzo[b]thiophen-2-yl]amino} formate (1.8 g, 3.9 mmol) was dissolved in 1,4-dioxane (18 mL). After the reaction system was purged with nitrogen, a solution of 1,4-dioxane (9 mL) containing phosgene (1.69 g, 11.7 mmol) was added dropwise at room temperature. The mixture was stirred at room temperature for 1 h, then heated to 80°C and stirred for 0.5 h. The system was added to water, extracted with ethyl acetate, dried, and concentrated to dryness. Prep-TLC was used to obtain 925 mg of target product (yield: 47%).
LC/MS: m/z = 505.1 $[M+H]^+$.

Step 5: Synthesis of 2-methylpropan-2-yl ({4-[6-chloro-2-chloro-8-fluoro-4-(6-hydroxy-6-methyl-1,4-oxazepan-4-yl)quinazolin-7-yl]-3-cyanob enzo[b]thiophen-2-yl}amino)formate

2-methylpropan-2-yl [4-(6-chloro-2-chloro-8-fluoro-4-hydroxyquinazolin-7-yl)-3-cyanobenzo[b]thiophen-2-yl] amino}form ate (900 mg, 1.78 mmol), 6-methyl-1,4-oxazepan-6-ol hydrochloride (440 mg, 2.67 mmol), DIPEA (918 mg, 7.12 mmol), and dichloromethane (10 mL) were added to a reaction flask. After the reaction system was purged with nitrogen, bis(2-oxo-3-oxazolyl)phosphine chloride (904 mg, 3.56 mmol) was added. The mixture was stirred at room temperature for 2 h. The reaction solution was poured into water (30 mL), and then extracted with dichloromethane. The organic phase was dried, concentrated under reduced pressure, and purified by silica gel column chromatography to obtain 659 mg of target product (yield: 60%).
LC/MS: m/z = 618.1 $[M+H]^+$.

Step 6: Synthesis of 2-methylpropan-2-yl ({4-[6-chloro-8-fluoro-4-(6-hydroxy-6-methyl-1,4-oxazacycloheptan-4-yl)-2-({[3-(3,4,5-trifluorophen yl)-2,3,5,6,7,7a-hexahydro-1H-pyrrolizin-7a-yl]methyl}oxy)quinazolin-7-yl]-3-cyanobenzo [b]thiophe n-2-yl}amino)formate (Trans isomer 2)

2-methylpropan-2-yl ({4-[6-chloro-2-chloro-8-fluoro-4-(6-hydroxy-6-methyl-1,4-oxazepan-4-yl)quinazolin-7-yl]-3-cyanob enzo[b]thiophen-2-yl}amino)formate (600 mg, 0.97 mmol) and [3-(3,4,5-trifluorophenyl)-2,3,5,6,7,7a-hexahydro-1H-pyrrolizin-7a-yl]methanol (Tran sisomer 2) (314 mg, 1.16 mmol) were added to a reaction flask, and then DMF/THF (7 mL/7 mL) was added, followed by the addition of $CS_2CO_3$ (632 mg, 1.94 mmol) and DABCO (32 mg, 0.29 mmol). The mixture was stirred at 45°C overnight. The reaction solution was poured into water, and then extracted with ethyl acetate. The organic phase was dried, concentrated under reduced pressure, and purified by silica gel column chromatography to obtain 330 mg of target product (yield: 40%).
LC/MS: m/z = 853.1 $[M+H]^+$.

Step 7: Synthesis of 2-amino-4-[6-chloro-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]benzo[b]thiophene-3-carb onitrile (Trans isomer 2)

2-methylpropan-2-yl ({4-[6-chloro-8-fluoro-4-(6-hydroxy-6-methyl-1,4-oxazepan-4-yl)-2-({[3-(3,4,5-trifluorophenyl)-2,3,5 ,6,7,7a-hexahydro-1H-pyrrolizin-7a-yl]methyl}oxy)quinazolin-7-yl]-3-cyanobenzo[b]thiophen-2-yl}a mino)formate (Trans isomer 2) (330 mg, 0.38 mmol) was added to a reaction flask and dissolved in dichloromethane (4 mL), and then trifluoroacetic acid (2 mL) was added. The mixture was stirred at room temperature for 4 h. The solution was concentrated under reduced pressure and added to a saturated $NaHCO_3$ aqueous solution (5 mL). The mixture was extracted with ethyl acetate. The organic phase was dried over anhydrous sodium sulfate, filtered, concentrated under reduced pressure, and purified by column chromatography (DCM/MeOH = 10/1) to obtain 120 mg of target product (yield: 42%).

LC/MS: m/z = 753.1 $[M+H]^+$.
$^1$H NMR (400 MHz, d6- DMSO) δ 8.54 (d, J = 26.0 Hz, 1H), 7.89 - 7.75 (m, 3H), 7.22 (ddd, J = 23.7, 19.3, 16.8 Hz, 4H), 4.19 (dd, J = 47.3, 14.9 Hz, 5H), 4.00 - 3.80 (m, 3H), 3.72 (d, J = 8.2 Hz, 1H), 3.56 (d, J = 5.6 Hz, 2H), 2.36 (s, 1H), 2.13 (s, 2H), 2.05 - 1.80 (m, 4H), 1.56 (d, J = 83.3 Hz, 3H), 1.15 (d, J = 8.3 Hz, 3H).

Step 8: Resolution of 2-amino-4-[6-chloro-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]benzo[b]thiophene-3-carb onitrile (Trans isomer 2)

Resolution method: Instrument: Waters SFC-80; Column: Chiralcel AD-3 (4.6*100 mm); Mobile phase: A (supercritical CO2), B (isopropanol, isopropanol solution containing 0.2% 7 M ammonia); Gradient: B% = 45%; Flow rate: 1.5 mL/min; Wavelength: 215 nm; Pressure: 2000 psi. Retention time of the first eluate (Rt) = 1.915 min, the second eluate (Rt) = 2.066 min, the third eluate (Rt) = 2.955 min, and the fourth eluate (Rt) = 3.606 min.

The first eluate (retention time Rt = 1.915 min) was defined as the compound in Example 19: LC/MS: m/z = 753.1[M+H]$^+$.

The second eluate (retention time Rt = 2.066 min) was defined as the compound in Example 20: LC/MS: m/z = 753.1 [M+H]$^+$.

The third eluate (retention time Rt = 2.955 min) was defined as the compound in Example 21:

LC/MS: m/z = 753.1 [M+H]$^+$.

$^1$H NMR (400 MHz, d6- DMSO) δ 8.50 (s, 1H), 7.89 - 7.76 (m, 3H), 7.25 (ddd, J = 26.0, 17.0, 7.3 Hz, 4H), 5.29 (s, 1H), 4.18 (ddd, J = 35.7, 22.5, 12.1 Hz, 5H), 3.96 (ddd, J = 32.8, 19.6, 10.8 Hz, 3H), 3.72 (dd, J = 9.6, 5.0 Hz, 1H), 3.60 - 3.50 (m, 2H), 2.39 - 2.31 (m, 1H), 2.20 (dd, J = 16.3, 8.3 Hz, 1H), 2.09 (dt, J = 21.8, 9.0 Hz, 2H), 1.96 - 1.79 (m, 3H), 1.69 - 1.49 (m, 3H), 1.13 (s, 3H).

The fourth eluate (retention time Rt = 3.606 min) was defined as the compound in Example 22: LC/MS: m/z = 797.2 [M+H]$^+$.

Example 23

2-amino-4-[6-bromo-8-fluoro-4-(6-hydroxy-6-methyl-1,4-oxazepan-4-yl)-2-({[1-(3,4,5-trifl uorophenyl)octahydrocyclopenta[1,2-a][5]annulen-3a-yl]methyl}oxy)quinazolin-7-yl]-7-fluorobenzo[ b]thiophene-3-carbonitrile (Trans isomer 2)

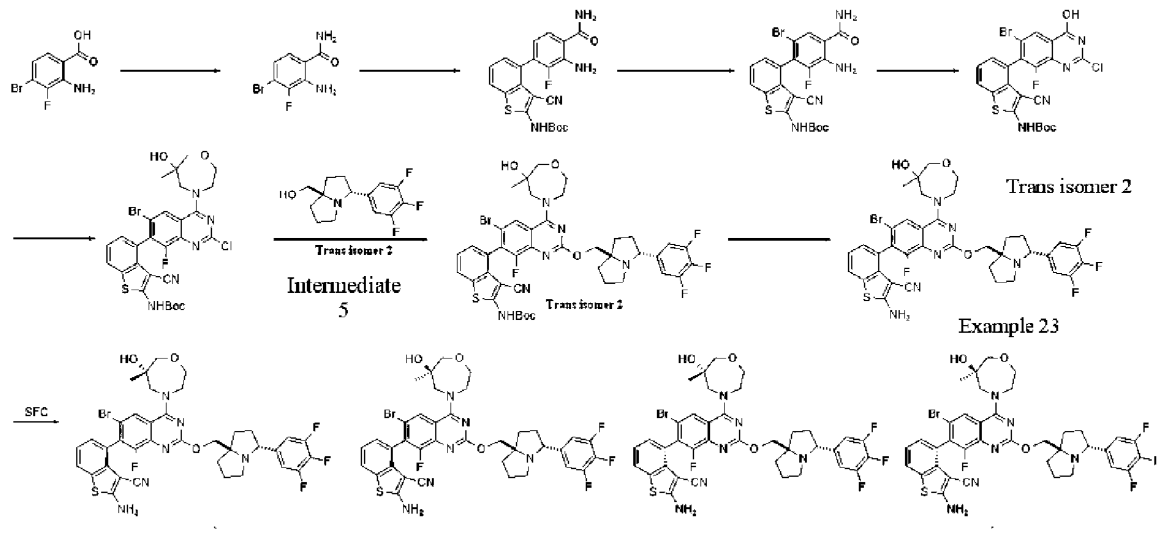


| | | | |
|---|---|---|---|
| Example 24: | Trans isomer 2. Axial chiral isomer 1 | Example 26: | Trans isomer 2. Axial chiral isomer 3 |
| Example 25: | Trans isomer 2. Axial chiral isomer 2 | Example 27: | Trans isomer 2, Axial chiral isomer 4 |

Step 1: Synthesis of 2-amino-4-bromo-3-fluorobenzamide

2-amino-4-bromo-3-fluorobenzene-1-carboxylic acid (350 g, 284.6 mol), ammonium chloride (280 g, 5.233 mol), and DMF (3.5 L) were added to a reaction flask, followed by the addition of DIPEA (868 g, 6.729 mmol) and HBTU (850 g, 2.243 mol). The mixture was stirred at room temperature for 12 h. The reaction solution was poured into water (14 L), and a large amount of brown solid precipitated. After stirring for 0.5 h, the mixture was filtered, and the filter cake was washed with a small amount of water and dried to obtain 210 g of target product (total yield: 60.2%).

LC/MS: m/z = 233.1 $[M+H]^+$.

Step 2: Synthesis of 2-methylpropan-2-yl {[4-(3-amino-4-carbamoyl-2-fluorophenyl)-3-cyanobenzo[b]thiophen-2-yl]amino}formate

2-amino-4-bromo-3-fluorobenzamide (68 g, 0.29 mol), potassium tert-butoxide (71.6 g, 0.73 mol), and pinacol diboronate (111.3 g, 0.44 mol) were added, and then dioxane (680 mL) was added to a reaction flask. After the reaction system was purged with nitrogen three times, Pd(dppf)$Cl_2$ (4.0 g, 0.005 mol) was added. The system was heated to 95°C and reacted for 3 h. Then, 2-methylpropan-2-yl {[3-cyano-4-(4,4,5,5-tetramethyl- 1,3,2-dioxaborolan-2-yl)benzo[b]thiophen-2-yl]amino }formate (8.5 g, 0.19 mol), $CS_2CO_3$ (142.4 g, 0.44 mol), and water (136 mL) were added to the system. Finally, Pd(dppf) $Cl_2$ (2.0 g, 0.002 mol) was added. The reaction mixture was heated to 95°C and stirred for 12 h under nitrogen. After the reaction system was cooled to room temperature, the reaction system was poured into water (1 L), and then extracted with ethyl acetate. The organic phase was dried and concentrated under reduced pressure to obtain 76.5 g of target product. The target product was used directly for the next step.
LC/MS: m/z=427.1 $[M+H]^+$.

Step 3: Synthesis of 2-methylpropan-2-yl {[4-(3-amino-6-bromo-4-carbamoyl-2-fluorophenyl)-3-cyanobenzo[b]thiophen-2-yl]amino}formate

2-methylpropan-2-yl {[4-(3-amino-4-carbamoyl-2-fluorophenyl)-3-cyanobenzo[b]thiophen-2-yl]amino}formate (197 mg, 0.46 mmol) was added to a reaction flask, followed by the addition of 2 mL of NMP, and then 82 mg of NBS (0.46 mmol) was slowly added. The system was allowed to react at room temperature for 6 min. The reaction system was then poured into water, extracted with ethyl acetate, dried, and concentrated to obtain a crude product. The crude product was purified by a silica gel agar plate to obtain 129 mg of target product (yield: 55%).
LC/MS: m/z = 505.2 $[M+H]^+$.

Step 4: Synthesis of 2-methylpropan-2-yl {[4-(6-bromo-2-chloro-8-fluoro-4-hydroxyquinazolin-7-yl)-3-cyanobenzo[b]thiophen-2-yl]amino} for mate

2-methylpropan-2-yl {[4-(3-amino-6-bromo-4-carbamoyl-2-fluorophenyl)-3-cyanobenzo[b]thiophen-2-yl]amino}formate (129 mg, 0.26 mmol) was dissolved in 1,4-dioxane (5 mL). After the reaction system was purged with nitrogen, a solution of 1,4-dioxane (2 mL) containing phosgene (88 mg, 0.78 mmol) was added dropwise at room temperature. The mixture was stirred at room temperature for 1 h, then heated to 80°C and stirred for 0.5 h. The system was added to water, extracted with ethyl acetate, dried, concentrated to dryness, and purified using silica gel agar to obtain 69 mg of target product (yield: 49%).
LC/MS: m/z=549.0 $[M+H]^+$.

Step 5: Synthesis of 2-methylpropan-2-yl ({4-[6-bromo-2-chloro-8-fluoro-4-(6-hydroxy-6-methyl-1,4-oxazepan-4-yl)quinazolin-7-yl]-3-cyanob enzo[b]thiophen-2-yl}amino)formate

2-methylpropan-2-yl [4-(6-bromo-2-chloro-8-fluoro-4-hydroxyquinazolin-7-yl)-3-cyanobenzo[b]thiophen-2-yl]amino}form ate (69 mg, 0.13 mmol), 6-methyl-1,4-oxazepan-6-ol hydrochloride (31 mg, 0.20 mmol), DIPEA (49 mg, 0.39 mmol), and dichloromethane (4 mL) were added. After the reaction system was purged with nitrogen, bis(2-oxo-3-oxazolyl)phosphine chloride (48 mg, 0.20 mmol) was added. The mixture was stirred at room temperature for 2 h. The reaction solution was poured into water (10 mL), and then extracted with dichloromethane. The organic phase was dried, concentrated under reduced pressure, and purified by silica gel column chromatography to obtain 49 mg of target product (yield: 59%).
LC/MS: m/z = 662.1 [M+H]+.

Step 6: Synthesis of 2-methylpropan-2-yl ({4-[6-bromo-8-fluoro-4-(6-hydroxy-6-methyl-1,4-oxazepan-4-yl)-2-({[3-(3,4,5-trifluorophenyl)-2,3,5 ,6,7,7a-hexahydro-1H-pyrrolizin-7a-yl]methyl}oxy)quinazolin-7-yl]-3-cyanobenzo[b]thiophen-2-yl}a mino)formate (Trans isomer 2)

2-methylpropan-2-yl ({4-[6-bromo-2-chloro-8-fluoro-4-(6-hydroxy-6-methyl-1,4-oxazepan-4-yl)quinazolin-7-yl]-3-cyanob enzo[b]thiophen-2-yl}amino)formate (49 mg, 0.07 mmol) and [3-(3,4,5-trifluorophenyl)-2,3,5,6,7,7a-hexahydro-1H-pyrrolizin-7a-yl]methanol (Trans isomer 2) (24 mg, 0.09 mmol) were added to a reaction flask, then DMF/THF (2 mL/2 mL) was added, followed by the addition of $CS_2CO_3$ (72 mg, 0.21 mmol) and DABCO (3 mg, 0.03 mmol). The mixture was stirred at 45°C for 6 h. The reaction solution was poured into water, and then extracted with ethyl acetate. The organic

phase was dried, concentrated under reduced pressure, and purified by column chromatography to obtain 29 mg of target product (yield: 44%).
LC/MS: m/z=897.1 [M+H]+.

Step 7: Synthesis of 2-amino-4-[6-bromo-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]benzo[b]thiophene-3-carb onitrile (Trans isomer 2)

2-methylpropan-2-yl ({4-[6-bromo-8-fluoro-4-(6-hydroxy-6-methyl-1,4-oxazepan-4-yl)-2-({[3-(3,4,5-trifluorophenyl)-2,3,5 ,6,7,7a-hexahydro-1H-pyrrolizin-7a-yl]methyl}oxy)quinazolin-7-yl]-3-cyanobenzo[b]thiophen-2-yl}amino)formate (Trans isomer 2) (29 mg, 0.03 mmol) was added to a reaction flask and dissolved in dichloromethane (3 mL). Trifluoroacetic acid (1 mL) was added, and the mixture was stirred at room temperature for 1 h. The solution was concentrated under reduced pressure and added to a saturated $NaHCO_3$ aqueous solution (5 mL). The mixture was extracted with ethyl acetate. The organic phase was dried over anhydrous sodium sulfate, filtered, concentrated under reduced pressure, and purified by column chromatography (DCM/MeOH = 10/1) to obtain 8 mg of target product (yield: 31%).

LC/MS: m/z = 797.2 [M+H]+.
$^1$H NMR (400 MHz, d6-DMSO) δ 1.15 (3H, s), 1.55-1.64 (4H, m), 1.82-1.87 (2H, m), 1.93-2.01 (1H, m), 2.07-2.13 (2H, m), 2.15-2.21 (1H, m), 2.33-2.37 (1H, m), 3.56 (2H, s), 3.68-3.73 (1H, m), 3.79-3.84 (1H, m), 3.93-3.97 (1H, m), 4.11-4.15 (2H, m), 4.19-4.27 (2H, m), 5.36 (3H, s), 7.12 (2H, d, J = 8.0Hz), 7.22 (1H, d, J = 8.0Hz), 7.25-7.30 (2H, m), 7.78 (1H, d, J=8.0Hz), 7.82 (2H, s), 8.72 (1H, s).

Step 8: Chiral resolution of 2-amino-4-[6-bromo-8-fluoro-4-(6-hydroxy-6-methyl-1,4-oxazepan-4-yl)-2-({[3-(3,4,5-trifluoropheny 1)-2,3,5,6,7,7a-hexahydro-1H-pyrrolizin-7a-yl]methyl}oxy)quinazolin-7-yl]benzo[b]thiophene-3-carb onitrile (Trans isomer 2)

The product from the previous step was subjected to SFC resolution to obtain a single-configuration compound.
Resolution method: Instrument: Waters SFC-80; Column: Chiralcel AD-3 (4.6*100 mm); Mobile phase: A (supercritical CO2), B (isopropanol, containing 0.2% DEA); Gradient: B% = 45%; Flow rate: 1.5 mL/min; Wavelength: 254 nm; Pressure: 2000 psi. Retention time of the first eluate (Rt) = 1.933 min, the second eluate (Rt) = 2.156 min, the third eluate (Rt) = 3.006 min, and the fourth eluate (Rt) = 3.485 min.
The first eluate (retention time Rt = 1.933 min) was defined as the compound in Example 24:

LC/MS: m/z = 797.2 [M+H]+.
$^1$H NMR (400 MHz, d6-DMSO) δ 1.16 (3H, s), 1.53-1.65 (4H, m), 1.83-1.87 (2H, m), 1.95-2.00 (1H, m), 2.09-2.12 (2H, m), 2.19-2.22 (1H, m), 2.33-2.36 (1H, m), 3.56 (2H, s), 3.69-3.73 (1H, m), 3.79-3.83 (1H, m), 3.94-3.96 (2H, m), 4.10-4.47 (3H, m), 5.42 (3H, s), 7.13 (2H, d, J = 8.0Hz), 7.22 (1H, t, J = 8.0Hz), 7.29-7.34 (2H, m), 7.79 (1H, d, J = 8.0Hz), 7.82 (2H, s), 8.72 (1H, s).

The second eluate (retention time Rt = 2.156 min) was defined as the compound in Example 25:

LC/MS: m/z = 797.2 [M+H]+.
$^1$H NMR (400 MHz, d6-DMSO) δ 1.15 (3H, s), 1.55-1.63 (4H, m), 1.84-1.86 (2H, m), 1.92-1.95 (1H, m), 2.08-2.12 (2H, m), 2.18-2.20 (1H, m), 2.34-2.36 (1H, m), 3.56 (2H, s), 3.69-3.72 (1H, m), 3.80-3.83 (1H, m), 3.94-3.96 (2H, m), 4.10-4.15 (2H, m), 4.19-4.27 (2H, m), 5.36 (3H, s), 7.12 (2H, d, J = 8.0Hz), 7.22 (1H, d, J = 8.0Hz), 7.24-7.30(2H, m), 7.78(1H, d, J = 8.0Hz), 7.82 (2H, s), 8.72 (1H, s).

The third eluate (retention time Rt = 3.006 min) was defined as the compound in Example 26:

LC/MS: m/z = 797.2 [M+H]+.
$^1$H NMR (400 MHz, d6-DMSO) δ 1.14 (3H, s), 1.55-1.64 (4H, m), 1.84-1.86 (2H, m), 1.94-2.00 (1H, m), 2.09-2.11 (2H, m), 2.19-2.21 (1H, m), 2.32-2.35 (1H, m), 3.55 (2H, s), 3.71-3.74 (1H, m), 3.88-3.92 (1H, m), 3.94-4.00 (2H, m), 4.08-4.23 (4H, m), 5.29 (3H, s), 7.16 (2H, d, J = 8.0Hz), 7.24 (1H, t, J = 8.0Hz), 7.29-7.33 (2H, m), 7.78(1H, d, J=8.0Hz), 7.82 (2H, s), 8.66 (1H, s).

The fourth eluate (retention time Rt = 3.485 min) was defined as the compound in Example 27:

LC/MS: m/z = 797.2 $[M+H]^+$.

$^1H$ NMR (400 MHz, d6-DMSO) δ 1.16 (3H, s), 1.53-1.63 (4H, m), 1.82-1.85 (2H, m), 1.90-1.95 (1H, m), 2.09-2.13 (2H, m), 2.19-2.22 (1H, m), 2.31-2.33 (1H, m), 3.54 (2H, s), 3.70-3.74 (1H, m), 3.88-3.93 (1H, m), 3.94-4.01 (2H, m), 4.05-4.21 (4H, m), 5.30 (3H, s), 7.15 (2H, d, J =8.0Hz), 7.22 (1H, t, J = 8.0Hz), 7.29-7.34 (2H, m), 7.79(1H, d, J = 8.0Hz), 7.81 (2H, s), 8.68 (1H, s).

Example 28

2-amino-4-[6-iodo-8-fluoro-4-(6-hydroxy-6-methyl-1,4-oxazepan-4-yl)-2-({[3-(3,4,5-trifluo rophenyl)-2,3,5,6,7,7a-hexahydro-1H-pyrrolizin-7a-yl]methyl}oxy)quinazolin-7-yl]benzo[b]thiophene -3-carbonitrile (Trans isomer2)

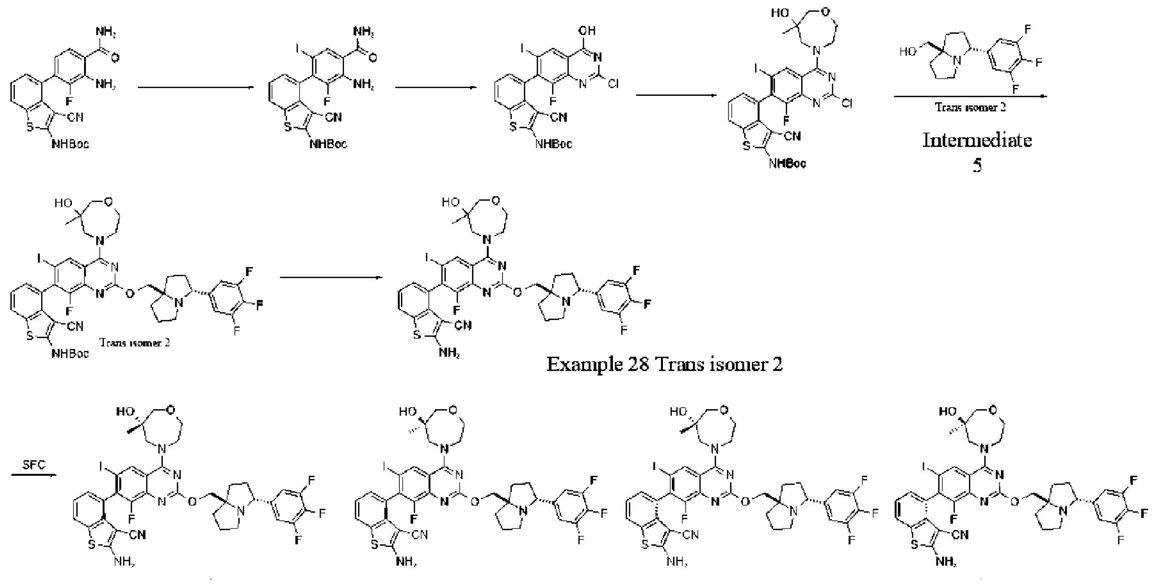


| | | | |
|---|---|---|---|
| Example 29: | Trans isomer 2, Axial chiral isomer 1 | Example 31: | Trans isomer 2, Axial chiral isomer 3 |
| Examiple30: | Trans isomer 2. Axial chiral isomer 2 | Example 32: | Trans isomer2, Axial chiral isomer 4 |

Step 1: Synthesis of 2-methylpropan-2-yl {[4-(3-amino-6-iodo-4-carbamoyl-2-fluorophenyl)-3-cyanobenzo[b]thiophen-2-yl]amino}formate

2-methylpropan-2-yl {[4-(3-amino-4-carbamoyl-2-fluorophenyl)-3-cyanobenzo[b]thiophen-2-yl]amino}formate (2.0 g, 4.69 mmol) was added to a reaction flask, then NMP (30 mL) was added, and NIS (2.1 g, 9.39 mmol) was slowly added. The system was reacted at room temperature for 12 h. The reaction system was poured into water to precipitate a solid. The solid was subjected to suction filtration and concentrated to obtain 3.3 g (crude product). The crude product was directly used for next-step reaction.

LC/MS: m/z = 553.2 $[M+H]^+$.

Step 2: Synthesis of 2-methylpropan-2-yl {[4-(6-iodo-2-chloro-8-fluoro-4-hydroxyquinazolin-7-yl)-3-cyanobenzo[b]thiophen-2-yl]amino}forma te

2-methylpropan-2-yl {[4-(3-amino-6-iodo-4-carbamoyl-2-fluorophenyl)-3-cyanobenzo [b]thiophen-2-yl]amino} formate (2.6 g, 4.7 mmol) was dissolved in 1,4-dioxane (26 mL). After the reaction system was purged with nitrogen, a solution of 1,4-dioxane (26 mL) containing phosgene (1.6 g, 14.1 mmol) was added dropwise at room temperature. After completion of the addition, the mixture was heated to 100°C and stirred for 1 h. The system was then added to water, extracted with ethyl acetate, dried, and concentrated to dryness to obtain 2.38 g (crude product).

LC/MS: m/z=597.1.0 $[M+H]^+$.

Step 3: Synthesis of 2-methylpropan-2-yl ({4-[6-iodo-2-chloro-8-fluoro-4-(6-hydroxy-6-methyl-1,4-oxazepan-4-yl)quinazolin-7-yl]-3-cyanoben zo[b]thiophen-2-yl}amino)formate

2-methylpropan-2-yl {[4-(6-iodo-2-chloro-8-fluoro-4-hydroxyquinazolin-7-yl)-3-cyanobenzo[b]thiophen-2-yl] amino}forma te (2.38 g, 3.99 mmol), 6-methyl-1,4-oxazepan-6-ol hydrochloride (800 mg, 4.79 mmol), DIPEA (1.03 mg, 7.98 mmol) and dichloromethane (25 mL) were added to a reaction flask. After the reaction system was purged with nitrogen, bis(2-oxo-3-oxazolyl)phosphine chloride (2.03 g, 7.98 mmol) was added. The mixture was stirred at room temperature for 2 h. The reaction solution was poured into water (100 mL), and then extracted with dichloromethane. The organic phase was dried, concentrated under reduced pressure, and purified by column chromatography to obtain 2.0 g of product (total three-step yield: 60.2%).
LC/MS: m/z=710.0 $[M+H]^+$.

Step 4: Synthesis of 2-methylpropan-2-yl ({4-[6-iodo-8-fluoro-4-(6-hydroxy-6-methyl-1,4-oxazepan-4-yl)-2-({[3-(3,4,5-trifluorophenyl)-2,3,5,6 ,7,7a-hexahydro-1H-pyrrolizin-7a-yl]methyl}oxy)quinazolin-7-yl]-3-cyanobenzo[b]thiophen-2-yl} ami no)formate (Trans isomer2)

2-methylpropan-2-yl ({4-[6-iodo-2-chloro-8-fluoro-4-(6-hydroxy-6-methyl-1,4-oxazepan-4-yl)quinazolin-7-yl]-3-cyanoben zo[b]thiophen-2-yl}amino)formate (2.0 g, 3.29 mmol) and [3-(3,4,5-trifluorophenyl)-2,3,5,6,7,7a-hexahydro-1H-pyrrolizin-7a-yl]methanol (Trans isomer 2) (1.07 g, 3.95 mmol) were added to a reaction flask, and then DMF/THF (5 mL/5 mL) was added, followed by the addition of $CS_2CO_3$ (2.14 g, 6.58 mmol) and DABCO (111 mg, 0.99 mmol). The reaction mixture was stirred at 45°C for 12 h. The reaction solution was poured into water, and then extracted with ethyl acetate. The organic phase was dried, concentrated under reduced pressure, and purified by column chromatography to obtain 1.8 g of target product (yield: 67.7%).
LC/MS: m/z=897.1 $[M+H]^+$.

Step 5: Synthesis of 2-amino-4-[6-iodo-8-fluoro-4-(6-hydroxy-6-methyl-1,4-oxazepan-4-yl)-2-({[3-(3,4,5-trifluorophenyl)-2,3,5,6,7,7a-hexahydro-1H-pyrrolizin-7a-yl]methyl}oxy)quinazolin-7-yl]benzo[b]thiophene-3-carboni trile (Trans isomer 2)

2-methylpropan-2-yl ({4-[6-iodo-8-fluoro-4-(6-hydroxy-6-methyl-1,4-oxazepan-4-yl)-2-({[3-(3,4,5-trifluorophenyl)-2,3,5,6 ,7,7a-hexahydro-1H-pyrrolizin-7a-yl]methyl}oxy)quinazolin-7-yl]-3-cyanobenzo[b]thiophen-2-yl}ami no)formate (Trans isomer 2) (1.8 g, 1.91 mmol) was added to a reaction flask and dissolved in dichloromethane (8 mL), and trifluoroacetic acid (2 mL) was added. The mixture was stirred at room temperature for 1 h. The solution was concentrated under reduced pressure, added to a saturated $NaHCO_3$ aqueous solution (20 mL), and then extracted with ethyl acetate. The organic phase was dried over anhydrous sodium sulfate, filtered, concentrated under reduced pressure, and purified by column chromatography (DCM/$NH_3$ in MeOH solution = 100/1) to obtain 1.5 g of target product (yield: 60.8%).

LC/MS: m/z = 845.1 $[M+H]^+$.
$^1$H NMR (400 MHz, d6-DMSO) $\delta$ 1.15 (3H, d, J = 8.0Hz), 1.54-1.63 (4H, m), 1.83-1.88 (2H, m), 1.92-1.98 (1H, m), 2.04-2.11 (2H, m), 2.13-2.18 (1H, m), 2.31-2.36 (1H, m), 3.55 (2H, s), 3.69-3.75 (1H, m), 3.80-3.84 (1H, m), 3.92-3.97 (2H, m), 4.12-4.23 (4H, m), 5.29-5.40(1H, m), 7.04-7.07(1H, m), 7.21-7.34 (3H, m), 7.77-7.81 (3H, m), 7.83-7.87 (1H, m).

Step 6: Resolution of 2-amino-4-[6-iodo-8-fluoro-4-(6-hydroxy-6-methyl-1,4-oxazepan-4-yl)-2-({[3-(3,4,5-trifluorophenyl)-2,3,5,6,7,7a-hexahydro-1H-pyrrolizin-7a-yl]methyl}oxy)quinazolin-7-yl]benzo[b]thiophene-3-carboni trile (Trans isomer2)

The product from the previous step was subjected to SFC resolution to obtain a single-configuration compound.
Resolution method: Instrument: Waters SFC-80; Column: Chiralcel AD-3 (4.6*100 mm); Mobile phase: A (supercritical CO2), B (isopropanol, containing 0.2% DEA); Gradient: B% = 45%; Flow rate: 1.5 mL/min; Wavelength: 254 nm; Pressure: 2000 psi. Retention time of the first eluate (Rt) = 1.988 min, the second eluate (Rt) = 2.290 min, the third eluate (Rt) = 3.095 min, and the fourth eluate (Rt) = 3.502 min.
The first eluate (retention time Rt = 1.988 min) was defined as the compound in Example 29:
LC/MS: m/z = 845.1 $[M+H]^+$.
The second eluate (retention time Rt = 2.290 min) was defined as the compound in Example 30:
LC/MS: m/z = 845.1 $[M+H]^+$.
The third eluate (retention time Rt = 3.095 min) was defined as the compound in Example 31:

LC/MS: m/z = 845.1 $[M+H]^+$.
$^1$H NMR (400 MHz, d6-DMSO) δ 8.83 (s, 1H), 7.79 (d, J = 8.1 Hz, 3H), 7.35 - 7.22 (m, 3H), 7.10 (d, J = 7.3 Hz, 1H), 5.28 (s, 1H), 4.17 (ddd, J = 22.0, 16.8, 8.2 Hz, 5H), 4.02 - 3.89 (m, 3H), 3.77 - 3.70 (m, 1H), 3.55 (q, J = 12.3 Hz, 2H), 2.38 - 2.33 (m, 1H), 2.21 (dd, J = 16.2, 8.3 Hz, 1H), 2.15 - 2.06 (m, 2H), 1.95 (s, 1H), 1.89 - 1.81 (m, 2H), 1.64 (s, 2H), 1.58 - 1.52 (m, 1H), 1.15 (s, 3H).

The fourth eluate (retention time Rt = 3.502 min) was defined as the compound in Example 32:
LC/MS: m/z = 845.1 $[M+H]^+$.

Example 33

2-amino-4-{6-[dimethyl(oxo)-15-methylphosphino]-8-fluoro-4-(6-hydroxy-6-methyl-1,4-ox azepan-4-yl)-2-({[3-(3,4,5-trifluorophenyl)-2,3,5,6,7,7a-hexahydro-1H-pyrrolizin-7a-yl]methyl}oxy)q uinazolin-7-yl}benzo[b]thiophene-3-carbonitrile (Trans isomer 2)

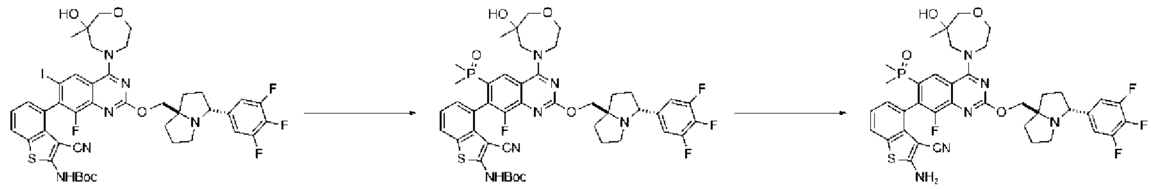

Example 28 Trans isomer 2

Trans isomer 2

Example 33 Trans isomer 2

Step 1: Synthesis of 2-methylpropan-2-yl [(3-cyano-4-{6-[dimethyl(oxo)-15-methylphosphino]-8-fluoro-4-(6-hydroxy-6-methyl-1,4-oxazepan-4 -yl)-2-({[3-(3,4,5-trifluorophenyl)-2,3,5,6,7,7a-hexahydro-1H-pyrrolizin-7a-yl]methyl}oxy)quinazolin -7-yl}benzo[b]thiophen-2-yl)amino]formate (Trans isomer 2)

2-amino-4-[6-iodo-8-fluoro-4-(6-hydroxy-6-methyl-1,4-oxazepan-4-yl)-2-({[3-(3,4,5-trifluo rophenyl)-2,3,5,6,7,7a-hexahydro-1H-pyrrolizin-7a-yl]methyl}oxy)quinazolin-7-yl]benzo[b]thiophene -3-carbonitrile (Trans isomer 2) (50 mg, 0.053 mmol), dimethylphosphine oxide (41 mg, 0.53 mmol), potassium phosphate (23 mg, 0.106 mmol), $Pd_2(dba)_3$ (3 mg, 0.0033 mmol), and xantphos (3 mg, 0.0066 mmol) were added to a reaction flask, and then 1,4-dioxane (1 mL) was added. After the system was purged with nitrogen, the system was heated to 100°C and stirred for 18 h. The reaction solution was added to water (5 mL), and then extracted twice with ethyl acetate. The organic phase was dried and concentrated under reduced pressure, and then purified by thin-layer silica gel chromatography (DCM/MeOH = 20/1) to obtain 20 mg of yellow solid (yield: 43.4%).
LC/MS: m/z = 895.3 $[M+H]^+$.

Step 2: Synthesis of 2-amino-4-{6-[dimethyl(oxo)-λ5-methylphosphino]-8-fluoro-4-(6-hydroxy-6-methyl-1,4-oxazepan-4-yl)-2-({[3-(3,4,5-trifluorophenyl)-2,3,5,6,7,7a-hexahydro-1H-pyrrolizin-7a-yl]methyl}oxy)quinazolin-7-yl}benzo[b] thiophene-3-carbonitrile (Trans isomer 2)

2-methylpropan-2-yl [(3-cyano-4-{6-[dimethyl(oxo)-λ5-methylphosphino]-8-fluoro-4-(6-hydroxy-6-methyl-1,4-oxazepan-4 -yl)-2-({[3-(3,4,5-trifluorophenyl)-2,3,5,6,7,7a-hexahydro-1H-pyrrolizin-7a-yl]methyl}oxy)quinazolin -7-yl} benzo[b]thiophen-2-yl)amino]formate (Trans isomer 2)(20 mg, 0.022 mmol) was weighed and dissolved in DCM (1.5 mL). TFA (0.5 mL) was added. The mixture was stirred at room temperature for 0.5 h. The pH was adjusted to 7-8 by adding a saturated sodium bicarbonate aqueous solution. The reaction solution was extracted with dichloromethane. The organic phase was dried and concentrated under reduced pressure, and then subjected to thin-layer silica gel chromatography (DCM/$NH_3$ MeOH solution = 30/1) to obtain 1 mg of yellow solid (yield: 5.6%).
LC/MS: m/z = 795.2 $[M+H]^+$.

Example 34

2-amino-4-[6-chloro-8-fluoro-4-(1,2,5-oxadiazepan-5-yl)-2-({[3-(3,4,5-trifluorophenyl)-2,3, 5,6,7,7a-hexahydro-1H-pyrrolizin-7a-yl]methyl}oxy)quinazolin-7-yl]benzo[b]thiophene-3-carbonitrile (Trans isomer 2)

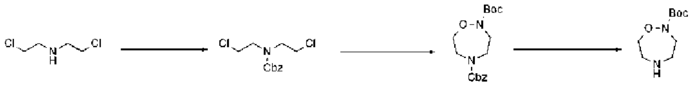

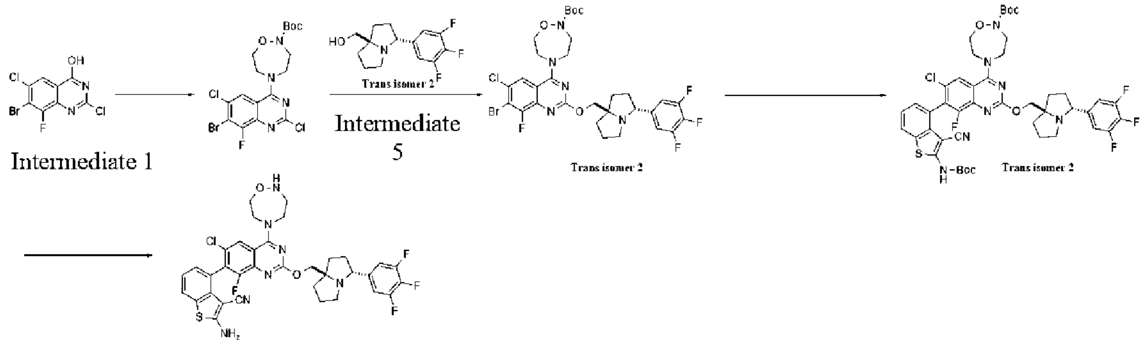


Example 34 Trans isomer 2

Step 1: Synthesis of benzyl [bis(2-chloroethyl)amino]formate

$NaHCO_3$ (5.96 g, 70.9 mmol) was weighed and dissolved in $H_2O$ (15 mL), and Cbz-Cl (6.3 g, 36.9 mmol) was added. After the materials were reacted at room temperature for 10 min, bis(2-chloroethyl)amine (5 g, 35.5 mmol) was dissolved in $H_2O$ (10 mL). After the system was cooled to 0°C, the aqueous solution of this compound was slowly added dropwise to the reaction system. After completion of the addition, the system was heated to room temperature and stirred for 1 h. The reaction solution was added to water (30 mL), and then extracted twice with ethyl acetate. The organic phase was dried, concentrated under reduced pressure, and purified by silica gel column chromatography (PE/EA = 3/1) to obtain 6.37 g of target product (yield: 65.3%).
LC/MS: m/z = 276.1 $[M+H]^+$.

Step 2: Synthesis of 2-methylpropan-2-yl 5-[(benzyloxy)carbonyl]-1,2,5-oxadiazepan-2-carboxylate

N-tert-butyloxycarbonylhydroxylamine (3.07 g, 23.1 mmol) was weighed and dissolved in DMF (40 mL). After the reaction system was purged with nitrogen, the system was cooled to 0°C. NaH (2.032 g, 50.7 mmol, 60%) was added, and kept 0°C for reaction for 1 h. Benzyl [bis(2-chloroethyl)amino]formate (6.37 g, 23.15 mmol) was dissolved in DMF (10 mL), and added to the reaction system. The reaction system was heated to 100°C and reacted for 0.5 h. The reaction solution was added to an ammonium chloride aqueous solution (50 mL) for quenching. The reaction mixture was extracted twice with ethyl acetate, and backwashed once with saturated brine. The organic phase was dried, concentrated under reduced pressure, and purified by silica gel column chromatography (PE/EA=50/1) to obtain 4.2 g of target product (yield: 53.9%).
LC/MS: m/z = 337.2 $[M+H]^+$.

Step 3: Synthesis of 2-methylpropan-2-yl 1,2,5-oxadiazepan-2-carboxylate

2-methylpropan-2-yl 5-[(benzyloxy)carbonyl]-1,2,5-oxadiazepan-2-carboxylate (2 g, 5.949 mmol) was weighed and dissolved in methanol (50 mL). Pd/C (200 mg, 10% w/w) was added. After the reaction system was purged with nitrogen, the reaction system was reacted at 50°C for 18 h. The reaction mixture was filtered. The filter cake was washed with methanol (20 mL). The mother liquor was concentrated under reduced pressure, and purified by silica gel column chromatography (PE/EA=20/1) to obtain 990 mg of target product (yield: 82.5%).
LC/MS: m/z = 203.1 $[M+H]^+$.

Step 4: Synthesis of 2-methylpropan-2-yl 5-(7-bromo-2,6-dichloro-8-fluoroquinazolin-4-yl)-1,2,5-oxadiazepan-2-carboxylate

7-bromo-2,6-dichloro-8-fluoroquinazolin-4-ol (200 mg, 0.645 mmol) and 1,2,5-oxadiazepan-2- carboxylate (156 mg, 0.774 mmol) were added to a reaction flask, and then DCM (3 mL) was added, followed by the addition of DIEA (166 mg, 1.29 mmol). After the materials were stirred at room temperature for 2 min, BoP-Cl (246 mg, 0.968 mmol) was added. The system was stirred at room temperature for 2 h, water (5 mL) was added, and the mixture was extracted with DCM. The organic phase was dried and concentrated under reduced pressure. The organic phase was dried, concentrated under reduced pressure, and then purified by thin-layer silica gel chromatography (PE/EA = 2/1) to obtain 140 mg of product

(yield: 43.9%).
LC/MS: m/z=495.0 $[M+H]^+$.

Step 5: Synthesis of 2-methylpropan-2-yl 5-[7-bromo-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-1,2,5-oxadiazepan-2-carboxylate (Trans isomer 2)

2-methylpropan-2-yl 5-(7-bromo-2,6-dichloro-8-fluoroquinazolin-4-yl)-1,2,5-oxadiazepan-2-carboxylate (140 mg, 0.283 mmol), [3-(3,4,5-trifluorophenyl)-2,3,5,6,7,7a-hexahydro-1H-pyrrolizin-7a-yl]methanol (Trans isomer 2) (115 mg, 0.425 mmol), $CS_2CO_3$ (186 mg, 0.566 mmol) and DABCO (13 mg, 0.113 mmol) were added to a reaction flask, and then DMF/THF (2 mL/2 mL) was added, After the reaction system was purged with nitrogen, the reaction system was reacted at 45°C for 18 h. The reaction system was added to water (10 mL). The reaction solution was extracted twice with ethyl acetate, and backwashed once with saturated brine. The organic phase was dried, concentrated under reduced pressure, and subjected to thin-layer silica gel chromatography (DCM/MeOH). =20/1) to obtain 85 mg of target product (yield: 41.1%).
LC/MS: m/z=730.1 $[M+H]^+$.

Step 6: Synthesis of 2-methylpropan-2-yl 5-{6-chloro-7-[3-cyano-2-({[(2-methylpropan-2-yl)oxy]carbonyl}amino)benzo [b]thiophen-4-yl]-8-flu oro-2-({[3-(3,4,5-trifluorophenyl)-2,3,5,6,7,7a-hexahydro-1H-pyrrolizin-7a-yl]methyl}oxy)quinazolin -4-yl}-1,2,5-oxadiazepane-2-carboxylate (Trans isomer 2)

2-methylpropan-2-yl 5-[7-bromo-6-chloro-8-fluoro-2-({[3-(3,4,5-trifluorophenyl)-2,3,5,6,7,7a-hexahydro-1H-pyrrolizin-7a-yl]methyl}oxy)quinazolin-4-yl]-1,2,5-oxadiazepane-2-carboxylate (Trans isomer 2) (85 mg, 0.117 mmol), potassium acetate (23 mg, 0.234 mol), and pinacol diboronate (45 mg, 0.176 mmol) were added to a reaction flask, and then 3 mL of dioxane solution was added. After the reaction system was purged with nitrogen three times, $Pd(dppf)Cl_2$ (8.8 mg, 0.012 mmol) was added. The system was heated to 95°C and reacted for 3 h. Then, 2-methylpropan-2-yl{[3-cyano-4-(4,4,5,5-tetramethyl-1,3,2-dioxaborolan-2-yl)benzo[b]thiophen-2-yl] amino} formate (47 mg, 0.117 mmol), $CS_2CO_3$ (76 mg, 0.234 mmol), and water (0.5 mL) were added to the system. Finally, $Pd(dppf)Cl_2$ (8.8 mg, 0.012 mmol) was added. The reaction mixture was heated to 95°C and stirred for 12 h under nitrogen. After being cooled to room temperature, the reaction system was poured into water (1 L), and then extracted with ethyl acetate. The organic phase was dried, and purified by silica gel thin-layer chromatography to obtain 18 mg of target product (yield: 16.7%).

Step 7: Synthesis of 2-amino-4-[6-chloro-8-fluoro-4-(1,2,5-oxadiazepan-5-yl)-2-({[3-(3,4,5-trifluorophenyl)-2,3,5,6,7,7a-h exahydro-1H-pyrrolizin-7a-yl]methyl}oxy)quinazolin-7-yl]benzo[b]thiophene-3-carbonitrile (Trans isomer 2)

5-{6-chloro-7-[3-cyano-2-({[(2-methylpropan-2-yl)oxy]carbonyl}amino)benzo[b]thiophen-4-yl]-8-fluoro-2-({[3-(3,4,5-trifluorophenyl)-2,3,5,6,7,7a-hexahydro-1H-pyrrolizin-7a-yl]methyl}oxy) quinazolin-4-yl}-1,2,5-oxadiazepan-2-carboxylate (Trans isomer 2)(18 mg, 0.019 mmol) was weighed and dissolved in DCM (1.5 mL). TFA (0.5 mL) was added. The reaction solution was stirred and reacted at room temperature for 0.5 h. The pH was adjusted to 7-8 by adding a saturated sodium bicarbonate aqueous solution. The reaction solution was extracted with dichloromethane. The organic phase was dried and concentrated under reduced pressure, and then purified by thin-layer silica gel chromatography (DCM/NH3 MeOH solution = 30/1) to obtain 3.2 mg of target product (yield: 22.6%).

LC/MS: m/z=724.1 $[M+H]^+$.
$^1$H NMR (400 MHz, d6-DMSO) δ 1.62-1.66 (2H, m), 1.82-1.88 (2H, m), 1.93-1.98 (2H, m), 2.10-2.14 (2H, m), 2.25-2.34 (2H, m), 3.20-3.25 (2H, m), 3.91-3.95 (2H, m), 4.00-4.06 (2H, m), 4.15-4.19 (4H, m), 4.45-4.49(1H, m), 7.10 (1H, s), 7.23-7.33 (4H, m), 7.79 (1H, d, J =8.0Hz), 7.84 (2H, brs), 7.98 (1H, s).

Example 35

2-amino-4-[6-chloro-8-fluoro-4-(3-hydroxy-3-methyl-2,3,4,5,5a,6a-hexahydrocyclopropano [1,2-b][1,4]oxazepin-5-yl)-2-({[3-(3,4,5-trifluorophenyl)-2,3,5,6,7,7a-hexahydro-1H-pyrrolizin-7a-yl] methyl}oxy)quinazolin-7-yl]benzo[b]thiophene-3-carbonitrile (Trans isomer 2)

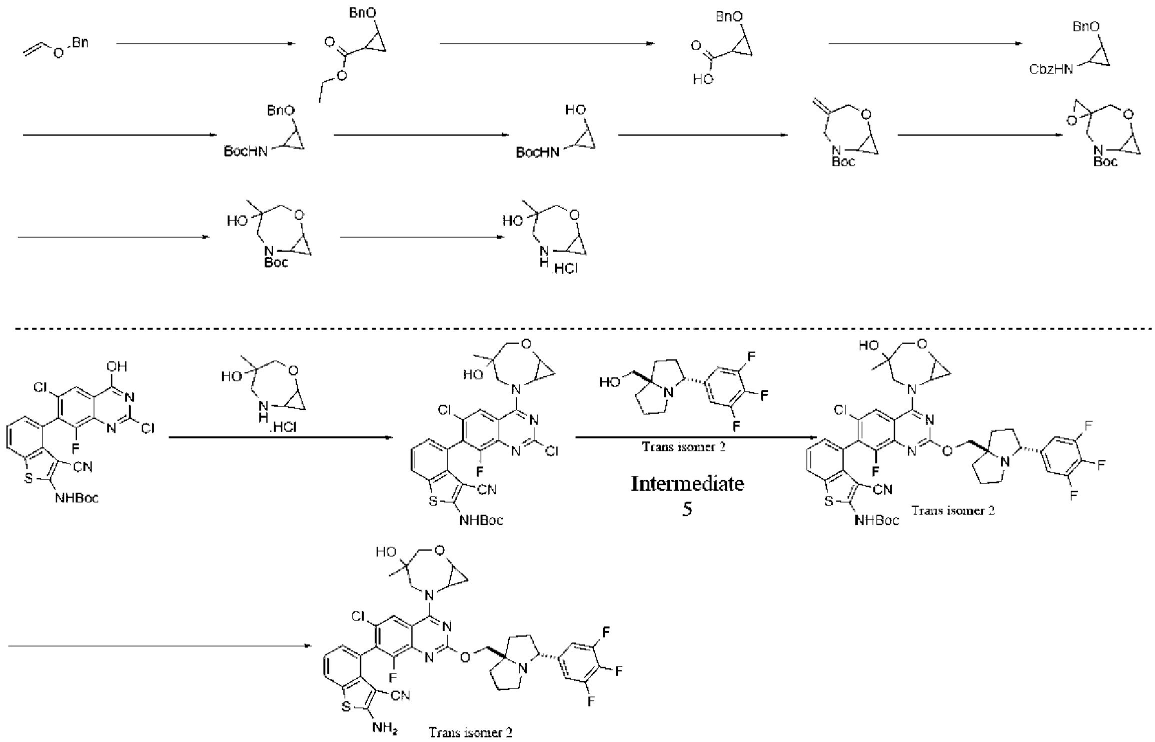


Step 1: Synthesis of ethyl 2-(benzyloxy)cyclopropane-1-carboxylate

Benzyl vinyl ether (9 g, 67.08 mmol), dimerized rhodium acetate (296 mg, 0.67 mmol) and DCM (90 mL) were added to a reaction flask. Ethyl diazoacetate (8.42 g, 73.78 mmol) was dissolved in DCM (40 mL). The solution of ethyl diazonate in DCM was slowly added dropwise (over 1 h) into the reaction system at room temperature, and stirred overnight. The reaction solution was concentrated to remove DCM, and ethyl acetate and water were added thereto. The phase separation was conducted, and organic phase was collected. The collected organic phase was washed once with saturated brine. The organic phase was dried, concentrated under reduced pressure, and purified by silica gel column chromatography (PE/EA=15/1) to obtain 4.1 g of colorless liquid compound (yield: 27%).

Step 2: Synthesis of 2-(benzyloxy)cyclopropane-1-carboxylic acid

Ethyl 2-(benzyloxy)cyclopropane-1-carboxylate (4g, 18.1mmol) was weighed and dissolved in methanol (40mL). LiOH (2.17g, 90.5mmol) and water (16mL) were added thereto. The reaction was carried out at room temperature for 12 h. The reaction solution was concentrated to dryness, and water and MTBE were added for phase separation. The aqueous phase was collected, and the pH was adjusted to approximately 2-3 with 2M HCl. The aqueous phase after pH adjustment was extracted by using DCM (3 × 75 mL). The organic phase was dried and concentrated under reduced pressure to obtain 3 g of colorless oily compound (yield: 86%).
LC/MS: m/z=191.1 $[M-H]^-$.

Step 3: Synthesis of benzyl {[2-(benzyloxy)cyclopropyl] amino } formate

2-(benzyloxy)cyclopropane-1-carboxylic acid (3 g, 15.6 mmol), TEA (4.72 g, 46.8 mmol), and DPPA (5.5 g, 20.2 mmol) were weighed and dissolved in dioxane (40 mL). The mixture was purged with nitrogen three times and reacted at room temperature for 20 min. Then, benzyl alcohol (5 g, 46.8 mmol) was added, and the reaction mixture was heated to 85°C and reacted for 12 h. The reaction solution was cooled to room temperature and concentrated to dryness. Water and ethyl acetate were added for phase separation. The organic phase was collected, washed once with saturated brine, dried, concentrated under reduced pressure, and purified by silica gel column chromatography to give 3.3 g of target product (yield: 71.7%).
LC/MS: m/z=298.1 $[M+H]^+$.

Step 4: Synthesis of 2-methylpropan-2-yl {[2-(benzyloxy)cyclopropyl] amino } formate

Benzyl {[2-(benzyloxy)cyclopropyl] amino } formate (3 g, 10.1 mmol) and di-tert-butyl dicarbonate (2.4 g, 11.1 mmol) were weighed and dissolved in methanol (40 mL), and then wetted palladium on carbon (300 mg) was added. The mixture was purged three times with hydrogen balloons, and reacted at room temperature for 12 h. The reaction mixture was concentrated to dryness and purified by silica gel column chromatography to obtain 2.1 g of target product (yield: 80%).
LC/MS: m/z = 286.1 $[M+Na]^+$.

Step 5: Synthesis of 2-methylpropan-2-yl [(2-hydroxycyclopropyl)amino]formate

2-methylpropan-2-yl {[2-(benzyloxy)cyclopropyl] amino } formate (2 g, 7.6 mmol) was weighed and dissolved in methanol (15 mL), and wet palladium carbon (250 mg) was added. The reaction mixture was purged three times with hydrogen balloons. The reaction mixture was then reacted at 40°C for 12 h. The reaction mixture was concentrated to dryness to obtain 1.1 g of target product (yield: 84%).
LC/MS: m/z = 174.1 $[M+H]^+$.

Step 6: Synthesis of 2-methylpropan-2-yl 3-methyl-2,3,4,5,5a,6a-hexahydrocyclopropano[1,2-b][1,4]oxazepine-5-carboxylate

3-chloro-2-chloromethylpropene (722 mg, 5.7 mmol) and NaH (524 mg, 13.1 mmol) were weighed and dissolved in DMF (12 mL). The reaction system was purged with nitrogen three times, and the reaction system was cooled to 0°C. 2-methylpropan-2-yl [(2-hydroxycyclopropyl)amino]formate (1g, 5.7mmol) was dissolved in DMF (4mL) and slowly added dropwise to the reaction system. After completion of the addition, the reaction system was allowed to warm to room temperature naturally and reacted for 4 h. The reaction system was quenched by slowly adding the reaction mixture to a saturated aqueous solution of ammonium chloride. Ethyl acetate was then added for phase separation. The organic phase was collected, washed three times with saturated brine, dried, and concentrated under reduced pressure, and then purified by silica gel column chromatography to obtain 560 mg of target product (yield: 43%).

Step 7: Synthesis of 2-methylpropan-2-yl 4,5,5a,6a-tetrahydro-2H-spiro[cyclopropa[1,2-*b*][1,4]oxazepine-3,2'-oxirane]-5-carboxylate

2-methylpropan-2-yl 3-methylidene-2,3,4,5,5a,6a-hexahydrocyclopropa[1,2-b][1,4]oxazepine-5-carboxylate (550 mg, 2.4 mmol) and m-chloroperbenzoic acid (619 mg, 3.6 mmol) were weighed and dissolved in DCM (8 mL). The reaction mixture was reacted at 25°C for 24 h. The reaction solution was filtered directly, and the filter cake was washed with a small amount of DCM. The filtrate was collected and washed once with saturated sodium bicarbonate aqueous solution and once with saturated brine. The organic phase was dried, concentrated under reduced pressure, and purified by silica gel column chromatography to obtain 420 mg of target product (yield: 72%).

Step 8: Synthesis of 2-methylpropan-2-yl 3-hydroxy-3-methyl-2,3,4,5,5a,6a-hexahydrocyclopropa[1,2-*b*][1,4]oxazepine-5-carboxylate

2-methylpropan-2-yl 4,5,5a,6a-tetrahydro-2*H*-spiro[cyclopropa[1,2-b][1,4]oxazepine-3,2'-oxlrane]-5-carboxylate (420 mg, 1.7 mmol) was weighed and dissolved in THF (6 mL). The reaction system was purged with nitrogen three times. The reaction system was cooled to 0°C. Then, $LiAlH_4$ (76 mg, 2.0 mmol) was added. After completion of the addition, the reaction system was allowed to warm to room temperature naturally and reacted for 3 h. The reaction system was slowly quenched by adding a saturated ammonium chloride aqueous solution, and then ethyl acetate was added for phase separation. The organic phase was collected, and washed once with saturated brine. The organic phase was dried, concentrated under reduced pressure, and purified by silica gel column chromatography to obtain 310 mg of target product (yield: 75%).

Step 9: Synthesis of 3-methyl-2,3,4,5,5a,6a-hexahydrocyclopropa[1,2-b][1,4]oxazepine-3-ol hydrochloride

2-methylpropan-2-yl 3-hydroxy-3-methyl-2,3,4,5,5a,6a-hexahydrocyclopropa[1,2-b][1,4]oxazepine-5-carboxylate (310 mg, 1.27 mmol) was weighed and dissolved in HCl/dioxane (4 M, 4 mL). The reaction system was reacted at room temperature for 3 h. The reaction system was directly concentrated under reduced pressure to obtain 180 mg of target product (yield: 85%).
LC/MS: m/z = 144.1 $[M+H]^+$.

Step 10: Synthesis of 2-methylpropan-2-yl ({3-cyano-4-[2,6-dichloro-8-fluoro-4-(3-hydroxy-3-methyl-2,3,4,5,5a,6a-hexahydrocyclopropa[1,2-b] [1,4]oxazepin-5-yl)quinazolin-7-yl]benzo[b]thiophen-2-yl}amino)formate

2-methylpropan-2-yl {[3-cyano-4-(2,6-dichloro-8-fluoro-4-hydroxyquinazolin-7-yl)benzo[b]thiophen-2-yl]amino}formate (500 mg, 0.99 mmol), DIEA (383 mg, 2.97 mmol), and 3-methyl-2,3,4,5,5a,6a-hexahydrocyclopropa[1,2-b] [1,4]oxazepine-3-ol hydrochloride (180 mg, 1.09 mmol) were weighed and dissolved in DCM (8 mL). Then, BOP-Cl (503 mg, 1.98 mmol) was added. The reaction system was reacted at room temperature for 4 h. DCM and water were added to the reaction system for layering. The organic phase was collected. The organic phase was dried, concentrated under reduced pressure, and purified by silica gel column chromatography to obtain 480 mg of target product (yield: 77%). LC/MS: m/z = 630.1 $[M+H]^+$.

Step 11: Synthesis of 2-methylpropan-2-yl ({4-[6-chloro-8-fluoro-4-(3-hydroxy-3-methyl-2,3,4,5,5a,6a-hexahydrocyclopropano[1,2-b][1,4]oxaze pin-5-yl)-2-({[3-(3,4,5-trifluorophenyl)-2,3,5,6,7,7a-hexahydro-1*H*-pyrrolizin-7a-yl]methyl}oxy)quin azolin-7-yl]-3-cyanobenzo[b]thiophen-2-yl}amino)formate (Trans isomer 2)

2-methylpropan-2-yl ({3-cyano-4-[2,6-dichloro-8-fluoro-4-(3-hydroxy-3-methyl-2,3,4,5,5a,6a-hexahydrocyclopropano[1,2-b][1,4]oxazepin-5-yl)quinazolin-7-yl]benzo[b]thiophen-2-yl}amino)formate (480 mg, 0.76 mmol) and [3-(3,4,5-trifluorophenyl)-2,3,5,6,7,7a-hexahydro-1H-pyrrolizin-7a-yl]methanol (Trans isomer2) (247 mg, 0.91 mmol) were weighed, and then DMF/THF (4 mL/4 mL) was added, followed by the addition of $CS_2CO_3$ (495 mg, 1.52 mmol) and DABCO (25 mg, 0.22 mmol). The reaction mixture was stirred at 45°C for 12 h. The reaction solution was poured into water, and extracted with ethyl acetate. The organic phase was dried, concentrated under reduced pressure, and purified by silica gel column chromatography to obtain 227 mg of target product (yield: 34%).
LC/MS: m/z=865.1 $[M+H]^+$.

Step 12: Synthesis of 2-amino-4-[6-chloro-8-fluoro-4-(3-hydroxy-3-methyl-2,3,4,5,5a,6a-hexahydrocyclopropa[1,2-*b*] [1,4]o xazepin-5-yl)-2-({[3-(3,4,5-trifluorophenyl)-2,3,5,6,7,7a-hexahydro-1*H*-pyrrolizin-7a-yl]methyl}oxy) quinazolin-7-yl]benzo[b]thiophene-3-carbonitrile (Trans isomer 2)

2-methylpropan-2-yl ({4-[6-chloro-8-fluoro-4-(3-hydroxy-3-methyl-2,3,4,5,5a,6a-hexahydrocyclopropa[1,2-*b*] [1,4]oxazepi n-5-y1)-2-({[3-(3,4,5-trifluorophenyl)-2,3,5,6,7,7a-hexahydro-1*H*-pyrrolizin-7a-yl]methyl}oxy)quinaz olin-7-yl]-3-cyanobenzo[b]thiophen-2-yl}amino)formate (Trans isomer 2) (220 mg, 0.25 mmol) was weighed and dissolved in DCM/TFA (2 mL/1 mL). The reaction was carried out at room temperature for 4 h. A saturated sodium bicarbonate aqueous solution was slowly added to the reaction system until the aqueous phase of the system was weakly alkaline. A small amount of DCM was added for extraction. The organic phase was collected, dried, concentrated under reduced pressure, and purified by pre-TLC to obtain 160 mg of target product (yield: 83%).
LC/MS: m/z=765.1 $[M+H]^+$.

Example 36

4-[4-(2,3,4,5,5a,6a-hexahydrocyclopropa[1,2-b][1,4]oxazepin-5-yl)-6-chloro-8-fluoro-2-({[3 -(3,4,5-trifluorophenyl)-2,3,5,6,7,7a-hexahydro-1H-pyrrolizin-7a-yl]methyl}oxy)quinazolin-7-yl]-2-a minobenzo[b]thiophene-3-carbonitrile (Trans isomer2)

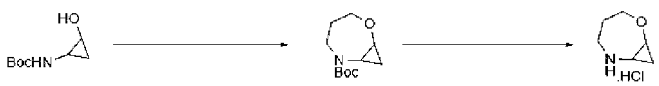

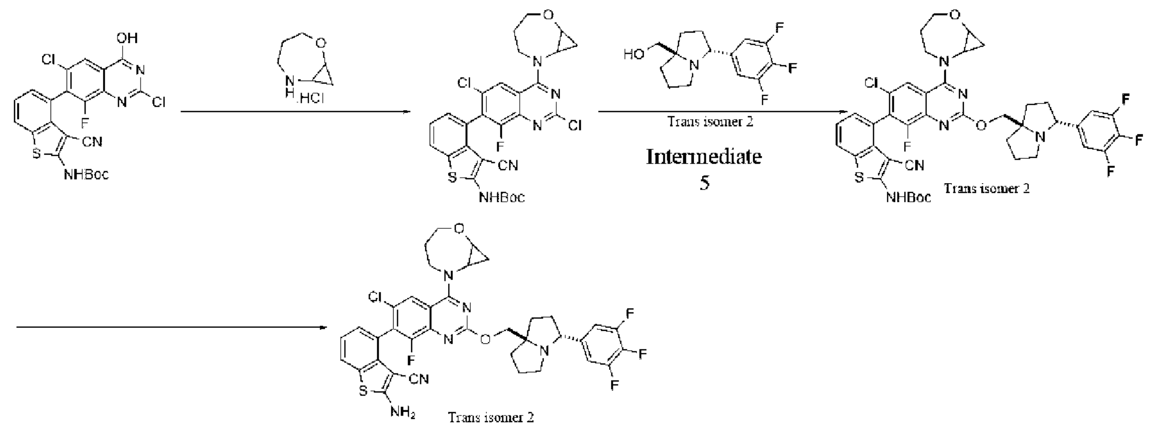


Step 1: Synthesis of 2-methylpropan-2-yl 2,3,4,5,5a,6a-hexahydrocyclopropa[1,2-b][1,4]oxazapyridine-5-carboxylate

1,3-dichloropropane (326 mg, 2.89 mmol) and NaH (254 mg, 6.35 mmol) were weighed and dissolved in DMF (6 mL). The mixture was purged with nitrogen three times, and the reaction system was cooled to 0°C. 2-methylpropan-2-yl [(2-hydroxycyclopropyl)amino]formate (500 mg, 2.89 mmol) was dissolved in DMF (4 mL) and then slowly added dropwise to the reaction system. After completion of the addition, the reaction system was allowed to warm to room temperature naturally and reacted for 8 h. The reaction system was quenched by slowly adding it to a saturated aqueous solution of ammonium chloride. Then, ethyl acetate was added for layering. The organic phase was collected, washed three times with saturated brine. The organic phase was dried, concentrated under reduced pressure, and purified by silica gel column chromatography to obtain 243 mg of target product, yield: 39%.

Step 2: Synthesis of 2,3,4,5,5a,6a-hexahydrocyclopropa[1,2-*b*][1,4]oxazepine hydrochloride

-2-methylpropan-2-yl 2,3,4,5,5a,6a-hexahydrocyclopropa[1,2-*b*][1,4] oxazepine-5-carboxylate (240 mg, 1.1 mmol) was weighed and dissolved in HCl/dioxane (4 M, 4 mL), and the reaction was carried out at room temperature for 3 h. The reaction mixture was directly concentrated under reduced pressure to obtain 156 mg of target product (yield: 96%).
LC/MS: m/z = 114.1 $[M+H]^+$.

Step 3: Synthesis of 2-methylpropan-2-yl ({4-[4-(2,3,4,5,5a,6a-hexahydrocyclopropa[1,2-*b*][1,4]oxazepin-5-yl)-2,6-dichloro-8-fluoroquinazolin -7-yl]-3-cyanobenzo[b]thiophen-2-yl}amino)formate

2-methylpropan-2-yl {[3-cyano-4-(2,6-dichloro-8-fluoro-4-hydroxyquinazolin-7-yl)benzo[*b*]thiophen-2-yl]amino}formate (500 mg, 0.99 mmol), DIEA (383 mg, 2.97 mmol), and 3-methyl-2,3,4,5,5a,6a-hexahydrocyclopropa[1,2-*b*][1,4]oxazepine-3-ol hydrochloride (150 mg, 1.01 mmol) were weighed and dissolved in DCM (8 mL). Then, BOP-Cl (503 mg, 1.98 mmol) was added. The mixture was reacted at room temperature for 4 h. DCM and water were added to the reaction system for phase separation. The organic phase was collected, dried, concentrated under reduced pressure, and purified by silica gel column chromatography to obtain 420 mg of target product (yield: 70%).
LC/MS: m/z = 600.1 $[M+H]^+$.

Step 4: Synthesis of 2-methylpropan-2-yl ({4-[4-(2,3,4,5,5a,6a-hexahydrocyclopropa[1,2-*b*][1,4]oxazepin-5-yl)-6-chloro-8-fluoro-2-({[3-(3,4,5-trifluorophenyl)-2,3,5,6,7,7a-hexahydro-1*H*-pyrrolizin-7a-yl]methyl}oxy)quinazolin-7-yl]-3-cyanoben zo[b]thiophen-2-yl}amino)formate (Trans isomer 2)

2-methylpropan-2-yl ({4-[4-(2,3,4,5,5a,6a-hexahydrocyclopropa[1,2-*b*][1,4]oxazepin-5-yl)-2,6-dichloro-8-fluoroquinazolin -7-yl]-3-cyanobenzo[b]thiophen-2-yl}amino)formate (420 mg, 0.7 mmol) and [3-(3,4,5-trifluorophenyl)-2,3,5,6,7,7a-hexahydro-1H-pyrrolizin-7a-yl]methanol (Trans isomer 2) (227 mg, 0.84 mmol) were weighed, and then DMF/THF (4 mL/4 mL) was added, followed by the addtion of $CS_2CO_3$ (456 mg, 1.4 mmol) and DABCO (22 mg, 0.2 mmol). The mixture was stirred at 45°C for 12 h. The reaction solution was poured into water, and then extracted with ethyl acetate. The organic phase was dried, concentrated under reduced pressure, and purified by silica gel column chromatography to obtain 189 mg of target product (yield: 32%).
LC/MS: m/z=835.1 $[M+H]^+$.

Step 5: Synthesis of 4-[4-(2,3,4,5,5a,6a-hexahydrocyclopropa[1,2-*b*][1,4]oxazepin-5-yl)-6-chloro-8-fluoro-2-({[3-(3,4,5-tri fluorophenyl)-2,3,5,6,7,7a-hexahydro-1*H*-pyrrolizin-7a-yl]methyl}oxy)quinazolin-7-yl]-2-aminobenz o[b]thiophene-3-carbonitrile (Trans isomer 2)

2-methylpropan-2-yl ({4-[4-(2,3,4,5,5a,6a-hexahydrocyclopropa[1,2-*b*][1,4]oxazepin-5-yl)-6-chloro-8-fluoro-2-({[3-(3,4,5-trifluorophenyl)-2,3,5,6,7,7a-hexahydro-1*H*-pyrrolizin-7a-yl]methyl}oxy)quinazolin-7-yl]-3-cyanoben zo[b]thiophen-2-yl}amino)formate (Trans isomer 2) (189 mg, 0.22 mmol) was weighed and dissolved in DCM/TFA (2 mL/1 mL). The reaction was carried out at room temperature for 4 h. A saturated sodium bicarbonate aqueous solution was slowly added to the reaction system until the aqueous phase of the system was weakly alkaline. A small amount of DCM was added for extraction. The organic phase was collected, dried, concentrated under reduced pressure, and purified by pre-TLC to obtain 135 mg of target product (yield: 83%).
LC/MS: m/z=735.1 $[M+H]^+$.

Example 37

Synthesis of 2-amino-4-[6-chloro-8-fluoro-4-(6-hydroxy-6-methyl-1,4-oxazepan-4-yl)-2-({[1-(3,4,5-trifluoropheny 1)octahydrocyclopenta[1,2-a][5]annulen-3a-yl]methyl}oxy)quinazolin-7-yl]thieno[2,3-c]pyridine-3-ca rbonitrile (Trans isomer 2)

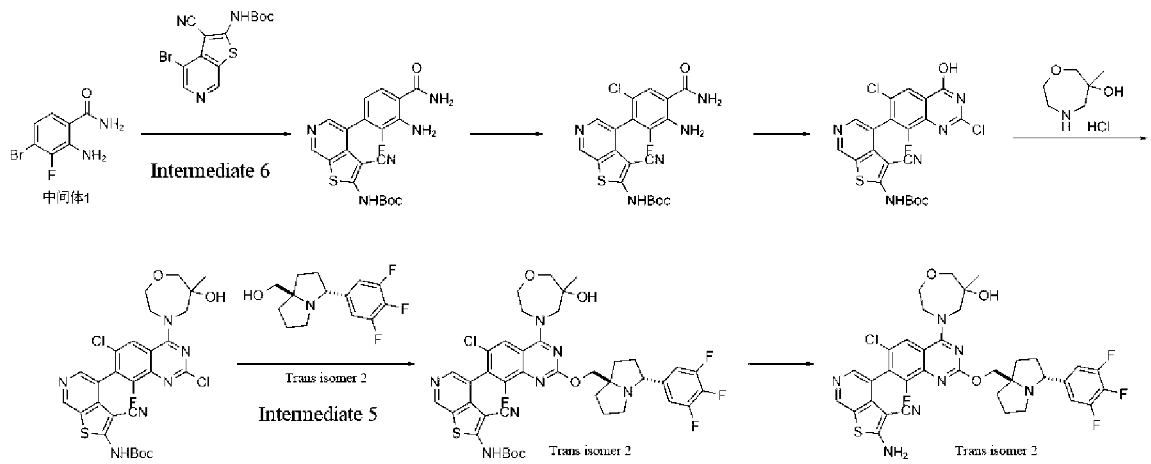


Step 1: Synthesis of 2-methylpropan-2-yl {[4-(3-amino-4-carbamoyl-2-fluorophenyl)-3-cyanothieno[2,3-c]pyridin-2-yl] amino}formate

2-amino-4-bromo-3-fluorobenzamide (437 mg, 1.88 mmol), pinacol diboronate (716 mg, 2.82 mol), and potassium acetate (368 mg, 3.76 mol) were added to a reaction flask, followed by the addition of a dioxane (20 mL) solution. After the system was purged with nitrogen three times, $Pd(OAc)_2$ (43 mg, 0.19 mmol) and n-butyldi(1-adamantyl) phosphine (68 mg, 0.19 mmol) were added. Then, the reaction system was heated to 85°C and reacted for 2 h. 2-methylpropan-2-yl [(4-bromo-3-cyanothieno[2,3-c]pyridin-2-yl)amino]formate (664 mg, 1.88 mmol), potassium carbonate (519 mg, 3.76 mol), and water (3 mL) were added. Finally, $Pd(OAc)_2$ (43 mg, 0.19 mmol) and n-butyldi(1-adamantyl) phosphine (68 mg, 0.19 mmol) were added to the system. The reaction mixture was heated to 85°C and reacted for 3 h under nitrogen. After the reaction system was cooled to room temperature, the reaction solution was poured into water (100 mL), and then extracted with ethyl acetate. The organic phase was dried, concentrated under reduced pressure, and purified by column chromatography (DCM/MeOH = 10/1) to obtain 286 mg of yellow solid (yield: 35.6%).
LC/MS: m/z=428.1 $[M+H]^+$.

Step 2: Synthesis of 2-methylpropan-2-yl {[4-(3-amino-4-carbamoyl-6-chloro-2-fluorophenyl)-3-cyanothieno[2,3-c]pyridin-2-yl]amino}formate

2-methylpropan-2-yl {[4-(3-amino-4-carbamoyl-2-fluorophenyl)-3-cyanothieno[2,3-c]pyridin-2-yl] amino}formate (286 mg, 0.67 mmol) was added to a reaction flask, and then DMF (10 mL) was added, followed by the slow addition of NCS (106 mg, 0.80 mmol). The system was reacted overnight at room temperature. The reaction system was poured into water, and extracted with ethyl acetate. The organic phase was dried and concentrated to obtain a crude product. The crude product was purified by silica gel column chromatography to obtain 198 mg of off-white solid (yield:

64.1%).
LC/MS: m/z = 462.1 $[M+H]^+$.

Step 3: Synthesis of 2-methylpropan-2-yl {[3-cyano-4-(2,6-dichloro-8-fluoro-4-hydroxyquinazolin-7-yl)thieno[2,3-c]pyridin-2-yl]amino}format e

2-methylpropan-2-yl {[4-(3-amino-4-carbamoyl-6-chloro-2-fluorophenyl)-3-cyanothieno[2,3-c]pyridin-2-yl]amino}formate (198 mg, 0.47 mmol) was dissolved in 1,4-dioxane (5 mL). After the reaction system was purged with nitrogen, a solution of 1,4-dioxane (2 mL) containing phosgene (162 mg, 0.78 mmol) was added dropwise at room temperature. After completion of the addition, the mixture was stirred at room temperature for 1 h, then heated to 80°C and stirred for 0.5 h. The system was added to water, extracted with ethyl acetate, dried, concentrated to dryness, and purified by silica gel column chromatography to obtain 96 mg of off-white solid (yield: 44.3%).
LC/MS: m/z=506.0 [M+H]+.

Step 4: Synthesis of 2-methylpropan-2-yl ({3-cyano-4-[2,6-dichloro-8-fluoro-4-(6-hydroxy-6-methyl-1,4-oxazepan-4-yl) quinazolin-7-yl]thieno[ 2,3-c]pyridin-2-yl}amino)formate

2-methylpropan-2-yl {[3-cyano-4-(2,6-dichloro-8-fluoro-4-hydroxyquinazolin-7-yl)thieno[2,3-c]pyridin-2-yl]amino}format e (96 mg, 0.19 mmol), 6-methyl-1,4-oxazepan-6-ol hydrochloride (38 mg, 0.23 mmol), DIPEA (74 mg, 0.57 mmol), and dichloromethane (4 mL) were added to a reaction flask. After the reaction system was purged with nitrogen, bis(2-oxo-3-oxazolyl)phosphine chloride (74 mg, 0.29 mmol) was added. The mixture was stirred at room temperature for 2 h. The reaction solution was poured into water (10 mL), and then extracted with dichloromethane. The organic phase was dried, concentrated under reduced pressure, and purified by silica gel thin-layer column chromatography to obtain 71 mg of target product (yield: 60.4%).
LC/MS: m/z=619.1 $[M+H]^+$.

Step 5: Synthesis of 2-methylpropan-2-yl ({4-[6-chloro-8-fluoro-4-(6-hydroxy-6-methyl-1,4-oxazepan-4-yl)-2-({[1-(3,4,5-trifluorophenyl)octah ydrocyclopenta[1,2-a][5]annulen-3a-yl]methyl}oxy)quinazolin-7-yl]-3-cyanothiopheno[2,3-c]pyridin-2-yl}amino)formate (Trans isomer 2)

2-methylpropan-2-yl ({3-cyano-4-[2,6-dichloro-8-fluoro-4-(6-hydroxy-6-methyl-1,4-oxazepan-4-yl)quinazolin-7-yl]thieno[ 2,3-c]pyridin-2-yl}amino)formate (71 mg, 0.12 mmol) and [3-(3,4,5-trifluorophenyl)-2,3,5,6,7,7a-hexahydro-1H-pyrrolizin-7a-yl]methanol (Trans isomer 2) (38 mg, 0.14 mmol) were added to a reaction flask, and then DMF/THF (2 mL/2 mL) was added, followed by the addition of $Cs_2CO_3$ (78 mg, 0.24 mmol) and DABCO (6 mg, 0.05 mmol). The mixture was stirred at 45°C for 6 h. The reaction solution was poured into water, and then extracted with ethyl acetate. The organic phase was dried, concentrated under reduced pressure, and purified by silica gel plate to give 39 mg of off-white solid (yield: 39.8%).
LC/MS: m/z = 854.2 $[M+H]^+$.

Step 6: Synthesis of 2-amino-4-[6-chloro-8-fluoro-4-(6-hydroxy-6-methyl-1,4-oxazepan-4-yl)-2-({[1-(3,4,5-trifluoropheny 1)octahydrocyclopenta[1,2-a][5]annulen-3a-yl]methyl}oxy)quinazolin-7-yl]thieno[2,3-c]pyridine-3-ca rbonitrile (Trans isomer 2)

2-methylpropan-2-yl ({4-[6-chloro-8-fluoro-4-(6-hydroxy-6-methyl-1,4-oxazepan-4-yl)-2-({[1-(3,4,5-trifluorophenyl)octah ydrocyclopenta[1,2-a][5]annulen-3a-yl]methyl}oxy)quinazolin-7-yl]-3-cyanothieno[2,3-c]pyridin-2-yl }amino)formate (Trans isomer 2) (39 mg, 0.05 mmol) was added to a reaction flask and dissolved in dichloromethane (3 mL). Trifluoroacetic acid (1 mL) was added, and the mixture was stirred at room temperature for 1 h. The solution was concentrated under reduced pressure and then added to a saturated aqueous sodium bicarbonate solution (5 mL). The mixture was extracted with ethyl acetate. The organic phase was dried over anhydrous sodium sulfate, filtered, concentrated under reduced pressure, and purified by silica gel plate to obtain 16 mg of target product (yield: 46.5%).
LC/MS: m/z = 754.2 $[M+H]^+$.

Drug Efficacy Test

**Test Example 1: Inhibitory activity test of compounds on KRAS G12D/G12V/G12C/G13D/G12R-mediated ERK phosphorylation**

1. Test method:

Day 1: AGS cells, SW480 cells, NCI H358 cells, HCT116 cells, and KP2 cells were seeded into 384-well cell culture plates and cultured overnight in a 37°C, 5% $CO_2$ incubator.

Day 2: The compounds were added to the plates using Echo550 and incubated for 3 h in a 37°C, 5% $CO_2$ incubator. Then, cells fixed with paraformaldehyde, permeated with methanol and blocked with a blocking solution were added. Finally, a mixed solution of primary antibodies (rabbit anti pERK, mouse anti GAPDH) was added, and the mixture was incubated overnight at 4°C.

Day 3: The mixed solution of primary antibodies was discarded, and a mixed solution of secondary antibodies (goat anti rabbit 800 CW, goat anti mouse 680 RD) was added. The resulting mixture was incubated at room temperature in the dark. Finally, the cell culture plate was washed with PBST, and then inverted for 1 minute of centrifugation. The fluorescence signal values were read with Odyssey CLx.

ERK phosphorylation was calculated according to the following formula:

Relative expression: (fluorescence signal ratio of compound - average value of positive control)/(average value of negative control - average value of positive control)

Negative control: DMSO
Positive control: 10 μM Reference
$IC_{50}$ value was fitted and calculated by XLFit 5.0 using the parametric formula.
Y = Bottom+(Top-Bottom)/(1+10^((LogIC50-X)*HillSlope))
X: log value of compound concentration.
Y: average value of relative expression of p-ERK between replicate wells.

2. The inhibitory activity of the compounds of the present disclosure on ERK phosphorylation is shown in Table 1.

Table 1: Inhibitory activity of the compounds of the present disclosure on ERK phosphorylation ($IC_{50}$, nm)

| Example | AGS-G12D | SW480-G12V | NCI H358-G12C | HCT116-G13D | KP2-G12R |
|---|---|---|---|---|---|
| **2** | >1000 | | | | |
| **3** | >1000 | | | | |
| **5** | 313 | | | | |
| **6** | 787 | | | | |
| **7** | 738 | | | | |
| **8** | >1000 | | | | |
| **9** | 27 | | | | |
| **10** | | >1000 | | | |
| **11** | | >1000 | | | |
| **12** | 3.3 | 2.4 | 3.1 | 63 | 148 |
| **13** | | 16 | | | |
| **14** | 758 | | | | |
| **15** | >1000 | | | | |
| **16** | 380 | | | | |
| **17** | 41 | | | | |
| **18** | 33 | | | | |
| **19** | >1000 | | | | |
| **20** | >1000 | | | | |
| **21** | 4.7 | 7.8 | 2.04 | 45 | 146 |
| **22** | 43 | | | | |

(continued)

| Example | AGS-G12D | SW480-G12V | NCI H358-G12C | HCT116-G13D | KP2-G12R |
|---|---|---|---|---|---|
| **23** | 4.2 | | 4.9 | 94 | 663 |
| **24** | 106 | | | | |
| **25** | >1000 | | | | |
| **26** | 2.7 | | | | |
| **27** | 355 | | | | |
| **28** | 4.2 | | | | |
| **29** | 33 | | | | |
| **30** | 94 | | | | |
| **31** | 1.9 | | | | |
| **32** | 24 | | | | |
| **33** | 196 | | | | |
| **34** | 67 | | | | |
| **37** | 193.01 | | | | |

Conclusion: From the above data, it can be seen that some compounds of the present disclosure can effectively inhibit the phosphorylation of the downstream target ERK of the KRAS pathway, and have broad use prospects.

Test Example 2: 3D Cell Proliferation Inhibition Test of Compounds

The diluted compounds to be tested were added to 384-well low-adhesion KRAS mutant types (such as GP2D, NCI H358, LoVo, RKN and KP2) of cell culture plates by using a nanoliter pipetting system (LABCYTE, P-0200). Then, the cells were plated and incubated in a 37°C, 5% $CO_2$ incubator for 7 days. Afterward, CellTiter-Glo® 3D reagent was added, and the luminescent values were read by an Envision multifunctional microplate reader (the luminescent signal is proportional to the amount of ATP in the system, and the ATP content directly represents the number of viable cells in the system). Finally, the $IC_{50}$ values (half-maximal inhibitory concentration) of the compounds were obtained by the XLFIT software using the nonlinear fitting formula.

1. Experimental Method

a) The diluted compounds to be tested were added to 384-well low-adhesion KRAS mutant types of cell culture plates by using a nanoliter pipetting system.
b) Cells were seeded into the 384-well plates in a) and then centrifuged at 1000 rpm for 1 min at room temperature, with a final DMSO concentration of compound being 0.5%. The plates were then incubated in a 5% $CO_2$ constant-temperature incubator at 37°C for 7 days.
c) CellTiter-Glo® 3D was added to the 384-well cell culture plates in b), shaken in the dark, and incubated at room temperature.
d) The luminescence values were read by using an Envision multifunctional microplate reader.

2. Data Analysis

The $IC_{50}$ (half-maximal inhibitory concentration) values of the compounds were obtained by using XLFIT software according to the following nonlinear fitting formula:

$$Y=Bottom+(Top-Bottom)/(1+10^{\wedge}((LogIC_{50}-X)\times HillSlope))$$

X: log value of compound concentration.
Y: inhibition rate (%)

Inhibition rate (%)=100×(average value of negative control - compound)/(average value of negative control - average value of positive control)

Negative control: DMSO.
Positive control: Medium only.

3. The 3D cell proliferation inhibition activity data of some compounds of the present disclosure on various mutant cells such as GP2D, NCI H358, LoVo, RKN and KP2 are shown in Table 2.

Table 2: Inhibitory activity of the compounds of the present disclosure on cell proliferation of various KRAS mutant subtypes ($IC_{50}$, nm)

| Example | GP2D (KRASG12D) | NCI H358 (KRAS G12C) | LoVo (KRAS G13D) | RKN (KRAS G12V) | KP2 (KRAS G12R) |
|---|---|---|---|---|---|
| **21** | 4.26 | / | / | 7.23 | / |
| **26** | 2.99 | 1.03 | 14.22 | 4.32 | 56.62 |
| **31** | 1.75 | 0.69 | 20.59 | 3.69 | 59.12 |

Experimental conclusion: some compounds of the present disclosure exhibit excellent anti-proliferative effects on mutant cells of various KRAS subtypes, including GP2D (KRAS G12D), NCIH358 (KRAS G12C), RKN (KRAS G12V), LoVo (KRAS G13D), and KP2 (KRAS G12R).

Test Example 3: In vivo pharmacokinetic test of compounds in mice

Each test compound was administered orally once to female BALB/c mice for pharmacokinetic study. The test compounds were prepared on the day of administration. The oral administration solvent: 50 mM sodium citrate (pH 5.0) + 10% captisol. The oral administration was conducted in a dose of 30 mg/kg. The animals were fasted for 10-14 h before oral administration, and the feeding was resumed 4 h after administration. Pharmacokinetic samples were collected through the orbit. The collection time points were: 15 min, 30 min, 1 h, 2 h, 4 h, 6 h, 8 h and 24 h after administration. Three whole blood samples were collected at each time point, with a collection volume of about 0.2 mL, and anticoagulated with sodium heparin. After blood samples were collected, they were immediately placed on ice and centrifuged within 1 h to isolate plasma (centrifugation conditions: 8000 rpm, 1 min, 2-8°C). The collected plasma was stored in a -20°C refrigerator before analysis. The concentrations of compounds in the plasma samples were determined using an LC-MS/MS analysis method.

Table 3: Pharmacokinetic test results of some compounds on BALB/c mice

| Example | Cmax (ng/mL) | AUC (ng/mL*h) |
|---|---|---|
| 9 | 1897 | 5181 |
| 12 | 5544 | 37427 |
| 17 | 1493 | 3991 |
| 18 | 2148 | 6017 |
| 21 | 5218 | 30872 |
| 23 | 3618 | 9227 |
| 26 | 4884 | 15796 |
| 28 | 3290 | 9224 |
| 31 | 5124 | 20953 |

Experimental conclusion: the compounds in some examples of the present disclosure have good oral exposure levels in mice.

Test Example 4: In vivo pharmacokinetic test of compounds in rats

Each test compound was administered orally once to male BALB/c rats for pharmacokinetic study. The test compounds were prepared on the day of administration. The intravenous administration solvent: 5% DMSO + 10% solutol + 85% saline; and the oral administration solvent: 50 mM sodium citrate (pH 5.0) + 10% captisol. The oral administration was conducted in a dose of 30 mg/kg. The animals were fasted for 10-14 h before oral administration, and the feeding was resumed 4 h after administration. Pharmacokinetic samples were collected through the orbit. The collection time points were: 5 min (intravenous administration), 15 min, 30 min, 1 h, 2 h, 4 h, 6 h, 8 h and 24 h after administration. Three whole blood samples were collected at each time point, with a collection volume of about 0.2 mL, and anticoagulated with sodium heparin. After blood samples were collected, they were immediately placed on ice and centrifuged within 1 h to isolate plasma (centrifugation conditions: 8000 rpm, 1 min, 2-8°C). The collected plasma was stored in a -20°C refrigerator before analysis. The concentrations of compounds in the plasma samples were determined using an LC-MS/MS analysis method.

Table 4: Pharmacokinetic test results of some compounds in SD Rats

| Example | Cmax (ng/mL) | AUC (ng/mL*h) | F(%) |
|---|---|---|---|
| 21 | 866 | 5287 | / |
| 26 | 1186 | 12373 | 22.3 |
| 31 | 1032 | 11251 | 21.5 |

Experimental conclusion: the compounds in some examples of the present disclosure have good oral exposure and bioavailability in rats.

Test Example 5: In vivo drug efficacy study of compounds in mice

Test method:

A human colon cancer GP2D cell subcutaneous xenograft tumor Balb/c nude mouse model was established. GP2D tumor cells resuspended in DMEM medium were inoculated subcutaneously on the right side of the experimental animal at $1\times10^7$ + gel/0.1 mL. When the tumor grew to about 150-200 $mm^3$, administration in groups began, with 5 animals in each group. On the day of the experiment, the animals were given corresponding drugs according to the groups. The first group G1 was set as a solvent group, and 50 mM citrate buffer (pH 5.0) + 10% Captisol was given by gavage alone. The second group G2 was given a 50 mM citrate buffer (pH 5.0) + 10% Captisol of compound 26. The third group G3 was given a 50 mM citrate buffer (pH 5.0) + 10% Captisol of compound 31. The dose levels and administration regimens are shown in Table 5.

Table 5: Study on effect of test substances on animal tumor size in human colon cancer GP2D mouse xenograft tumor model

| Group | Animal number | Test substance | Dosage (mg/kg) | Route of administration | Dosing frequency |
|---|---|---|---|---|---|
| G1 | 5 | Solvent | / | PO | BID × 28 |
| G2 | 5 | Compound 26 | 30 | PO | BID × 28 |
| G3 | 5 | Compound 31 | 30 | PO | BID × 28 |

Note: PO indicates oral administration, and BID indicates twice daily.
During the experiment, the body weight and tumor size of the animals were measured twice a week, and the clinical symptoms of the animals were observed and recorded every day. Each drug administration was based on the most recent weighing of the animals.
The tumor was measured with a digital vernier caliper to measure the length (a) and width (b). The tumor volume (TV) was calculated using the following formula:

$$TV = a \times b^2/2.$$

Experimental results:

Compound 26 and compound 31 had significant inhibitory effects on human colon cancer GP2D mouse xenograft tumors. After 28 days of administration, the tumor volume inhibition rate TGI (%) of the second group G2 (30 mg/kg, PO, BID) was 72.7 on the 28th day; the tumor volume inhibition rate TGI (%) of the third group G3 (30 mg/kg, PO, BID) was 86.7 on the 28th day. The detailed results are shown in Table 6.

Table 6: Effects of test substances on animal tumor size in human colon cancer GP2D mice xenograft tumor model

| Group | Animal number | Test substance | Dose (mg/kg) | Route and frequencyof administration | Tumor volume inhibition rate (TGI) (%) |
|---|---|---|---|---|---|
| G1 | 5 | Solvent | / | PO, BID × 28 | / |
| G2 | 5 | Compound 26 | 30 | PO, BID × 28 | 72.7 |
| G3 | 5 | Compound 31 | 30 | PO, BID × 28 | 86.7 |

Note: "/" indicates not detected.

Experimental conclusion: in terms of in vivo drug efficacy, the compounds in some examples of the present disclosure showed good anti-tumor effects in the GP2D mice drug efficacy study.

Test Example 6: In vivo drug efficacy study of compounds in mice

Test method:

A subcutaneous xenograft tumor model of human ovarian cancer RKN (derived from uterine leiomyosarcoma) cells was established in Balb/c nude mice. RKN tumor cells resuspended in Ham's F12 medium were inoculated subcutaneously on the right side of the experimental animals at $5\times10^6$ + gel/0.1 mL. When the tumor grew to about 150-200 $mm^3$, 20 animals having relatively uniform tumor volumes were selected for group administration, with a total of 4 groups, 5 animals in each group. On the day of the experiment, the animals were given the corresponding drugs according to the groups. The first group G1 was set as a solvent group, and 50 mM citrate buffer (pH 5.0) + 10% Captisol was given by gavage alone. The second group G2 was given a 50 mM citrate buffer (pH 5.0) + 10% Captisol of compound 21. The third group G3 was given a 50 mM citrate buffer (pH 5.0) + 10% Captisol of compound 26. The fourth group G4 was given a 50mM citrate buffer (pH 5.0) + 10% Captisol of compound 31. The dosage and schedule of administration are shown in Table 7.

Table 7: Study on effect of test substances on animal tumor size in the human ovarian cancer RKN mouse xenograft tumor model

| Group | Animal number | Test substance | Dose (mg/kg) | Route of administration | Dosing frequency |
|---|---|---|---|---|---|
| G1 | 5 | Solvent | / | PO | BID × 28 |
| G2 | 5 | Compound 21 | 50 | PO | BID × 28 |
| G3 | 5 | Compound 26 | 50 | PO | BID × 28 |
| G4 | 5 | Compound 31 | 50 | PO | BID × 28 |

Note: PO indicates oral administration, and BID indicates twice daily.

During the experiment, the body weight and tumor size of the animals were measured twice a week, and the clinical symptoms of the animals were observed and recorded every day. Each drug administration was based on the most recent weighing of the animals.

The tumor was measured with a digital vernier caliper to measure the length (a) and width (b). The tumor volume (TV) was calculated using the following formula:

$$TV = a \times b^2/2.$$

Experimental results:

Compound 21, Compound 26 and Compound 31 had significant inhibitory effects on human ovarian cancer RKN mouse xenograft tumors. After 28 days of administration, the tumor volume inhibition rate TGI (%) of the second group G2 (50 mg/kg, PO, BID) was 109 on the $28^{th}$ day; the tumor volume inhibition rate TGI (%) of the third group G3 (50 mg/kg, PO, BID) was 116 on the $28^{th}$ day; the tumor volume inhibition rate TGI (%) of the fourth group G4 (50 mg/kg, PO, BID) was 116 on the $28^{th}$ day. The detailed results are shown in Table 8.

Table 8: Effects of test substances on animal tumor size in the human ovarian cancer RKN mouse xenograft tumor model

| Group | Animal number | Test substance | Dose (mg/kg) | Routeand frequency of administration | Tumor volume inhibition rate (TGI) (%) |
|---|---|---|---|---|---|
| G1 | 5 | Solvent | / | PO, BID × 28 | / |
| G2 | 5 | Compound 21 | 50 | PO, BID × 28 | 109 |
| G3 | 5 | Compound 26 | 50 | PO, BID × 28 | 116 |
| G4 | 5 | Compound 31 | 50 | PO, BID × 28 | 116 |

Experimental conclusion: in terms of in vivo drug efficacy, the compounds in some examples of the present disclosure showed good anti-tumor effects in the RKN mice drug efficacy study.

The above-mentioned compound provided by the present disclosure can be used to treat one or more cancers caused by KRAS mutation, has high in vitro cell proliferation and ERK phosphorylation inhibitory activity, has good exposure and bioavailability in mice and rats, shows excellent anti-tumor effect in vivo drug efficacy study in mice, can be used to prepare drugs for preventing and/or treating KRAS-mediated diseases, and has good use prospects.

The above are only preferred embodiments of the present disclosure. It should be noted that the above preferred embodiments should not be regarded as limiting the present disclosure, and the protection scope of the present disclosure should be based on the scope defined by the claims. For ordinary technicians in this technical field, several improvements and modifications can be made without departing from the spirit and scope of the present disclosure, and these improvements and modifications should also be regarded as the protection scope of the present disclosure.

## Claims

1. A compound represented by Formula I, or a tautomer, a mesomer, a racemate, an enantiomer, a diastereoisomer or a mixture thereof, a metabolite, a metabolic precursor, an isotope substitution form, a pharmaceutically acceptable salt, a hydrate, a solvate, a polymorph or a cocrystal thereof:

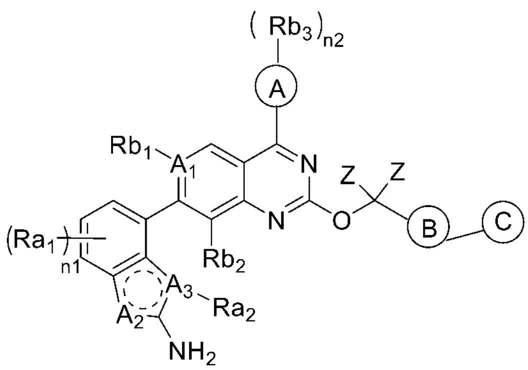

I

wherein,

$A_1$ is selected from C or N;
$A_2$ is selected from S, N or C; $A_3$ is selected from N, O or C; and $A_2$ and $A_3$ are not C at the same time;
Z at each occurrence is independently selected from H or deuterium;

$Ra_1$ is selected from H, halogen, $C(CH_3)=CH-$ or $-CH=CH_2$, $-CF=CH_2$, -CN, OH, $NH_2$, $-CF_3$, $-CHF_2$, $-CH_2F$, $-CF_2CH_3$, $-CF_2CF_3$, $-OCHF_2$, $-OCF_3$, aroyl, ethynyl, propynyl, butynyl, pentynyl, hexynyl, cyclopropyl, cyclopentenyl, cyclohexyl, cycloheptyl, methyl, ethyl, propyl, butyl, pentyl, vinyl, propenyl, allyl, butenyl, butenyl, pentadienyl, dipentenyl, hexenyl, hexadienyl, methoxy, ethoxy, propoxy, isopropoxy, butoxy, tert-butoxy, pentyloxy, or hexyloxy;

$n_1$ is an integer from 0 to 3;

$R_{a2}$ is selected from -CN, H, or absent;

$Rb_1$, $Rb_2$, and $Rb_3$ are selected from absent, hydrogen, deuterium, halogen, substituted or unsubstituted alkyl or cycloalkyl, substituted or unsubstituted heteroalkyl or heterocycloalkyl, substituted or unsubstituted alkoxy, hydroxyl, cyano, amino, ester group, amide, aryl, heteroaryl, acylguanidyl, sulfonyl, phosphoryl, sulfonic acid group, and phosphate group, wherein the substituents are each independently selected from deuterium, halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group;

$n_2$ is an integer from 0 to 6;

Ring A is selected from one of the following substituted or unsubstituted structures:

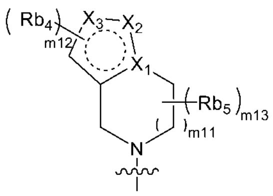
,
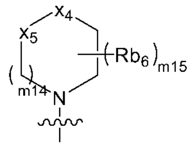
,
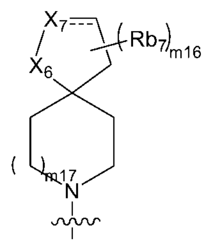
,
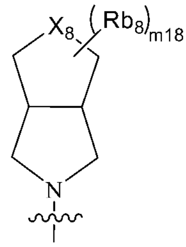
,

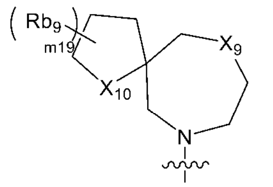
,
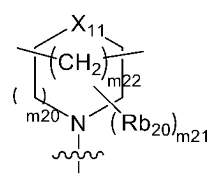
,
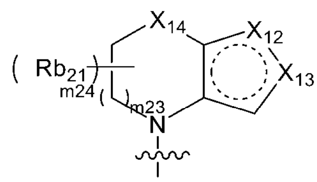
,
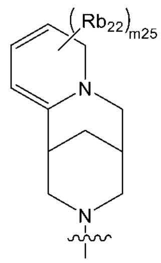
,

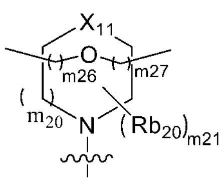
,
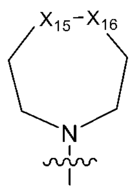
, and
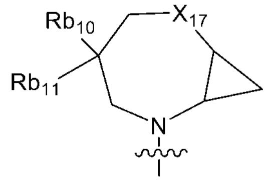
;

$X_1$ and $X_2$ are each independently selected from N or C; $X_3$ is selected from N, O, or C; and only one of $X_1$, $X_2$, and $X_3$ is C; $Rb_4$ and $Rb_5$ at each occurrence are each independently selected from absent, H, halogen, deuterium, substituted or unsubstituted alkyl, hydroxyl, cyano, $-C(=O)NRb_{41}Rb_{42}$, $-(CH_2)m_{121}C(=O)NRb_{41}Rb_{42}$, $-(CH_2)m_{121}NRb_{41}Rb_{42}$, $-NRb_{41}Rb_{42}$, $-C(=O)Rb_{41}$, $-C(=O)ORb_{41}$; $Rb_{41}$ and $Rb_{42}$ are each independently selected from H, substituted or unsubstituted alkyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{11}$ is an integer from 0 to 3; and $m_{12}$, $m_{13}$, and $m_{121}$ are each independently an integer from 0 to 6;

$X_4$ and $X_5$ are each independently selected from N, O, or C; $Rb_6$ at each occurrence is independently selected from absent, H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{14}$ is an integer from 0 to 3; and $m_{15}$ is an integer from 0 to 6;

$X_6$ is selected from N or O; $X_7$ is selected from N, O or C; $Rb_7$ at each occurrence is independently selected from absent, H, halogen, deuterium, substituted or unsubstituted alkyl, hydroxyl, cyano, carbonyl, $-C(=O)NRb_{41}Rb_{42}$, $-(CH_2)m_{121}C(=O)NRb_{41}Rb_{42}$, $-(CH_2)m_{121}NRb_{41}Rb_{42}$, $-NRb_{41}Rb_{42}$, $-C(=O)Rb_{41}$, $-C(=O)ORb_{41}$; $Rb_{41}$ and $Rb_{42}$ are each independently selected from H, substituted or unsubstituted alkyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{16}$ is an integer from 0 to 6; and $m_{17}$ is an integer from 0 to 3;

$X_8$ is selected from N, O, or C; $Rb_8$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{18}$ is an integer from 0 to 6;

$X_9$ and $X_{10}$ are each independently selected from N or O; $Rb_9$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{19}$ is an integer from 0 to 6;

$X_{11}$ is selected from N, O, or C; $Rb_{20}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{21}$ is an integer from 0 to 6; and $m_{20}$ and $m_{22}$ are integers from 0 to 3;

$X_{12}$, $X_{13}$, and $X_{14}$ are each independently selected from N or O; $Rb_{21}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; $m_{23}$ is an integer from 1 to 3; and $m_{24}$ is an integer from 0 to 6;

$Rb_{22}$ at each occurrence is independently selected from H, deuterium, halogen, hydroxyl, cyano, amino, alkoxy, sulfonyl, phosphoryl, sulfonic acid group, phosphate group, carbonyl, substituted or unsubstituted: alkyl or cycloalkyl, heteroalkyl or heterocycloalkyl, ester group, acyl, amide, aryl, heteroaryl, and acylguanidyl, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group; and $m_{25}$ is an integer from 0 to 6;

$m_{26}$ and $m_{27}$ are integers from 0 to 3;

$X_{15}$ and $X_{16}$ are each independently selected from O and -NH;

$X_{17}$ is selected from O or N; $Rb_{10}$ and $Rb_{11}$ are independently selected from -OH, $-CH_3$, and H;

Ring B has the following structure:

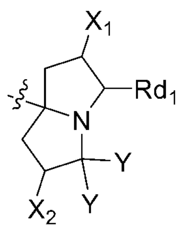
,

wherein $X_1$ and $X_2$ are independently selected from hydrogen, deuterium, and halogen; Y is selected from hydrogen or deuterium; and $Rd_1$ is Ring C;

the Ring C is selected from one of the following:

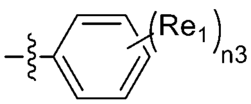, 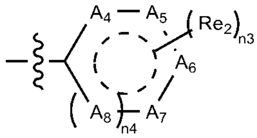, and 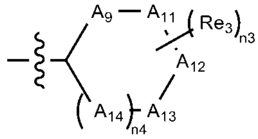

$Re_1$, $Re_2$, and $Re_3$ at each occurrence are each independently selected from -H, -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl, substituted or unsubstituted alkoxy, alkynyl, ester group, amide, aryl, heteroaryl, acylguanidyl, sulfonyl, phosphoryl, sulfonic acid group, and phosphate group, wherein the substituents are each independently selected from halogen, alkyl, cycloalkyl, heteroalkyl, heterocycloalkyl, hydroxyl, cyano, amino, ester group, amide, aryl, heteroaryl, acylguanidyl, sulfonyl, phosphoryl, sulfonic acid group, and phosphate group;

$n_3$ at each occurrence is independently an integer from 0 to 5; $n_4$ at each occurrence is independently selected from 0 or 1.

or, when $n_3$ is greater than or equal to 2, two adjacent $Re_1$, $Re_2$ or $Re_3$ together with atoms connected therebetween also form a 4 to 7-membered ring;

$A_4$ to $A_8$ are each independently selected from a heteroatom or C, and are not all C, the number of heteroatoms in $A_4$ to $A_8$ is 1, 2 or 3, and the heteroatoms are selected from N and/or O;

$A_9$ to $A_{14}$ are each independently selected from a heteroatom or C, and are not all C, the number of heteroatoms in $A_9$ to $A_{14}$ is 1 or 2, and the heteroatoms are selected from N and/or O;

the substituents of the Ring A, Ring B, and Ring C are selected from -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl, alkoxy, alkynyl, ester group, amide, aryl, heteroaryl, acylguanidyl, sulfonyl, phosphoryl, sulfonic acid group, and phosphate group; and

the aforementioned alkyl is selected from $C_{0-10}$ alkyl.

2. The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein the substituted alkyl is selected from $-CF_3$ and $-C_2F_5$.

3. The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein $X_4$ and $X_5$ are each independently selected from N, O or C, and are not simultaneously O;

$X_6$ is selected from N or O; $X_7$ is selected from N, O or C, and $X_6$ and $X_7$ are not simultaneously O; and $X_{12}$, $X_{13}$, and $X_{14}$ are each independently selected from N or O, and $X_{12}$ and $X_{13}$ are not simultaneously O.

4. The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein $Re_1$, $Re_2$, and $Re_3$ at each occurrence are each independently selected from -H, -OH, halogen, $-NO_2$, -CN, $-CF_3$, $-C_2F_5$, $-OCF_3$, $-OCHF_2$, $-OCH_2F$, phosphate group, monomethyl phosphate group, dimethyl phosphate group, linear or branched $C_{1-5}$ alkyl, $C_{1-5}$ alkoxy, $C_{3-10}$ cycloalkyl, $C_{3-10}$ heterocycloalkyl, -N ($C_{0-10}$ alkyl)($C_{0-10}$ alkyl), -S ($C_{0-10}$ alkyl), -OCON ($C_{0-10}$ alkyl)($C_{0-10}$ alkyl), $C_{5-6}$ aryl, five-membered heteroaryl, and six-membered heteroaryl;

or, when $n_3$ is greater than or equal to 2, two adjacent $Re_1$, $Re_2$ or $Re_3$ together with atoms connected therebetween also form saturated or unsaturated $C_{3-8}$ cycloalkyl, saturated or unsaturated $C_{3-8}$ heterocycloalkyl, saturated or unsaturated cyclolactone, $C_{5-6}$ aryl, bridged cycloalkyl, and spirocycloalkyl, wherein the H in the above-mentioned groups is substituted by -D, -OH, -F, $-NO_2$, -CN, $-CF_3$, $-C_2F_5$, $C_{1-3}$ alkyl, or amide.

5. The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein the Ring C is selected from:

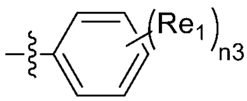

$Re_1$ at each occurrence is independently selected from -H, -OH, -F, -Cl, -CN, $-CF_3$, $-C_2F_5$, $-OCF_3$, monomethylphosphoryl, dimethylphosphoryl, linear or branched $C_{1-5}$ alkyl, $C_{1-5}$ alkoxy, $C_{3-10}$ cycloalkyl, $C_{3-10}$ heterocycloalkyl, -S ($C_{0-10}$ alkyl), -OCON ($C_{0-10}$ alkyl)($C_{0-10}$ alkyl), $C_{5-6}$ aryl, five-membered heteroaryl, and six-membered heteroaryl;

or, when $n_3$ is greater than or equal to 2, the two adjacent $Re_1$ together with carbon atoms therebetween form saturated or unsaturated $C_{3-8}$ cycloalkyl, saturated or unsaturated $C_{3-8}$ heterocycloalkyl, five-membered cyclolactone, $C_{5-6}$ aryl, bridged cycloalkyl, and spirocycloalkyl, wherein the H in the above-mentioned groups is substituted by -D, -OH, -F, $-NO_2$, -CN, $-CF_3$, $-C_2F_5$, $C_{1-3}$ alkyl, or amide.

**6.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 5, wherein when $n_3$ is greater than or equal to 2, the two adjacent $Re_1$ together with a benzene ring connected therebetween also form one of the following structures:

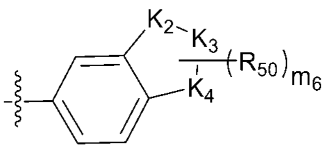 , 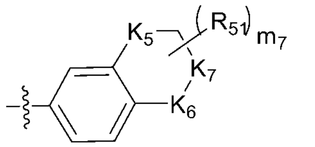 , 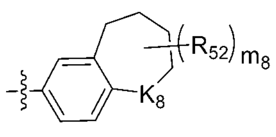 ,

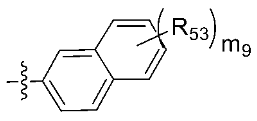 , 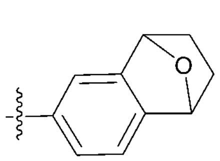 , 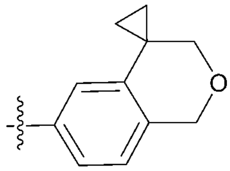 , 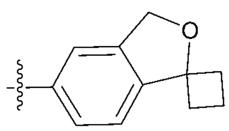 ,

and

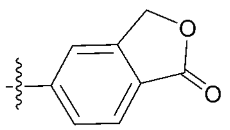 ;

wherein, $K_2$, $K_3$, and $K_4$ are fused with the benzene ring to form a saturated or unsaturated five-membered ring, $K_2$, $K_3$, and $K_4$ are independently selected from C, N, O, and S; $K_5$, $K_6$, and $K_7$ are independently selected from C, N, O, and S; $K_8$ is independently selected from C, N, O, and S; $R_{50}$, $R_{51}$, $R_{52}$, and $R_{53}$ are independently selected from -H, -D, -OH, -F, $-NO_2$, -CN, $-CF_3$, $-C_2F_5$, $C_{1-3}$ alkyl, $C_{1-3}$ alkoxy, and $C_{3-6}$ cycloalkyl; and $m_6$, $m_7$, $m_8$, and $m_9$ are integers from 0 to 6.

**7.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein the Ring C is selected from:

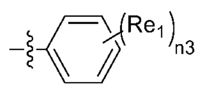

$Re_1$ at each occurrence is independently selected from halogen, $-CF_3$, $-C_2F_5$, $-OCF_3$, $-OCHF_2$, and $-OCH_2F$.

**8.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein

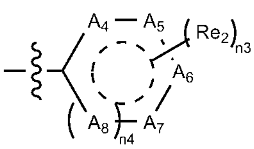 is selected from 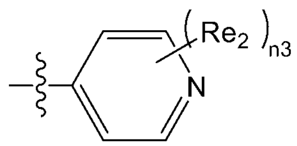 , 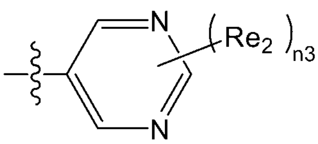 ,

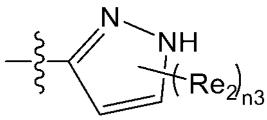, and 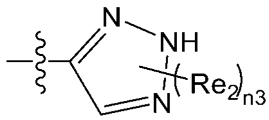;

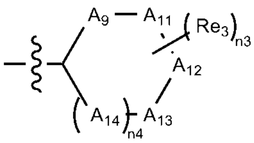 is selected from 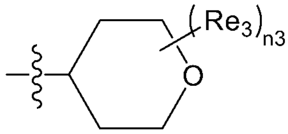, 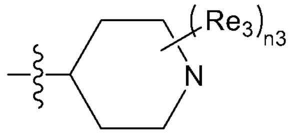,

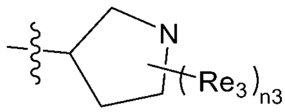, and 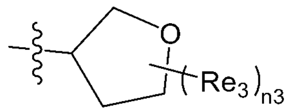.

**9.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein

in

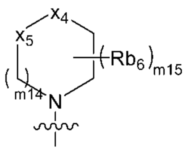,

$X_4$ and $X_5$ are selected from one of the following combinations:

(1) $X_4$ is O, and $X_5$ is C;
(2) $X_4$ and $X_5$ are C;
(3) $X_4$ is N, and $X_5$ is C;
(4) $X_4$ is O, and $X_5$ is N;

$m_{14}$ is selected from 0, 1, or 2;
in

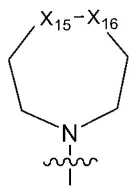,

$X_{15}$ and $X_{16}$ are each independently selected from O and -NH, and different from each other;
in

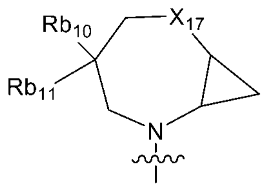,

$X_{17}$ is selected from O; $Rb_{10}$ and $Rb_{11}$ are selected from one of the following combinations:

(1) $Rb_{10}$ and $Rb_{11}$ are each independently selected from -OH and $-CH_3$, and different from each other;

(2) $Rb_{10}$ and $Rb_{11}$ are selected from H.

**10.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 9, wherein

in

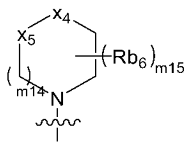,

$m_{14}$ is selected from 2, and $X_4$ is O and $X_5$ is C;
$m_{15}$ is selected from 2, and $Rb_6$ at each occurrence is independently selected from hydroxyl, substituted or unsubstituted $C_{1-3}$ alkyl, substituted or unsubstituted amide; and the substituents are each independently selected from halogen, alkyl, and hydroxyl.

**11.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 10, wherein

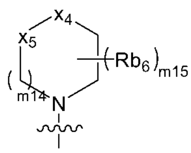

has the following structure:

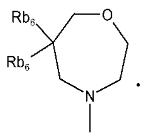.

**12.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 11, wherein $Rb_6$ at each occurrence is independently selected from hydroxyl, substituted or unsubstituted $C_{1-3}$ alkyl; and the substituents are each independently selected from halogen and hydroxyl.

**13.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein $A_1$ is selected from C; $A_2$ is selected from S; $A_3$ is selected from C; $Rb_1$ is selected from halogen, substituted or unsubstituted alkyl or cycloalkyl, -CN, and phosphoryl, wherein the substituents are each independently selected from deuterium and halogen; $Rb_2$ is selected from halogen; $Ra_1$ is selected from H and halogen; and $Ra_2$ is selected from -CN.

**14.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein $Rb_1$ is selected from Cl, Br, I, $-CF_3$, cyclopropyl, and -CN; $Rb_2$ is selected from F; and $Ra_1$ is selected from H, F, Cl, and Br.

**15.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein $n_1$ is selected from 1 or 2; $n_2$ is selected from 0; and $n_3$ is selected from 1, 2, 3, 4 or 5.

**16.** The compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein the Formula I is selected from one of the following:

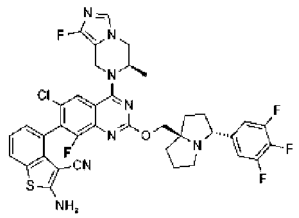
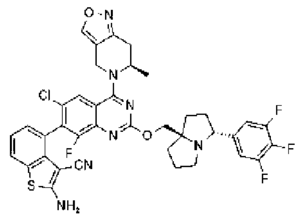
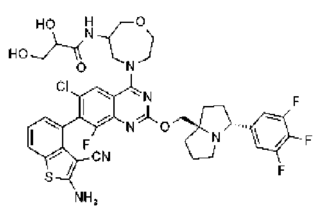

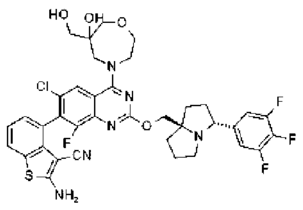
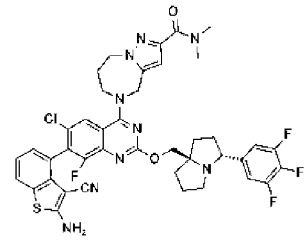

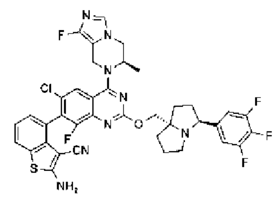
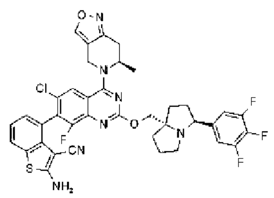
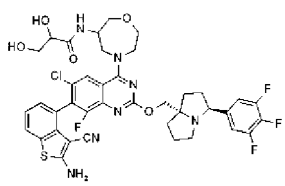

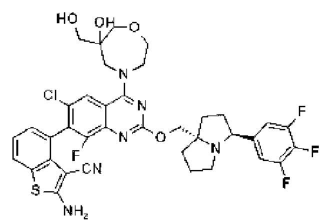
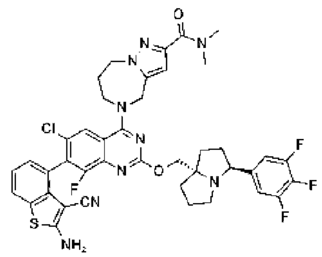

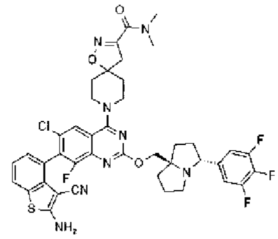
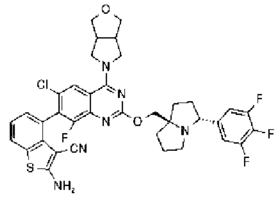
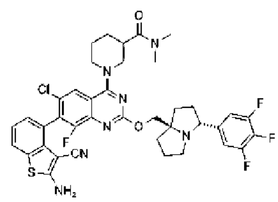

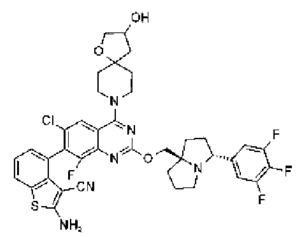
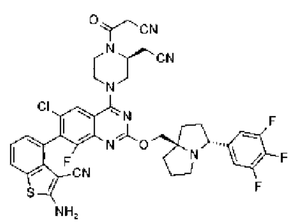

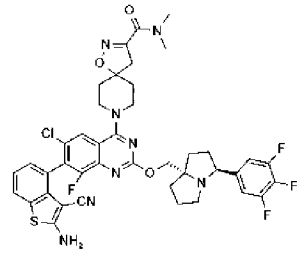
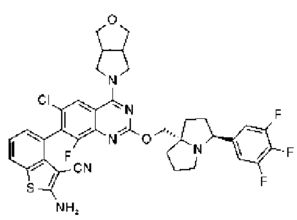
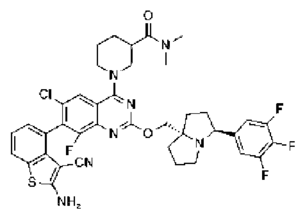

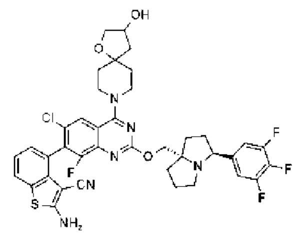
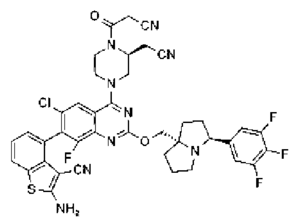

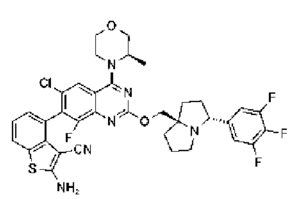
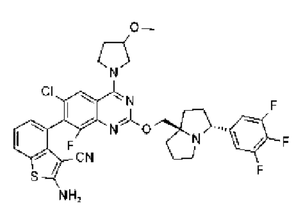
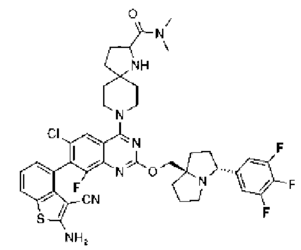

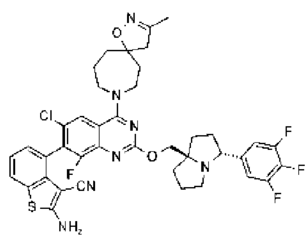
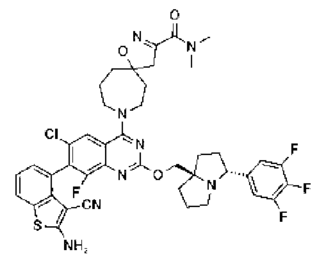

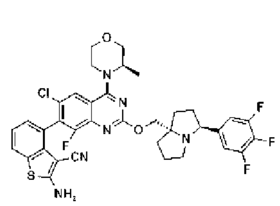
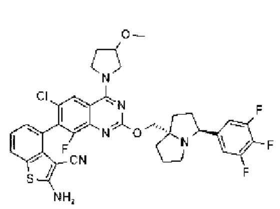
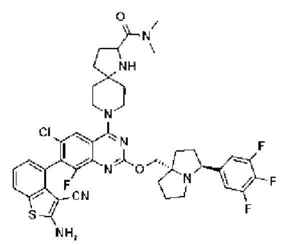

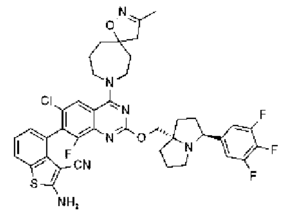
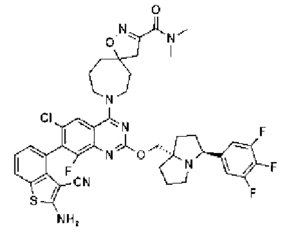

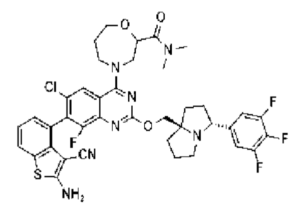
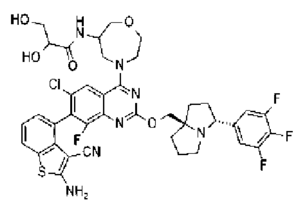
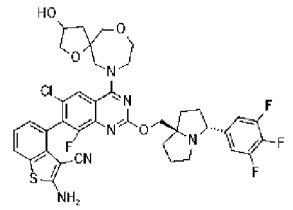

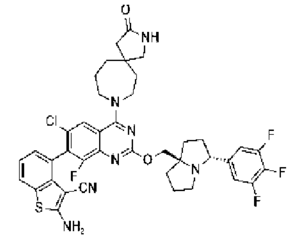
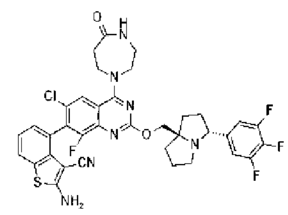

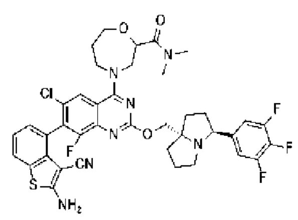
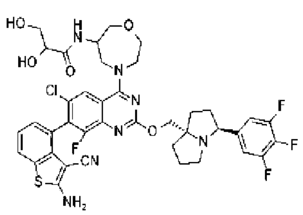
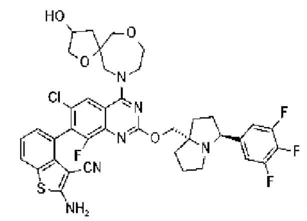

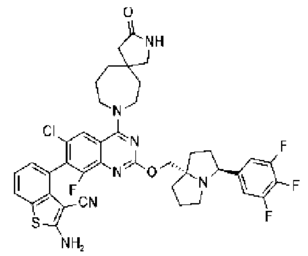
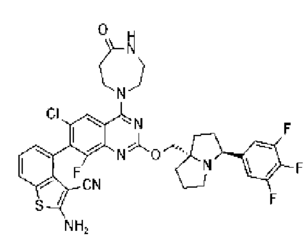

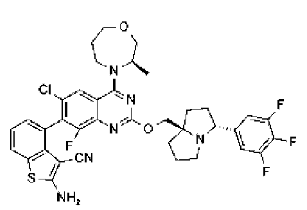
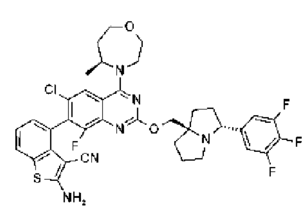
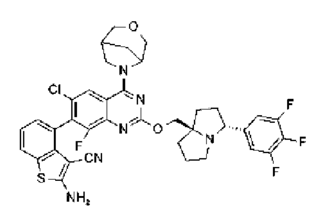

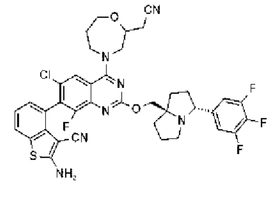
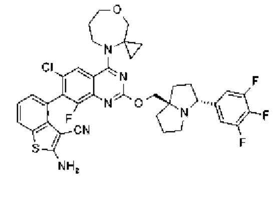

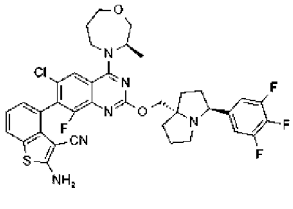
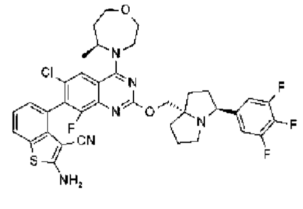
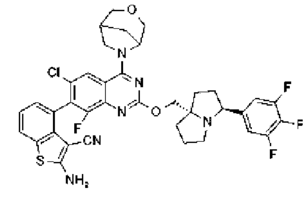

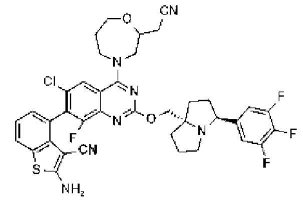
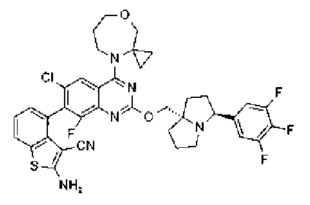

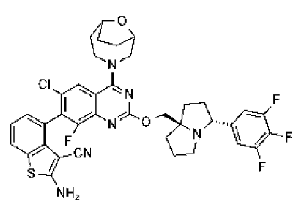
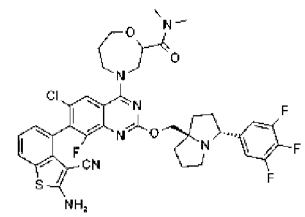
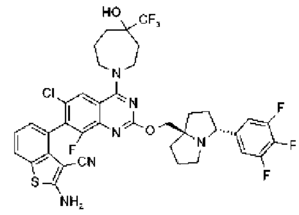

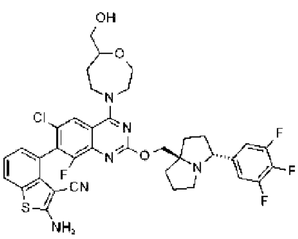
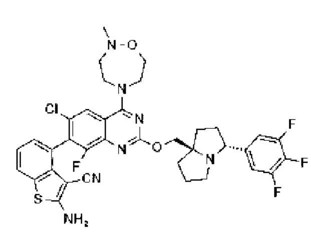

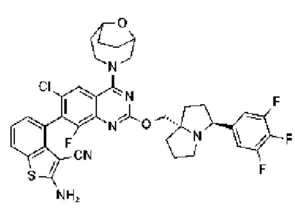
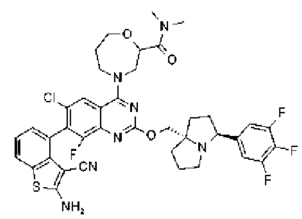
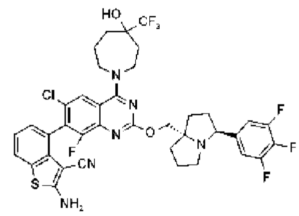

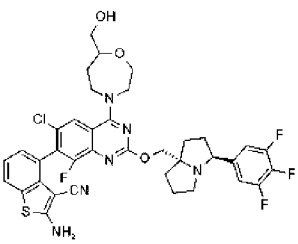
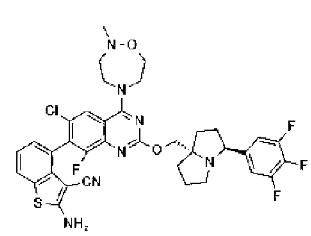

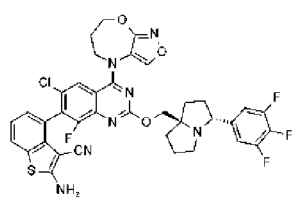
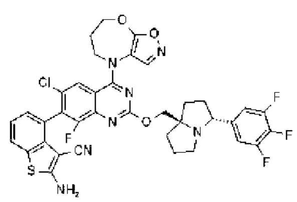
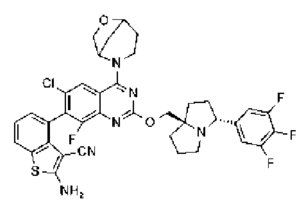

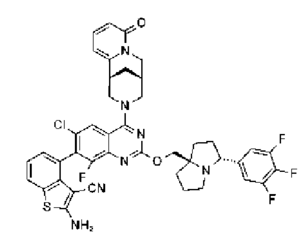
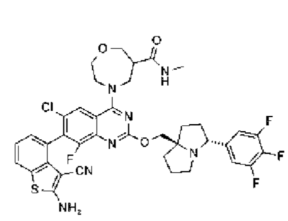

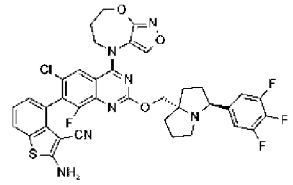

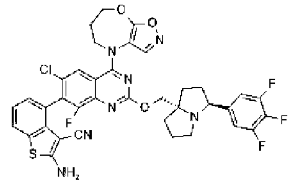

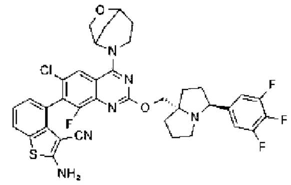

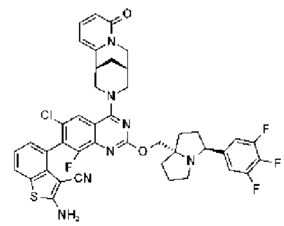

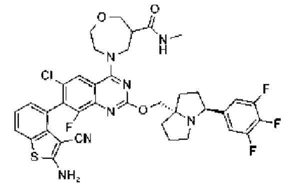

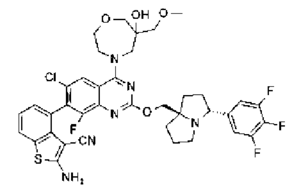

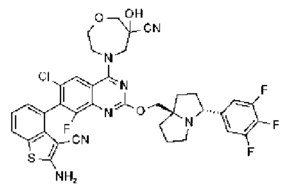

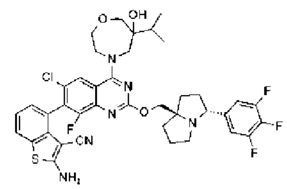

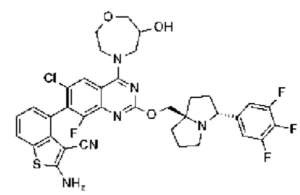

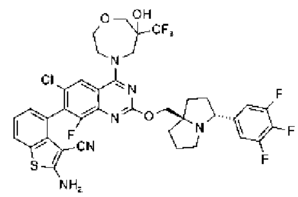

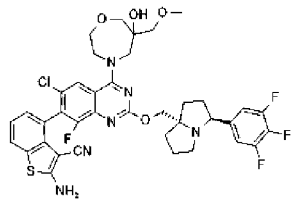

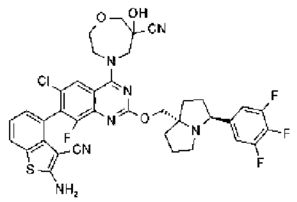

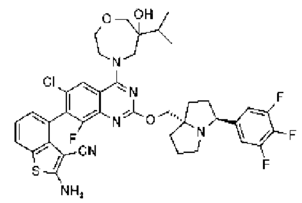

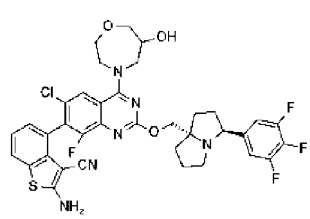

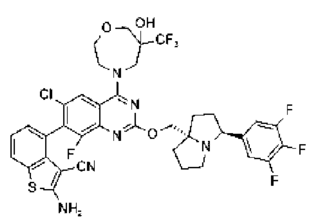

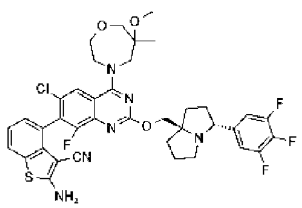

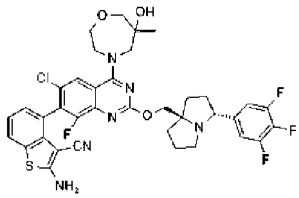

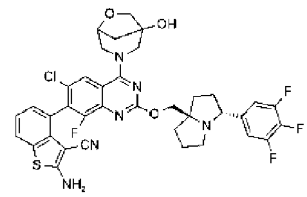

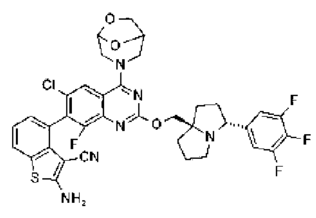
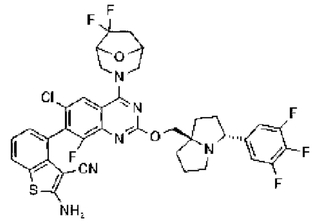

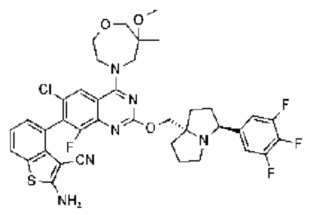
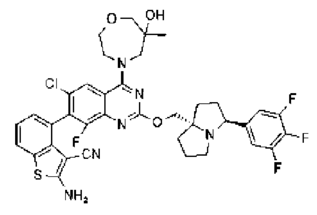
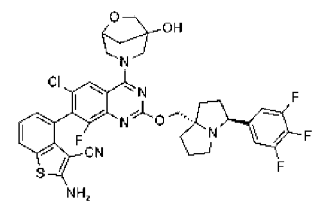

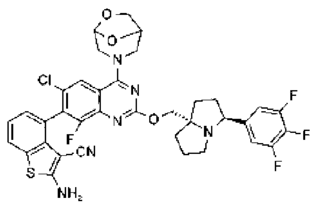
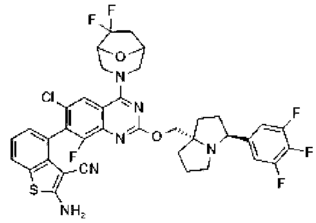

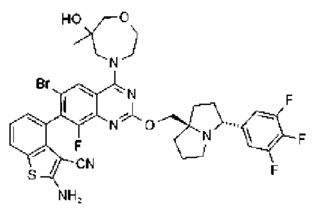
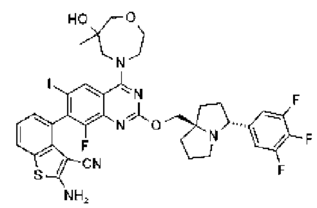
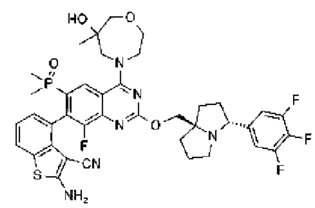

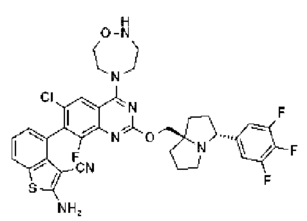
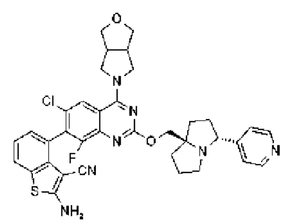

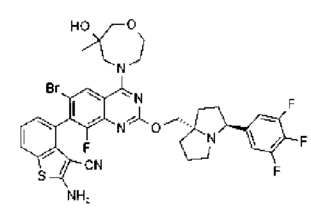
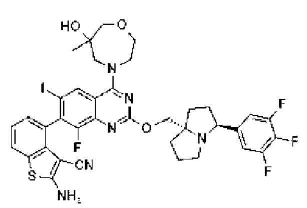
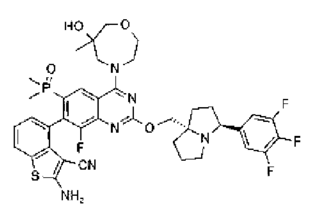

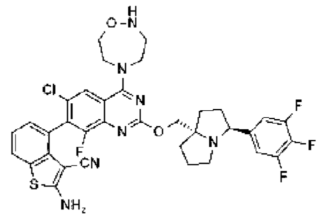
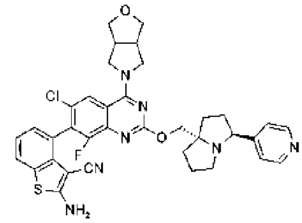

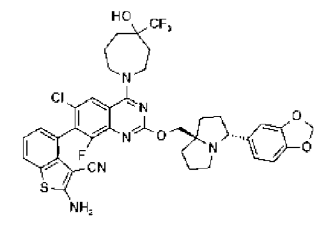
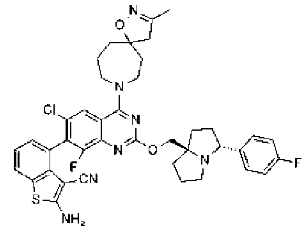
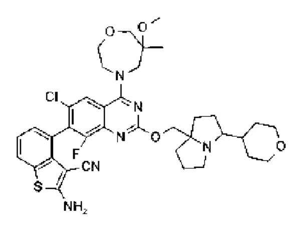

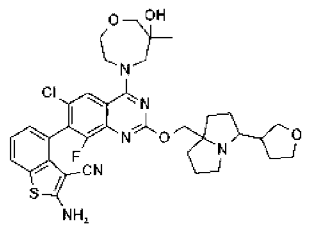
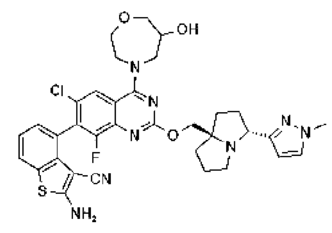

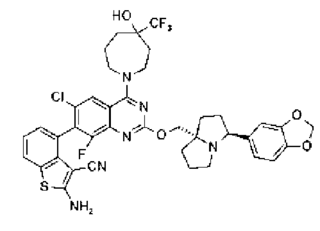
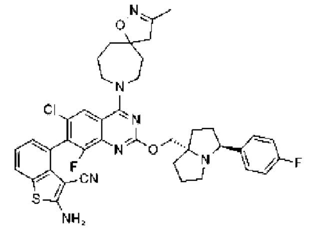
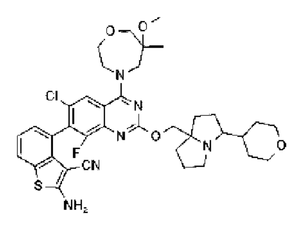

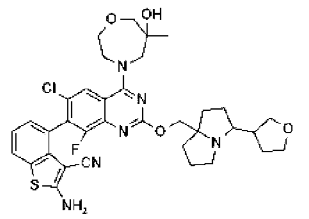
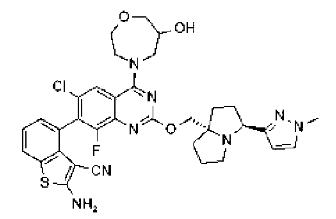

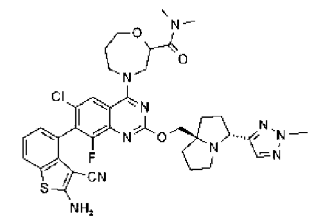
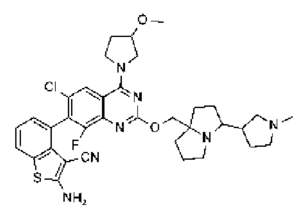
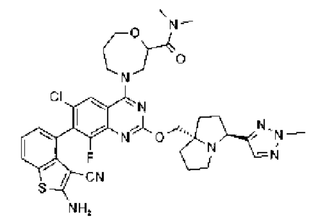

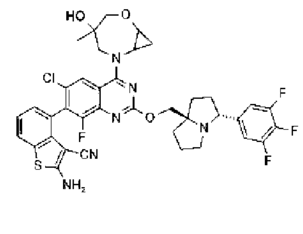
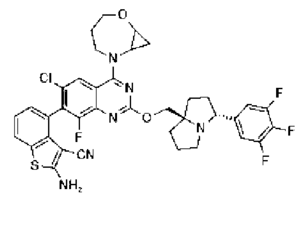

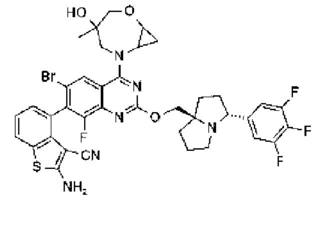
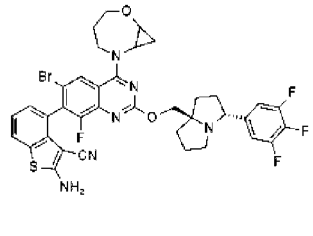
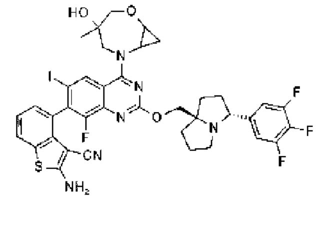

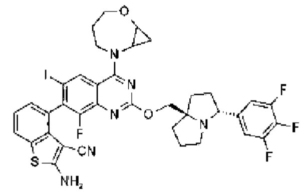
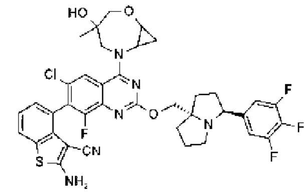

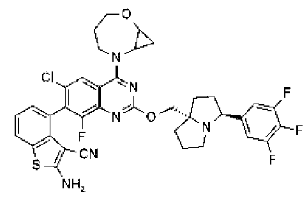
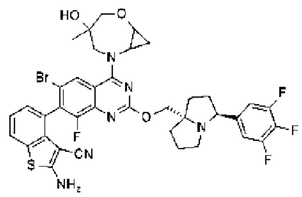
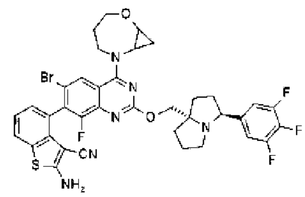

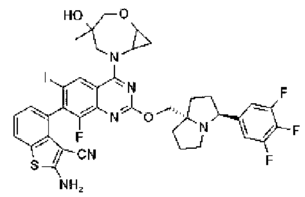
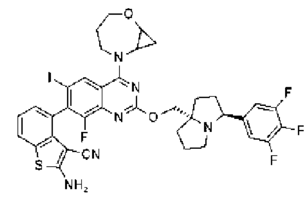

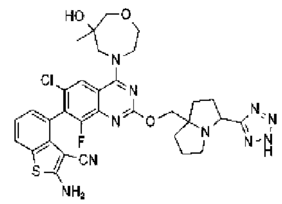
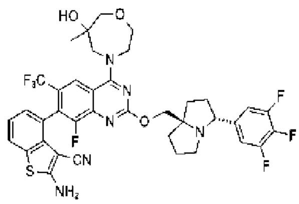
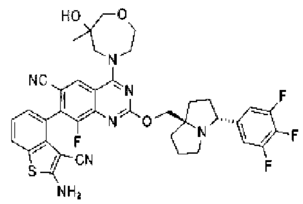

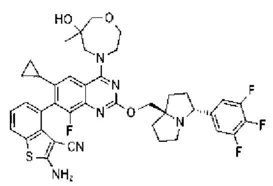
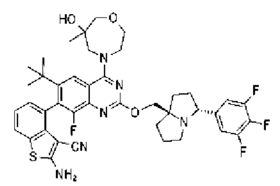

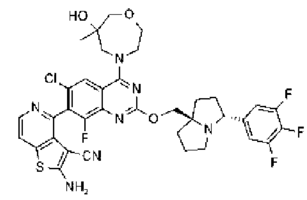
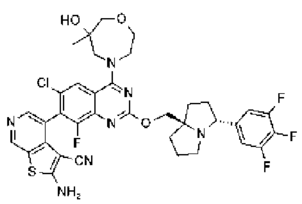
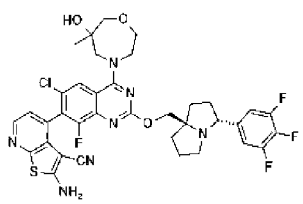

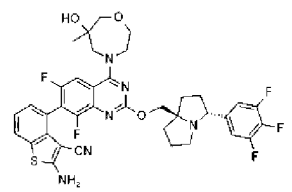
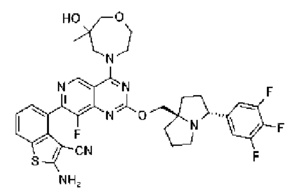

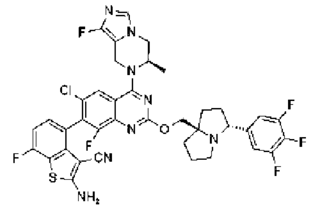
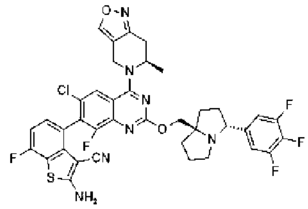
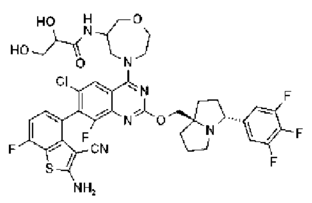

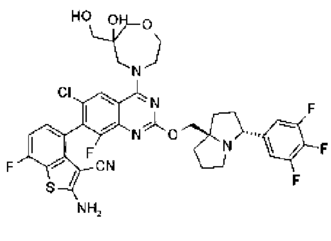
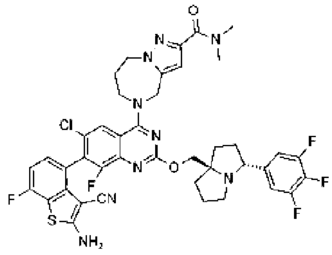

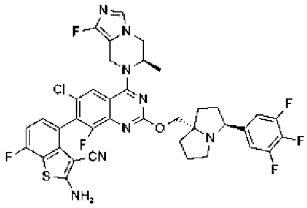
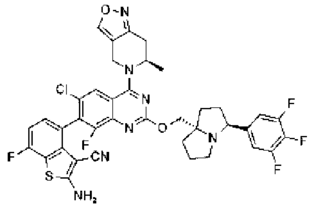
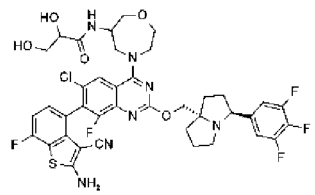

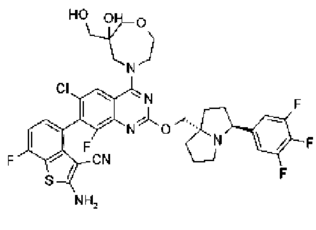
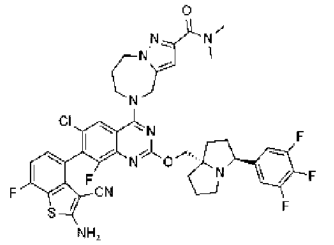

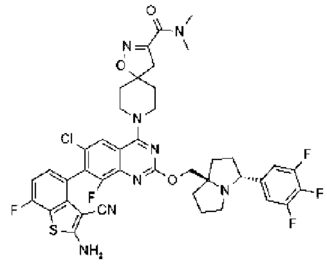
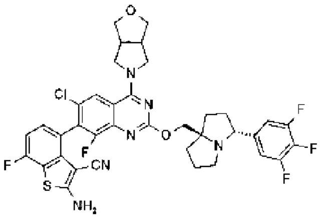
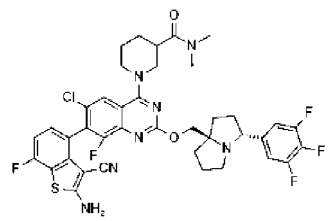

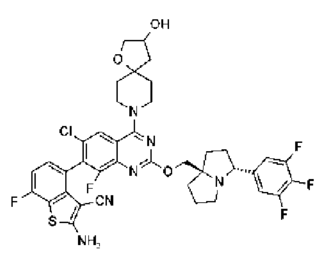
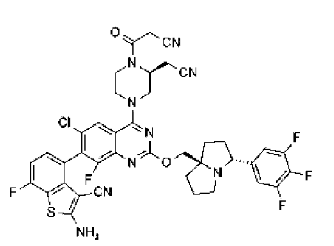

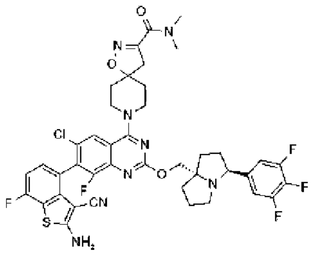
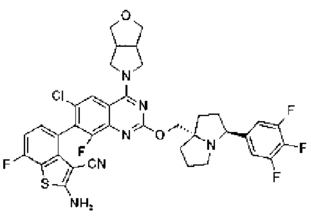
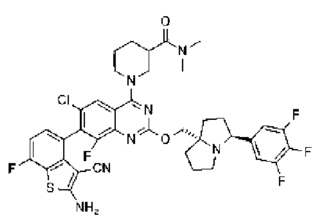

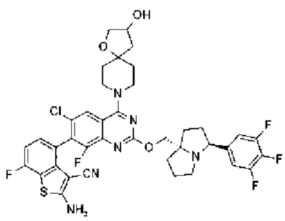
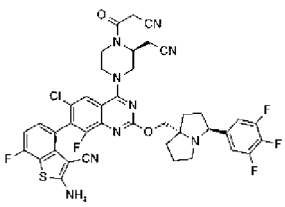

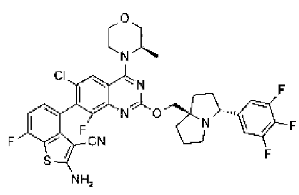
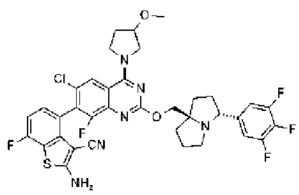
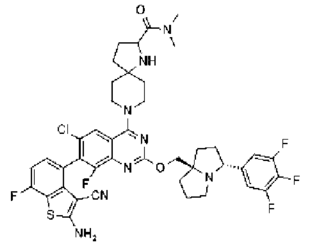

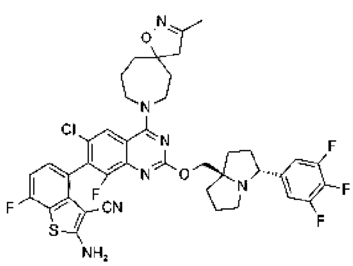
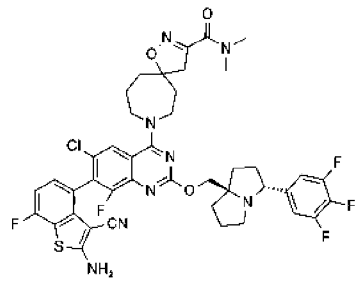

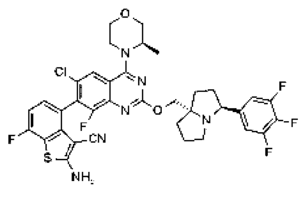
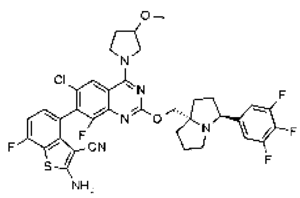
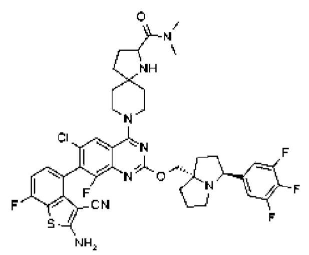

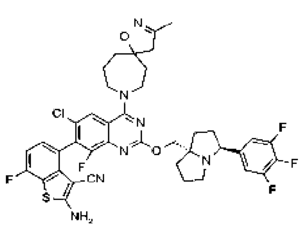
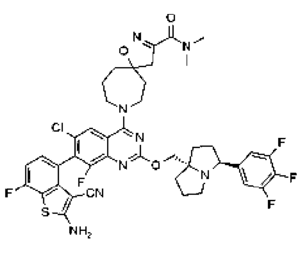

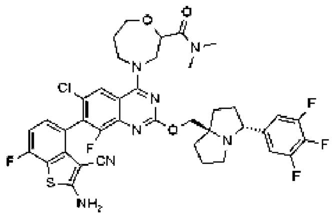
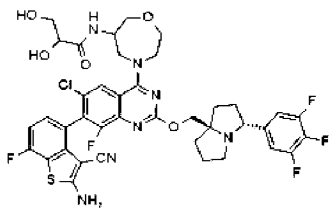
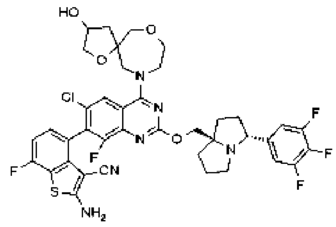

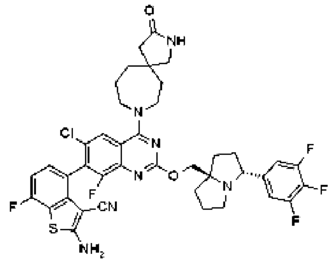
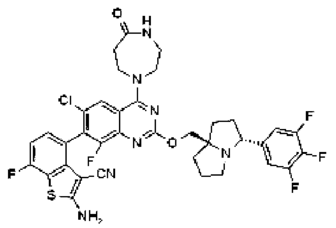

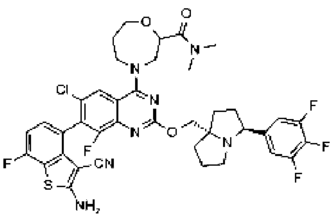
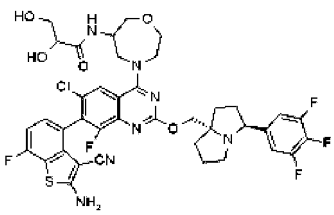
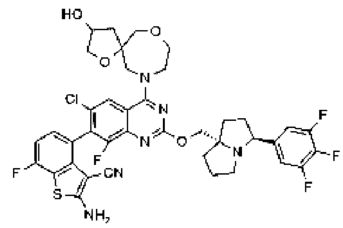

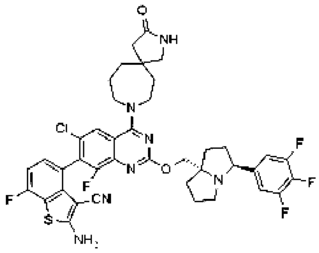
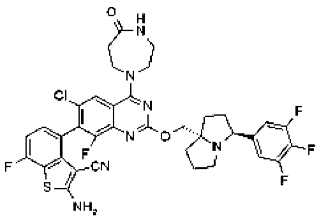

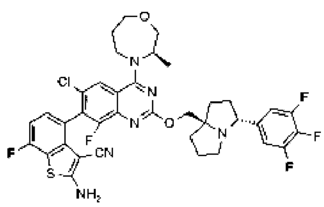
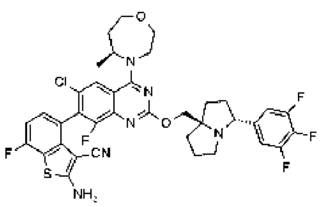
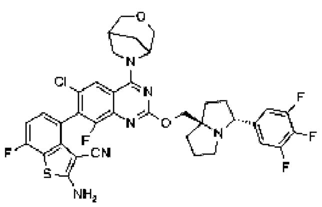

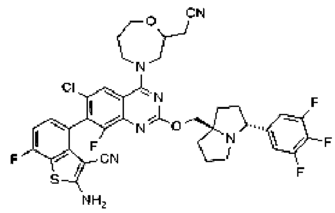
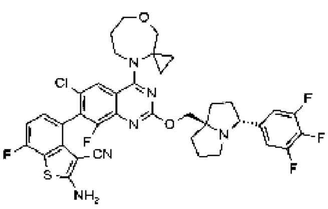

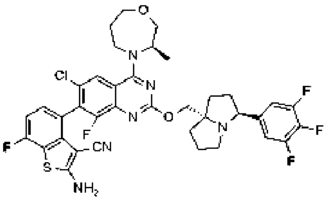
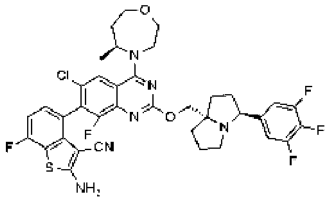
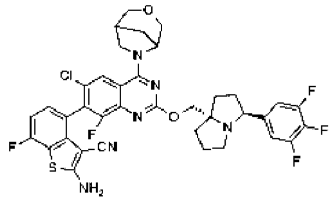

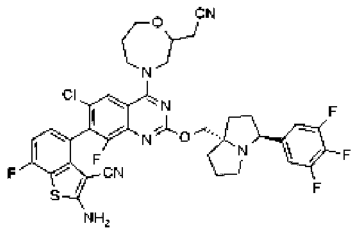
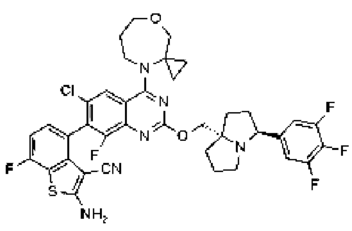

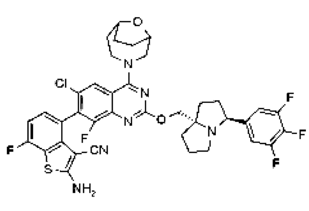
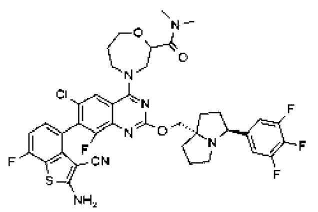
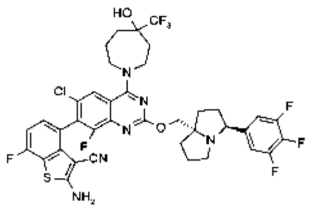

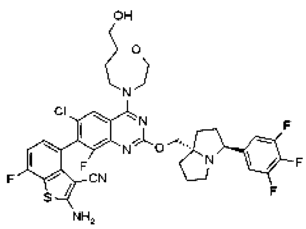
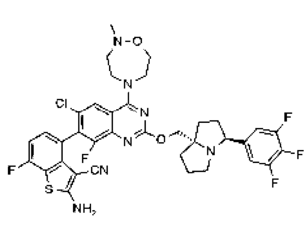

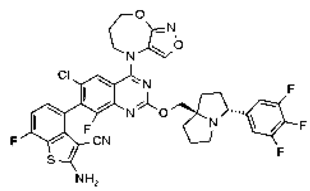
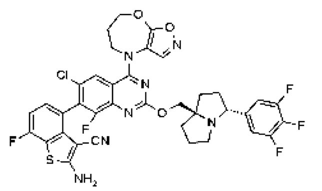
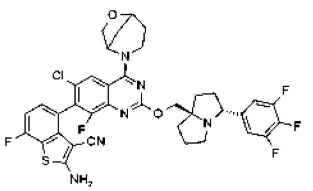

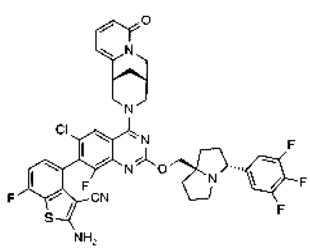
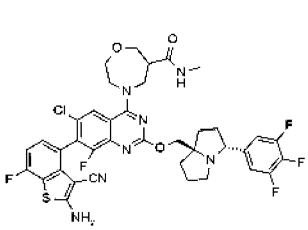

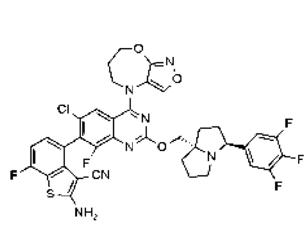
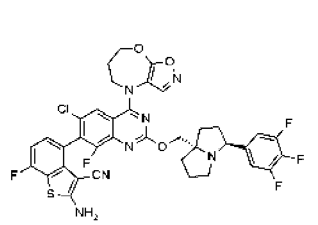
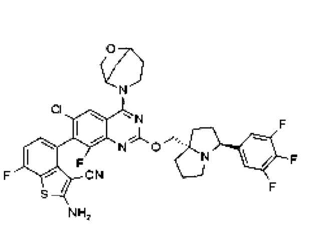

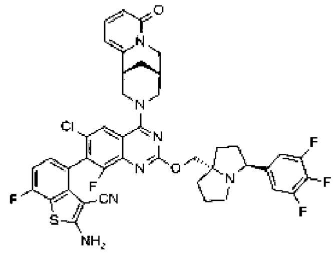
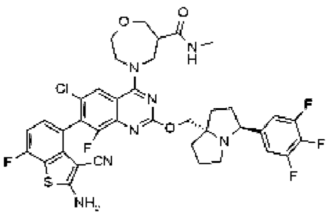

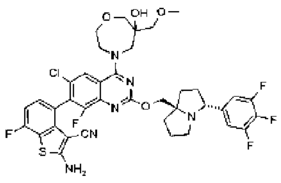
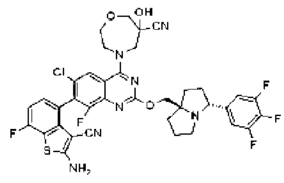
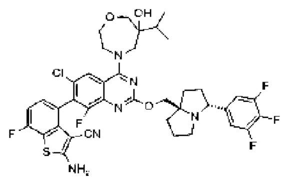

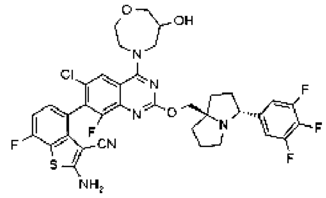
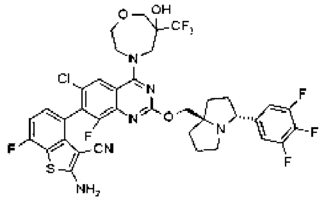

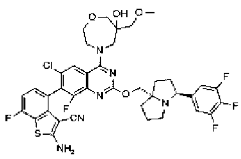
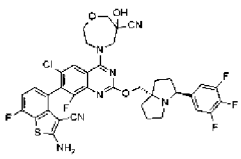
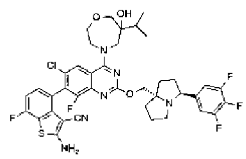

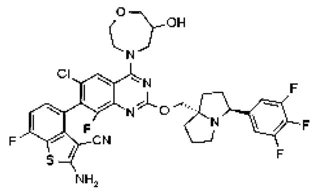
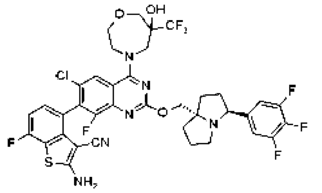

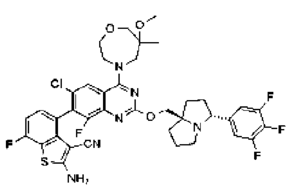
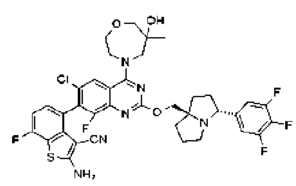
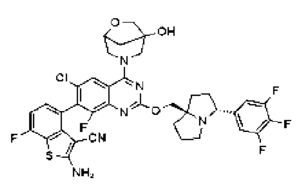

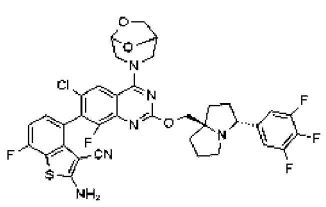
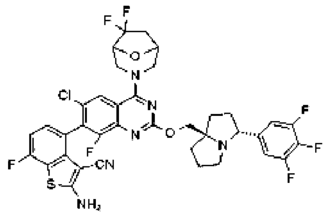

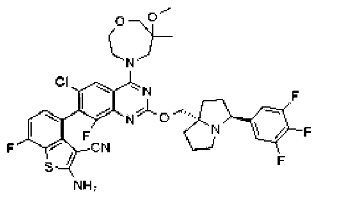
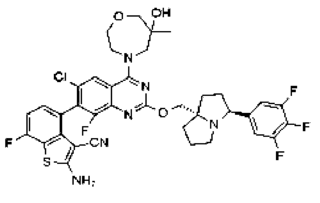
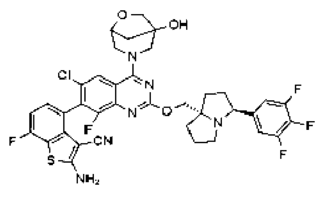

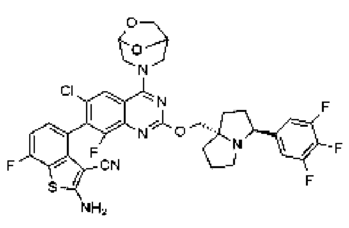
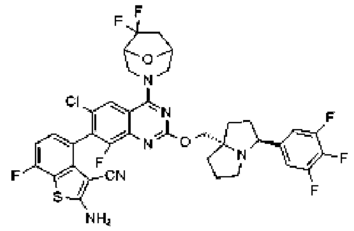

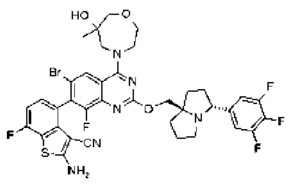
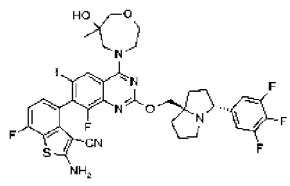
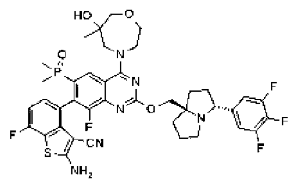

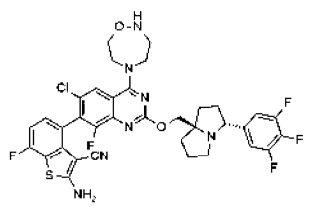
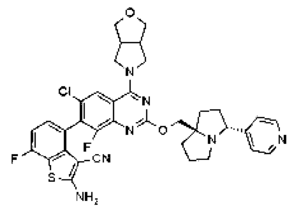

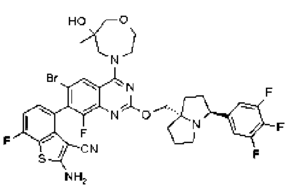
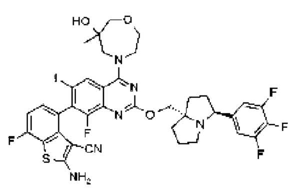
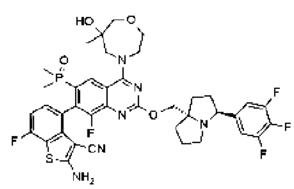

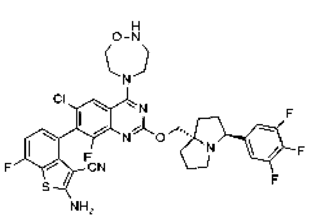
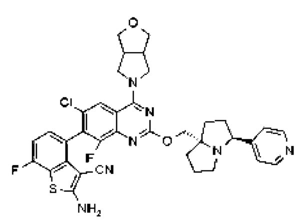

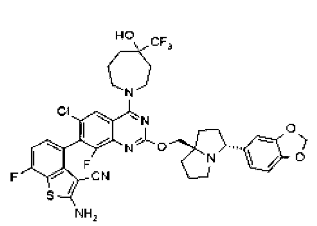
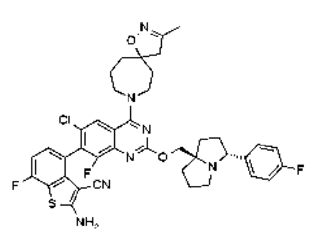
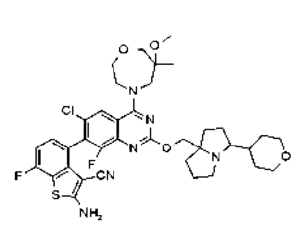

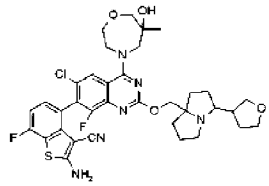
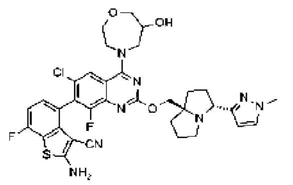

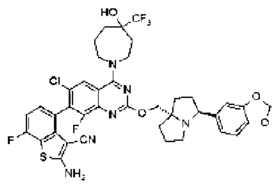
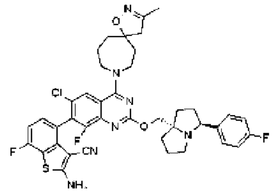
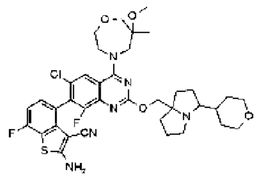

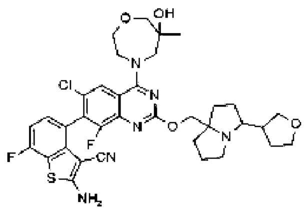
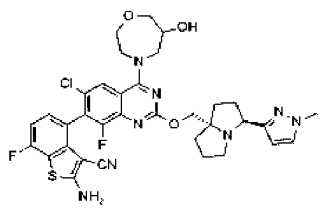

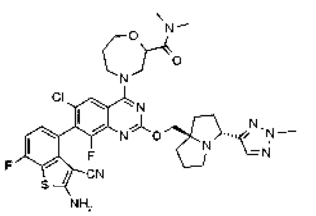
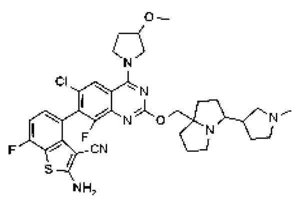
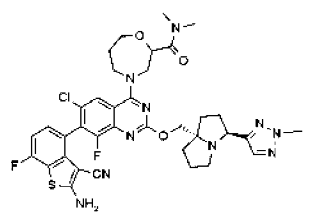

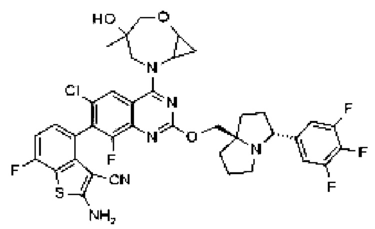
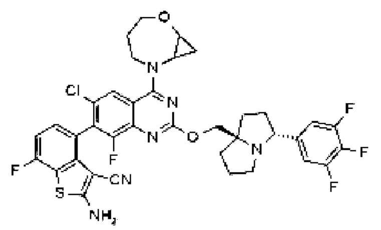

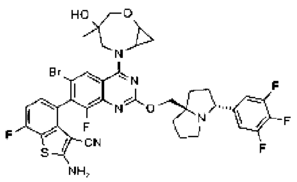
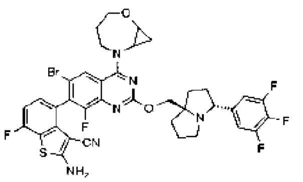
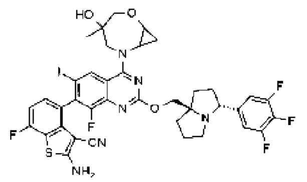

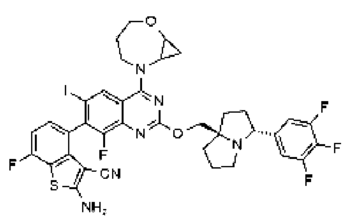
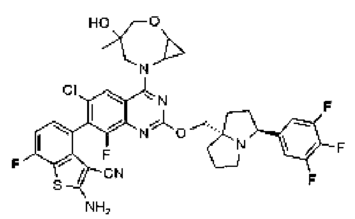

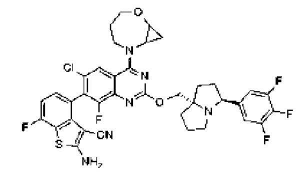
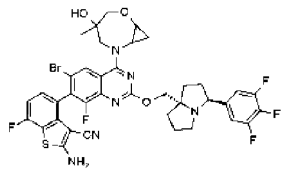
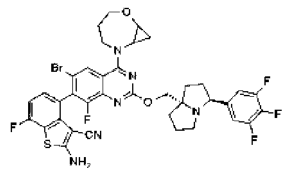

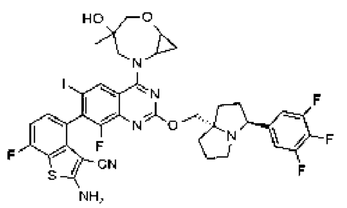
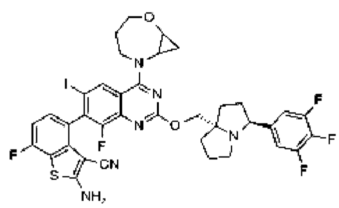

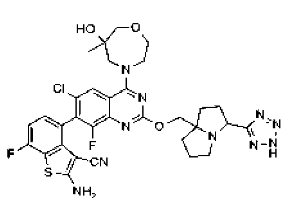
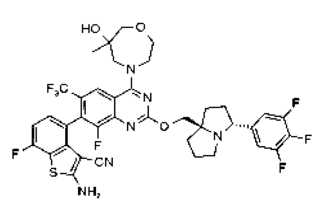
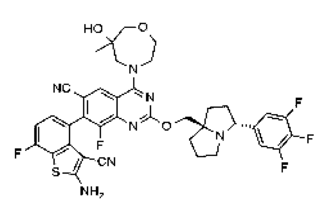

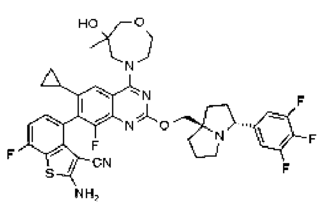
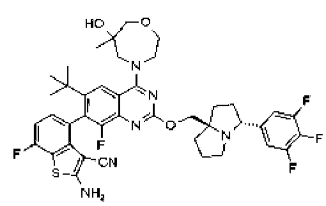

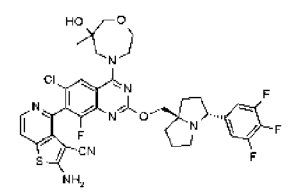
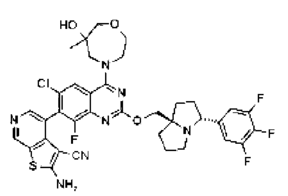
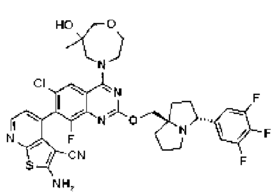

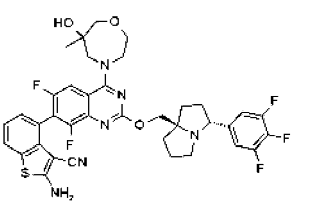
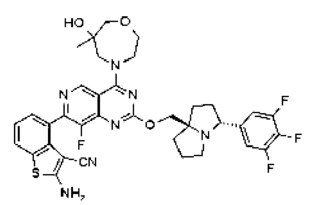

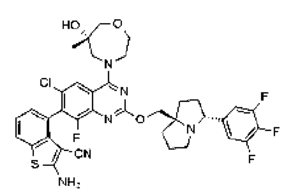
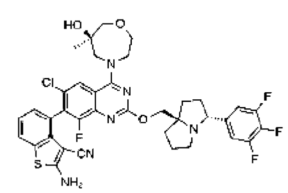
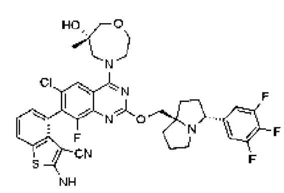

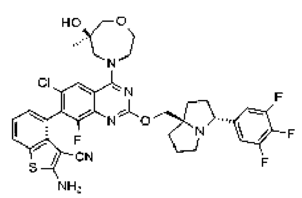
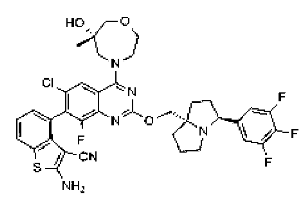

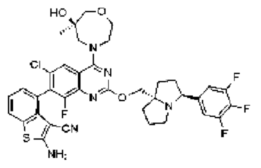
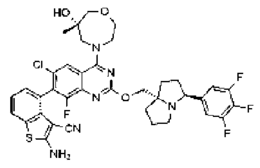
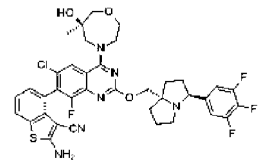

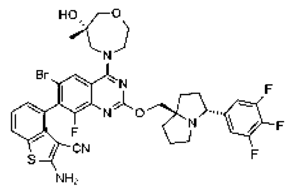
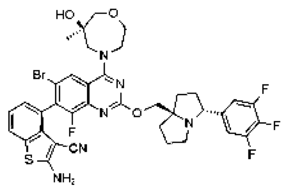

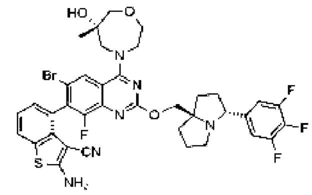
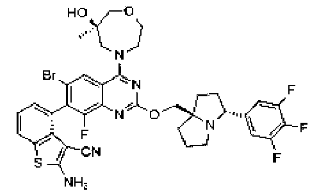
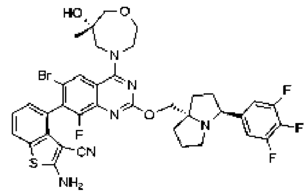

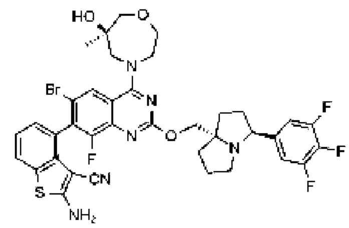
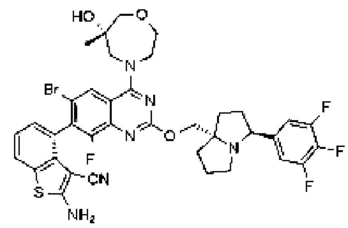

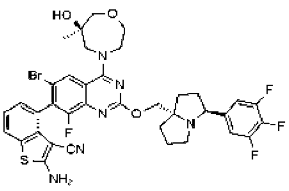
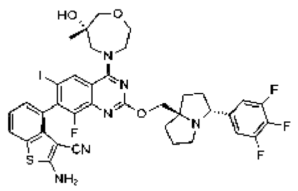
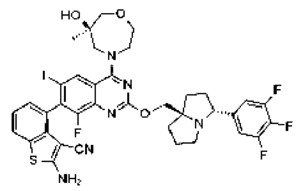

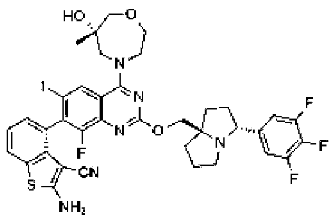
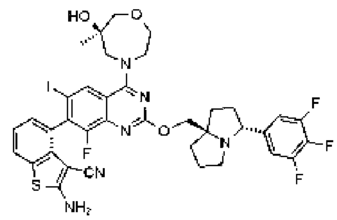

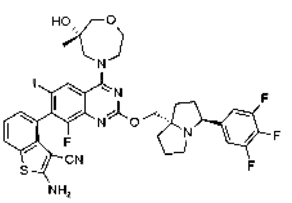
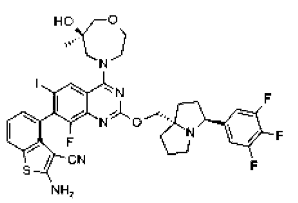
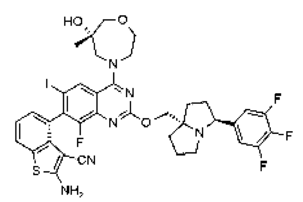

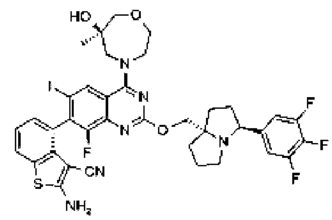
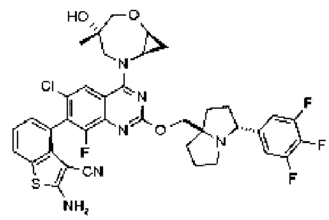

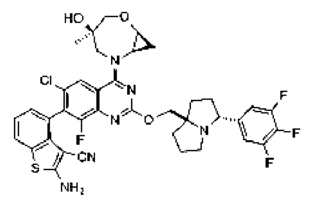
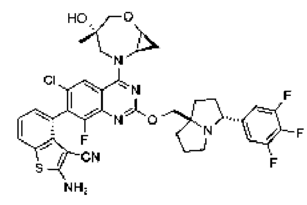
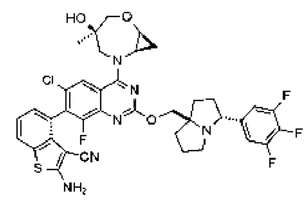

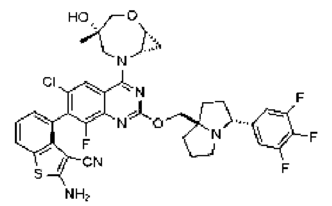
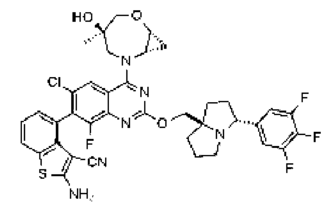

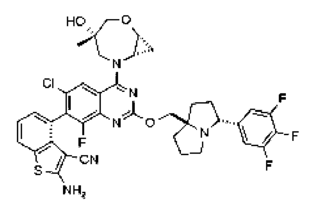
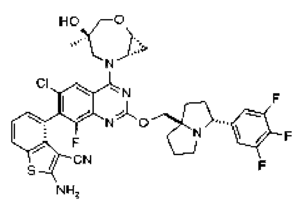
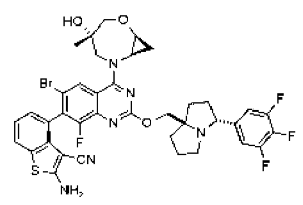

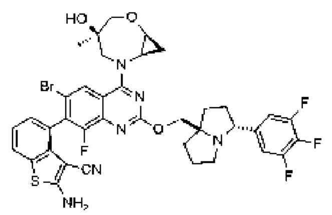
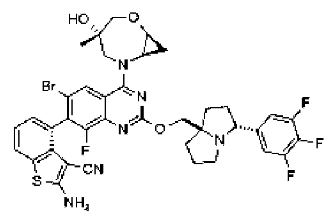

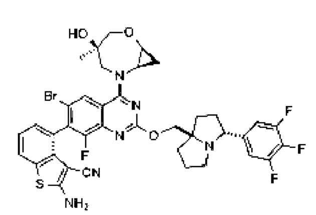
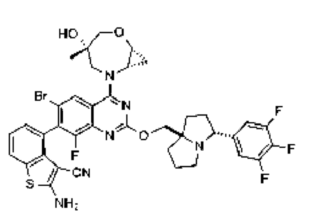
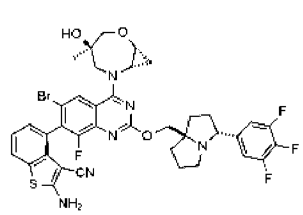

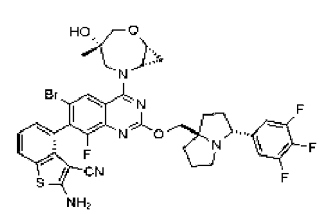
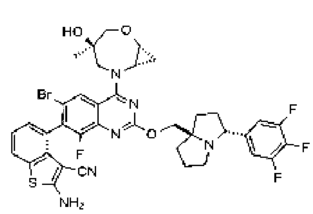

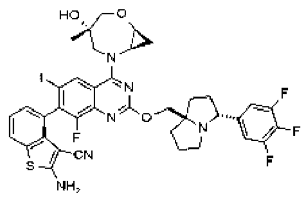 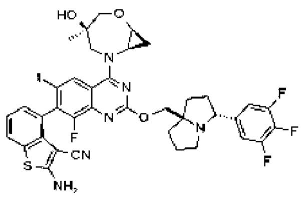 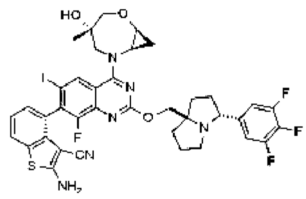

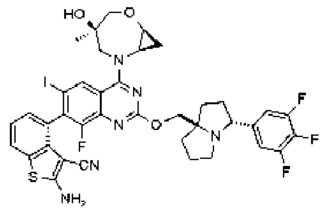 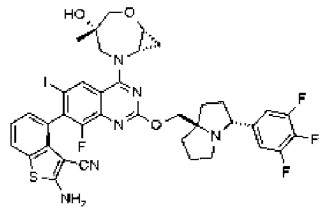

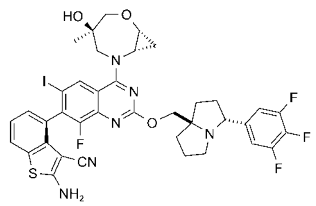 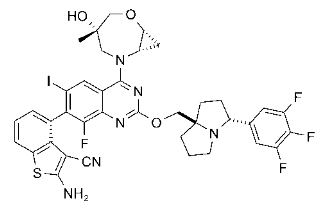 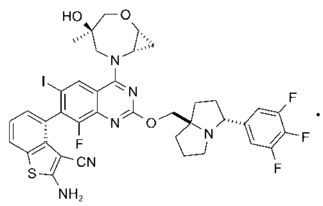

**17.** A pharmaceutical composition, wherein an active compound in the pharmaceutical composition comprises one or more selected from the compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to any one of claims 1-16.

**18.** Use of the compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to any one of claims 1-16 in the preparation of a drug for inhibiting KRAS.

**19.** The use according to claim 18, wherein the drug for inhibiting KRAS is a drug that inhibits KRAS G12D, KRAS G12V, KRAS G12S, KRAS G12C, KRAS G12R, KRAS G12A or KRAS G13D.

**20.** The use according to claim 18, wherein the compound, tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof is used in the preparation of a drug for the treatment, relief or prevention of diseases or disorders associated with Noonan syndrome, LEOPARD syndrome, leukemia, neuroblastoma, lymphoma, melanoma, esophageal cancer, head and neck tumors, breast cancer, lung cancer, bone cancer, biliary tract cancer, glioma, nasopharyngeal carcinoma, gastric cancer, kidney cancer, liver cancer, cervical cancer, thyroid cancer, bladder cancer, prostate cancer, testicular cancer, endometrial cancer, pancreatic cancer, pancreatic duct adenocarcinoma, ovarian cancer, rectal cancer and colon cancer.

**INTERNATIONAL SEARCH REPORT**

International application No.
**PCT/CN2024/123898**

**A. CLASSIFICATION OF SUBJECT MATTER**

C07D519/00(2006.01)i；C07D401/14(2006.01)i；C07D487/04(2006.01)i；C07D487/08(2006.01)i；C07D495/00(2006.01)i；A61K31/505(2006.01)i；A61K31/435(2006.01)i；A61K31/553(2006.01)i；A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS(STN), REGISTRY(STN): 喹唑啉, 吡呤, 肿瘤, 癌, quinazol+, pyrroliz+, KRAS, tumor, cancer

**C. DOCUMENTS CONSIDERED TO BE RELEVANT**

| Category* | Citation of document, with indication, where appropriate, of the relevant passages | Relevant to claim No. |
|---|---|---|
| X | WO 2023072188 A1 (BETTA PHARMACEUTICALS CO., LTD.) 04 May 2023 (2023-05-04) description, page 1, paragraph 5 to page 3, 2nd-to-last line, pages 15-18, the compounds, and page 18, text section, paragraph 1 to page 19, paragraph 1 | 1-20 |
| X | WO 2022184178 A1 (JACOBIO PHARMACEUTICALS CO., LTD.) 09 September 2022 (2022-09-09) claims 1 and 39-42, and description, tables 10-11 | 1-20 |
| X | WO 2022221739 A1 (MERCK SHARP & DOHME CORP.) 20 October 2022 (2022-10-20) description, paragraphs [0007]-[0008], [0026]-[0027], [0076], [0084]-[0085], and [0093]-[0095] | 1-20 |
| X | CN 114615981 A (MIRATI THERAPEUTICS, INC. et al.) 10 June 2022 (2022-06-10) claims 1 and 45-52 | 1-20 |
| X | WO 2023278600 A1 (DANA-FARBER CANCER INSTITUTE, INC.) 05 January 2023 (2023-01-05) claims 1 and 20-25 | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 January 2025** | **08 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/123898**

**C. DOCUMENTS CONSIDERED TO BE RELEVANT**

| Category* | Citation of document, with indication, where appropriate, of the relevant passages | Relevant to claim No. |
|---|---|---|
| X | CN 115490709 A (SICHUAN HAISCO PHARMACEUTICAL CO., LTD.) 20 December 2022 (2022-12-20)<br>claims 1 and 11-15 | 1-20 |
| X | CN 115836055 A (INVENTISBIO CO., LTD.) 21 March 2023 (2023-03-21)<br>claims 1-15, 31-36, and 51-54, and description, paragraphs [0232]-[0233] and [0237] | 1-20 |
| X | CN 115974896 A (SUNSHINE LAKE PHARMA CO., LTD.) 18 April 2023 (2023-04-18)<br>claims 1 and 14-18 | 1-20 |
| X | CN 116102559 A (SICHUAN HUIYU PHARMACEUTICAL CO., LTD. et al.) 12 May 2023 (2023-05-12)<br>claims 1 and 27-32 | 1-20 |
| X | CN 116217591 A (3D MEDICINES (SHANGHAI) CO., LTD.) 06 June 2023 (2023-06-06)<br>claims 1, 4-6, and 13-15, and description, paragraphs [0087]-[0088] | 1-20 |
| X | WO 2022148422 A1 (BEIGENE, LTD. et al.) 14 July 2022 (2022-07-14)<br>claims 1, 6, 13-17, 22, and 39-41, and description, paragraph [0130], table 1, and paragraphs [0198]-[0202] | 1-20 |

Form PCT/ISA/210 (second sheet) (July 2022)

**INTERNATIONAL SEARCH REPORT**
**Information on patent family members**

International application No.
**PCT/CN2024/123898**

| Patent document cited in search report | Publication date (day/month/year) | Patent family member(s) | Publication date (day/month/year) |
|---|---|---|---|
| WO 2023072188 A1 | 04 May 2023 | None | |
| WO 2022184178 A1 | 09 September 2022 | None | |
| WO 2022221739 A1 | 20 October 2022 | US 2024239788 A1 | 18 July 2024 |
| | | EP 4322954 A1 | 21 February 2024 |
| CN 114615981 A | 10 June 2022 | MX 2022002465 A | 19 May 2022 |
| | | IL 290845 A | 01 April 2022 |
| | | BR 112022003543 A2 | 24 May 2022 |
| | | EP 4021444 A1 | 06 July 2022 |
| | | EP 4021444 A4 | 04 January 2023 |
| | | US 11453683 B1 | 27 September 2022 |
| | | AU 2020337938 A1 | 17 March 2022 |
| | | CA 3148745 A1 | 04 March 2021 |
| | | CO 2022003782 A2 | 08 July 2022 |
| | | ZA 202202362 B | 28 September 2022 |
| | | TW 202122396 A | 16 June 2021 |
| | | KR 20220071193 A | 31 May 2022 |
| | | JP 2022546043 A | 02 November 2022 |
| | | US 2024309020 A1 | 19 September 2024 |
| | | US 2023077225 A1 | 09 March 2023 |
| | | US 11964989 B2 | 23 April 2024 |
| | | CL 2022000447 A1 | 21 October 2022 |
| | | WO 2021041671 A1 | 04 March 2021 |
| WO 2023278600 A1 | 05 January 2023 | AU 2022303357 A1 | 14 December 2023 |
| | | CA 3221811 A1 | 05 January 2023 |
| | | EP 4363412 A1 | 08 May 2024 |
| CN 115490709 A | 20 December 2022 | None | |
| CN 115836055 A | 21 March 2023 | US 2023242544 A1 | 03 August 2023 |
| | | WO 2022002102 A1 | 06 January 2022 |
| | | JP 2023531269 A | 21 July 2023 |
| | | EP 4175947 A1 | 10 May 2023 |
| | | EP 4175947 A4 | 03 July 2024 |
| CN 115974896 A | 18 April 2023 | None | |
| CN 116102559 A | 12 May 2023 | None | |
| CN 116217591 A | 06 June 2023 | None | |
| WO 2022148422 A1 | 14 July 2022 | US 2024140957 A1 | 02 May 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022173870 A
- WO 2023020519 A